# EUROPEAN PATENT SPECIFICATION

(11) **EP 2 560 568 B1**
(45) Date of publication and mention of the grant of the patent: **21.09.2016**
(21) Application number: 11718569.4
(22) Date of filing: 19.04.2011
(51) Int. Cl.: A61B 18/12, A61B 18/14, A61B 18/18, A61N 1/40, A61M 35/00, A61N 1/32, A61N 1/36, A61B 18/00

(54) **COMBINED ENERGY AND TOPICAL COMPOSITION APPLICATION FOR REGULATING THE CONDITION OF MAMMALIAN SKIN**
KOMIBINIERTE ANWENDUNG VON ENERGIE UND TOPISCHER ZUSAMMENSETZUNG ZUR REGULIERUNG DES ZUSTANDES VON SÄUGERHAUT
APPLICATION COMBINÉE D'ÉNERGIE ET D'UNE COMPOSITION TOPIQUE POUR RÉGULER L'ÉTAT DE LA PEAU D'UN MAMMIFÈRE

(30) Priority: 19.04.2010 US 325645 P
(43) Date of publication of application: 27.02.2013
(73) Proprietor: Syneron Medical Ltd., 20692 Yokneam Ilit (IL)
(72) Inventor: WEISGERBER, David, John, Cincinnati, Ohio 45239 (US); ANNUNZIATA, Nikki, Elizabeth, Harrison, Ohio 45030 (US); MAURER, Tia, Janinne, Liberty Township, Ohio 45044 (US)
(74) Representative: Nederlandsch Octrooibureau
(86) International application number: PCT/US2011/033034
(87) International publication number: WO 2011/133539

(56) References cited:
- WO-A1-02/092168
- WO-A2-2005/060354
- WO-A2-2007/099460
- US-A1- 2005 197 681

## Description

### FIELD OF THE INVENTION

The present invention relates to combined applications of energy and topical compositions to mammalian skin for regulating the condition of the skin. Regimens for the most efficient use of energy delivery devices and methods for determining efficacy of an energy delivery device, the composition and the regimen are also disclosed.

### BACKGROUND OF THE INVENTION

Treatment of skin to avoid or reduce effects of intrinsic chronological and extrinsic environmental aging of skin is a multi-billion dollar commercial industry underpinned by even greater dollar investment in the development and validation of new technologies. Administration of electromagnetic (EM) energy to skin via application to the surface of skin has been known for decades and implemented in a wide range of forms and through a variety of delivery devices. Generally EM-based skin treatment methods may be divided into ablative and nonablative procedures although both exploit the thermolytic effect of EM energy application.

A variety of products are available to consumers to improve the condition of skin and to delay and/or prevent typical signs of aging. Such signs include, for example, fine lines, wrinkles, hyper-pigmentation, shallowness, sagging, dark under-eye circles, puffy eyes, uneven skin tone, enlarged pores, diminished rate of epidermal cell turnover, and abnormal desquamation or exfoliation. For some consumers, however, the wide variety of available products and the advancements in skin care technology still fail to produce the desired results, and some feel the need to turn to more invasive medical procedures.

Ablative procedures such as ablative laser have proven to be effective methods for gross morphological resurfacing or removal of skin, such as in scar and tattoo removal procedures, and have also proven effective for treating and improving appearance of aged and photo-damaged skin. Although ablative procedures are effective for improving the appearance of fine lines and wrinkles in the cosmetically vulnerable perioral and periorbital areas of facial skin, major disadvantages include prolonged periods of healing and recuperation which impose a seriously compromised cosmetic appearance to the consumer for undesirably long time periods. Further, the potential side effects of infection, scarring and pigmentation irregularities which may result are often considered cosmetically unacceptable to consumers in particular where facial skin is implicated.

Recent research and development efforts have therefore focused on providing consumers with cheaper, more convenient and safer nonablative skin anti-aging and rejuvenation treatments. Cosmetic regimens involving administration of thermal energy to the skin for the purpose of promoting improved appearance of the skin are well known in the art. Electromagnetic energy delivery technology in the form of handheld devices targeted for consumer home use have been available on the market for nearly a decade. Improvements and advances in the technology center around maximizing a thermally induced benefit to deeper target skin tissues while minimizing undesirable damage both to the target tissue to peripheral and surface tissues. It is believed that selective thermal treatment induces new collagen formation and selective thermal damage induces, inter alia, dermal matrix remodeling. Currently available technologies, however, are known to result in undesirable sustained negative side effects of problematic damage including overheating, burning, erythema and pigment irregularities.

In the past, efficacy of these devices and compositions was determined by exterior methods only. That is, if there was a visible improvement in the exterior layer of the skin the energy delivery device must be working. But there are few methods for determining the optimal time, temperature and composition for these energy delivery devices. Moreover, it is generally accepted that many of the visible changes that occur on the surface of the skin are the results of changes that occur below the surface in the dermis and epidermis layers of the skin. It is difficult to know exactly how to control and maximize the performance of a device without knowing how it affects the underlying layers of skin.

Selective photothermolysis of skin tissue is a widely practiced cosmetic treatment form, in particular in treatments comprising administration of monochromatic laser energy and broad spectrum intense pulsed light (IPL) energy. In these technologies, optical energy is applied directly to the surface of the skin and penetration relies on transmission through the epidermis and absorption in the dermis. Dark skin and hyper-pigmented spots on the epidermis may impede transmission and hinder efficacy of the treatment by absorbing energy, and may also result in overheating of the pigmented areas resulting in blistering, burning, and other cosmetically undesirable effects.

As an alternative to EM-based thermolysis, electrically conducted radio frequency (RF) current has also been investigated as a cosmetic skin treatment modality. The use of RF current and pulsed radio frequencies (pRF) in the medicinal arts is known, although the use of RF current as a nonablative skin rejuvenation technology for self-delivery by consumers is still relatively innovative. In the application of RF current to skin, a transfer of biopolar RF current takes place through two electrodes that are applied directly to the skin. The current, therefore, runs directly through the dermal layer conducted from electrode to electrode, distinguishing it from application of EM energy which is focused through the epidermis and limited by factors which affect wavelength penetration depth.

RF current administration theoretically appears to offer significant skin treatment advantages over EM energy application. Unlike electromagnetic energy, for example, electrically conducted RF energy is chromophore-independent, which avoids complications and efficacy problems relating to the existence of an absorption differential between pigmented and non-pigmented skin and the resulting problems in treating darker skin, which has more energy-absorbing melanin and lighter skin, which may reflect optical wavelengths. In both cases, consistency of results is compromised and thermal control in chromophore-containing skin remains problematic.

RF current administration to skin at energy levels which may provide thermal treatment efficacy, however, is plagued by an inability by investigators to optimize parameters to achieve a desired benefit in the absence of undesirable skin damage. RF current is delivered through the dermal tissue below the skin surface, whereas effect-monitoring by temperature or moisture sensors is limited to the accessible surface of skin. RF current impedance is a function of tissue composition and various skin tissue attributes including collagen density and integrity, hydration level, and the like. Although the distance between electrodes and control of parameters such as pulse length and frequency may theoretically be adjusted to optimize effect and avoid safety concerns, such adjustments are nearly impossible without benefit of an apparatus or other means to monitor changes in tissue condition. In the case of EM energy-based delivery, the surface skin typically reaches the highest treatment temperatures so that temperature monitoring at the surface can prevent undesired damage to sub-epidermal tissue. However, in the case of RF current the sub-epidermal tissues actually reach a higher treatment temperature than the surface skin so that damage may occur to deeper tissues without being measurably manifest at the surface. Outside of clinical settings under the supervision of highly trained medical personnel and using sophisticated instrumentation, devices and regimens targeted for personal use by consumers based on delivery of RF current alone have therefore been generally avoided since safety considerations continue to exist at effective treatment levels in the absence of an appropriate sub-epidermal monitoring mechanism. Handheld energy delivery devices which provide RF-current as a sole treatment modality and targeted for home consumer use are virtually unknown. One such purported device (STOP™, Ultragen Ltd) is marketed to consumers for personal use in Europe, but the treatment tolerances of the device are set so low in order to avoid undesirable damage, that objective evidence of clinical efficacy under controlled conditions is not available.

Hence, the role of RF in skin treatment is substantially limited to an adjunctive or preparative function in combination with other thermolytic procedures. For example, in 2002, Bitter and Mulholland ("Report of a new technique for enhanced non-invasive skin rejuvenation using a dual mode pulsed light and radio frequency energy sources: selective radiothermolysis," J. Cosmet Dermatol 2002; 1: 142-145) proposed a treatment protocol based on a combination of RF current and IPL and reported results based on facial treatment of 100 test subjects, although the authors failed to disclose specific treatment design parameters. In that study, RF was reported to augment the effects of IPL treatment. Side effects included reports of cosmetically undesirable pigmentation effects, and consumer perception of pain was controlled by superficial cooling.

RF has also been suggested and investigated as useful for cosmetic skin treatment in conjunction with targeted optical energy application. Generally, according to this treatment protocol design, the RF is used adjunctively to the optical energy and is applied in accordance with some parameter of the optical energy. For example, in Hammes et al. ("Electro-optical synergy (ELOS™) for nonablative skin rejuvenation: a preliminary prospective study," Journal of European Academy of Dermatology. and Venereology 2006, 20, 1070-1075), the authors focus on a coordinating pulse frequency between the RF current and the optical energy and suggest that synergy exists between these energy forms which may permit use of lower, less invasive levels of optical energy and further suggest that side effects associated with RF application alone are reduced or avoided by the combined protocol. Further, the regimens which employ these devices include means to mechanically cool the skin in response to overheating, or to prevent overheating.

Another document describing a method of combined RF and cosmetic composition treatment is WO 2005/8060354.

In another example, U.S. Published Application No. 2008/0033516 A1 to Altshuler discloses "temperature controlled photobiostimulation" of skin tissue which involves a combination of heating skin to a target depth and irradiation of a target area with electromagnetic radiation. Altshuler notes the existing technologies of low-level light, low-level laser, monochromatic and quasi-monochromatic photostimulation based skin treatment methods, which are generally thought to increase ATP production, cellular proliferation and protein production, as well as trigger a growth response by induction of a low-grade inflammatory response, but notes reports of inconsistent results and lack of clinical confirmation of efficacy. Altshuler posits that application thermal energy may enhance the photostimulatory response. Altshuler teaches that hyperthermia of a volume of skin may be achieved by any known source capable of raising the temperature of the volume to preferable between 37° and 45°C, and specifically exemplifies heating by hot air, AC or DC electrical current, use of a conductive heat source, ultrasound or microwave radiation or any suitable wavelength or wavelengths of EM radiation in the range of 380-2700 nm. In all Altshuler embodiments, however, EM energy is relied upon to achieve the desired treatment effect. Altshuler teaches that heat provides synergistic enhancement of the desired effects of photostimulation, but also suggests that heat in the absence of EM may result in undesirable biostimulation such as slowing repair of radiation-induced DNA damage, production of heat shock proteins, which build tolerance to subsequent heat applications, and modification of enzymatic processes including those involved in skin tissue regeneration and repair and generally teaches away from heat in the absence of light as a skin treatment modality. Altshuler does not suggest how to overcome deficiencies relating to an inability to assess or monitor sub-epidermal skin conditions.

Moreover, consumer compliance is always an issue for currently available devices. Consumers have a limited amount of time each day for their beauty regimen. While device manufacturers would like to recommend that the consumers use there devices for extended periods to insure they get the maximum benefit, the consumers are unlikely to comply. Hence there is a trade off between recommending extended use of the device and recognizing that consumers have a limited amount of free time in their day to use the device.

The consumer experience is also important when designing a device, a composition and a regimen. For example, sonograms are commonly performed procedures and provide an enormous medical benefit. But the gel used in sonogram procedures is thick and difficult to remove causing consumer discomfort. Moreover, many energy delivery devices heat the exterior skin too quickly or too hot causing an unpleasant consumer experience.

Therefore, there is a continuing need for methods of improving the condition of skin sufficiently to avoid the need for more invasive procedures and the risks associated therewith. And there exists a need for better methods of determining the efficacy of energy delivery devices, which methods can then be used to develop more consumer acceptable experiences, while achieving the desired results of improved skin appearance.

There remains a need in the art for safe and effective nonablative skin treatment and rejuvenation devices, therapies and regimens suitable for personal use by consumers. In particular, there remains a need for a means to treat sub-epidermal skin tissue by enhancing collagen synthesis and dermal remodeling without causing undesirable damage to the treated target tissue or to surrounding tissue. There is a specific need in the art for methods of assessing and monitoring the effects of RF-current based consumer-conducted treatments in order to optimize RF-current administration for provision of desired benefits, and there remains a need for optimized RF-current based therapies which avoid the problems associated with EM-based therapies and which do not rely on mechanical cooling in conjunction with treatment.

### SUMMARY OF THE INVENTION

The present invention relates to a method for regulating the condition of mammalian skin. The skin has at least three layers: a stratum corneum exterior layer; an epidermis; and a dermis. And the method comprises the steps of, applying a first personal care composition to an area of skin where regulation is desired, wherein the first personal care composition comprises a gel composition. Another step comprises delivering energy to the dermis to heat collagen in the dermis such that the heated collagen in the dermis heats the epidermis and stratum corneum until the stratum corneum reaches an external temperature of from about 37°C to about 48°C. The energy delivery to the dermis is then controlled to maintain the temperature of the stratum corneum in the range of from about 37°C to about 48°C. The energy is delivered from an RF energy device via two or more electrodes that contact the stratum corneum via the gel composition. It is sometimes preferred that the energy delivery device does not emit light and it does not produce electromagnetic energy. And in one embodiment the gel composition has an electrical conductivity of from about 1,000 to about 2, 000 µS/cm.

In another embodiment of the present invention, when the RF energy device is turned on it delivers the RF energy in the range of 35% to about 65% of full power for the for about 20 to about 50 seconds, then the power is increased to from about 65% to about 100% for about 20 to about 50 seconds, then energy delivery is controlled such that the temperature of the stratum corneum is maintained in the range of from about 37°C to about 48°C.

In yet another embodiment of the present invention the energy delivery device is handheld and is applied under an eye of a consumer and moved underneath the eye to just above the crows feet area, and then the direction is reversed, and the energy delivery device is moved back and forth across this path for from about 3 to about 6 minutes. This method can be repeated underneath the other eye of the consumer. Moreover, the energy delivery device can be applied above an eye of a consumer and moved over the eye to just below the crows feet area, and then the direction is reversed, and the energy delivery device is moved back and forth across this path for from about 3 to about 6 minutes. This method can be repeated above the other eye of the consumer.

A multi-step regimen is disclosed comprising the steps of applying the energy delivery device under one eye of a consumer and moving it to just above the crows feet area in a continuous back and forth motion for from about 3 to about 6 minutes, then applying the energy delivery device above an eye of the consumer and moving it to just below the crows feet area in a continuous back and forth motion for from about 3 to about 6 minutes, repeating these two steps on the other eye of the consumer such that the crows feet area adjacent both eyes of the consumer are each treated for from about 6 to about 12 minutes. This multi-step regimen can be completed at least once per day, for a regiment period of 3 to 5 days per week, for from about 3 weeks to bout 6 weeks. The consumer can then waits for about 2 to about 8 months and then repeats the multi step regimen for the regimen period. Preferably, the fine line and wrinkles around the eyes of the consumer are visibly reduced after each regimen period.

The invention is defined in the claims. Other embodiments are merely exemplary.

### BRIEF DESCRIPTION OF THE DRAWINGS

While the specification concludes with claims particularly pointing out and distinctly claiming the present invention, it is believed the same will be better understood from the following description taken in conjunction with the accompanying drawings in which:
**Figure** 1 is a schematic representation of naturally occurring damage and repair cycle in human skin;
**Figure 2A, 2B** illustrates the properties of Human Collagen;
**Figure 3** is a schematic of Biological Model of RF current administration efficacy;
**Figure 4A, 4B** is the 24 hour average fold-change for compilation genes;
**Figure 5A, 5B** is the 24 hour average fold-change for exemplary genes;
**Figure 6A, 6B** is the 1 month average fold-change for compilation genes;
**Figure 7A, 7B** is the 1 month average fold-change for exemplary genes;
**Figure 8A, 8B** are two graphs of RF simple heat transfer;
**Figure 9** is a schematic of RF simple heat transfer;
**Figure 10** is a schematic of the Crow's Feet area around a consumer's eyes;
**Figure 11** is zone A of a consumer's skin;

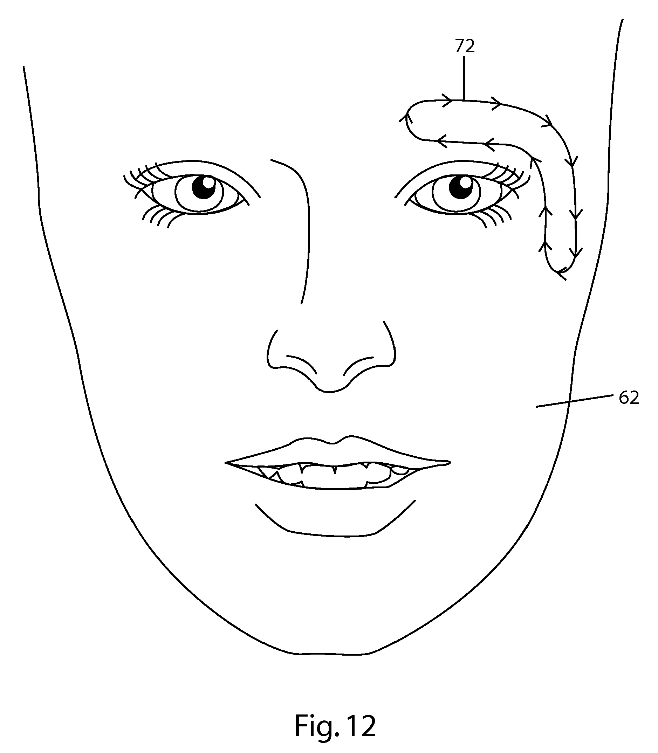

**Figure 12** is zone B of a consumer's skin;
**Figure 13** is zone C of a consumer's skin;
**Figure 14** is zone D of a consumer's skin;
**Figure 15** is zone E of a consumer's skin; and
**Figure 16** is zone F of a consumer's skin.

### DETAILED DESCRIPTION OF THE INVENTION

In all embodiments of the present invention, all percentages are by weight of the total composition, unless specifically stated otherwise. All ratios are weight ratios, unless specifically stated otherwise. All ranges are inclusive and combinable. The number of significant digits conveys neither limitations on the indicated amounts nor on the accuracy of the measurements. All numerical amounts are understood to be modified by the word "about" unless otherwise specifically indicated. All measurements are understood to be made at 25°C and at ambient conditions, where "ambient conditions" means conditions under one atmosphere of pressure and at 50% relative humidity.

It is to be understood that the steps recited in any method can be performed in any order unless specified otherwise. For example, in a method reciting steps (a), (b) and (c), step (c) could be performed prior to or between steps (a) and (b). Furthermore, the individual steps, although recited as distinct steps, can be performed during time periods with some or complete overlap.

Herein, "regulating the condition of skin" means improving the condition of skin and/or prophylactically regulating the condition of skin, and includes, for example, protecting the tissue from ultraviolet radiation, and regulating the signs of skin aging. Herein, "improving the condition of mammalian skin" means effecting a visually and/or tactilely perceptible positive change in the appearance and feel of the tissue. Conditions that may be regulated and/or improved include, but are not limited to, one or more of the following: Reducing the appearance of wrinkles and coarse deep lines, fine lines, crevices, bumps, and large pores; thickening of skin (e.g., building the epidermis and/or dermis and/or sub-dermal layers of the skin, and where applicable the keratinous layers of the nail and hair shaft, to reduce skin, hair, or nail atrophy); increasing the convolution of the dermal-epidermal border (also known as the rete ridges); preventing loss of skin or hair elasticity, for example, due to loss, damage and/or inactivation of functional skin elastin, resulting in such conditions as elastosis, sagging, loss of skin or hair recoil from deformation; reduction in cellulite; change in coloration to the skin, hair, or nails, for example, under-eye circles, blotchiness (e.g., uneven red coloration due to, for example, rosacea), sallowness, discoloration caused by telangiectasia or spider vessels, dryness, brittleness, and graying hair.

As used herein, "signs of skin aging," include, but are not limited to, outward visibly and tactilely perceptible manifestations, as well as any macro- or micro effects, due to skin aging. These signs may result from processes which include, but are not limited to, the development of textural discontinuities such as wrinkles and coarse deep wrinkles, fine lines, skin lines, crevices, bumps, large pores, unevenness or roughness; flaking; dryness; loss of skin elasticity; discoloration (including under eye circles); blotchiness; shallowness; hyperpigmented skin regions such as age spots and freckles; keratoses; abnormal differentiation; hyperkeratinization; elastosis; collagen breakdown, and other histological changes in the stratum corneum, dermis, epidermis, vascular system (e.g., telangiectasia or spider vessels), and underlying tissues (e.g., fat and/or muscle), especially those proximate to the skin.

"Hyperpigmentation," as used herein, refers to an area of skin wherein the pigmentation is greater than that of an adjacent area of skin (e.g., a pigment spot, an age spot, and the like).

Herein, "personal care composition" means compositions suitable for topical application on mammalian skin. The personal care compositions described herein may contain one or more skin care actives. "Skin care actives," or "actives," as used herein, means compounds that aid in regulating the condition of skin and of other manmialian skin, for example, by providing a benefit or improvement to the skin.

"Energy delivery device," as used herein, means any device used to deliver energy to mammalian skin and/or hair. Herein, "delivery of energy," means that the surface and/or layers of the skin are exposed to the energy emanating from the energy delivery device, where it may penetrate to desired layers of the skin, including the hair shaft and/or hair follicle.

"Continuous level," as used herein, means that the energy delivered by the device, or energy output, remains at an essentially constant level between the time of device activation and the time of device deactivation.

"Pulsed," as used herein, means that between the time of device activation and the time of device deactivation, the energy output varies in a predictable manner, characterized by periods of higher output (pulses) alternating with periods of lower output. The onset of pulses may be sudden or gradual. "Predictable" means that the pulse peak intensities, pulse shapes, pulse durations, and the temporal spacing between the pulses are substantially identical. The duration of the pulses and the time between pulses may vary.

"Hand-held," as used herein, means that the device is of a weight and dimension suitable for an average adult human to comfortably hold.

The human skin may be divided into two major structural layers: the epidermis and the sub-epidermal or underlying dermis. The epidermis with the stratum corneum serves as a biological barrier to the environment. In the basilar layer of the epidermis, pigment-forming cells called melanocytes are present, which are the main determinants of skin color.

The underlying dermis provides the main structural support of the skin. It is composed mainly of an extra-cellular protein called collagen. Collagen is produced by fibroblasts and synthesized as a triple helix with three polypeptide chains that are connected with heat labile and heat stable chemical bonds. When collagen-containing tissue is heated, alterations in the physical properties of this protein matrix occur at a characteristic temperature. Structural transition of collagen contraction and remodeling of the collagen matrix occurs with heat.

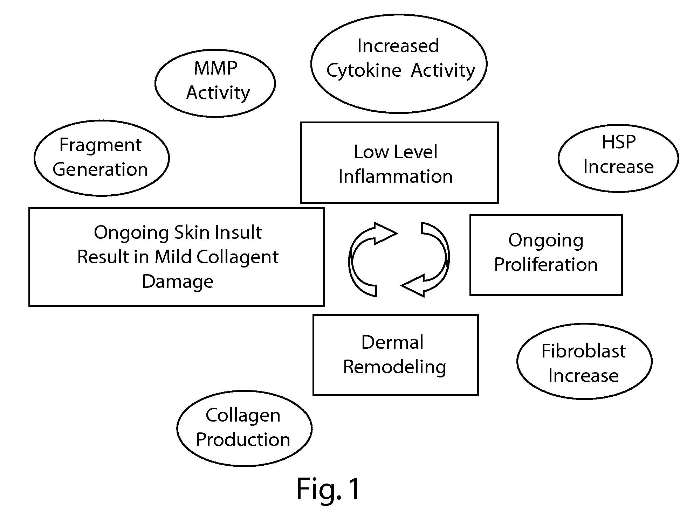

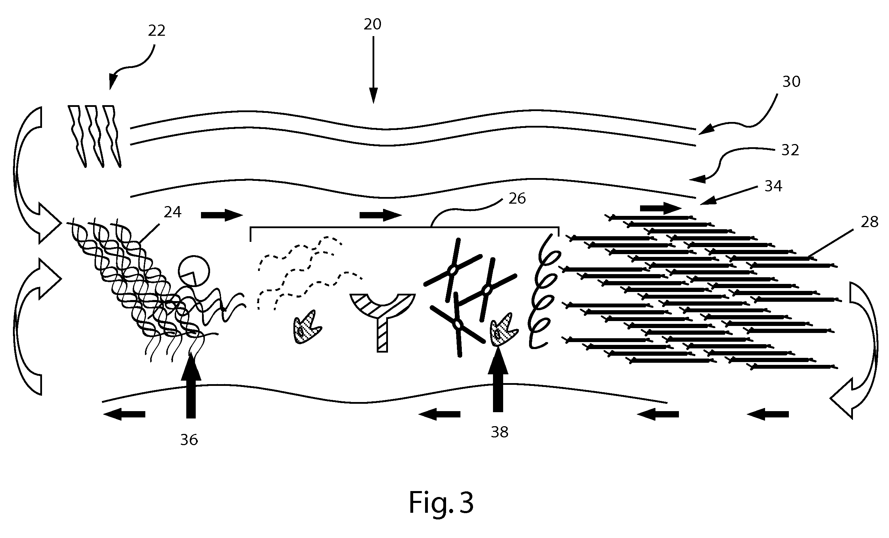

Within the skin, some amount of repair activity occurs to promote continual collagen production. During aging the rate at which damage occurs may increase faster than repair activity, or damage may continue to occur at substantially constant rates, but repair activity slows. In either case the result is reduced collagen and compromised appearance manifest as signs of aging, including appearance of surface defects such as fine lines, wrinkles and hyper-pigmented spots. The normal repair activity cycle and resultant impact on appearance over time is illustrated in Figures 1 and 3.

In Fig. 1 the boxes represent normal causes of collagen production. For many reasons, the increase of collagen in the skin, and the speed with which it is repaired and replaced contributes to fuller, healthier, and more attractive looking skin. Ongoing proliferation and dermal remodeling occur naturally, but unfortunately, these processes slow as we age. Collagen is also produced when skin is damaged, for example, after inflammation or insult from, for example, radiation from the sun. The circles in Fig. 1 represent some of the mechanisms by which collagen is formed and how its formation can be tracked. MMP and cytokine activity are just two measurable quantities that help track the collagen repair and replenishment cycle.

Fig. 3 is a schematic of the dual action biological model for elure efficacy 20. Skin 21 is divided into three layers, the stratum corneum 30, the epidermis 32 and the dermis 34. The collagen remodeling and production of new collagen occurs in the dermis layer. Normal insult 22 to skin 21 occurs constantly and includes normal aging, UV insult, changes in pH, chemical insults and others. Normal insults 22 result in damaged collagen 24. Likewise, normal low level inflammation 26 occurs that results in cytokine, HSP and HSF activity. Ultimately, repeated low level thermal energy yields increased MMP activity 36 results in the production of collagen fragments. The increase in heat cause an up regulation 38 of cellular activity that causes the formation and repair of collagen. Both mechanisms 36 and 38 result in the formation and remodeling of healthy collagen 28.

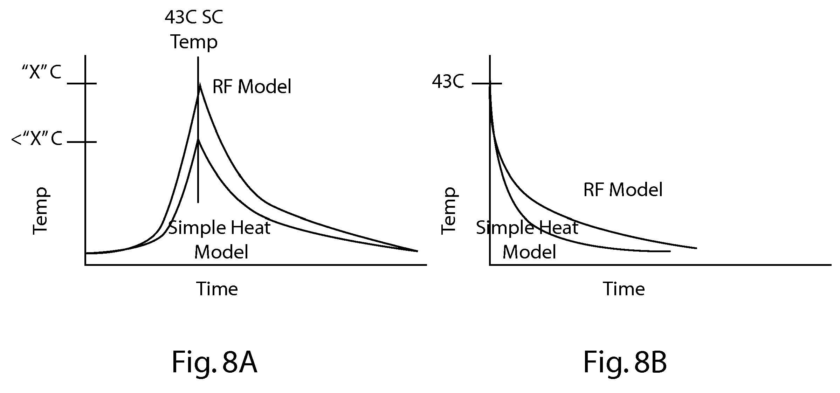

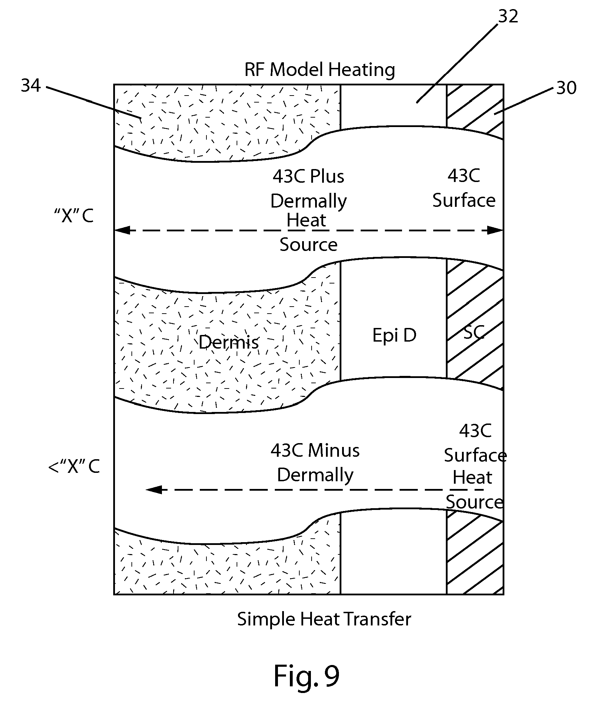

Although collagen is measured to have a melting temperature of up to 50°C, the repair cycle may be altered and enhanced by the addition of heat at lower levels. Thermal cleavage of intramolecular hydrogen bonded crosslinking is created by the balance between cleavage events and relaxation events (reforming of hydrogen bonds). No external force is required for this process to occur. As a result, intermolecular stress is created by the thermal cleavage of intramolecular hydrogen bonds. Contraction of the tertiary structure of the cross-linked molecule creates the initial intermolecular vector of contraction. RF model heating curves are illustrated in Figures 8A, 8B and 9.

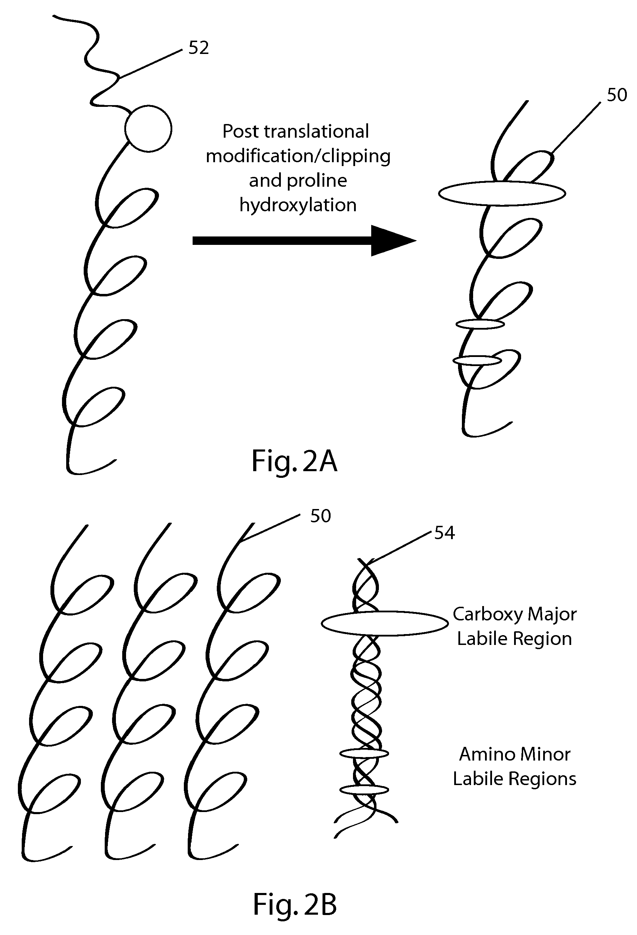

The dermal structure is predominantly comprised of collagen 1, 50, Figures 2A and 2B. Collagen is expressed as procollagen 52, a single stranded protein, by fibroblasts. Procollagen 52 is clipped upon expression to collagen 1 50 and folded into a triple helix conformation called "tropocollagen" 54. This process is illustrated in Figures 2A and 2B.

Collagen crosslinking may be intramolecular (covalent or hydrogen bond) or intermolecular (covalent or ionic bonds). Causes of collagen denaturation as a function of age include thermal energy insult, mechanical insult, effects of pH on collagenase and MMP rate, hydration status, and general disruption in the natural equilibrium of collagen microfibrils which may "zip" or "unzip," making them vulnerable to MMP digestion. Although these represent multiple insult types, all are rate controlled by temperature. Further, the normal collagen turnover cycle may be regulated within a temperature range of <37°C - 43°C.

Cleavage of collagen bonds also occurs at lower temperatures but at a lower rate. Low-level thermal cleavage is frequently associated with relaxation phenomena in which bonds are reformed without a net change in molecular length.

Dermal remodeling is a biophysical phenomenon that occurs at cellular and molecular levels. Molecular contraction or partial denaturization of collagen involves the application of an energy source, which destabilizes the longitudinal axis of the molecule by cleaving the heat labile bonds of the triple helix. As a result, stress is created to break the intermolecular bonds of the matrix. This is essentially an extra-cellular process, whereas cellular contraction requires a lag period for the migration and multiplication of fibroblasts into a damaged area. A healing response generally involves an initial inflammatory process, which consists of infiltration by white blood cells or leukocytes that dispose of cellular debris. This is followed by proliferation of fibroblasts at the injured site and/or an increase in turnover with an ultimate increase in collagen available for deposition. Fibroblast cells differentiate into contractile myofibroblasts, which are the source of cellular soft tissue contraction. Following cellular contraction, collagen is laid down as a static supporting matrix in the tightened soft tissue structure. The deposition and subsequent remodeling of this nascent scar matrix provides the means to alter the consistency and geometry of soft tissue for aesthetic purposes.

Application of thermal energy to initiate the damage and repair cascade in order to ultimately achieve an improvement in surface appearance of skin is known in the art, however currently available technologies are associated with known deficiencies. For example, laser delivery devices use specific wavelengths of light that penetrate the skin, bind to specific chromophores and, through a process called selective photothermolysis, remove various colors and pigments from the skin. The lasers are large, expensive pieces of capital equipment, only attack specific problems or colors in the skin, are prone to laser burns, scars, can cause hyper and/or hypopigmentation and may result in user and patient ocular injuries. Intense broad band light systems emit multiple wavelengths of light, and through selective photothermolysis, also improve skin discoloration and, through skin heating, non-specific skin texture improvement. The systems are also larger and expensive, the skin textures and wrinkle improvements are minimal and there is also the risk of skin burns, hypo or hyperpigmentation and scars. Generally, application of electromagnetic energy is achieved through the epidermis with penetration limited by pigmentation factors at the surface and composition of the dermal layer.

Radio frequency technologies are also known in the art of skin treatment. RF technology uses electrical current to heat the dermis and stimulate production of collagen and elastin fibers that firm and tighten the skin. Substantial drawbacks exist, however, in the current state of the art due to an inability to optimize treatment parameters. RF current application creates a thermal gradient in the skin that is reverse to other thermal energy delivery technologies. Administration of RF current is conducted between electrodes placed some distance apart on the skin. The current is conducted between the electrodes, through the dermis so that the temperature of the dermis rises more rapidly than the temperature at the skin surface. Since most skin parameter measuring devices are designed for skin surface measurement, excessive heating of the dermis may occur before realization.

Hence, treatment by RF current in the absence of thermal quenching, mechanical cooling or other technologies designed to control internal heating has been avoided in the art.

However, utilizing recent advances in the biotechnologies of genomics and proteonomics, the present inventors developed methods for assessing the effects of dermal administration of RF current. In particular, the present inventors screened a group of potential genes identified as involved in the dermal collagen matrix, dermal inflammation and remodeling, and in epidermal differentiation. Genetic signatures and gene chip constituents based on resultant genetic expression profiles were determined by analysis and inspection of differential regulation of the potential genes when subject to specific RF current treatment parameters, conditions and regimens.

Energy delivered to and/or into layers of the skin is in the form of RF energy, including, for example, radiofrequency waves and microwaves. Exemplary RF energy devices are disclosed in the following U.S. Patent Nos.: 6,889,090; 6,702,808; 6,662,054; 5,569,242; 5,755,753; 6,241,753; 6,430,446; 6,350,276; 5,919,219; 5,660,836; 6,413,255; 6,228,078; 5,366,443; and 6,766,202.

The method of the present invention comprises the step of applying a first personal care composition and optionally a second personal care composition to an area of mammalian skin.

The first personal care composition comprises a gel composition. The second personal care compositions may be in a variety of forms, including but not limited to lotions, creams, serums, foams, gels, sprays, ointments, masks, sticks, moisturizers, patches, powders, and/or wipes. The first personal care composition is applied prior to and/or during delivery of energy. In an alternative embodiment, the second personal care composition is applied after the application of the first composition and the delivery of energy. Optionally, the method of the present invention may comprise the step of applying a third personal care composition to the skin, wherein the third composition comprises a conditioning agent. In one embodiment, the third personal care composition is applied prior to application of the first personal care composition. Preferably, the third personal care composition is applied at least 24 hours prior to the delivery of energy. In an alternative embodiment, the first personal care composition is applied twice daily and energy is delivered once daily, alternatively once weekly, and alternatively once monthly. In one embodiment, the first personal care composition is applied to the skin twice daily and energy is delivered to the skin once weekly. The first, second and third personal care compositions may contain a variety of ingredients, non-limiting examples of which may be found in The CTFA International Cosmetic Ingredient Dictionary and Handbook, Tenth Edition (2004).

The compositions of the present invention may comprise from 50% to 99.9% of a dermatologically acceptable carrier. The carrier of the present invention is in the form of an emulsion. Herein, "emulsions" generally contain an aqueous phase and an oil phase. The oils may be derived from animals, plants, or petroleum, may be natural or synthetic, and may include silicone oils. Emulsion carriers include, but are not limited to, oil-in-water, water-in-oil, water-in-oil-in-water, and oil-in-water-in-silicone emulsions. In one embodiment, the dermatologically acceptable carrier comprises an oil-in-water emulsion, and alternatively, a silicone-in-water emulsion. The emulsion further may comprise a humectant, for example, glycerin and a non-ionic, cationic and/or anionic emulsifier. Suitable emulsifiers are disclosed in, for example, U.S. Patent No. 3,755,560 issued to Dickert et al., U.S. Patent No. 4,421,769, issued to Dixon et al., and McCutcheon's Detergents and Emulsifiers, North American Edition, pages 317-324 (1986).

A wide range of quantities of the compositions of the present invention can be employed to improve the condition of the skin. The quantity of the personal care composition that is applied to the skin can vary depending on the bodily location and desired benefit. Exemplary quantities include from 0.1 mg/cm² to 40 mg/cm². One useful application amount is 0.5 mg/cm² to 10 mg/cm².

A temperature change may be simultaneously induced in the skin or alternatively, in a composition applied to the surface of the skin. This temperature change is in addition to any temperature change induced by the delivered energy itself. For example, the skin may be heated prior to delivery of energy, or alternatively, the skin may be cooled before, during, and/or after delivery of energy.

Using the gene panels and genetic signatures after treatment, as well as extensive consumer research, regimen zones were developed that optimize size of the treatment area and the optimal treatment time were developed. It was discovered, contrary to the teachings in the art, using the device over large areas is not the best treatment method. When too large an area is treated, the beginning portion can cool down and recover before the consumer sweeps over it again. If too small an area is treated the consumer risks overtreatment and unnecessary damage. Moreover, the treatment itself is work intensive for the consumer and to breaking the treatment into discreet chunks makes it easier for the consumer to fully comply with the regimen. Likewise, the personal care composition used with the device is much more appealing to the consumer if they can apply it to a small area, treat that area and then remove any remaining personal care composition. Putting the personal care composition on the entire face, or even half of the face, can render the treatment experience unpleasant. This, in turn, has a negative impact on consumer compliance.

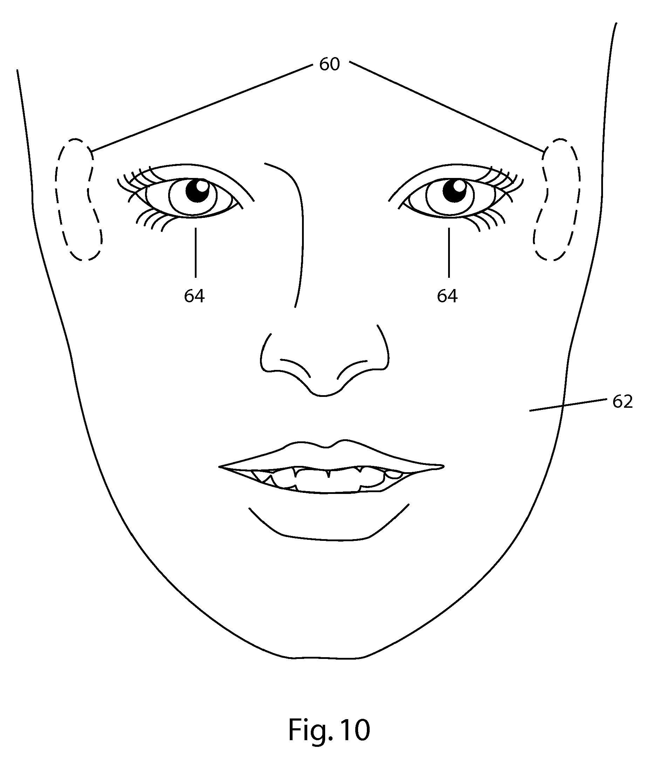

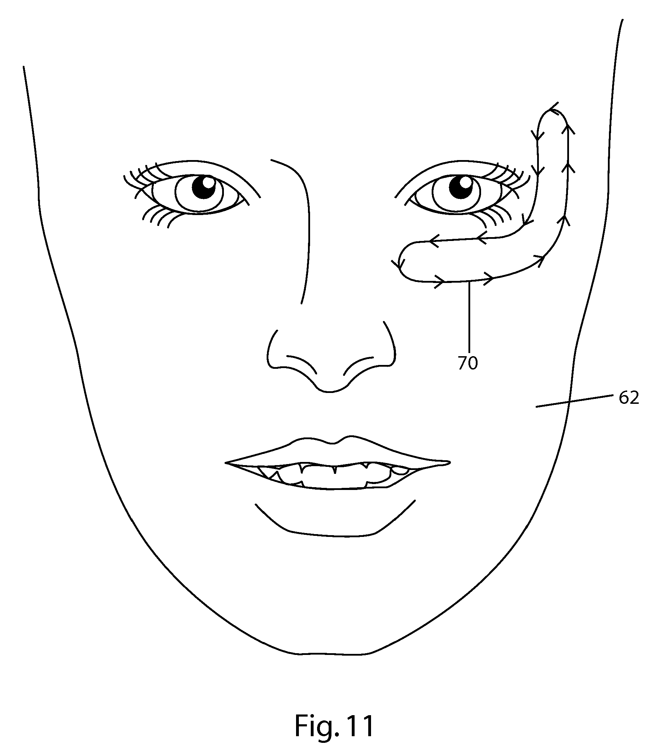

Turning now to Figure 10 wherein crow's feet area 60 of consumer 62 is identified with a dashed circle and occur above and below eyes 64 and adjacent the outside corner of each eye. It is well known that crow's feet areas 60 are prone to wrinkle and fine line formation as human age and are subject to environmental insults. Figure 11 shows Zone A 70 wherein arrows within Zone A are showed to demonstrate a exemplary treatment path for the device (not shown). The times of treatment are given above. Figure 10 illustrates Zone B 72 which is over the same eye of consumer 62 and overlaps crow's feet area 60. Exemplary arrows are shown to illustrate a possible path of treatment. By defining Zone A 70 and Zone B 73 in this manner crow's feet area 60 is treated twice as long as non-crow's feet portion of Zones A and B. This is an important discovery because most of the fine lines and wrinkles on a consumer's face that need treating are in the crow's feet area.

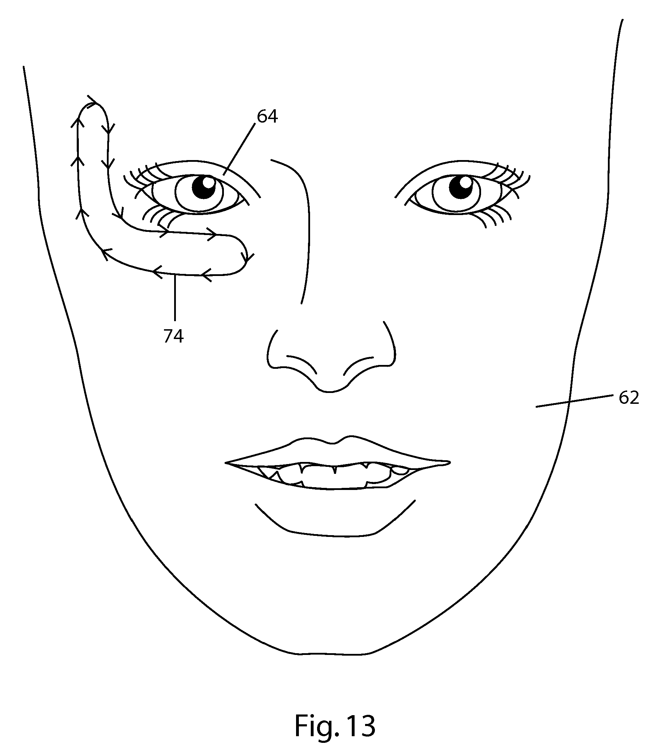

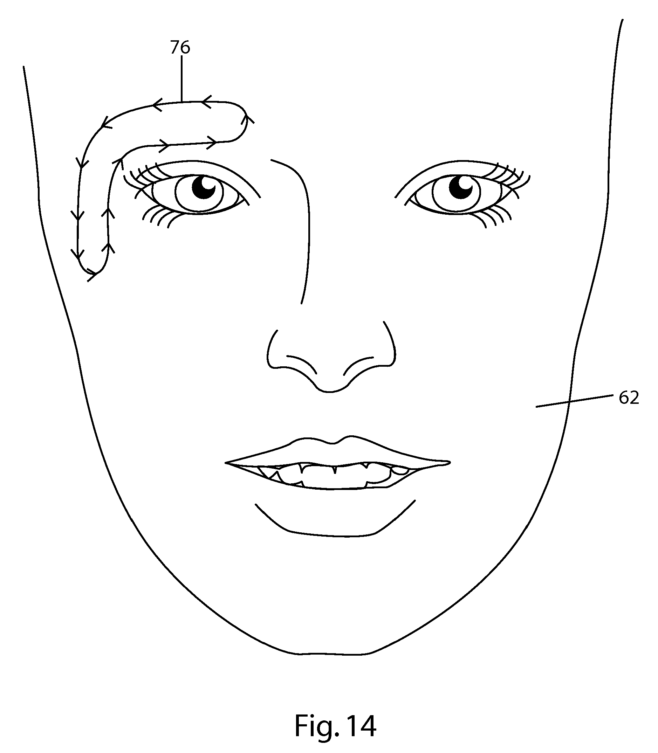

Figures 13 and 14 illustrate Zones C 74 and D 76 on the other eye 64 of the consumer 62. As one would expect, these treatment areas are substantially similar and treated in the same manner as Zones A and B.

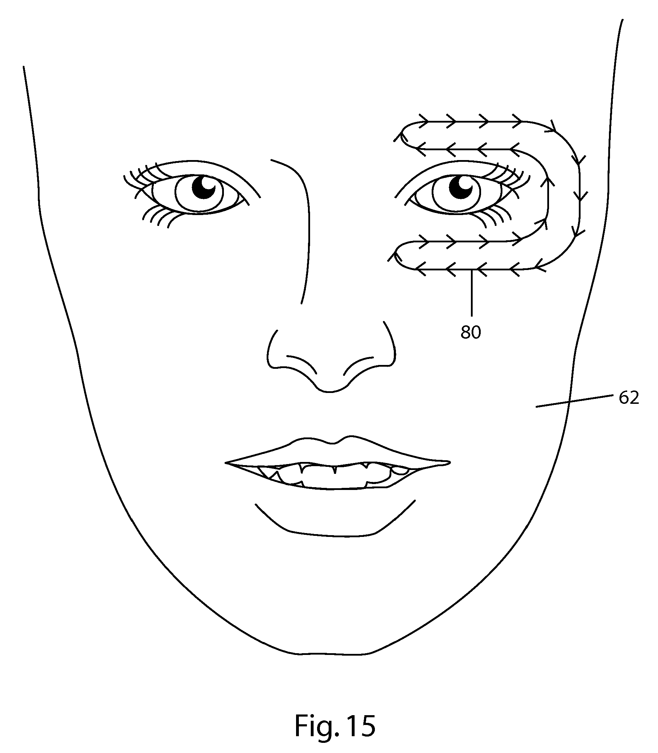

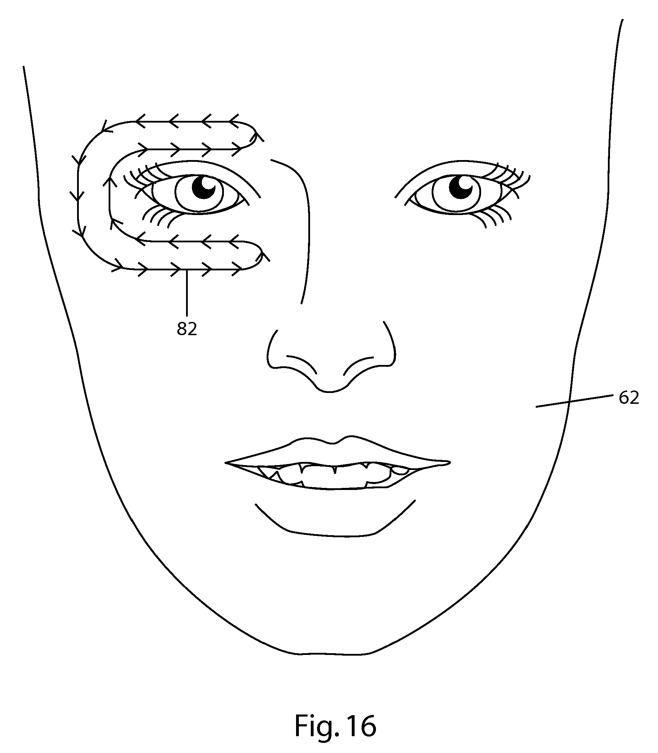

An alternate treatment protocol is shown in Figures 15 and 16 wherein Zones E 80 and F 82 are shown on consumer 62. These "C" shaped treatment areas treat the crow's feet area 60, Figure 10, only once on each pass, but the longer "C" shaped treatment area allows for each area of skin that is treated to cool a bit before the device returns for another pass. Thus, consumer's comfort is increased, but the crow's feet area 60, Figure 10, is treated only during the one treatment cycle.

A second personal care composition may optionally be used in conjunction with the above-described method. The second personal care composition may be used between successive treatment periods that employ the first personal care composition and thermal heat device. The second personal care composition preferably comprises at least one skin care active not present in the first personal care composition.

Application of RF and treatment regimens comprising administration of RF current through the dermis may be designed for the first time in order to optimize the desired effects of RF treatment. Upon analysis and inspection of the genetic expression data, it was surprisingly discovered that the potential gene dataset could be reduced into three subsets with particular utility in optimizing treatment regimens to provide increase in dermal collagen and desired hormetic stress-induced dermal remodeling, in the absence of a more problematic inflammatory cytokine damage response. To the best of the present inventors knowledge, this represents the first time that genomics has been applied to cosmetic treatment employing RF current technology. The expression profiles reveal that controlled and optimized administration results in the provision of positive hormetic stress which initiates and sustains desirable dermal remodeling, while avoiding the traditional damage associated with undesirable biological effects. The present invention provides novel gene chips, genetic signatures, methods of screening and optimizable regimens based on these discoveries.

Genes investigated as potential genes include genes associated with integrity of the dermal matrix (FBN1, FBLN1, TNXB, FN1, LOXL2, COL3A1, COL1A1, ELN and LOXL1), genes associated with dermal inflammation initiated remodeling (TIMP2, IL1A, TIMP1, TNF, MMP1, MMP9, MMP3, SOD2 and IL1B) and genes associated with epidermal barrier function (KRT2, KRT6A, CLDN1, LOR, FLG, IVL, DRT10, AQP3, and KRT14). Subsets derived from analysis of the expression data for these genes are set forth in Table 1 (dermal markers), Table 2 (matrix remodeling markers, i.e. positive hormetic stress initiators) and Table 3 (markers of an inflammatory cytokine response, i.e. damage outside the repair response).

A genetic expression profile provides information about cellular response to a set of conditions. Genes contain the instructions for making messenger RNA (mRNA). At any given point in time, however, each cell makes mRNA from only a fraction of the genes it carries. A gene is referred to as being turned "on" if it is being used to produce mRNA and is otherwise referred to as being turned "off." The term "regulation" refers to triggering a transcriptional status that is different from a gene's control status. For example, "up-regulation" may include merely turning on, or may refer to increasing a transcriptional rate over a base line rate derived from a control or reference condition.

In expression profiling, the relative amount of mRNA expressed in two or more experimental conditions is measured. Altered levels of mRNA suggest a changed need for the protein coded for by the mRNA. For example, increased transcription of enzyme catalysts or cofactors is observed in response to increased levels of the enzyme's substrate in the cellular environment.

In general, a gene expression profile includes those genes that demonstrate significant differences under changed experimental conditions. This is typically a subset of some dataset, which may include the entire genome. For a type of cell, a group of genes whose combined expression pattern is uniquely characteristic to a given condition constitutes a gene signature of the condition. Gene signatures may be used, for example, to select patients which may benefit from a particular treatment, or to design treatment protocols to maximize a desired signature.

Genetic expression profiles for the potential gene dataset may be determined using a microarray. Exemplary cDNA microarrays are commercially available and may be purchased from such companies as Agilent Technologies, Affymetrix Inc. (Santa Clara, Calif.), Nanogen (San Diego, Calif.) and Protogene Laboratories (Palo Alto, Calif.). Specific hybridization technology which also may be practiced to generate the expression profiles employed in the subject methods includes the technology described in U.S. Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992; as well as WO 95/21265; WO 96/31622; WO 97/10365; WO 97/27317; EP 373 203; and EP 785 280. Generally in these methods, an array of "probe" nucleic acids that includes a probe for each of the phenotype determinative genes whose expression is being assayed is contacted with target nucleic acids as set forth above. Contact is carried out under hybridization conditions, e.g., stringent hybridization conditions and unbound nucleic acid is then removed. The resultant pattern of hybridized nucleic acid provides information regarding expression for each of the genes that have been probed, where the expression information is in terms of whether or not the gene is expressed and, typically, at what level, where the expression data, i.e., expression profile, may be both qualitative and quantitative. Alternatively, the expression profile is determined by quantitative PCR or other quantitative methods for measuring mRNA.

The invention provides a gene panel comprising genes regulated in mammalian skin in response to generation of a radio frequency current in a tissue volume of the mammalian skin sufficient to heat the tissue volume to a treatment temperature. At least one gene is selected from Table 1 or Table 2 and at least one gene is selected from Table 3. In specific embodiments at least one gene is selected from each of the three tables. All gene panels according to the invention are contemplated to include at least one gene from Table 3, since lack of expression shift in these genes indicates lack of a "bad" damage response. Probes may be designed to target each gene constituting a gene chip of the invention in order to construct very specific microarrays with utility in designing, screening, adapting or monitoring treatment regimens for the desired effect, or for validation of the treatment regimen in treated subjects. Microarrays comprising a set of immobilized nucleic acid probes capable of hybridizing to and detecting genes constituting a gene panel according to the invention are contemplated.

As used herein, a "probe" refers to an oligonucleotide, polynucleotide or DNA molecule, whether occurring naturally or produced synthetically, which is capable of specifically hybridizing to a nucleic acid with sequences complementary to the probe. The probes of the present invention refer specifically to the oligonucleotides attached to a solid support in the DNA microarray substrate. A probe may be either single-stranded or double-stranded. The probe typically contains 15-25 or more nucleotides, although it may contain fewer nucleotides. The probes herein are selected to be complementary to different strands of a particular target nucleic acid sequence and therefore must be sufficiently complementary so as to be able to specifically hybridize with their respective target strands under a set of pre-determined conditions. Therefore, the probe sequence need not reflect the exact complementary sequence of the target. For example, a non-complementary nucleotide fragment may be attached to the 5' or 3' end of the probe, with the remainder of the probe sequence being complementary to the target strand. Alternatively, non-complementary bases or longer sequences can be interspersed into the probe, provided that the probe sequence has sufficient complementarity with the sequence of the target nucleic acid to anneal therewith specifically.

Another embodiment of the invention includes methods for providing a benefit to mammalian skin. The benefit comprises inducing collagen formation and/or dermal remodeling in a dermal layer of the mammalian skin in the absence of a skin-damaging inflammatory cytokine response and the method comprises generating a radio frequency current in a tissue volume of the mammalian skin for a treatment cycle sufficient to heat the tissue volume to a treatment temperature while avoiding an upregulation in expression of genes listed in Table 3. The RF current may be generated a plurality of times in one treatment cycle.

As used herein the terms "treat" and "treatment" and the like generally refer to obtaining a desired cosmetic or aesthetic effect, underpinned by a targeted biological response. "Treatment" as used herein covers treatment in a mammal, particularly a human, and includes: (a) preventing or avoiding development of a cosmetically undesirable skin condition, for example fine lines, wrinkles, hyper-pigmented spots, and other skin irregularities that result from either chronological or environmental aging or impact on the skin, (b) inhibiting, ameliorating or delaying appearance of a cosmetically undesirable skin condition; (c) reversing or causing regression of the cosmetically undesirable skin condition.

One or more treatment cycles according to the invention may be conducted across a treatment period. Treatment cycles may be as short in duration as necessary to effectuate a desired response. In specific embodiments the treatment cycle is about one minute or less, while in other specific embodiments the treatment cycle is greater than about one minute. In other specific embodiments a treatment cycle lasts between about 1 and about 6 minutes and in other embodiments lasts between about 2 and about 6 minutes. A treatment period according to the invention comprises one or more treatment cycles and in specific embodiments is at least one week and comprises at least one treatment cycle. In other specific embodiments the treatment period is between one week and 12 weeks. In more specific embodiments the treatment period is between 3 and 8 weeks. In certain embodiments each week of a treatment period comprises between one and six treatment cycles, although it is understood in the art that this may vary with unique features of individual being treated.

Generally, although RF current administration effectuates a higher temperature in the dermis than at the skin surface, skin temperature is most conveniently and noninvasively measured at the surface. Hence, in certain embodiments a treatment temperature, defined herein as the temperature of the tissue volume through which the RF current is conducted, effectuates a skin surface temperature over the tissue volume of less than about 45°C. In more specific embodiments the treatment temperature effectuates a skin surface temperature over the tissue volume of between about 37°C and about 43°C.

Desired benefits according to the invention may be assessed by extracting mRNA from a sample obtained from the tissue volume through which the RF current is conducted; and determining an expression profile of a gene panel consisting of at least one gene selected from Table 1 and/or Table 2 and at least one gene selected from Table 3. A benefit is indicated where an expression profile reflects upregulation of genes selected from Tables 1 and/or 2 combined with substantially no change in expression of genes selected from Table 3 is indicative of a benefit being provided.

Generally, regulation of genes in accordance with the invention is reflected in expression fold change data. It is known in the art that many methods exist for analysis of microarray based experiments to identify genes that are differentially expressed between conditions, and that choice of methods may affect the set of genes that are identified. Fold-change appears to provide the most reproducible results. Fold-change may be defined as the ratio of the mean control and mean treatment observations (or as the difference of the mean log control and mean log treatment data). A fold-change of 1, therefore, represents no change over the control observation. A positive fold-change indicates an increase in expression across a time period referred to herein as upregulation, and a negative fold change indicates a decrease in expression across a time period referred to herein as downregulation. The significance of a fold-change may be determined by ordinary statistical methods.

According to certain aspects of the invention, the benefit may be optimized by adjusting the treatment temperature and/or other treatment parameters in response to the expression profile wherein upregulation of the genes selected from Table 1 and/or Table 2 is maximized while expression of genes selected from Table 3 is maintained at substantially no change. The treatment regimens of the invention may comprise maintenance treatments wherein a benefit is maintained beyond the treatment period by one or more maintenance treatments, each maintenance treatment comprising at least one maintenance cycle.

The inventive skin treatment regimens contemplate application of thermal energy by electrically conducted RF current via a handheld RF current delivery device. Preferred RF delivery devices according to the invention deliver RF as a current electrically conducted between two electrodes placed as some distance from one another on the skin. The delivery device that effectuates the benefits of the invention conducts the radio frequency current through the tissue volume in the absence of electromagnetic radiation in the visible light or infrared frequencies of the electromagnetic spectrum and in the absence of supplemental monochromatic or polychromatic light sources directed toward the tissue volume, as well as in the absence of mechanical cooling.

Certain embodiments of the invention provide the capability for assessing efficacy of any energy delivery device or combination of energy delivery technologies in providing benefits according to the invention. Hence, specific methods for assessing treatment efficacy of an energy delivery device designed to provide a benefit to skin by heating the skin comprise: treating the skin by application of the energy delivery device; extracting mRNA from a sample of the treated skin; and generating an expression profile for a gene panel according to the invention. As illustrated in **Example 3,** below, RF current delivery devices exist comprising parameter specification tolerances capable of being dialed in to yield the expression profile according to the invention. A suitable device is manufactured by Syneron. An example of a device that is not designed to achieve the genomic response goals of the instant invention is a handheld personal use RF delivery device manufactured by Ultragen Ltd for distribution in Europe under the brand STOP™. This device, also illustrated in Example 3 for comparative purposes, fails to elicit the upregulation of genes in Tables 1 and 2. The device specifications are designed to accommodate a regimen that is based on provision of substantially less thermal energy applied over a larger skin area and may not reach the levels required to achieve the positive homeotic effect achieved by devices and regimens in accordance with the present invention.

According to another embodiment of the invention, methods of screening a facial skin treatment regimen are provided. The methods are screened for efficacy in providing a collagen and/or dermal remodeling benefit to mammalian skin without stimulating a skin-damaging inflammatory cytokine response. The methods generally comprise treating facial skin according to a treatment regimen; extracting mRNA from a sample of the treated facial skin; generating a gene expression profile for a gene panel according to the invention; comparing the gene expression profile to a reference profile; and determining that the facial treatment regimen is efficacious where the expression profile reflects upregulation of genes selected from Table 1 and/or Table 2 and a substantial lack of regulation of genes selected from Table 3. In specific embodiments the facial skin treatment regimen comprises generating a pulsed radio frequency current through a first tissue volume of the facial skin over a treatment cycle with a radio frequency current generating device. The treatment regimen may comprise moving the radio frequency current generating device and generating a pulsed radio frequency current through a second tissue volume of the facial skin during the treatment cycle.

In screening methods, areas of skin are selected for concordance with characteristics of target treatment areas, in particular of the face. Cosmetically vulnerable areas of facial skin include the periorbital and perioral areas. Hence, facial skin around the ear, known as periauricular skin, is close in structure to the treatment target areas but not readily visible in the case of blemishes which may result from minor biopsy procedures. In aspects of the invention requiring biopsy therefore, the required sample is obtained by biopsy of treated periauricular skin and the reference is obtained by biopsy of pre-treated or non-treated periauricular skin. Generally, the pre-treated or non-treated periauricular reference skin comprises skin substantially adjacent to the treated periauricular skin. In very specific embodiments the expression profile of genes selected from Table 1 and/or Table 2 reveals a fold-change over the reference that is statistically greater than one.

Notably, the instant invention may provide benefits to skin other than facial skin, however facial skin is highlighted in illustrative examples because it poses the greatest cosmetic treatment challenges in that it manifests most readily the effects of both chronological and environmental aging and damage, and is initially thinner and more vulnerable than skin over other areas of the body. Further, facial skin is an area of paramount aesthetic significance to consumers of cosmetic treatment technologies.

In certain embodiments the benefit provided by application of RF-current may be augmented or enhanced by application of a cosmetic active or composition to the facial skin in conjunction with generating the pulsed radio frequency current. The active or composition may have a potentiating or synergistic effect on the gene expression profiles according to the invention. Non-limiting examples of suitable cosmetic actives include Retinol Propionate and derivatives thereof, caffeine, Hyaluronic Acid, and generally plant extracts.

Screening methods according to the invention comprise substantially the same parameters as treatment methods according to the invention with respect to treatment cycles, treatment periods, treatment temperatures and delivery devices, adjusted for desired screen tolerances.

A gene signature of differentially expressed genes suitable for identifying a cosmetic skin benefit is provided. The benefit comprises induction of collagen formation and/or dermal remodeling in a dermal layer of mammalian skin in the absence of a skin-damaging inflammatory cytokine response, the genetic signature comprising at least one gene selected from each of Table 1, Table 2, and Table 3. Generally a gene signature is a subset of genes obtained from a dataset of genes related to a particular characteristic, trait or biological function. Gene signatures may be obtained from all or a part of a gene dataset and signatures according to the invention may comprise information from at least about two genes, or any number of genes up to the number constituting the total dataset. Where a subset of the dataset is used, the subset may comprise upregulated genes, downregulated genes, substantially unregulated genes or combinations thereof.

The following Examples are provided to illustrate certain features and advantages of various embodiments of the invention and should not be construed as limiting the scope thereof.

### EXAMPLES

### Example 1: Treatment protocol

This example illustrates treatment of the periauricular region of facial skin for purposes of conducting genomic and histological assessments. Therefore, all treatments are technician-administered in a clinical setting for control purposes. Treatments are administered to four zones within the treatment area for a total of 16 minutes with 4 minutes per zone, 3 times per week for six weeks. The protocol is according to a split face/neck study in a within-subject control design with the treatment applied only to the periauricular region of one side and a sham treatment consisting of gel plus an unpowered device applied to the corresponding periauricular region of the other side. Treatments are monitored with thermal imaging cameras to ensure that target skin surface temperatures of 40-43°C are reached within the first minute and maintained but not exceeded during each 4 minute treatment.

The treatment site is located and centered just below the ear. The site is approximately 1.5 inches wide and about 4 inches long. If an area of treated skin is too dry or gets too hot, extra gel may be applied. The face and neck of the subject are clean and free of lotions, perfumes and the like. Hair is clipped back from the treatment area.

### Device treatment:

1. One pump (approximately 1.4 g gel) is applied to treatment site and spread evenly without rubbing. An additional pump of gel to the cheek above the treatment area may be applied as needed during treatment to cool skin if it gets too hot, as expressed by the subject.
2. The device is turned on and a 4 minute timer is set.
3. The device applicator head is applied to the skin and moved along the treatment site using a back and forth motion and a light touch. Connectivity is maintained while maneuvering the device head across the treatment area. Proper contact with the skin is indicated by a lens on the applicator which should continue to flash red to indicate contact.
4. The thermal camera image is monitored during treatment. Within 30-60 seconds, the skin in the treatment area reaches the optimal temperature of 40-43°C. Color patterns on the thermal image indicate temperature. Generally, as the device warms up the applicator is moved rapidly across the skin to avoid discomfort from the rising temperature. As the treatment continues, movement is slowed to maintain ideal temperature. Subjects are warned that the device will feel warm, but will not burn them.
5. The temperature is in excess of 43°C if the thermal image shows solid red areas. Temperature is controlled by increasing treatment speed in the back and forth motion, by slightly increasing the size of the treatment area, by alternating the back and forth motion with a figure eight pattern, or by adding more gel by touching the applicator head to extra gel deposited on the cheek for this contingency.
6. If the treatment area fails to reach optimal temperature, or if it cools off, the back and forth motion should be slowed until the bulk of the treatment area is at the target temperature.

### Example 2: Biopsy protocol

This example illustrates the relatively gentle biopsy protocol used to collect samples for assessment and monitoring purposes in accordance with certain aspects of the invention and support data disclosed herein.

A clinical biopsy study was conduced using an RF-current delivery device in 30 adult female subjects. Treatments were provided in the periauricular (around the ear) region of the facial skin. Periauricular skin is known in the cosmetic arts as a suitable perorbital substitute. Treatments were clinically administered using a Syneron V8 device (see Example 3), and a four minute heating profile as the target profile. Subjects received treatment three times per week for a total of six weeks. Biopsies were conducted to inspect skin histology as a function of treatment, and to generate a genomics profile.

### Biopsy procedure:

One 4mm biopsy was taken from the periauricular area from both the right and left sides of the neck (this area is located just below the ear). Using a 30 guage sterile needle, an anesthetic containing 2% xylocaine with epinephrine is injected just under the skin to be biopsied. Once the subject indicates that the area is numb, an appropriate size punch biopsy is collected using standard aseptic techniques, followed by suture closure. A 20% aqueous solution of aluminum chloride is used from homeostasis, as needed. Following biopsy the punch site is monitored for normal healing and sutures are removed after 7 days.

### Post biopsy sample handling:

The biopsied tissue is split into two separate samples, one for gene expression testing and the other for histological evaluation. The sample is divided into equal halves in a line parallel to a line drawn from the stratum corneum to the dermis. Sample handling is in accordance with industry standards.

### Example 3: mRNA extraction and analysis

This example illustrates RNA biomarker identification and analysis that underpins certain embodiments of the invention including gene chips, genetic signatures and discovery of the biological model that guides RF delivery regimen design.

4mm biopsies in accordance with Example 2 were taken from both sides of the face/neck just below the ear as described above 2 times, once at 24 hours post final treatment, and once at 4 weeks post treatment.

The biopsied samples were transferred to 2 ml centrifuge tubes containing 1.5 ml tissue storage reagent (RNA*later*® solution, invitrogen, Life Technologies, Carlsbad, CA). Tubes were refrigerated overnight at 2-8°C. Storage reagent was removed and the samples were placed in a freezer at -80°C until processing. Just prior to processing, samples were removed from the freezer and 1.5 ml monophasic solution of phenol and guanidine isothiocynate (TRIzol® reagent, Invitrogen, Life Technologies, Carlsbad, CA) and one 3 mM Tungsten carbide bead (3 mM Tungsten Carbide beads, Qiagen, Catalog #69997) were added to each tube. The samples were immediately homogenized in a mixer mill (Quiagen Inc.) with four 3 minutes shades at 30/second, flipping the adapter after each. Samples were centrifuged for 10 minutes at 12,000 rpm to remove debris. The supernatant was then transferred to pre-spun Phase Lock gel heavy tubes (Phase Lock gel heavy tubes, Eppendorf, New York, NY, catalog #0032-005-152), 300 µL chloroform (Sigma) was added, and the tubes shaken vigorously without over-vortexing. The samples were centrifuged for 10 minutes at 12,000 rpm and the supernatant was thereafter transferred into new 2 ml centrifuge tubes.

Binding conditions were adjusted by adding 800 µl 70% ethanol, and the tubes were vortexed to mix and then spun briefly. 830 ml of the sample was transferred to an RNeasy mini spin column in a vacuum manifold, and a vacuum was applied. The remaining sample (approx. 830 mil) was transferred onto the same RNeasy column and a vacuum was applied. Contaminants were removed by sequentially pitpetting 700µl and 500µl of buffer RW1 onto the RNeasy column with vacuum applied after each rinse.

The RNeasy spin column was transferred to a new 2mil collection tube and the tube was centrifuged for 2 minutes at 14,000 rpm, and the column was transferred into anew 1.5 ml collection tube. Residual ethanol was aspirated from the inside ridge of each column. Thirty µl of pre-heated Rnase-free water was pipetted directly onto the RNeasy membrane and the membrane was incubated for 5 minutes and then centrifuged for 2 minutes at 14,000 rpm. The elute was then collected, providing ready-to-use RNA in water.

After isolation, RNA yield was determined using the RNA 6000 Nano LabChip® Kit #5065-4476, available from Agilent Technologies, Inc. of Santa Clara, CA. RNA was evaluated by one-step RT-PCR. For the RT-PCR biomarker analysis, RNA was diluted to a final concentration of 5 ng/well.

Purified RNA was converted to cDNA with an RT-PCR kit (QScript™ One-Step Fast MGB qRT-PCR kit, available from Quanta BioScience, Inc., Gaithersburg, MD). Five hundred nanograms of RNA was then mixed with the QScript enzyme/buffer mix and run on a thermal cycler according to kit instructions. One µl of the resulting cDNA was then mixed with the Quanta Perfecta Master Mix, and aliquoted across a custom array plate (Custom RT Profiler™ PCR Array available from SABiosciences, Corp., Federick, MD) containing pre-validated primers for the following genes: KRT2, KRT6A, CLDN1, LOR, FLG, IVL, KRT10, AQP3, KRT14, FBN1, FBNL1, TNXB, FN1, LOXL2, COL3A1, COL1A1, ELN, LOXL1, TIMP2, IL1A, TIMP1, TNF, MMP1, MMP9, MMP3, SOD2, and IL1B. The array plates were then sealed and run on a thermal cycler (StepOnePlus™ Real-Time PCR System Upgrade, from Applied Biosystems, Inc. Foster City, CA).

Data analysis was performed using the data analysis software provided by SABiosciences of Frederick, MD.

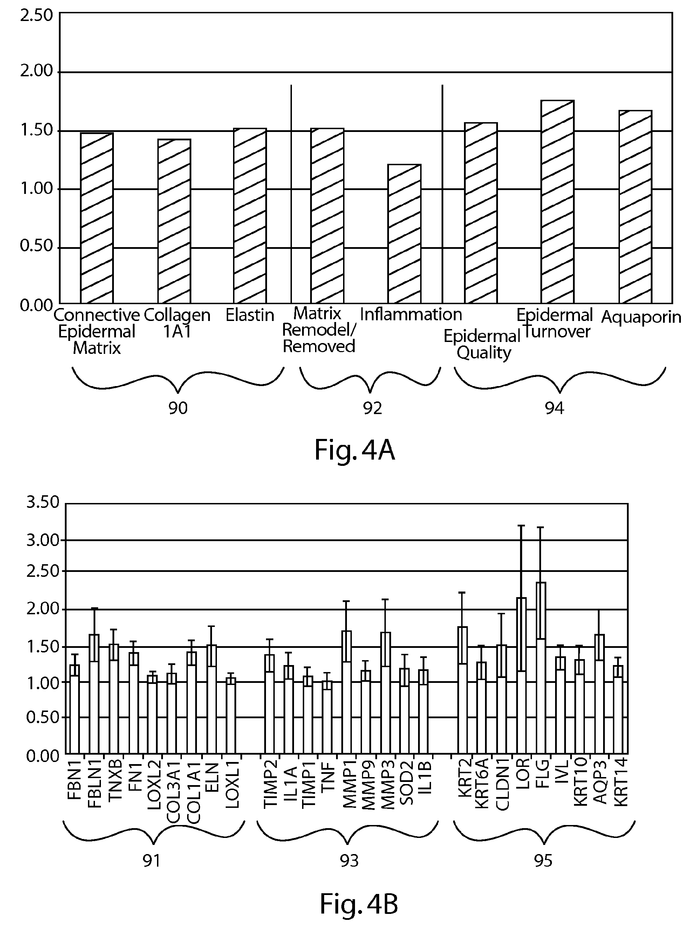

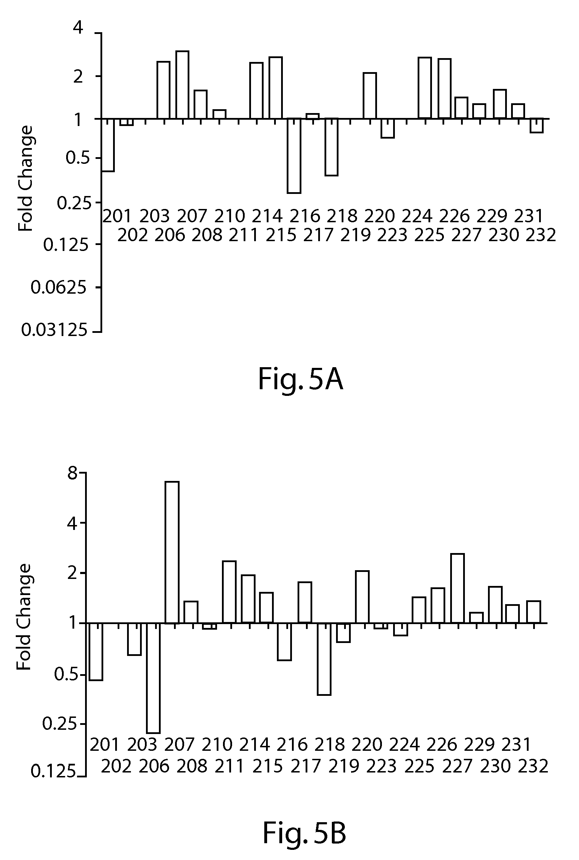

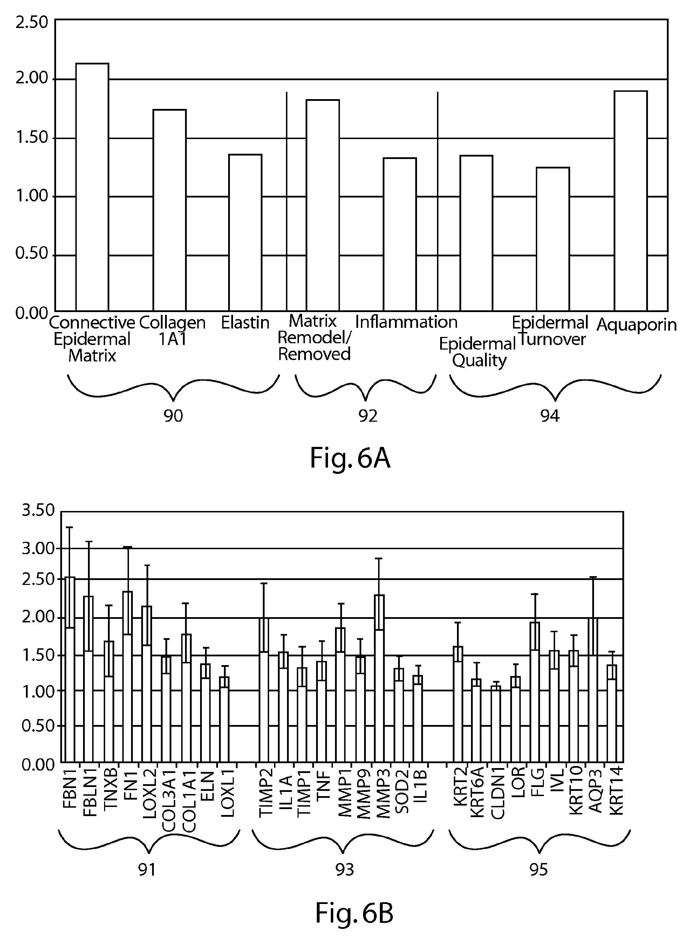

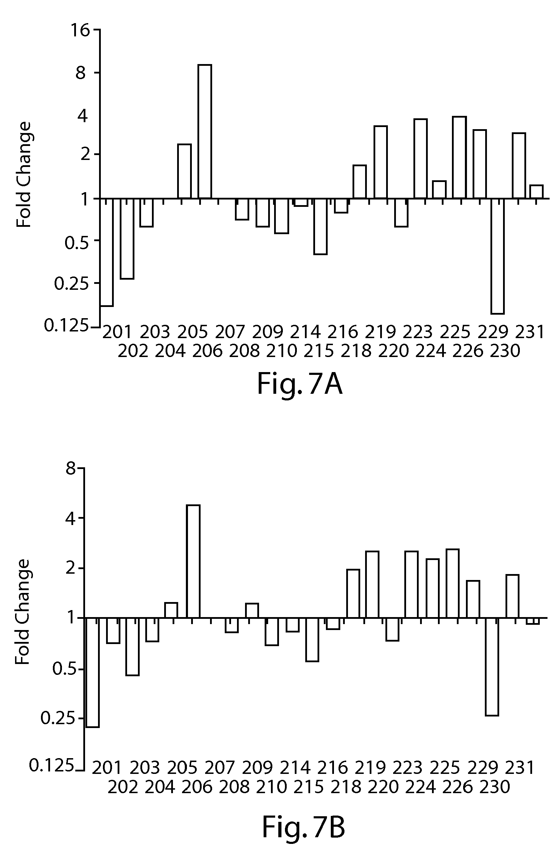

Expression fold change data for the 27 compilation genes listed above at the 24 hour and 4 week target times are set forth in Figures 4A, 4B and Figures 6A, 6B, respectively, above. Genes associated with the dermal matrix are grouped as 90 and 91, the genes associated with dermal inflammation remodeling are grouped as 92 and 93 , and the genes associated with epidermal differentiation are grouped together as 94 and 95. Fold change data for exemplary genes are set forth in Figures 5A collgen 1A1, 5B elastin, and Figure 7A collagen 1A1 and 7B elastin. Note the co-regulation between many of the collagene1A1 genes and the elastin genes.

The fold change data was subject to inspection and analysis and groupings it was surprisingly discovered that when genes demonstrating very little regulation in response to the treatment were segregated (IL1A, IL1B and TNF), they comprised the inflammatory cytokine response genes, while genes relating to collagen synthesis and turnover and dermal remodeling were upregulated under treatment conditions. The genes selected on the basis of fold change data are tabled accordingly in Tables 1, 2 and 3, below.

Further, it was surprisingly found that the positive impact of the treatment regimen on gene expression that existed at 24 months were maintained nearly unabated at the 1 month out target. Maintenance treatments may be desirable to maintain the genomic effects for periods beyond this.

It is contemplated that the gene expression profile reflects a downstream upregulation in the translation products. Although the observed fold change may be relatively low, the sustained upregulation in synthesis of certain proteins and enzymes over an extended period of time is considered to confer substantially enduring cosmetic benefit to the skin.

### Example 4 Personal Care Composition

The following is an exemplary personal care composition for use with the devices of the present invention.

| **TRADE OR COMMON NAME** | **INCI NAME** | **Function** | **PERCENT COMPOSITION** | | **By Wt** % |
|---|---|---|---|---|---|
| | | | **As Added** | **Chem. Content** | **Possible Range** |
| Purified Water | Water (Aqua) | Vehicle | 87.75 | 87.75 | 50.0-96.0 |
| Glycerin | Glycerin | Humectant | 6.00 | 6.00 | 1.0-50.0 |
| Simulgel INS 100 | Hydroxyethyl acrylate Sodium Acryloyldimethyl taurate Copolymer | Thickener | 3.00 | 1.08 | 0.0-3.0 |
| | Isohexadecane | Skin Conditioner | | 0.72 | 0.0-2.5 |
| | Polysorbate 60 | Emulsifier Surfactant | | 0.12 | 0.0-0.30 |
| | Water | Vehicle | | 1.08 | 0.0-3.0 |
| Dow Corning 1503 | Dimethicone | Skin Conditioner | 1.00 | 0.88 | 0.0-3.0 |
| | Dimethiconol | Skin Conditioner | | 0.12 | 0.0-0.03 |
| Hexylene Glycol | Hexylene Glycol | Emulsifier Surfactant | 1.00 | 1.00 | 0.0-1.00 |
| Phenoxyethanol | Phenoxyethanol | Emulsifier Surfactant | 0.50 | 0.50 | 0.0-2.0 |
| Nipagin M | Methylparaben | Preservative | 0.20 | 0.20 | 0.0-2.0 |
| Dissolvine NA2 S | Disodium EDTA | Chelating Agent | 0.05 | 0.05 | 0.0-1.0 |
| Microthene FN-510 | Polyethylene | Acute Powder | 0.50 | 0.50 | 0.0-1.0 |
| | | | 100.0 | 100.0 | 100.0 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

All documents cited in the Detailed Description of the Invention are, in relevant part, incorporated herein by reference; the citation of any document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

**Table 1**

| Dermal Markers | | |
|---|---|---|
| **Gene** | **Expression Product** | **Function** |
| **FBN1** (**SEQ ID NO: 1)** | A member of the fibrillin family; a large, extracellular matrix glycoprotein that serve as a structural component of 10-12 nm calcium-binding microfibrils which provide force bearing structural support in elastic and nonelastic connective tissue throughout the body. | Fibrillins are structural components of 10-12 nm extracellular calcium-binding microfibrils, which occur either in association with elastin or in elastin-free bundles. Fibrillin-1-containing microfibrils provide long-term force bearing structural support |
| **FBLN1** (**SEQ ID NO: 2)** | A secreted glycoprotein that becomes incorporated into a fibrillar extracellular matrix. Calcium-binding is apparently required to mediate its binding to laminin and nidogen. It mediates platelet adhesion via binding fibrinogen. | Incorporated into fibronectin-containing matrix fibers. May play a role in cell adhesion and migration along protein fibers within the extracellular matrix (ECM). Could be important for certain developmental processes and contribute to the supramolecular organization of ECM architecture, in particular to those of basement membranes. Implicated in a role in cellular transformation and tumor invasion; appears to be a tumor suppressor. May play a role in haemostasis and thrombosis owing to its ability to bind fibrinogen and incorporate into clots. Could play a significant role in modulating |
| | | the neurotrophic activities of APP, particularly soluble APP |
| **TNXB** (**SEQ ID NO:** 3) | A member of the tenascin family of extracellular matrix glycoproteins. The tenascins have anti-adhesive effects, as opposed to fibronectin which is adhesive. This protein is thought to function in matrix maturation during wound healing. | Appears to mediate interactions between cells and the extracellular matrix. Substrate-adhesion molecule that appears to inhibit cell migration. Accelerates collagen fibril formation. May play a role in supporting the growth of epithelial tumors |
| **FN1** (**SEQ ID NO: 4)** | fibronectin, a glycoprotein present in a soluble dimeric form in plasma, and in a dimeric or multimeric form at the cell surface and in extracellular matrix. Fibronectin is involved in cell adhesion and migration processes including embryogenesis, wound healing, blood coagulation, host defense, and metastasis. | Fibronectins bind cell surfaces and various compounds including collagen, fibrin, heparin, DNA, and actin. Fibronectins are involved in cell adhesion, cell motility, opsonization, wound healing, and maintenance of cell shape. Interaction with TNR mediates inhibition of cell adhesion and neurite outgrowth (By similarity) |
| **LOXL2** (**SEQ ID NO: 5)** | A member of the lysyl oxidase gene family. | The prototypic member of the family is essential to the biogenesis of connective tissue, encoding an extracellular copper-dependent amine oxidase that catalyses the first step in the formation of crosslinks in collagens and elastin. A highly conserved amino acid sequence at the C-terminus end appears to be sufficient for amine oxidase activity, suggesting that each family member may retain this function. The N-terminus is poorly conserved and may impart additional roles in developmental regulation, senescence, tumor suppression, cell growth control, and chemotaxis to each member of the family. |
| **COL 3A1 (SEQ ID NO: 6)** | The pro-alpha1 chains of type III collagen, a fibrillar collagen that is found in extensible connective tissues such as skin, lung, uterus, intestine and the vascular system, frequently in association with type I collagen. | Collagen type III occurs in most soft connective tissues along with type I collagen |
| **COL 1A1** (**SEQ ID NO: 7)** | The pro-alpha1 chains of type I collagen whose triple helix comprises two alpha1 chains and one alpha2 chain. | Type I is a fibril-forming collagen found in most connective tissues and is abundant in bone, cornea, dermis and tendon. |
| **ELN** (**SEQ ID NO: 8)** | A protein that is one of the two components of elastic fibers. The encoded protein is rich in hydrophobic amino acids such as glycine and proline, which form mobile hydrophobic regions bounded by crosslinks between lysine residues. | Major structural protein of tissues such as aorta and nuchal ligament, which must expand rapidly and recover completely. Molecular determinant of the late arterial morphogenesis, stabilizing arterial structure by regulating proliferation and organization of vascular smooth muscle. |
| **LOXL1** (**SEQ ID NO: 9)** | A member of the lysyl oxidase gene family. | The prototypic member of the family is essential to the biogenesis of connective tissue, encoding an extracellular copper-dependent amine oxidase that catalyses the first step in the formation of crosslinks in collagens and elastin. |

**Table 2**

| Matrix Remodeling Markers | | |
|---|---|---|
| **Gene** | **Expression Product** | **Function** |
| **TIMP2** (**SEQ** | Inhibitors of the matrix metalloproteinases: Complexes with metalloproteinases (such as | Has a unique role among TIMP family members in its ability to directly suppress the |
| **ID NO: 10)** | collagenases) and irreversibly inactivates them. Known to act on MMP-1, MMP-2, MMP-3, MMP-7, MMP-8, MMP-9, MMP-10, MMP-13, MMP-14, MMP-15, MMP-16 and MMP-19 | proliferation of endothelial cells. As a result, the encoded protein may be critical to the maintenance of tissue homeostasis by suppressing the proliferation of quiescent tissues in response to angiogenic factors, and by inhibiting protease activity in tissues undergoing remodelling of the extracellular matrix |
| **TIMP1** (**SEQ ID NO: 11)** | Complexes with metalloproteinases (such as collagenases) and irreversibly inactivates them. Also mediates erythropoiesis in vitro; but, unlike IL-3, it is species-specific, stimulating the growth and differentiation of only human and murine erythroid progenitors. Known to act on MMP-1, MMP-2, MMP-3, MMP-7, MMP-8, MMP-9, MMP-10, MMP-11, MMP-12, MMP-13 and MMP-16. Does not act on MMP-14 | Promotes cell proliferation in a wide range of cell types, and may also have an anti-apoptotic function. Transcription of this gene is highly inducible in response to many cytokines and hormones. |
| **MMP1** (**SEQ ID NO: 12)** | Matrix metalloproteinase (MMP) family of proteins: Cleaves collagens of types I, II, and III at one site in the helical domain. Also cleaves collagens of types VII and X. In case of HIV infection, interacts and cleaves the secreted viral Tat protein, leading to a decrease in neuronal Tat's mediated neurotoxicity | Proteins of the Matrix metalloproteinase (MMP) family of proteins are involved in the breakdown of extracellular matrix in normal physiological processes, such as embryonic development, reproduction, and tissue remodeling, as well as in disease processes, such as arthritis and metastasis. Activated when cleaved by extracellular proteinases. A secreted enzyme which breaks down the interstitial collagens, types I, II, and III. |
| **MMP9** (**SEQ ID NO: 13)** | Matrix metalloproteinase (MMP) family of proteinsCleaves KiSS1 at a Gly- -Leu bond. Cleaves type IV and type V collagen into large C-terminal three quarter fragments and shorter N-terminal one quarter fragments. Degrades fibronectin but not laminin or Pz-peptide | Degrades type IV and V collagens; The enzyme may be involved in IL-8-induced mobilization of hematopoietic progenitor cells from bone marrow, and may have a role in tumor-associated tissue remodeling. May play an essential role in local proteolysis of the extracellular matrix and in leukocyte migration. Could play a role in bone osteoclastic resorption. |
| **MMP3 (SEQ ID** NO: **14)** | Matrix metalloproteinase (MMP) family of proteins | Degrades fibronectin, laminin, collagens III, IV, IX, and X, and cartilage proteoglycans. The enzyme is thought to be involved in wound repair, progression of atherosclerosis, and tumor initiation. |

**Table 3**

| Markers of Inflammatory Cytokine Response | | |
|---|---|---|
| **Gene** | Expression Product | Function |
| **IL1B** (**SEQ ID NO: 15)** | Member of the interleukin 1 cytokine family. This cytokine is produced by activated macrophages as a proprotein, which is proteolytically processed to its active form by caspase 1 (CASP1/ICE) | This cytokine is an Important mediator of the inflammatory response, and is involved in a variety of cellular activities, including cell proliferation, differentiation, and apoptosis. The induction of cyclooxygenase-2 (PTGS2/COX2) by this cytokine in the central nervous system (CNS) is found to contribute to inflammatory pain hypersensitivity. Produced by activated macrophages, IL-1 stimulates thymocyte proliferation by inducing IL-2 release, B-cell maturation and proliferation, and fibroblast growth factor activity. IL-1 proteins are involved in the inflammatory response, being identified as endogenous pyrogens, and are |
| | | reported to stimulate the release of prostaglandin and collagenase from synovial cells |
| **TNF** (**SEQ ID NO: 16)** | A multifunctional proinflammatory cytokine that belongs to the tumor necrosis factor **(TNF)** superfamily and mainly secreted by macrophages; binds to and thus function through its receptors **TNF**RSF1A/**TNF**R1 and **TNF**RSF1 B/**TNF**BR. | Involved in the regulation of a wide spectrum of biological processes including cell proliferation, differentiation, apoptosis, lipid metabolism, and coagulation; Implicated in a variety of diseases, including autoimmune diseases, insulin resistance, and cancer. May have a neuroprotective function. It is mainly secreted by macrophages and can induce cell death of certain tumor cell lines. It is potent pyrogen causing fever by direct action or by stimulation of interleukin-1 secretion and is implicated in the induction of cachexia, Under certain conditions it can stimulate cell proliferation and induce cell differentiation |
| **IL1A** (**SEQ ID NO: 17)** | A member of the interleukin 1 cytokine family; produced by monocytes and macrophages as a proprotein, which is proteolytically processed and released in response to cell injury, and thus induces apoptosis. | Stimulates thymocyte proliferation by inducing IL-2 release, B-cell maturation and proliferation, and fibroblast growth factor activity. IL-1 proteins are involved in the inflammatory response, being identified as endogenous pyrogens, and are reported to stimulate the release of prostaglandin and collagenase from synovial cells |

### Gene Sequences

NCBI Reference Sequence: NM_000138.3
   **Homo sapiens fibrillin 1 (FBN1), mRNA**
   >gi|93589095|ref|NM-000138.3| Homo sapiens fibrillin 1 (FBN1), mRNA 
GGGAGCCGCGGCAGAGACTGTGGGTGCCACAAGCGGACAGGAGCCACAGCTGGACAGCTGCGAGCGGAG
CCGAGCAGTGGCTGTAGCGGCCACGACTGGGAGCAGCCGCCGCCTCCTCGGGAGTCGGAGCCGCCGC
TTCTCCACTGGCAGGGGCCGCCTGAAGTGGGAGCAGCGCCTGGAGAAGGCGGAGGAGCCCGGCCCGGGG
GACGGGCGGCGGGATAGCGGGACCCCGGCGGCGCGGTGCGCTTCAGGGCGCAGCGGCGGCCGCAGACCGA
GCCCCGGGCGCGGCAAGAGGCGGCGGGAGCCGGTGGCGGCTCGGCATCATGCGTCGAGGGCGTCTGCTGG
AGATCGCCCTGGGATTTACCGTGCTTTTAGCGTCCTACACGAGCCATGGGGCGGACGCCAATTTGGAGGC
TGGGAACGTGAAGGAAACCAGAGCCAGTCGGGCCAAGAGAAGAGGCGGTGGAGGACACGACGCGCTTAAA
GGACCCAATGTCTGTGGATCACGTTATAATGCTTACTGTTGCCCTGGATGGAAAACCTTACCTGGCGGAA
ATCAGTGTATTGTCCCCATTTGCCGGCATTCCTGTGGGGATGGATTTTGTTCGAGGCCAAATATGTGCAC
TTGCCCATCTGGTCAGATAGCTCCTTCCTGTGGCTCCAGATCCATACAACACTGCAATATTCGCTGTATG
AATGGAGGTAGCTGCAGTGACGATCACTGTCTATGCCAGAAAGGATACATAGGGACTCACTGTGGACAAC
CTGTTTGTGAAAGTGGCTGTCTCAATGGAGGAAGGTGTGTGGCCCCAAATCGATGTGCATGCACTTACGG
ATTTACTGGACCCCAGTGTGAAAGAGATTACAGGACAGGCCCATGTTTTACTGTGATCAGCAACCAGATG
TGCCAGGGACAACTCAGCGGGATTGTCTGCACAAAAACGCTCTGCTGTGCCACAGTCGGCCGAGCCTGGG
GCCACCCCTGTGAGATGTGTCCTGCCCAGCCTCACCCCTGCCGCCGTGGCTTCATTCCAAATATCCGCAC
GGGAGCTTGTCAAGATGTGGATGAATGCCAGGCCATCCCCGGGCTCTGTCAGGGAGGAAATTGCATTAAT
ACTGTTGGGTCTTTTGAGTGCAAATGCCCTGCTGGACACAAACTTAATGAAGTGTCACAAAAATGTGAAG
ATATTGATGAATGCAGCACCATTCCTGGAATCTGTGAAGGGGGTGAATGTACAAACACAGTCAGCAGTTA
CTTTTGCAAATGTCCCCCTGGTTTTACACCTCTCCAGATGGTACCAGATGCATAGATGTTCGCCCAGGA
TACTGTTACACAGCTCTGACAAACGGGCGCTGCTCTAACCAGCTGCCACAGTCCATAACCAAAATGCAGT
GCTGCTGTGATGCCGGCCGATGCTGGTCTCCAGGGGTCACTGTCGCCCCTGAGATGTGTCCCATCAGAGC
AACCGAGGATTTCAACAAGCTGTGCTCTGTTCCTATGGTAATTCCTGGGAGACCAGAATATCCTCCCCCA
CCCCTTGGCCCCATTCCTCCAGTTCTCCCTGTTCCTCCTGGCTTTCCTCCTGGACCTCAAATTCCGGTCC
CTCGACCACCAGTGGAATATCTGTATCCATCTCGGGAGCCACCAAGGGTGCTGCCAGTAAACGTTACTGA
TTACTGCCAGTTGGTCCGCTATCTCTGTCAAAATGGACGCTGCATTCCAACTCCTGGGAGTTGCCGGTGT
GAGTGCAACAAAGGGTTCCAGCTGGACCTCCGTGGGGAGTGTATTGATGTTGATGAATGTGAGAAAAACC
CCTGTGCTGGTGGTGAGTGTATTAACAACCAGGGTTCGTACACCTGTCAGTGCCGAGCTGGATATCAGAG
CACACTCACGCGGACAGAATGCCGAGACATTGATGAGTGTTTACAGAATGGCCGGATCTGCAATAATGGA
CGCTGCATCAACACAGATGGCAGTTTTCATTGCGTGTGTAATGCGGGCTTTCATGTTACACGAGATGGGA
AGAACTGTGAAGATATGGATGAATGCAGCATAAGGAACATGTGCCTTAATGGAATGTGTATCAATGAAGA
TGGCAGTTTTAAATGTATTTGCAAACCTGGATTCCAGCTGGCATCAGATGGACGTTATTGCAAAGACATT
AACGAGTGTGAAACCCCTGGGATCTCCATCAATCCCCCTTGCGTCAACACTGATGGCTCCTACAGATGTG
AATGCTTCCCTGGACTGGCTGTGGGTCTGGATGGCCGTGTGTGTGTTGACACACACATGCGGAGCACATG
CTATGGTGGATACAAGAGAGGCCAGTGTATCAAACCTTTGTTTGGTGCTGTCACTAAATCTGAATGCTGT
TGCGCCAGCACTGAGTATGCATTTGGGGAACCTTGCCAGCCGTGTCCTGCACAGAATTCAGCGGAATATC
AGGCACTCTGCAGCAGTGGGCCAGGAATGACGTCAGCAGGCAGTGATATAAATGAATGTGCACTAGATCC
TGATATTTGCCCAAATGGAATCTGTGAAAACCTTCGTGGGACCTATAAATGTATATGCAATTCAGGATAT
GAAGTGGATTCAACTGGGAAAAACTGCGTTGATATTAATGAATGTGTACTGAACAGTCTCCTTTGTGACA
ATGGACAATGTAGAAATACTCCTGGAAGTTTTGTCTGTACCTGCCCCAAGGGATTTATCTACAAACCTGA
TCTAAAAACATGTGAAGACATTGATGAATGCGAATCAAGTCCTTGCATTAATGGAGTCTGCAAGAACAGC
CCAGGCTCTTTTATTTGTGAATGTTCTTCTGAAAGTACTTTGGATCCAACAAAAACCATCTGCATAGAAA
CCATCAAGGGCACTTGCTGGCAGACTGTCATTGATGGGCGATGTGAGATCAACATCAATGGAGCCACCTT
AAAGTCCCAGTGCTGCTCCTCCCTCGGTGCTGCGTGGGGAAGCCCGTGCACCCTATGCCAAGTTGATCCC
ATATGTGGTAAAGGGTACTCAAGAATTAAAGGAACACAATGTGAAGATATAGATGAATGTGAAGTGTTCC
CAGGAGTGTGTAAAAATGGCCTGTGTGTTAACACTAGGGGGTCATTCAAGTGTCAGTGTCCCAGTGGAAT
GACTTTGGATGCCACAGGAAGGATCTGTCTTGATATCCGCCTGGAAACCTGCTTCCTGAGGTACGAGGAC
GAGGAGTGCACCCTGCCTATTGCTGGCCGCCACCGCATGGACGCCTGCTGCTGCTCCGTCGGGGCAGCCT
GGGGTACTGAGGAATGCGAGGAGTGTCCCATGAGAAATACTCCTGAGTACGAGGAGCTGTGTCCGAGAGG
 
ACCCGGATTTGCCACAAAAGAAATTACAAATGGAAAGCCTTTCTTCAAAGATATCAATGAGTGCAAGATG
ATACCCAGCCTCTGCACCCACGGCAAGTGCAGAAACACCATTGGCAGCTTTAAGTGCAGGTGTGACAGCG
GCTTTGCTCTTGATTCTGAAGAAAGGAACTGCACAGACATTGACGAATGCCGCATATCTCCTGACCTCTG
TGGCAGAGGCCAGTGTGTGAACACCCCTGGGGACTTTGAATGCAAGTGTGACGAAGGCTATGAAAGTGGA
TTCATGATGATGAAGAACTGCATGGATATTGATGAGTGTCAGAGAGATCCTCTCCTATGCCGAGGTGGTG
TTTGCCATAACACAGAGGGAAGTTACCGCTGTGAATGCCCGCCTGGCCATCAGCTGTCCCCCAACATCTC
CGCGTGTATCGACATCAATGAATGTGAGCTGAGTGCACACCTGTGCCCCAATGGCCGTTGCGTGAACCTC
ATAGGGAAGTATCAGTGTGCCTGCAACCCTGGCTACCATTCAACTCCCGATAGGCTATTTTGTGTTGACA
TTGATGAATGCAGCATAATGAATGGTGGTTGTGAAACCTTCTGCACAAACTCTGAAGGCAGCTATGAATG
TAGCTGTCAGCCGGGATTTGCACTAATGCCTGACCAGAGATCATGCACCGACATCGATGAGTGTGAAGAT
AATCCCAATATCTGTGATGGTGGTCAGTGCACAAATATCCCTGGAGAGTACAGGTGCTTGTGTTATGATG
GATTCATGGCATCTGAAGACATGAAGACTTGTGTAGATGTCAATGAGTGTGACCTGAATCCAAATATCTG
CCTAAGTGGGACCTGTGAAAACACGAAAGGCTCATTTATCTGCCACTGTGATATGGGCTACTCCGGCAAA
AAAGGAAAAACTGGCTGTACAGACATCAATGAATGTGAAATTGGAGCACACAACTGTGGCAAACATGCTG
TATGTACCAATACAGCAGGAAGCTTCAAATGTAGCTGCAGTCCCGGGTGGATTGGAGATGGCATTAAGTG
CACTGATCTGGACGAATGTTCCAATGGAACCCATATGTGCAGCCAGCATGCAGACTGCAAGAATACCATG
GGATCTTACCGCTGTCTGTGCAAGGAAGGATACACAGGTGATGGCTTCACTTGTACAGACCTTGATGAGT
GCTCTGAGAACCTGAATCTCTGTGGCAATGGCCAGTGCCTCAATGCACCAGGAGGATACCGCTGTGAATG
CGACATGGGCTTCGTGCCCAGTGCTGACGGAAAGCCTGTGAAGATATTGATGAGTGCTCCCTTCCGAAC
ATCTGTGTCTTTGGAACTTGCCACAACCTCCCTGGCCTGTTCCGCTGTGAGTGTGAGATAGGCTACGAAC
TGGACAGAAGCGGCGGGAACTGCACAGATGTGAATGAATGCCTGGATCCAACCACGTGCATCAGTGGAA
CTGTGTCAACACTCCAGGCAGCTATATCTGTGACTGCCCACCTGATTTTGAACTGAACCCAACTCGAGTT
GGCTGTGTTGATACCCGCTCTGGAAATTGCTATTTGGATATTCGACCTCGAGGAGACAATGGAGATACAG
CCTGCAGCAATGAAATTGGAGTTGGTGTTTCCAAAGCTTCCTGCTGCTGTTCTCTGGGTAAAGCCTGGG
TACTCCTTGTGAGATGTGTCCTGCTGTGAACACATCCGAGTACAAAATTCTTTGTCCTGGAGGGGAAGGT
TTCCGACCAAATCCTATCACCGTTATATTGGAAGATATTGATGAGTGCCAGGAGCTACCAGGGCTGTGCC
AAGGAGGAAAATGTATCAACACCTTTGGGAGTTTCCAGTGCCGCTGTCCAACCGGCTACTACCTGAATGA
AGATACACGAGTGTGTGATGATGTGAATGAATGTGAGACTCCTGGAATCTGTGGTCCAGGGACATGTTAC
AACACCGTTGGCAACTACACCTGTATCTGTCCTCCAGACTACATGCAAGTGAATGGGGGAAATAATTGCA
TGGATATGAGAAGAAGTTTGTGCTACAGAAACTACTATGCTGACAACCAGACCTGTGATGGAGAATTGTT
ATTCAACATGACCAAGAAGATGTGCTGCTGTTCCTACAACATTGGCCGGGCGTGGAACAAGCCCTGTGAA
CAGTGTCCCATCCCAAGTACAGATGAGTTTGCTACACTCTGTGGAAGTCAAAGGCCAGGCTTTGTCATCG
ACATTTATACCGGTTTACCCGTTGATATTGATGAGTGCCGGGAGATCCCAGGGGTCTGTGAAAATGGAGT
GTGTATCAACATGGTTGGCAGCTTCCGATGTGAATGTCCAGTGGGATTCTTCTATAATGACAAGTTGTTG
GTTTGTGAAGATATTGACGAGTGTCAGAACGGCCCAGTGTGCCAGCGCAACGCCGAATGCATCAACACTG
CAGGCAGCTACCGCTGTGACTGTAAGCCCGGCTACCGCTTCACCTCCACAGGACAGTGCAATGATCGTAA
TGAATGTCAAGAAATCCCCAATATATGCAGTCATGGGCAGTGCATTGACACAGTTGGAAGCTTTTATTGC
CTTTGCCACACTGGTTTTAAAACAAATGATGACCAAACCATGTGCTTGGACATAAATGAATGTGAAAGAG
ATGCCTGTGGGAATGGAACTTGCCGGAACACAATTGGTTCCTTCAACTGCCGCTGCAATCATGGTTTCAT
CCTTTCTCACAACAATCACTCTATACATCTTCATCAATGTCCAACTCCAAATGGCAATCTTTGCAGAAAT
GGCCAATGCATTAATACAGTGGGGTCTTTCCAGTGCCAGTGCAATGAAGGCTATGAGGTGGCTCCAGATG
GGAGGACCTGTGTGGATATCAATGAATGTCTTCTAGAACCCAGAAAATGTGCACCAGGTACCTGTCAAAA
CTTGGATGGGTCCTACAGATGCATTTGCCCACCTGGATACAGTCTTCAAAATGAGAAGTGTGAAGATATT
GATGAGTGTGTCGAAGAGCCAGAAATTTGTGCCCTGGGCACATGCAGTAACACTGAAGGCAGCTTCAAAT
GTCTGTGTCCAGAAGGGTTTTCCTTGTCCTCCAGTGGAAGAAGGTGCCAAGATTTGCGAATGAGCTACTG
TTATGCGAAGTTTGAAGGAGGAAAGTGTTCATCACCCAAATCCAGAAATCACTCCAAGCAGGAATGCTGC
TGTGCCTTGAAGGGAGAAGGCTGGGGAGACCCCTGCGAGCTCTGCCCCACGGAACCTGATGAGGCCTTCC
GCCAGATATGTCCTTATGGAAGTGGGATCATCGTGGGACCTGATGATTCAGCAGTTGATATGGACGAATG
CAAAGAACCCGATGTCTGTAAACATGGACAGTGCATCAATACAGATGGTTCCTATCGCTGCGAGTGTCCC
TTTGGTTATATTCTAGCAGGGAATGAATGTGTAGATACTGATGAATGTTCTGTTGGCAATCCTTGTGGAA
ATGGAACCTGCAAGAATGTGATTGGAGGTTTTGAATGCACCTGCGAGGAGGGATTTGAGCCCGGTCCAAT
GATGACATGTGAAGATATAAATGAATGTGCCCAGAATCCTCTGCTCTGTGCCTTCCGATGTGTGAACACT
TATGGGTCATATGAATGCAAATGTCCCGTGGGATATGTGCTCAGAGAAGACCGTAGGATGTGCAAAGATG
AGGATGAGTGTGAAGAGGGAAAACATGACTGTACTGAAAAACAAATGGAATGCAAGAACCTCATTGGCAC
ATATATGTGCATCTGTGGACCCGGGTATCAGCGGAGACCTGATGGAGAAGGCTGTGTAGATGAGAATGAA
TGTCAGACGAAGCCAGGGATCTGTGAGAATGGGCGCTGCCTCAACACCCGTGGGAGCTACACCTGTGAGT
 
GTAATGATGGGTTTACCGCCAGCCCCAACCAGGACGAGTGCCTTGACAATCGGAAGGGTACTGCTTCAC
AGAGGTGCTACAAAACATGTGTCAGATCGGCTCCAGCAACAGGAACCCCGTCACCAAATCGGAATGCTGC
TGTGACGGAGGGAGAGGCTGGGGTCCCCACTGTGAGATCTGCCCTTTCCAGGGGACTGTGGCTTTCAAGA
AACTCTGTCCCCATGGCCGAGGATTCATGACCAATGGAGCAGATATCGATGAATGCAAGGTTATTCACGA
TGTTTGCCGAAATGGGGAATGTGTCAATGACAGAGGATCATATCATTGCATTTGTAAAACTGGGTACACT
CCAGATATAACTGGGACTTCCTGTGTAGATCTGAACGAGTGCAACCAGGCTCCCAAACCCTGCAATTTTA
TCTGCAAAAACACAGAAGGGAGTTACCAGTGTTCATGCCCGAAAGGCTACATTCTGCAAGAGGATGGAAG
GAGCTGCAAAGATCTTGATGAGTGTGCAACCAAGCAACACAACTGCCAGTTCCTATGTGTTAACACCATT
GGCGGCTTCACATGCAAATGTCCTCCCGGATTTACCCAACACCATACGTCCTGCATTGATAACAATGAAT
GCACCTCTGACATCAATCTGTGCGGGTCTAAGGGCATTTGCCAGAACACTCCTGGAAGCTTCACCTGTGA
ATGCCAGCGGGGATTCTCACTTGATCAGACCGGCTCCAGCTGTGAAGACGTGGACGAGTGTGAGGGTAAC
CACCGCTGCCAGCATGGCTGCCAGAACATCATTGGGGGCTACAGGTGCAGCTGCCCCCAGGGCTACCTCC
AGCACTACCAGTGGAACCAGTGTGTTGATGAAAACGAATGCCTCAGCGCTCACATCTGCGGAGGAGCCTC
CTGTCACAACACCCTGGGGAGCTACAAGTGCATGTGTCCCGCCGGCTTCCAGTATGAACAGTTCAGTGGA
GGATGCCAAGACATCAATGAATGTGGCTCTGCGCAGGCCCCCTGCAGCTATGGCTGTTCCAATACCGAGG
GCGGTTACCTGTGTGGCTGTCCACCTGGTTACTTCCGCATAGGCCAAGGGCACTGTGTTTCTGGAATGGG
CATGGGCCGAGGAAACCCAGAGCCACCTGTCAGTGGTGAAATGGATGACAATTCACTCTCCCCAGAGGCT
TGTTACGAGTGTAAGATCAATGGCTACCCCAAACGGGGCAGGAAACGGAGAAGCACAAACGAAACTGATG
CCTCCAATATCGAGGATCAGTCTGAGACAGAAGCCAATGTGAGTCTTGCAAGTTGGGATGTTGAGAAGAC
AGCCATCTTTGCTTTCAATATTTCCCACGTCAGTAACAAGGTTCGAATCCTAGAACTCCTTCCAGCTCTT
ACAACTCTGACGAATCACAACAGATACTTGATCGAATCTGGAAATGAAGATGGCTTCTTTAAAATCAACC
AAAAGGAAGGGATCAGCTACCTCCACTTCACAAAGAAGAAGCCAGTGGCTGGAACCTATTCATTACAAAT
CAGTAGTACTCCACTTTATAAAAAGAAAGAACTTAACCAACTAGAAGACAAATATGACAAAGACTACCTC
AGTGGTGAACTGGGTGATAATCTGAAGATGAAAATCCAGGTTTTGCTTCATTAATTCACCATCCAGAGAC
CAAATAATTAAAAGAAAAACAAATATAGATAGGTAGAACTATATTTCCCCCAATCAGAATCATCATATC
ATAGGTACAATCTTTCACCAAGTAAATTTGTATAAATAAGCACTATTCTTTGTATTACCAAAGCAAGGTA
CAGGTGACTACCCTAGTTCAAAACAACCACTTTCTCAGGCTTCTCATGTGTGTAGCTAAGCTACCTTGTC
ATATGTGTTGATTCTTGAAAACTGGACGTGTATTTCCATTGGGGGTTGGCCATTTATGCTGACATGCCA
TCCTTCCAGCAAACGTACGGGAATGTGCTTTCAATTGATGGACTACTCTATTTTTTGCAAATTTGTAAAC
TTTGCTTCTCCAAATACAAGTACTAGGTTGTCCATTTATGGTACCTATTTGGTGCTAGTAAATTTTCAAA
CTAGATTTATAAATGCACTGTAATATGTACACAACTTAGAAACCAAATTACAAGTATTCAGTTCCAATAC
TTCATTAATTTCAATCAACCAAAGTTAGTTCAGTAGCTTATCTCAGTTATGAGTATAATACATTACATGT
AAATTAAGTGTGTGTATACTGTAATCGTGCTATTTTTTATCATTGAAACATTTATAAACTAGAATAATAA
TGCCCTTAATGTGAGGGTTTGTAATGGTGCTTATTAAGACCAAAGACTTGTTAAATGTATACACCAAGTG
GTAATGAAATTTCGGTGACTGGCCCACACGTGCATAGAGGTCTGGGAGGACCAGGAAACAGCCTCAGTGG
CCAGAGGATCACCAGTGCATCCTTCATCACAGCATGTGCAATATGCCAAGATTACCCTCGGTCATTCCTG
TCAACAAGGGGTCAATGTCATAAATGTCACAATAAAACAATCTCTTCTTTTTTTTAGTTTACCCCTTGGC
TTTGTGTTCTTGCATGGATTTGGGGTTGGAGGGGCCATTCCGGAGGCTAAATAAAGTCTCCTGGATTTAA
ATTATCCTGGGTCTCTTACTTATGGCTTATGAAAGTACCAAATGTATAACCACTAGAAGAAAATTTAACA
TATGAGTCGATCCCTTCTTTTATCCATTCAAACTACCAGAGTCTGGTGTCATTAACCTGACTTGCTTGTG
AGAAATTTAGATTGTAGAGTCATTTCTGAAACATGACCTAATTCATCTTGTGACTTTTAAATAGTCTTAA
ATACCAAGTTCAGTCATTGTCTTAGAGCACATGAATTTCATTATAATAGATTTATCATGCCCCCCTCTCA
AATATACACAGTTTTGGCAAGCCTTAGGTGTTCTGTTCCATTTTTTTTCCCCTAAACATCTTTCGTTAG
TCAATGCTCATCTAATTACAAAGGATAATCCCAGACTGTATCCAATTGCTGTAACTTTTGGTTTCTTAA
TGTCATAATTTTTAAAGTCTGTTTTATTTTAAGTGCAATATTGAGTATTTAGCTGTTAGGCTCAATCCGT
CGATATGAAATAATTTTTTAAATCCCTAAGGGCAGGAAAGCATTTCGTGGTAGTGAAAATAAGAGGAAAT
AAGATGGCATGAAGGTGGTGGGCGGAGAAACTAGGTAGGACACAGGAAAGTGCTCTCAAAAATCTTTGAA
GAGCTCAGCTGAAAAAAATGGAGTAGATTTGGCTCATACTATTCCGGAAGGCAAAACCAGGGTCAGCTGA
TGTCAGCCCCAGTTTAATACACACGGTCCCAATTATAGAGCTACTCACTGAAAGAATGGGTTTCCTTGCA
TTGTGGTGAGCTCCCTGTCACAAGATAGAAGAGTTTCAGTCTAGGCTTAATGGCAACCATTGGACAAAGA
TGCTTTCTTCCACCTAACAGGCCATTAACATCTTAAAGGTATTTTTGTATCTCTAATTTTGTTTATAATA
GGTGCTCAACAGAATGAGCTGAATGGCTGTTACAAAGGGGGTTTGTACCTTGGGTAAGAGATTAAAATAT
AACTCAAAATTTCCTTCTAACGCTGCACCTATGGAACCATGTGATAGAGGTGTATTAAAATTGTTATCGA
AGAATATATAGCATATGGTAAACAACAGTTTGCATATGGAAAATGTCTTTGATAATTTAACCAGAACTGC
ATTATATTCAATAACGGATTTTCTTTATAACAAACAACAGGGGAAAATGGAGTTGGCACACAGTGGATCA
CTTTGATATTTTTAATAGTCCAAGTCTGGATTTTATTTATTCCTGAGCCAACAATTTTGAACAGCATATT
 
TTCCATGTTTCTGACTGTAACAAAACATTTTCCTCATTGTTCCATTGTAAATATTCCTCTTGTTGGAACT
CTTTTTAATCCTGAGATTTAAACCTGTACCTTTCAATTGTCTGTGACCTTTCAATTTCACTTTCAATAGT
TGAAGAACTTGGCTTTGTAAATCTCTCAGAAGCTTGAAAATATCTTGTCTCTACCCCCTCAGCCCATTTC
ATTTGCCAATAATTATTTTGTAAGTAGGGTTGAAATGAACTCAGCTGGCCTTGTGAAATGTTTAAACTTG
CACAAACAACTACATTTTTGTTCAACAAATAGCAGTTTACTCAGCCAAAATCACTTTGGATATTGCCATT
ACAAATACTGTTAAACTTCAGAAATCATGTCTGTAAATTAGATGAGCCAAAATAAAGGACAATTGGGTTG
ATGCTG
 **Homo sapiens fibulin 1 (FBLN1), transcript variant D, mRNA**
   >gi|34734065|ref|NM-006486.2| Homo sapiens fibulin 1 (FBLN1), transcript variant D, mRNA 
CTCCTCCCGGGCGGATAATTGAACGGCGCGGCCCTGGCCCAGCGTTGGCTGCCGAGGCTCGGCCGGAGC
GTGGAGCCCGCGCCGCTGCCCCAGGACCGCGCCCGCGCCTTTGTCCGCCGCCGCCCACCGCCCGTCGCCC
GCCGCCCATGGAGCGCGCCGCGCCGTCGCGCCGGGTCCCGCTTCCGCTGCTGCTGCTCGGCGGCCTTGCG
CTGCTGGCGGCCGGAGTGGACGCGGATGTCCTCCTGGAGGCCTGCTGTGCGGACGGACACCGGATGGCCA
CTCATCAGAAGGACTGCTCGCTGCCATATGCTACGGAATCCAAAGAATGCAGGATGGTGCAGGAGCAGTG
CTGCCACAGCCAGCTGGAGGAGCTGCACTGTGCCACGGGCATCAGCCTGGCCAACGAGCAGGACCGCTGT
GCCACGCCCCACGGTGACAACGCCAGCCTGGAGGCCACATTTGTGAAGAGGTGCTGCCATTGCTGTCTGC
TGGGGAGGGCGGCCCAGGCCCAGGGCCAGAGCTGCGAGTACAGCCTCATGGTTGGCTACCAGTGTGGACA
GGTCTTCCGGGCATGCTGTGTCAAGAGCCAGGAGACCGGAGATTTGGATGTCGGGGGCCTCCAAGAAACG
GATAAGATCATTGAGGTTGAGGAGGAACAAGAGGACCCATATCTGAATGACCGCTGCCGAGGAGGCGGGC
CCTGCAAGCAGCAGTGCCGAGACACGGGTGACGAGGTGGTCTGCTCCTGCTTCGTGGGCTACCAGCTGCT
GTCTGATGGTGTCTCCTGTGAAGATGTCAATGAATGCATCACGGGCAGCCACAGCTGCCGGCTTGGAGAA
TCCTGCATCAACACAGTGGGCTCTTTCCGCTGCCAGCGGGACAGCAGCTGCGGGACTGGCTATGAGCTCA
CAGAGGACAATAGCTGCAAAGATATTGACGAGTGTGAGAGTGGTATTCATAACTGCCTCCCCGATTTTAT
CTGTCAGAATACTCTGGGATCCTTCCGCTGCCGACCCAAGCTACAGTGCAAGAGTGGCTTTATACAAGAT
GCTCTAGGCAACTGTATTGATATCAATGAGTGTTTGAGTATCAGTGCCCCGTGCCCTATCGGGCATACAT
GCATCAACACAGAGGGCTCCTACACGTGCCAGAAGAACGTGCCCAACTGTGGCCGTGGCTACCATCTCAA
CGAGGAGGGAACGCGCTGTGTTGATGTGGACGAGTGCGCGCCACCTGCTGAGCCCTGTGGGAAGGGACAT
CGCTGCGTGAACTCTCCCGGCAGTTTCCGCTGCGAATGCAAGACGGGTTACTATTTTGACGGCATCAGCA
GGATGTGTGTCGATGTCAACGAGTGCCAGCGCTACCCCGGGCGCCTGTGTGGCCACAAGTGCGAGAACAC
GCTGGGCTCCTACCTCTGCAGCTGTTCCGTGGGCTTCCGGCTCTCTGTGGATGGCAGGTCATGTGAAGAC
ATCAATGAGTGCAGCAGCAGCCCCTGTAGCCAGGAGTGTGCCAACGTCTACGGCTCCTACCAGTGTTACT
GCCGGCGAGGCTACCAGCTCAGCGATGTGGATGGAGTCACCTGTGAAGACATCGACGAGTGCGCCCTGCC
CACCGGGGGCCACATCTGCTCCTACCGCTGCATCAACATCCCTGGAAGCTTCCAGTGCAGCTGCCCCTCG
TCTGGCTACAGGCTGGCCCCCAATGGCCGCAACTGCCAAGACATTGATGAGTGTGTGACTGGCATCCACA
ACTGCTCCATCAACGAGACCTGCTTCAACATCCACCGCCGCTTCCCCTCCCTCCCCTTCCACTCCCCTCA
GAACTACCGCCGCTCCGCAGCCACGCTCCAGCAGGAGAAGACAGACACGGTCCGCTGCATCAAGTCCTGC
CGCCCCAACGATGTCACATGCGTGTTCGACCCCGTGCACACCATCTCCCACACCGTCATCTCGCTGCCTA
CCTTCCGCGAGTTCACCCGCCCTGAAGAGATCATCTTCCTCCGGGCCATCACGCCACCGCATCCTGCCAG
CCAGGCTAACATCATCTTCGACATCACGGAAGGGAACCTGCGGGACTCTTTTGACATCATCAAGCGTTAC
ATGGACGGCATGACCGTGGGTGTCGTGCGCCAGGTGCGGCCCATCGTGGGCCCATTTCATGCCGTCCTGA
AGCTGGAGATGAACTATGTGGTCGGGGGCGTGGTCTCCCACCGAAATGTTGTCAACGTCCACATCTTCGT
CTCTGAGTACTGGTTCTGAGGGCTGGTCTGCCGCACAGCCGCAGGTGCACCTCCAGGCCAAATCATTGCT
GCCAGTGACTGTGGTCTGTACTTGTTTATACCCTCAGACTTTTTTAATGTTAGGTATTTGTAGCATTAGG
CCAACATGTATTAAGCTGAGCCAGATGAATAAGTCCATCTGATGTATTTCGGTGTTTAAAAAATGAGCC
CAGTTGCTCAACTGTTTGGTTGAAAACCTTGCTCATTTTTTAATGCGAAGGCTAAGTGTCACCCCCTTTC
TCTGCCTCTGGCTGGGCCTTGCTAAGGGCCAAGGAAAGAAAGACATTTTTTAGGGGGCAGCCAGTCCAAA
TGCCAAAAGAAGACCAGTTCTTGCCCTGATTGTATGAAATTTGACATTTTGGCACTTTTTTTTTTTTTT
GGCCAATCAGATTTTCTATGTTCTAAGGACATGGCTGCTGTAGAATAGCACAGACGTGGATGATAAATTA
TCCCCAGAAGCAGCATGACAGAATGCCTCGGGGAGCACTTGGAAGGGAAATTGCAGTTCTGTTGAAATAG
AGGAAAATCCCTTGGTAAAGACACAGCCTGTTAGGCTCGTGTGGGCCTCCAGTATGTTCACCAGGGGAAT
GGCTGGGATTTCTCGGCACTCTGCATCATCCATCTTTTCTTATAGGTGGAAAATAAACAACTTTGTGAT
 CCTCCTG
NCBI Reference Sequence: NM_006485.3
   **Homo sapiens fibulin 1 (FBLN1), transcript variant B, mRNA**
   >gi|154091330|ref|NM-006485.3| Homo sapiens fibulin 1 (FBLN1), transcript variant B, mRNA 
CTCCTCCCGGGCGGATAATTGAACGGCGCGGCCCTGGCCCAGCGTTGGCTGCCGAGGCTCGGCCGGAGC
GTGGAGCCCGCGCCGCTGCCCCAGGACCGCGCCCGCGCCTTTGTCCGCCGCCGCCCACCGCCCGTCGCCC
GCCGCCCATGGAGCGCGCCGCGCCGTCGCGCCGGGTCCCGCTTCCGCTGCTGCTGCTCGGCGGCCTTGCG
CTGCTGGCGGCCGGAGTGGACGCGGATGTCCTCCTGGAGGCCTGCTGTGCGGACGGACACCGGATGGCCA
CTCATCAGAAGGACTGCTCGCTGCCATATGCTACGGAATCCAAAGAATGCAGGATGGTGCAGGAGCAGTG
CTGCCACAGCCAGCTGGAGGAGCTGCACTGTGCCACGGGCATCAGCCTGGCCAACGAGCAGGACCGCTGT
GCCACGCCCCACGGTGACAACGCCAGCCTGGAGGCCACATTTGTGAAGAGGTGCTGCCATTGCTGTCTGC
TGGGGAGGGCGGCCCAGGCCCAGGGCCAGAGCTGCGAGTACAGCCTCATGGTTGGCTACCAGTGTGGACA
GGTCTTCCGGGCATGCTGTGTCAAGAGCCAGGAGACCGGAGATTTGGATGTCGGGGGCCTCCAAGAAACG
GATAAGATCATTGAGGTTGAGGAGGAACAAGAGGACCCATATCTGAATGACCGCTGCCGAGGAGGCGGGC
CCTGCAAGCAGCAGTGCCGAGACACGGGTGACGAGGTGGTCTGCTCCTGCTTCGTGGGCTACCAGCTGCT
GTCTGATGGTGTCTCCTGTGAAGATGTCAATGAATGCATCACGGGCAGCCACAGCTGCCGGCTTGGAGAA
TCCTGCATCAACACAGTGGGCTCTTTCCGCTGCCAGCGGGACAGCAGCTGCGGGACTGGCTATGAGCTCA
CAGAGGACAATAGCTGCAAAGATATTGACGAGTGTGAGAGTGGTATTCATAACTGCCTCCCCGATTTTAT
CTGTCAGAATACTCTGGGATCCTTCCGCTGCCGACCCAAGCTACAGTGCAAGAGTGGCTTTATACAAGAT
GCTCTAGGCAACTGTATTGATATCAATGAGTGTTTGAGTATCAGTGCCCCGTGCCCTATCGGGCATACAT
GCATCAACACAGAGGGCTCCTACACGTGCCAGAAGAACGTGCCCAACTGTGGCCGTGGCTACCATCTCAA
CGAGGAGGGAACGCGCTGTGTTGATGTGGACGAGTGCGCGCCACCTGCTGAGCCCTGTGGGAAGGGACAT
CGCTGCGTGAACTCTCCCGGCAGTTTCCGCTGCGAATGCAAGACGGGTTACTATTTTGACGGCATCAGCA
GGATGTGTGTCGATGTCAACGAGTGCCAGCGCTACCCCGGGCGCCTGTGTGGCCACAAGTGCGAGAACAC
GCTGGGCTCCTACCTCTGCAGCTGTTCCGTGGGCTTCCGGCTCTCTGTGGATGGCAGGTCATGTGAAGAC
ATCAATGAGTGCAGCAGCAGCCCCTGTAGCCAGGAGTGTGCCAACGTCTACGGCTCCTACCAGTGTTACT
GCCGGCGAGGCTACCAGCTCAGCGATGTGGATGGAGTCACCTGTGAAGACATCGACGAGTGCGCCCTGCC
CACCGGGGGCCACATCTGCTCCTACCGCTGCATCAACATCCCTGGAAGCTTCCAGTGCAGCTGCCCCTCG
TCTGGCTACAGGCTGGCCCCCAATGGCCGCAACTGCCAAGACATTGATGAGTGTGTGACTGGCATCCACA
ACTGCTCCATCAACGAGACCTGCTTCAACATCCAGGGCGGCTTCCGCTGCCTGGCCTTCGAGTGCCCTGA
GAACTACCGCCGCTCCGCAGCCACGCAGAAATCCAAGAAGGGAAGGCAGAACACCCCAGCGGGATCAAGT
AAAGAGGACTGCAGGGTTCTTCCATGGAAGCAGGGGTTGGAGGATACCCACCTTGATGCCTAGTGAGGAA
GATGGACCTGGACAGACAGTCAGCTCCACACCTTGCGCTGAGCAGCTGTGATTGTGCCACGGAGCATGA
GCCCTTTTCCCCACGGCCCTTGCCACTGTCTCCTGGCCCTCTCTCTGATCATGCCAGGTTTGCACCAGCC
TCGAGTCTCCCATGTTGTAGTACATTCTCCAAGATGCAGCCCAGGAGCCTCTCTGAAGGACCAGTCTGGT
TACGATGGTCTGAGCTTCCTTAGAACCTTCCATGGTTGTCTTTTCCCAGCAGATGAAGCATAGCCTCCTT
GGAATGGCATGGGAGGCCTGGCCTGATCTGGCCTCTGCCCACCCTTTGAGCTGCACCTGCCCCACCCCAG
CTCATCCATGTGCTTGTACCCTGGCCCCACGGGGAGGCTTGCCCTTCCCTGAATCTGCCTTCTTGTGGCT
TTAGCATGTGCCATGCTGTCCCCCTGCAGACTGCCATTCTCCTTGCAGACTTGGCTCAGAAGTCACCTCC
TCAGTGCAGTTAGCCTGAGCTCCCCTGGCCCCAGGTGCCTCCATCAGAGCATTTACCCCATTGTGTTGTG
GCTGTTCCTTAACGTCCCCACTAGCCAGGCTCTTTGAGGGCAGGGATTGTGTCTGGTTAATTTCTGTATT
CTCTGCAACTTTGCAATGTTTGGCTTGAAGAAGGAGCTCAGTAAACATCTGAATAAACATGCAGGTTGAT
GG
NCBI Reference Sequence: NM_001996.2
   **Homo sapiens fibulin 1 (FBLN1), transcript variant C, mRNA**
   >gi|34734061|ref|NM-001996-2| Homo sapiens fibulin 1 (FBLN1), transcript variant C, mRNA 
CTCCTCCCGGCGGATAATTGAACGGCGCGGCCCTGGCCCAGCGTTGGCTGCCGAGGCTCGGCCGGAGC
GTGGAGCCCGCGCCGCTGCCCCAGGACCGCGCCCGCGCCTTTGTCCGCCGCCGCCCACCGCCCGTCGCCC
GCCGCCCATGGAGCGCGCCGCGCCGTCGCGCCGGGTCCCGCTTCCGCTGCTGCTGCTCGGCGGCCTTGCG
CTGCTGGCGGCCGGAGTGGACGCGGATGTCCTCCTGGAGGCCTGCTGTGCGGACGGACACCGGATGGCCA
CTCATCAGAAGGACTGCTCGCTGCCATATGCTACGGAATCCAAAGAATGCAGGATGGTGCAGGAGCAGTG
CTGCCACAGCCAGCTGGAGGAGCTGCACTGTGCCACGGGCATCAGCCTGGCCAACGAGCAGGACCGCTGT
GCCACGCCCCACGGTGACAACGCCAGCCTGGAGGCCACATTTGTGAAGAGGTGCTGCCATTGCTGTCTGC
TGGGGAGGGCGGCCCAGGCCCAGGGCCAGAGCTGCGAGTACAGCCTCATGGTTGGCTACCAGTGTGGACA
GGTCTTCCGGGCATGCTGTGTCAAGAGCCAGGAGACCGGAGATTTGGATGTCGGGGGCCTCCAAGAAACG
GATAAGATCATTGAGGTTGAGGAGGAACAAGAGGACCCATATCTGAATGACCGCTGCCGAGGAGGCGGGC
CCTGCAAGCAGCAGTGCCGAGACACGGGTGACGAGGTGGTCTGCTCCTGCTTCGTGGGCTACCAGCTGCT
GTCTGATGGTGTCTCCTGTGAAGATGTCAATGAATGCATCACGGGCAGCCACAGCTGCCGGCTTGGAGAA
TCCTGCATCAACACAGTGGGCTCTTTCCGCTGCCAGCGGACAGCAGCTGCGGACTGGCTATGAGCTCA
CAGAGGACAATAGCTGCAAAGATATTGACGAGTGTGAGAGTGGTATTCATAACTGCCTCCCCGATTTTAT
CTGTCAGAATACTCTGGGATCCTTCCGCTGCCGACCCAAGCTACAGTGCAAGAGTGGCTTTATACAAGAT
GCTCTAGGCAACTGTATTGATATCAATGAGTGTTTGAGTATCAGTGCCCCGTGCCCTATCGGGCATACAT
GCATCAACACAGAGGGCTCCTACACGTGCCAGAAGAACGTGCCCAACTGTGGCCGTGGCTACCATCTCAA
CGAGGAGGGAACGCGCTGTGTTGATGTGGACGAGTGCGCGCCACCTGCTGAGCCCTGTGGGAAGGGACAT
CGCTGCGTGAACTCTCCCGGCAGTTTCCGCTGCGAATGCAAGACGGGTTACTATTTTGACGGCATCAGCA
GGATGTGTGTCGATGTCAACGAGTGCCAGCGCTACCCCGGGCGCCTGTGTGGCCACAAGTGCGAGAACAC
GCTGGGCTCCTACCTCTGCAGCTGTTCCGTGGGCTTCCGGCTCTCTGTGGATGGCAGGTCATGTGAAGAC
ATCAATGAGTGCAGCAGCAGCCCCTGTAGCCAGGAGTGTGCCAACGTCTACGGCTCCTACCAGTGTTACT
GCCGGCGAGGCTACCAGCTCAGCGATGTGGATGGAGTCACCTGTGAAGACATCGACGAGTGCGCCCTGCC
CACCGGGGGCCACATCTGCTCCTACCGCTGCATCAACATCCCTGGAAGCTTCCAGTGCAGCTGCCCCTCG
TCTGGCTACAGGCTGGCCCCCAATGGCCGCAACTGCCAAGACATTGATGAGTGTGTGACTGGCATCCACA
ACTGCTCCATCAACGAGACCTGCTTCAACATCCAGGGCGGCTTCCGCTGCCTGGCCTTCGAGTGCCCTGA
GAACTACCGCCGCTCCGCAGCCACCCGCTGTGAGCGCTTGCCTTGCCATGAGAATCGGGAGTGCTCCAAG
CTGCCTCTGAGAATAACCTACTACCACCTCTCTTTCCCCACCAACATCCAAGCGCCCGCGGTGGTTTTCC
GCATGGGCCCCTCCAGTGCTGTCCCCGGGGACAGCATGCAGCTGGCCATCACCGGCGGCAATGAGGAGGG
CTTTTTCACCACCCGGAAGGTGAGCCCCCACAGTGGGGTGGTGGCCCTCACCAAGCCTGTCCCCGAGCCC
AGGGACTTGCTCCTGACCGTCAAGATGGATCTCTCTCGCCACGGCACCGTCAGCTCCTTTGTGGCCAAGC
TTTTCATCTTTGTGTCTGCAGAGCTCTGAGCACTCGCTTCGCGTCGCGGGTCTCCCTCCTGTTGCTTTC
CTAACCCTGCCCTCCGGGGCGTTAATAAAGTCTTAGCAAGCGTCCCACACAGTGAAAAAAAAAAAAAAAA
AAA
NCBI Reference Sequence: NM_006487.2
   **Homo sapiens fibulin 1 (FBLN1), transcript variant A, mRNA**
   >gi|34734067|ref|NM-006487.2| Homo sapiens fibulin 1 (FBLN1), transcript variant A, mRNA 
CTCCTCCCGGGCGGGATAATTGAACGGCGCGGCCCTGGCCCAGCGTTGGCTGCCGAGGCTCGGCCGGAGC
GTGGAGCCCGCGCCGCTGCCCCAGGACCGCGCCCGCGCCTTTGTCCGCCGCCGCCCACCGCCCGTCGCCC
GCCGCCCATGGAGCGCGCCGCGCCGTCGCGCCGGGTCCCGCTTCCGCTGCTGCTGCTCGGCGGCCTTGCG
CTGCTGGCGGCCGGAGTGGACGCGGATGTCCTCCTGGAGGCCTGCTGTGCGGACGGACACCGGATGGCCA
CTCATCAGAAGGACTGCTCGCTGCCATATGCTACGGAATCCAAAGAATGCAGGATGGTGCAGGAGCAGTG
CTGCCACAGCCAGCTGGAGGAGCTGCACTGTGCCACGGGCATCAGCCTGGCCAACGAGCAGGACCGCTGT
GCCACGCCCCACGGTGACAACGCCAGCCTGGAGGCCACATTTGTGAAGAGGTGCTGCCATTGCTGTCTGC
TGGGGAGGGCGGCCCAGGCCCAGGGCCAGAGCTGCGAGTACAGCCTCATGGTTGGCTACCAGTGTGGACA
GGTCTTCCGGGCATGCTGTGTCAAGAGCCAGGAGACCGGAGATTTGGATGTCGGGGGCCTCCAAGAAACG
GATAAGATCATTGAGGTTGAGGAGGAACAAGAGGACCCATATCTGAATGACCGCTGCCGAGGAGGCGGGC
CCTGCAAGCAGCAGTGCCGAGACACGGGTGACGAGGTGGTCTGCTCCTGCTTCGTGGGCTACCAGCTGCT
 
GTCTGATGGTGTCTCCTGTGAAGATGTCAATGAATGCATCACGGGCAGCCACAGCTGCCGGCTTGGAGAA
TCCTGCATCAACACAGTGGGCTCTTTCCGCTGCCAGCGGGACAGCAGCTGCGGGACTGGCTATGAGCTCA
CAGAGGACAATAGCTGCAAAGATATTGACGAGTGTGAGAGTGGTATTCATAACTGCCTCCCCGATTTTAT
CTGTCAGAATACTCTGGGATCCTTCCGCTGCCGACCCAAGCTACAGTGCAAGAGTGGCTTTATACAAGAT
GCTCTAGGCAACTGTATTGATATCAATGAGTGTTTGAGTATCAGTGCCCCGTGCCCTATCGGGCATACAT
GCATCAACACAGAGGGCTCCTACACGTGCCAGAAGAACGTGCCCAACTGTGGCCGTGGCTACCATCTCAA
CGAGGAGGGAACGCGCTGTGTTGATGTGGACGAGTGCGCGCCACCTGCTGAGCCCTGTGGGAAGGGACAT
CGCTGCGTGAACTCTCCCGGCAGTTTCCGCTGCGAATGCAAGACGGGTTACTATTTTGACGGCATCAGCA
GGATGTGTGTCGATGTCAACGAGTGCCAGCGCTACCCCGGGCGCCTGTGTGGCCACAAGTGCGAGAACAC
GCTGGGCTCCTACCTCTGCAGCTGTTCCGTGGGCTTCCGGCTCTCTGTGGATGGCAGGTCATGTGAAGAC
ATCAATGAGTGCAGCAGCAGCCCCTGTAGCCAGGAGTGTGCCAACGTCTACGGCTCCTACCAGTGTTACT
GCCGGCGAGGCTACCAGCTCAGCGATGTGGATGGAGTCACCTGTGAAGACATCGACGAGTGCGCCCTGCC
CACCGGGGGCCACATCTGCTCCTACCGCTGCATCAACATCCCTGGAAGCTTCCAGTGCAGCTGCCCCTCG
TCTGGCTACAGGCTGGCCCCCAATGGCCGCAACTGCCAAGACATTGATGAGTGTGTGACTGGCATCCACA
ACTGCTCCATCAACGAGACCTGCTTCAACATCCAGGGCGGCTTCCGCTGCCTGGCCTTCGAGTGCCCTGA
GAACTACCGCCGCTCCGCAGCCACATGATCGTAGGGAACTCTGCATGAGGCCATCGGTGCAGGCTGGAGA
AGAGAAGGCAAGTTGGCAGGAGTGGAGACCACGGGCATTTGAGCCACTTCCTCATGTAACTTAACTTGTG
CCTTCAGGACCTGCTCAAGCCCGATCACGTATATACCACTTCCATTTGATGATGGAATGCTGCTGTTCAT
GACCAACTTTATGGCTAGATGGGTCAGAAAGCACCCAGTTCATGATAGGCAGTTCAGGTCATATGGTGAC
TTGATGACCCAGAGTCAAACATTCAGTTTCCACCAAAGCCCAGTAACAGGCCAAGAGCTGTCTCTCAAAA
GGAGAGTAGTTATCTGCAGAAGATGGCAGGGCCTTGCTCCGAAAGCCTAGAGACCGCCACTGTGATTCAC
CTATGGGGGCCTGCCAAAGGCTGCAGCCAGCATCCTTATCTGCCACTGACACCTCAAGCAACATTGGATC
TGCTGGGTCATATGGCCCAAGTGGCAGAGCAACTTGCACAACAGCCTGGACCTGTCATAGAGCTTTCTCC
TGTTCTGGACCCCACTCAAAACTGGCAGCCTTTCAGGTCACTCGATAAATGTGCTGGAGTAACACTCAAA
CGAGGAATGTGTTGCCTCCAAAATCCAATAGGCCC
NCBI Reference Sequence: NM_019105.6
   **Homo sapiens tenascin XB (TNXB), transcript variant XB, mRNA**
   >gi|188528647|ref|NM_019105.6| Homo sapiens tenascin XB (TNXB), transcript variant XB, mRNA 
TCCTCCCCTTCTCCTCCCCTGCTCGCTGCAGACTCCTCCTCACTGTCGCTGCCGAGATCCACAGTCGGT
TGTGGCTCAGCCCCTGTTGCAGGGGACAAGTGAGGGAGACTTCCCTGTCCTGCCCTGAGACGCCGCCCTC
CCGGGGTTGGGGACAGAGCAGGTGCAGAGGCACTGCAGCTGCTCGGTTGCCCAGCCTCCTGAATGATGCC
AGCCCAGTATGCTCTAACCTCCAGCCTGGTTCTCCTGGTGCTGCTGAGCACAGCCAGAGCAGGCCCCTTC
TCTTCACGGTCCAATGTGACACTGCCAGCCCCCCGGCCCCCTCCCCAGCCAGGGGGCCACACAGTGGGGG
CTGGAGTGGGAAGCCCCTCTTCTCAGCTTTACGAGCACACAGTGGAAGGAGGGGAGAAGCAGGTGGTATT
CACCCACCGCATTAACCTGCCCCCTTCCACTGGCTGTGGCTGTCCCCCAGGCACCGAGCCCCCAGTCCTT
GCTTCAGAGGTACAGGCCCTGAGGGTCCGTCTAGAGATCCTGGAGGAGTTGGTGAAGGGGCTCAAGGAAC
AGTGCACTGGGGGATGTTGTCCTGCCTCTGCCCAAGCTGGCACAGGTCAGACAGATGTGCGGACCCTCTG
CAGTCTCCATGGTGTGTTTGATCTGAGCCGCTGCACCTGTTCCTGTGAGCCAGGCTGGGGTGGCCCACC
TGCTCAGACCCCACAGATGCTGAGATCCCTCCCTCTTCCCACCCTCAGCCTCGGGGTCCTGCCCAGATG
ACTGCAATGATCAGGGTCGCTGTGTCCGTGGTCGTTGCGTGTGCTTTCCCGGCTACACTGGCCCCAGCTG
TGGCTGGCCATCCTGTCCCGGGGACTGCCAAGGCCGTGGGCGCTGCGTGCAGGGCGTGTGTGTGCCGG
GCAGGCTTCTCAGGCCCCGACTGCAGCCAGCGCTCCTGCCCTCGAGGTTGCAGCCAGAGGGGACGCTGTG
AGGGTGGGCGCTGCGTGTGTGACCCAGGCTACACTGGTGACGACTGTGGCATGAGGAGCTGCCCTCGCGG
TTGCAGTCAGAGGGGGCGCTGTGAGAATGGGCGCTGCGTGTGTAACCCCGGCTACACTGGCGAGGACTGT
GGGGTGAGGAGCTGCCCTCGGGGCTGCAGCCAGCGGGGACGCTGCAAGGACGGGCGCTGCGTGTGTGACC
CCGGCTACACTGGCGAGGACTGTGGTACGCGGAGCTGCCCCTGGGACTGTGGCGAGGGCGGGCGCTGCGT
GGACGGCCGCTGCGTGTGCTGGCCCGGGTACACAGGCGAGGACTGCAGCACGCGGACATGTCCGAGGGAC
TGCCGGGGCCGCGGGCGCTGCGAGGACGGCGAATGCATTTGCGACACGGGCTACAGCGGGGACGACTGCG
GCGTGCGCAGCTGCCCTGGCGACTGCAACCAAAGGGGCCGCTGCGAGGACGGCCGCTGCGTGTGCTGGCC
GGGGTACACTGGAACCGATTGCGGCTCGCGCGCCTGCCCACGCGACTGTAGAGGTCGCGGGCGCTGCGAG
 
AACGGCGTGTGTGTTTGCAATGCGGGCTACAGCGGCGAGGACTGCGGTGTGCGCAGCTGTCCTGGGACT
GTCGTGGCCGGGGCCGCTGTGAGAGTGGCCGCTGCATGTGTTGGCCGGGGTACACAGGCCGGGACTGCGG
CACGCGCGCCTGTCCTGGCGACTGTCGCGGGCGCGGGCGCTGCGTGGATGGCCGCTGCGTGTGCAACCCG
GGCTTCACCGGTGAGGACTGTGGGAGCCGTCGCTGTCCCGGGGACTGCCGTGGGCACGGCCTTTGCGAGG
ATGGCGTGTGCGTGTGTGACGCAGGCTACTCAGGGGAAGACTGCAGCACGCGCAGCTGCCCCGGGGGCTG
CCGAGGCCGCGGCCAGTGCCTAGATGGGCGGTGTGTGTGCGAGGACGGCTACTCTGGCGAGGATTGCGGT
GTGAGGCAGTGCCCGAATGACTGCAGCCAGCACGGCGTGTGCCAGGACGGTGTGTGCATCTGTTGGGAAG
GCTACGTGAGTGAGGACTGCAGCATCCGCACCTGCCCCTCCAACTGCCACGGAGGGCCGCTGTGAGGA
AGGGCGCTGCCTGTGCGACCCAGGCTACACCGGCCCTACCTGTGCCACCCGCATGTGCCCGGCTGACTGC
CGGGGACGTGGGCGGTGTGTGCAAGGAGTGTGCCTGTGCCACGTGGGCTATGGCGGTGAGGACTGCGGC
AGGAAGAGCCTCCAGCCAGCGCCTGCCCTGGAGGCTGCGGGCCCCGGGAACTGTGCCGGGCAGGCCAGTG
TGTGTGTGTAGAGGGCTTCCGAGGCCCTGACTGTGCCATCCAGACATGCCCAGGGCACTGCCGTGGCCGA
GGAGAGTGTCACGATGGCAGCTGTGTCTGCAAAGATGGGTATGCTGGCGAAGACTGCGGAGAAGAGGTGC
CAACCATTGAGGGCATGAGGATGCATCTCTTGGAGGAGACAACAGTTCGGACAGAGTGGACCCCGGCTCC
TGGCCCCGTGGATGCCTATGAAATTCAGTTCATCCCCACGACAGAGGGGGCGAGCCCCCCATTCACAGCA
CGGGTTCCAAGCTCTGCCTCAGCCTATGACCAGAGAGGACTGGCCCCTGGACAGGAGTACCAGGTCACTG
TCCGAGCCCTTCGAGGGACCAGCTGGGGCCTTCCTGCCTCCAAGACCATCACCACCATGATCGATGGCC
CCAGGACCTCCGAGTGGTGGCTGTGACACCGACAACACTGGAGCTTGGCTGGCTGCGTCCCCAGGCTGAG
GTGGACCGATTTGTGGTGTCCTACGTCAGTGCCGGCAACCAGAGGGTGAGGCTGGAAGTGCCCCCTGAAG
CAGACGGGACGCTGCTGACTGACCTGATGCCAGGCGTAGAATATGTGGTGACTGTCACAGCGGAGCGGGG
CCGGGCAGTCAGCTACCCAGCTTCTGTCAGGGCCAACACAGGGTCCTCACCCTTGGGCCTCTTGGGACT
ACCGATGAGCCTCCTCCCTCAGGCCCCTCGACGACGCAAGGGGCCCAGGCTCCTCTCCTGCAGCAGCGCC
CCCAGGAGCTGGGAGAGTTGAGGGTGCTGGGCAGAGATGAGACAGGGCGCCTCCGTGTGGTCTGGACCGC
CCAGCCTGACACCTTTGCCTACTTCCAACTGCGCATGCGGGTGCCCGAGGGGCCGGGGGCACATGAGGAA
GTGCTGCCAGGGGACGTCCGCCAGGCTCTGGTGCCTCCACCCCCTCCTGGAACCCCGTATGAGCTGTCAC
TTCATGGGGTCCCTCCTGGGGGCAAGCCCTCTGACCCCATCATCTACCAAGGCATTATGGACAAGGATGA
GGAGAAGCCTGGGAAGTCCTCAGGCCCACCACGCCTGGGTGAGCTGACGGTGACAGACAGGACCTCCGAC
TCCTTGCTCCTGCGCTGGACGGTCCCCGAGGGCGAGTTTGACTCCTTCGTGATCCAGTACAAAGACAGGG
ACGGGCAGCCCCAGGTGGTGCCCGTGGAAGGACCCCAGCGCTCGGCCGTCATCACCTCCCTGGATCCTGG
CCGCAAGTACAAATTTGTCCTGTATGGGTTTGTTGGCAAGAAGAGGCATGGTCCGCTGGTGGCTGAAGCC
AAGATCTTGCCTCAGAGTGACCCAAGTCCAGGGACTCCACCCCACCTGGGAAACCTGTGGGTGACAGACC
CTACCCCAGATTCACTGCACCTCTCCTGGACTGTCCCTGAGGGCCAGTTTGACACCTTCATGGTCCAGTA
CAGGGACAGGGATGGACGGCCCCAGGTGGTACCTGTGGAAGGGCCCGAGCGTTCATTTGTTGTCTCCTCA
CTGGACCCTGACCACAAGTACAGATTCACTCTGTTTGGAATTGCGAACAAGAAGCGGTATGGCCCCCTCA
CGGCCGATGGCACCACTGCTCCAGAGAGGAAAGAGGAGCCCCCCCGCCCTGAGTTCCTGGAGCAGCCCCT
CCTGGGGGAACTGACAGTGACCGGCGTGACCCCAGACTCCTTGCGTCTCTCATGGACAGTGGCCCAGGGC
CCCTTCGACTCATTCATGGTCCAGTACAAGGATGCACAGGGGCAGCCCCAGGCAGTGCCTGTTGCGGGGG
ATGAGAATGAGGTTACTGTCCCCGGCCTGGATCCCGACCGGAAGTATAAGATGAACCTCTACGGGCTTCG
TGGCAGGCAGCGTGTGGGGCCCGAGTCTGTGGTGGCCAAGACTGCTCCTCAGGAGGATGTGGACGAGACC
CCCACCCCCACAGAACTCCCCACCCACCCCCCCCACTCCCCCACCACCCCCTCCTCCCCCACCTCACAG
TGACAGGATCCTCCCCTGATTCGCTGAGCCTCTTCTGGACCGTCCCCCAGGGCAGCTTCGACTCTTTCAC
CGTGCAGTACAAGGACAGGGATGGGCGGCCCCGGGCGGTGCGTGTTGGGGGCAAGGAGAGTGAGGTCACC
GTGGGAGGCCTAGAGCCCGGGCACAAGTACAAGATGCACCTGTACGGCCTCCACGAGGGGCAGCGCGTGG
GCCCGGTGTCCGCCGTGGGCGTGACAGCCCCACAACAAGAAGAGACCCCTCCAGCCACTGAGTCCCCGCT
GGAGCCACGCCTAGGAGAGCTGACAGTGACAGATGTGACCCCCAACTCTGTGGGCCTCTCCTGGACAGTC
CCCGAGGGCCAGTTTGACTCCTTCATAGTCCAGTACAAGGACAAGGACGGGCAGCCCCAGGTGGTGCCGG
TGGCGGCAGACCAGCGAGAGGTCACAGTCTACAACCTGGAGCCTGAGAGAAAATATAAGATGAACATGTA
TGGACTACATGATGGGCAACGCATGGGCCCCCTGTCTGTGGTCATCGTGACGGCTCCCCTCCCACCAGCC
CCAGCCACAGAGGCCTCCAAGCCTCCCCTGGAGCCACGCCTAGGGGAGCTGACAGTGACGGATATAACCC
CTGACTCTGTGGGCCTCTCATGGACAGTCCCTGAGGGTGAATTCGACTCCTTTGTGGTTCAGTACAAGGA
CAGGGACGGGCAGCCCCAGGTGGTGCCCGTGGCTGCAGATCAGCGGGAGGTCACTATCCCTGACCTGGAA
CCCTCCCGCAAGTACAAGTTCCTGCTCTTTGGGATCCAGGATGGGAAACGACGCAGCCCAGTCTCTGTGG
AGGCAAAGACGGTTGCCCGAGGTGACGCCAGCCCAGGGGCCCCACCCCGCCTTGGGGAGCTGTGGGTGAC
AGACCCCACCCCAGACTCACTGCGCCTCTCCTGGACGGTTCCTGAGGGCCAGTTCGACTCTTTTGTGGTC
CAGTTCAAGGACAAAGACGGCCCCCAGGTGGTGCCCGTGGAGGGCCATGAGCGCTCTGTCACTGTCACCC
CTCTGGATGCCGGCCGCAAGTACAGATTCCTCCTCTATGGCCTCCTGGGCAAGAAGCGCCATGGCCCTCT
 
CACTGCCGACGGCACCACGGAAGCCGGAGTGCTATGGATGATACTGGAACAAAGCGTCCCCCAAAACCC
CGTCTGGGGGAGGAGCTGCAGGTGACCACCGTGACCCAGAACTCCGTGGGCCTCTCCTGGACAGTCCCTG
AGGGCCAGTTTGACTCCTTTGTGGTCCAGTACAAAGACAGGGACGGCAGCCCCAGGTGGTGCCCGTGGA
GGGCAGCCTCAGGGAGGTCAGCGTGCCGGGCCTGGACCCTGCCCACAGGTACAAGCTGCTGCTCTACGGG
CTGCACCACGGCAAGCGTGTGGGCCCCATCTCGGCCGTCGCCATTACTGCCGGCAGGGAAGAAACGGAAA
CTGAGACCACGGCCCCGACCCCTCCAGCGCCTGAGCCCACCTCGGGGAGTTGACAGTGGAGGAGGCCAC
GTCACACACCCTGCATCTCTCCTGGATGGTGACTGAGGGAGAATTTGACTCCTTCGAAATCCAGTACACA
GATAGAGACGGGCAACTCCAAATGGTCCGCATAGGAGGTGACCGGAATGACATCACCCTCTCTGGCCTGG
AATCCGACCACAGATACCTGGTGACCCTGTATGGTTTCAGTGATGGGAAGCATGTAGGTCCTGTCCATGT
CGAGGCCCTGACAGTCCCGGAGGAGGAGAAGCCTTCAGAACCTCCCACCGCAACCCCCGAGCCCCCCATC
AAGCCTCGCCTGGGGGAGCTGACCGTGACAGATGCCACCCCTGACTCCCTCAGCCTGTCCTGGACAGTTC
CCGAGGGACAGTTTGACCACTTCCTGGTCCAGTACAGGAATGGAGATGGGCAGCCCAAGGCAGTGAGGGT
GCCAGGGCACGAGGAAGGGGTCACCATCTCGGGCCTGGAGCCAGACCATAAATACAAGATGAACCTGTAC
GGCTTCCACGGTGGCCAGCGCATGGGCCCTGTGTCTGTCGTCGGGGTGACAGCTGCAGAGGAAGAGACCC
CCAGCCCCACAGAACCCAGCATGGAGGCCCCGGAGCCCGCTGAGGAGCCGCTCCTGGGGGAGCTAACAGT
GACAGGATCCTCCCCTGACTCGCTGAGCCTCTCCTGGACCGTCCCCCAGGGCCGCTTCGACTCCTTCACC
GTGCAGTACAAGGACAGGGACGGGCGGCCCCAGGTGGTGCGTGTTGGGGGCGAGGAGAGTGAAGTCACCG
TGGGGGGCCTGGAGCCTGGGCGCAAGTACAAGATGCACCTGTACGGCCTCCACGAGGGGCGGCGCGTGGG
CCCAGTGTCTGCTGTGGGCGTCACGGCCCCGAAGAGGAGTCCCCTGATGCTCCTCTTGCAAAGCTGCGC
CTAGGGCAGATGACAGTGAGAGACATCACCTCCGACTCCCTCAGCCTCTCCTGGACAGTCCCCGAGGGCC
AGTTTGACCATTTCTTGGTCCAGTTTAAGAATGGGGACGGCAGCCCAAGGCGGTGCGGGTGCCGGGACA
CGAGGATGGGGTCACCATCTCGGGCCTGGAGCCAGACCACAAGTACAAGATGAACCTGTACGGCTTCCAC
GGTGGCCAGCGCGTGGGCCCCGTGTCTGCTGTTGGTTTAACTGCCCCAGGAAAGGATGAAGAAATGGCCC
CAGCCTCGACAGAACCTCCCACCCCTGAACCCCCCATCAAGCCTCGCCTGGAGGAGCTGACCGTGACAGA
TGCGACCCCTGACTCCCTCAGCCTGTCCTGGACGGTTCCCGAGGGACAGTTTGACCACTTCCTGGTCCAG
TACAAGAATGGGGATGGGCAGCCCAAGGCAACACGGGTGCCAGGACATGAGGACAGGGTCACCATCTCCG
GCCTGGAGCCAGACAACAAGTACAAGATGAACCTGTACGGCTTCCACGGTGGCCAGCGTGTGGGCCCCGT
GTCTGCCATCGGGGTGACAGCTGCAGAGGAAGAGACCCCCAGCCCCACAGAACCCAGCATGGAGGCCCCG
GAGCCCCCTGAGGAGCCGCTCCTGGGGGAGCTAACAGTGACAGGATCCTCCCCTGACTCGCTGAGCCTCT
CCTGGACCGTCCCCCAGGGCCGCTTCGACTCCTTCACCGTGCAGTACAAGGACAGGGACGGGCGGCCCCA
GGTGGTGCGTGTTGGGGGCGAGGAGAGCGAGGTCACCGTGGGGGGCCTGGAGCCTGGGCGCAAATACAAG
ATGCACCTGTATGGCCTCCACGAGGGGCGGCGCGTGGGCCCGGTGTCCACCGTGGGCGTGACTGCCCCAC
AAGAGGATGTGGACGAGACCCCCAGCCCTACAGAACCAGGCACAGAGGCCCCAGGGCCCCCCGAGGAGCC
TCTCCTGGGGGAGCTGACAGTGACAGGATCCTCCCCTGACTCGCTGAGCCTTTCCTGGACCGTCCCCCAG
GGCCGCTTTGACTCCTTCACCGTGCAGTACAAGGACAGGGACGGGCGGCCCCAGGCGGTGCGTGTTGGGG
GCCAGGAGAGCAAGGTCACTGTGAGGGGCCTGGAGCCTGGGCGCAAGTACAAGATGCACCTGTACGGCCT
CCACGAGGGGCGGCGCCTGGGCCCGGTGTCTGCCGTGGGCGTCACAGAGGATGAAGCCGAGACCACCCAA
GCAGTGCCTACCATGACCCCTGAGCCCCCCATCAAGCCTCGCCTGGGGGAGCTGACCATGACAGATGCCA
CCCCTGACTCCCTCAGCCTGTCCTGGACGGTTCCCGAGGGCCAGTTTGACCACTTCCTGGTCCAGTACAG
GAATGGGGATGGGCAGCCCAAGGCGGTGCGGCTCCCCCCCCACCACCACCCCCTCACCATCTCAGGCCTG
GAGCCAGACCATAAATACAAGATGAACCTGTACGGCTTCCACGGTGGCCAGCGCGTGGGCCCCATCTCTG
TCATTGGGGTGACGGCTGCAGAGGAAGAGACCCCCAGCCCCACGGAACTCAGCACTGAGGCCCCGGAGCC
CCCTGAGGAGCCGCTCCTGGGGGAGCTGACAGTGACAGGATCCTCCCCTGACTCGCTGAGCCTCTCCTGG
ACCATCCCCCAGGGCCACTTCGACTCCTTCACCGTGCAGTACAAGGACAGGGACGGGCGGCCCCAGGTGA
TGCGTGTCAGGGGCGAGGAGAGCGAGGTCACCGTGGGGGGCCTGGAGCCCGGGCGCAAATACAAGATGCA
CCTGTACGGCCTCCACGAGGGGCGGCGTGTGGGCCCGGTGTCCACCGTGGGTGTGACAGAGGATGAAGCA
GAGACCACCCAAGCAGTGCCCACCACAACCCCTGAGCCCCCCAACAAGCCTCGCCTCGGGGAGCTGACCG
TGACAGATGCCACCCCTGACTCCCTCAGCCTGTCCTGGATGGTCCCCGAGGGCCAGTTTGACCACTTCCT
GGTCCAGTACAGGAATGGGGATGGGCAGCCCAAGGTGGTGCGGGTGCCGGGGCACGAGGACGGGGTCACC
ATCTCAGGCCTGGAGCCAGACCACAAGTACAAGATGAACCTGTACGGCTTCCACGGTGGCCAGCGCGTGG
GCCCCATCTCTGTCATTGGGGTGACAGCTGCAGAGGAAGAAACTCCCGCCCCCACAGAACCCAGCACGGA
GGCCCCGGAGCCCCCTGAGGAGCCGCTCCTGGGGGAGCTGACAGTGACAGGATCCTCCCCTGACTCGCTG
AGCCTCTCCTGGACCATCCCCCAGGGCCGCTTCGACTCCTTCACTGTGCAGTACAAGGACAGGGACGGGC
GGCCCCAGGTGGTGCGTGTCAGGGGCGAGGAGAGCGAGGTCACCGTGGGGGGCCTGGAGCCCGGGTGCAA
ATACAAGATGCACCTGTACGGCCTCCACGAGGGGCAGCGCGTGGGCCCAGTGTCCGCTGTGGGTGTGACA
GCTCCAAAGGATGAAGCCGAGACCACCCAAGCAGTGCCTACCATGACCCCTGAGCCCCCCATCAAGCCTC
 
GCCTGGGGGAGCTGACCGTGACAGATGCCACCCCCGACTCCCTCAGCCTGTCCTGGATGGTTCCCGAGGG
CCAGTTTGACCACTTCCTGGTCCAGTACAGGAATGGGGATGGGCAGCCCAAGGCGGTGCGGGTGCCGGGG
CACGAGGACGGGGTCACCATCTCAGGCCTGGAGCCAGACCATAAATACAAGATGAACCTGTACGGCTTCC
ACGGTGGCCAGCGCGTAGGCCCTGTGTCTGCCATTGGGGTGACGGAGGAAGAGACCCCCAGCCCCACAGA
ACCCAGCACTGAGGCCCCGGAGGCCCCTGAGGAGCCGCTCCTGGGGGAGTTGACAGTGACAGGATCCTCC
CCTGACTCGCTGAGCCTCTCCTGGACCGTCCCCCAGGGCCGCTTCGACTCCTTCACCGTGCAGTACAAGG
ACAGGGACGGGCAGCCCCAGGTGGTGCGTGTCAGGGGCGAGGAGAGCGAGGTCACCGTGGGGGGCCTGGA
GCCCGGGCGCAAATACAAGATGCATCTGTACGGCCTCCACGAGGGGCAGCGCGTGGGCCCAGTGTCCACC
GTGGGCATCACGGCGCCCCTGCCCACACCACTGCCGGTGGAGCCCCGCCTGGGGGAGCTGGCGGTGGCGG
CCGTGACCTCGGACTCAGTGGGCCTCTCATGGACGGTGGCCCAGGGCCCCTTTGACTCCTTCCTGGTACA
GTACAGGGACGCGCAGGGGCAGCCCCAGGCAGTGCCTGTGAGCGGAGACCTCCGAGCGGTCGCCGTCTCG
GGGCTGGACCCGGCCCGCAAGTACAAGTTCCTGCTCTTTGGACTCCAGAATGGGAAACGCCACGGCCCAG
TCCCTGTGGAGGCCAGGACCGCCCCAGACACCAAACCGTCTCCCCGCCTGGGGGAGCTGACTGTGACAGA
TGCGACCCCTGACTCCGTGGGCCTCTCGTGGACGGTCCCTGAGGGCGAATTCGACTCCTTCGTGGTCCAG
TACAAGGATAAGGATGGTCGGCTCCAGGTGGTGCCGGTGGCAGCCAACCAGCGGGAGGTCACAGTCCAGG
GCCTGGAGCCCAGTAGGAAATACAGGTTCCTGCTCTATGGTCTGTCAGGCAGGAAACGACTGGGCCCCAT
CTCTGCTGACAGCACCACAGCTCCCCTGGAGAAGGAGCTACCTCCCCACCTGGGGGAACTGACCGTGGCT
GAGGAGACCTCCAGCTCTCTGCGCCTGTCCTGGACGGTAGCCCAGGGCCCCTTTGACTCCTTCGTGGTCC
AGTACAGGGACACGGACGGGCAGCCCAGGGCAGTGCCTGTGGCCGCAGACCAGCGCACAGTCACCGTAGA
GGACCTGGAGCCTGGCAAGAAATACAAGTTTCTGCTCTACGGGCTCCTTGGGGGAAAGCGCCTGGGCCCG
GTCTCTGCCCTGGGAATGACAGCCCCAGAAGAGGACACACCAGCCCCAGAGTTAGCCCCAGAGGCCCCTG
AGCCTCCTGAAGAGCCCCGCCTAGGAGTGCTGACCGTGACCGACACAACCCCAGACTCCATGCGCCTCTC
GTGGAGCGTGGCCCAGGGCCCCTTTGATTCCTTCGTGGTCCAGTATGAGGACACGAACGGGCAGCCCCAG
GCCTTGCTCGTGGACGGCGACCAGAGCAAGATCCTCATCTCAGGCCTGGAGCCCAGCACCCCCTACAGGT
TCCTCCTCTATGGCCTCCATGAAGGGAAGCGCCTGGGGCCCCTCTCAGCTGAGGGCACCACAGGGCTGGC
TCCTGCTGGTCAGACCTCAGAGGAGTCAAGGCCCCGCCTGTCCCAGCTGTCTGTGACTGACGTGACCACC
AGTTCACTGAGGCTCAACTGGGAGGCCCCACCGGGGGCCTTCGACTCCTTCCTGCTCCGCTTTGGGGTTC
CATCACCAAGCACTCTGGAGCCGCATCCGCGTCCACTGCTGCAGCGCGAGCTGATGGTGCCGGGGACGCG
GCACTCGGCCGTGCTCCGGGACCTGCGTTCCGGGACTCTGTACAGCCTGACACTGTATGGGCTGCGAGGA
CCCCACAAGGCCGACAGCATCCAGGGAACCGCCCGCACCCTCAGCCCAGTTCTGGAGAGCCCCCGTGACC
TCCAATTCAGTGAAATCAGGGAGACCTCAGCCAAGGTCAACTGGATGCCCCCACCATCCCGGGCGGACAG
CTTCAAAGTCTCCTACCAGCTGGCGGACGGAGGGGAGCCTCAGAGTGTGCAGGTGGATGGCCAGGCCCGG
ACCCAGAAACTCCAGGGGCTGATCCCAGGCGCTCGCTATGAGGTGACCGTGGTCTCGGTCCGAGGCTTTG
AGGAGAGTGAGCCTCTCACAGGCTTCCTCACCACGGTTCCTGACGGTCCCACACAGTTGCGTGCACTGAA
CTTGACCGAGGGATTCGCCGTGCTGCACTGGAAGCCCCCCCAGAATCCTGTGGACACCTATGACGTCCAG
GTCACAGCCCCTGGGGCCCCGCCTCTGCAGGCGGAGACCCCAGGCAGCGCGGTGGACTACCCCCTGCATG
ACCTTGTCCTCCACACCAACTACACCGCCACAGTGCGTGGCCTGCGGGGCCCCAACCTCACTTCCCCAGC
CAGCATCACCTTCACCACAGGGCTAGAGGCCCCTCGGGACTTGGAGGCCAAGGAAGTGACCCCCCGCACC
GCCCTGCTCACTTGGACTGAGCCCCCAGTCCGGCCCGCAGGCTACCTGCTCAGCTTCCACACCCCTGGTG
GACAGAACCAGGAGATCCTGCTCCCAGGAGGGATCACATCTCACCAGCTCCTTCCCCTCTTTCCCTCCAC
CTCCTACAATGCACGGCTCCAGGCCATGTGGGGCCAGAGCCTCCTGCCGCCCGTGTCCACCTCTTTCACC
ACGGGTGGGCTGCGGATCCCCTTCCCCAGGGACTGCGGGGAGGAGATGCAGAACGGAGCCGGTGCCTCCA
GGACCAGCACCATCTTCCTCAACGGCAACCGCGAGCGGCCCCTGAACGTGTTTTGCGACATGGAGACTGA
TGGGGGCGGCTGGCTGGTGTTCCAGCGCCGCATGGATGGACAGACAGACTTCTGGAGGGACTGGGAGGAC
TATGCCCATGGTTTTGGGAACATCTCTGGAGAGTTCTGGCTGGGCAATGAGGCCCTGCACAGCCTGACAC
AGGCAGGTGACTACTCCATGCGCGTGGACCTGCGGGCTGGGGACGAGGCTGTGTTCGCCCAGTACGACTC
CTTCCACGTAGACTCGGCTGCGGAGTACTACCGCCTCCACTTGGAGGGCTACCACGGCACCGCAGGGGAC
TCCATGAGCTACCACAGCGGCAGTGTCTTCTCTGCCCGTGATCGGGACCCCAACAGCTTGCTCATCTCCT
GCGCTGTCTCCTACCGAGGGGCCTGGTGGTACAGGAACTGCCACTACGCCAACCTCAACGGGCTCTACGG
GAGCACAGTGGACCATCAGGGAGTGAGCTGGTACCACTGGAAGGGCTTCGAGTTCTCGGTGCCCTTCACG
GAAATGAAGCTGAGACCAAGAAACTTTCGCTCCCCAGCGGGGGAGGCTGAGCTGCTGCCCACCTCTCTC
GCACCCCAGTATGACTGCCGAGCACTGAGGGGTCGCCCCGAGAGAAGAGCCAGGGTCCTTCACCACCCAG
CCGCTGGAGGAAGCCTTCTCTGCCAGCGATCTCGCAGCACTGTGTTTACAGGGGGGAGGGGAGGGGTTCG
TACGGGAGCAATAAAGGAGAAACTGAGGTACCCGGAAAAAAAAAAAAAAAAAA
NCBI Reference Sequence: NM_032470.3
   **Homo sapiens tenascin XB (TNXB), transcript variant XB-S, mRNA**
   >gi|885286491|ref|NM-032470.31 Homo sapiens tenascin XB (TNXB), transcript variant XB-S, mRNA 
CCTTGTGCATTTGGGCTGAAGACAAAGATGACTGCAGGAGTGGCAGGCCGGAGTGGGGCGCCCTGGCCT
GTCCCCAGGAAGGAGGAGGAGTCTGCAGCCCTGTGGGCTTCAACATCCATCAAGGAGTCCAGAGCAGGAG
CCAGGCCAGGCGGGAGGGAAAGGCCCTGGAGGGGCTCTCTAATCTCCCAGCCCCGACTCTGCCCCGTCA
CTGCCACTGCTCCTCATTACTCGCTGGGGCTGCTGTCGCCTCCCCGAAGGGTGGCCTTGTCCAGATAGCG
GCAAACCTCCCTGCCGTGGATGAGTCAGGAGCATTTTCTTAAGAGGAACATCACTGGAAAACAAAATGAG
CGGGGACACAGAAACCAACAGCAGTGGCTGCATTTGTGGTACAGGCTCCTCTTCCAGAGCTCGCTGATGC
CCACCTCAGACAGGCCTGACCACGGCACGGCTGGTGGGATTTGCCAGTCACCTCAACCAGCCAGTTCCAC
CCTCAGCTTCTCTCAGAAGGGAGCACCACACTCCTCAAGCTCAGTGAATGTATCCCGGCATGGGTGGGGC
CAGAGCCTGTGATATCTCGAGGTGGGCTCGGCAGGACACCGGGGTGTGGAAGGGGGAAGCGAGCACCTGA
CTCAGACAGCGCGGGAGCTCGCAGGAGTCACGAGGCCACAGCGACTTCATTGTCTGACTGGGCCTGGACC
TATAAACTTCCCACCTCAGCCTTGGGCCAAGCCTGGAAGATAAAAATGGAGCACCCCATGGCGCCCCTCA
CTCAGATTCTCCCCTGGGCTTCTCCCACGCAGCCCCAGAAGAGGACACACCAGCCCCAGAGTTAGCCCCA
GAGGCCCCTGAGCCTCCTGAAGAGCCCCGCCTAGGAGTGCTGACCGTGACCGACACAACCCCAGACTCCA
TGCGCCTCTCGTGGAGCGTGGCCCAGGGCCCCTTTGATTCCTTCGTGGTCCAGTATGAGGACACGAACGG
GCAGCCCCAGGCCTTGCTCGTGGACGGCGACCAGAGCAAGATCCTCATCTCAGGCCTGGAGCCCAGCACC
CCCTACAGGTTCCTCCTCTATGGCCTCCATGAAGGGAAGCGCCTGGGGCCCCTCTCAGCTGAGGGCACCA
CAGGGCTGGCTCCTGCTGGTCAGACCTCAGAGGAGTCAAGGCCCCGCCTGTCCCAGCTGTCTGTGACTGA
CGTGACCACCAGTTCACTGAGGCTCAACTGGGAGGCCCCACCGGGGGCCTTCGACTCCTTCCTGCTCCGC
TTTGGGGTTCCATCACCAAGCACTCTGGAGCCGCATCCGCGTCCACTGCTGCAGCGCGAGCTGATGGTGC
CGGGGACGCGGCACTCGGCCGTGCTCCGGGACCTGCGTTCCGGGACTCTGTACAGCCTGACACTGTATGG
GCTGCGAGGACCCCACAAGGCCGACAGCATCCAGGGAACCGCCCGCACCCTCAGCCCAGTTCTGGAGAGC
CCCCGTGACCTCCAATTCAGTGAAATCAGGGAGACCTCAGCCAAGGTCAACTGGATGCCCCCACCATCCC
GGGCGGACAGCTTCAAAGTCTCCTACCAGCTGGCGGACGGAGGGGAGCCTCAGAGTGTGCAGGTGGATGG
CCAGGCCCGGACCCAGAAACTCCAGGGGCTGATCCCAGGCGCTCGCTATGAGGTGACCGTGGTCTCGGTC
CGAGGCTTTGAGGAGAGTGAGCCTCTCACAGGCTTCCTCACCACGGTTCCTGACGGTCCCACACAGTTGC
GTGCACTGAACTTGACCGAGGGATTCGCCGTGCTGCACTGGAAGCCCCCCCAGAATCCTGTGGACACCTA
TGACGTCCAGGTCACAGCCCCTGGGGCCCCGCCTCTGCAGGCGGAGACCCCAGGCAGCGCGGTGGACTAC
CCCCTGCATGACCTTGTCCTCCACACCAACTACACCGCCACAGTGCGTGGCCTGCGGGGCCCCAACCTCA
CTTCCCCAGCCAGCATCACCTTCACCACAGGGCTAGAGGCCCCTCGGGACTTGGAGGCCAAGGAAGTGAC
CCCCCGCACCGCCCTGCTCACTTGGACTGAGCCCCCAGTCCGGCCCGCAGGCTACCTGCTCAGCTTCCAC
ACCCCTGGTGGACAGAACCAGGAGATCCTGCTCCCAGGAGGGATCACATCTCACCAGCTCCTTGGCCTCT
TTCCCTCCACCTCCTACAATGCACGGCTCCAGGCCATGTGGGGCCAGAGCCTCCTGCCGCCCGTGTCCAC
CTCTTTCACCACGGGTGGGCTGCGGATCCCCTTCCCCAGGGACTGCGGGGAGGAGATGCAGAACGGAGCC
CCTCCCTCCAGGACCAGCACCATCTTCCTCAACGGCAACCGCGAGCGGCCCCTGAACGTGTTTTGCGACA
TGGAGACTGATGGGGGCGGCTGGCTGGTGTTCCAGCGCCGCATGGATGGACAGACAGACTTCTGGAGGGA
CTGGGAGGACTATGCCCATGGTTTTGGGAACATCTCTGGAGAGTTCTGGCTGGGCAATGAGGCCCTGCAC
AGCCTGACACAGGCAGGTGACTACTCCATGCGCGTGGACCTGCGGGCTGGGGACGAGGCTGTGTTCGCCC
AGTACGACTCCTTCCACGTAGACTCGGCTGCGGAGTACTACCGCCTCCACTTGGAGGGCTACCACGGCAC
CGCAGGGGACTCCATGAGCTACCACAGCGGCAGTGTCTTCTCTGCCCGTGATCGGGACCCCAACAGCTTG
CTCATCTCCTGCGCTGTCTCCTACCGAGGGGCCTGGTGGTACAGGAACTGCCACTACGCCAACCTCAACG
GGCTCTACGGGAGCACAGTGGACCATCAGGGAGTGAGCTGGTACCACTGGAAGGGCTTCGAGTTCTCGGT
GCCCTTCACGGAAATGAAGCTGAGACCAAGAAACTTTCGCTCCCCAGCGGGGGGAGGCTGAGCTGCTGCC
CACCTCTCTCGCACCCCAGTATGACTGCCGAGCACTGAGGGGTCGCCCCGAGAGAAGAGCCAGGGTCCTT
CACCACCCAGCCGCTGGAGGAAGCCTTCTCTGCCAGCGATCTCGCAGCACTGTGTTTACAGGGGGGAGGG
GAGGGGTTCGTACGGGAGCAATAAAGGAGAAACTGAGGTACCCGGAAAAAAAAAAAAAAAAAA
NCBI Reference Sequence: NM_212476.1 **Homo sapiens fibronectin 1 (FN1), transcript variant 5, mRNA**
   >gi|47132552|ref|NM_212476.1| Homo sapiens fibronectin 1 (FN1), transcript variant 5, mRNA 
GCCCGCGCCGGCTGTGCTGCACAGGGGGAGGAGAGGGAACCCCAGGCGCGAGCGGGAAGAGGGGACCTGC
AGCCACAACTTCTCTGGTCCTCTGCATCCCTTCTGTCCCTCCACCCGTCCCCTTCCCCACCCTCTGGCCC
CCACCTTCTTGGAGGCGACAACCCCCGGGAGGCATTAGAAGGGATTTTTCCCGCAGGTTGCGAAGGGAAG
CAAACTTGGTGGCAACTTGCCTCCCGGTGCGGGCGTCTCTCCCCCACCGTCTCAACATGCTTAGGGGTCC
GGGGCCCGGGCTGCTGCTGCTGGCCGTCCAGTGCCTGGGGACAGCGGTGCCCTCCACGGAGCCTCGAAG
AGCAAGAGGCAGGCTCAGCAAATGGTTCAGCCCCAGTCCCCGGTGGCTGTCAGTCAAAGCAAGCCCGGTT
GTTATGACAATGGAAAACACTATCAGATAAATCAACAGTGGGAGCGGACCTACCTAGGCAATGCGTTGGT
TTGTACTTGTTATGGAGGAAGCCGAGGTTTTAACTGCGAGAGTAAACCTGAAGCTGAAGAGACTTGCTTT
GACAAGTACACTGGGAACACTTACCGAGTGGGTGACACTTATGAGCGTCCTAAAGACTCCATGATCTGGG
ACTGTACCTGCATCGGGGCTGGGCGAGGGAGAATAAGCTGTACCATCGCAAACCGCTGCCATGAAGGGGG
TCAGTCCTACAAGATTGGTGACACCTGGAGGAGACCACATGAGACTGGTGGTTACATGTTAGAGTGTGTG
TGTCTTGGTAATGGAAAAGGAGAATGGACCTGCAAGCCCATAGCTGAGAAGTGTTTTGATCATGCTGCTG
GGACTTCCTATGTGGTCGGAGAAACGTGGGAGAAGCCCTACCAAGGCTGGATGATGGTAGATTGTACTTG
CCTGGGAGAAGGCAGCGGACGCATCACTTGCACTTCTAGAAATAGATGCAACGATCAGGACACAAGGACA
TCCTATAGAATTGGAGACACCTGGAGCAAGAAGGATAATCGAGGAAACCTGCTCCAGTGCATCTGCACAG
GCAACGGCCGAGGAGAGTGGAAGTGTGAGAGGCACACCTCTGTGCAGACCACATCGAGCGGATCTGGCCC
CTTCACCGATGTTCGTGCAGCTGTTTACCAACCGCAGCCTCACCCCCAGCCTCCTCCCTATGGCCACTGT
GTCACAGACAGTGGTGTGGTCTACTCTGTGGGGATGCAGTGGCTGAAGACACAAGGAAATAAGCAAATGC
TTTGCACGTGCCTGGGCAACGGAGTCAGCTGCCAAGAGACAGCTGTAACCCAGACTTACGGTGGCAACTC
AAATGGAGAGCCATGTGTCTTACCATTCACCTACAATGGCAGGACGTTCTACTCCTGCACCACAGAAGGG
CGACAGGACGGACATCTTTGGTGCAGCACAACTTCGAATTATGAGCAGGACCAGAAATACTCTTTCTGCA
CAGACCACACTGTTTTGGTTCAGACTCGAGGAGGAAATTCCAATGGTGCCTTGTGCCACTTCCCCTTCCT
ATACAACAACCACAATTACACTGATTGCACTTCTGAGGGCAGAAGAGACAACATGAAGTGGTGTGGGACC
ACACAGAACTATGATGCCGACCAGAAGTTTGGGTTCTGCCCCATGGCTGCCCACGAGGAAATCTGCACAA
CCAATGAAGGGGTCATGTACCGCATTGGAGATCAGTGGGATAAGCAGCATGACATGGGTCACATGATGAG
GTGCACGTGTGTTGGGAATGGTCGTGGGGAATGGACATGCATTGCCTACTCGCAGCTTCGAGATCAGTGC
ATTGTTGATGACATCACTTACAATGTGAACGACACATTCCACAAGCGTCATGAAGAGGGGCACATGCTGA
ACTGTACATGCTTCGGTCAGGGTCGGGGCAGGTGGAAGTGTGATCCCGTCGACCAATGCCAGGATTCAGA
GACTGGGACGTTTTATCAAATTGGAGATTCATGGGAGAAGTATGTGCATGGTGTCAGATACCAGTGCTAC
TGCTATGGCCGTGGCATTGGGGAGTGGCATTGCCAACCTTTACAGACCTATCCAAGCTCAAGTGGTCCTG
TCGAAGTATTTATCACTGAGACTCCGAGTCAGCCCAACTCCCACCCCATCCAGTGGAATGCACCACAGCC
ATCTCACATTTCCAAGTACATTCTCAGGTGGAGACCTAAAAATTCTGTAGGCCGTTGGAAGGAAGCTACC
ATACCAGGCCACTTAAACTCCTACACCATCAAAGGCCTGAAGCCTGGTGTGGTATACGAGGGCCAGCTCA
TCAGCATCCAGCAGTACGGCCACCAAGAAGTGACTCGCTTTGACTTCACCACCACCAGCACCAGCACACC
TGTGACCAGCAACACCGTGACAGGAGAGACGACTCCCTTTTCTCCTCTTGTGGCCACTTCTGAATCTGTG
ACCGAAATCACAGCCAGTAGCTTTGTGGTCTCCTGGGTCTCAGCTTCCGACACCGTGTCGGGATTCCGGG
TGGAATATGAGCTGAGTGAGGAGGGAGATGAGCCACAGTACCTGGATCTTCCAAGCACAGCCACTTCTGT
GAACATCCCTGACCTGCTTCCTGGCCGAAAATACATTGTAAATGTCTATCAGATATCTGAGGATGGGGAG
CAGAGTTTGATCCTGTCTACTTCACAAACAACAGCGCCTGATGCCCCTCCTGACCCGACTGTGGACCAAG
TTGATGACACCTCAATTGTTGTTCGCTGGAGCAGACCCCAGGCTCCCATCACAGGGTACAGAATAGTCTA
TTCGCCATCAGTAGAAGGTAGCAGCACAGAACTCAACCTTCCTGAAACTGCAAACTCCGTCACCCTCAGT
GACTTGCAACCTGGTGTTCAGTATAACATCACTATCTATGCTGTGGAAGAAAATCAAGAAAGTACACCTG
TTGTCATTCAACAAGAAACCACTGGCACCCCACGCTCAGATACAGTGCCCTCTCCCAGGGACCTGCAGTT
TGTGGAAGTGACAGACGTGAAGGTCACCATCATGTGGACACCGCCTGAGAGTGCAGTGACCGGCTACCGT
GTGGATGTGATCCCCGTCAACCTGCCTGGCGAGCACGGGCAGAGGCTGCCCATCAGCAGGAACACCTTTG
CAGAAGTCACCGGGCTGTCCCCTGGGGTCACCTATTACTTCAAAGTCTTTGCAGTGAGCCATGGGAGGGA
GAGCAAGCCTCTGACTGCTCAACAGACAACCAAACTGGATGCTCCCACTAACCTCCAGTTTGTCAATGAA
ACTGATTCTACTGTCCTGGTGAGATGGACTCCACCTCGGGCCCAGATAACAGGATACCGACTGACCGTGG
GCCTTACCCGAAGAGGACAGCCCAGGCAGTACAATGTGGGTCCCTCTGTCTCCAAGTACCCACTGAGGAA
TCTGCAGCCTGCATCTGAGTACACCGTATCCCTCGTGGCCATAAAGGGCAACCAAGAGAGCCCCAAAGCC
ACTGGAGTCTTTACCACACTGCAGCCTGGGAGCTCTATTCCACCTTACAACACCGAGGTGACTGAGACCA
CCATTGTGATCACATGGACGCCTGCTCCAAGAATTGGTTTTAAGCTGGGTGTACGACCAAGCCAGGGAGG
AGAGGCACCACGAGAAGTGACTTCAGACTCAGGAAGCATCGTTGTGTCCGGCTTGACTCCAGGAGTAGAA
 
TACGTCTACACCATCCAAGTCCTGAGAGATGGACAGGAAAGATGCGCCAATTGTAAACAAAGTGGTGA
CACCATTGTCTCCACCAACAAACTTGCATCTGGAGGCAAACCCTGACACTGGAGTGCTCACAGTCTCCTG
GGAGAGGAGCACCACCCCAGACATTACTGGTTATAGAATTACCACAACCCCTACAAACGGCCAGCAGGGA
AATTCTTTGGAAGAAGTGGTCCATGCTGATCAGAGCTCCTGCACTTTTGATAACCTGAGTCCCGGCCTGG
AGTACAATGTCAGTGTTTACACTGTCAAGGATGACAAGGAAAGTGTCCCTATCTCTGATACCATCATCCC
AGCTGTTCCTCCTCCCACTGACCTGCGATTCACCAACATTGGTCCAGACACCATGCGTGTCACCTGGGCT
CCACCCCCATCCATTGATTTAACCAACTTCCTGGTGCGTTACTCACCTGTGAAAAATGAGGAAGATGTTG
CAGAGTTGTCAATTTCTCCTTCAGACAATGCAGTGGTCTTAACAAATCTCCTGCCTGGTACAGAATATGT
AGTGAGTGTCTCCAGTGTCTACGAACAACATGAGAGCACACCTCTTAGAGGAAGACAGAAAACAGGTCTT
GATTCCCCAACTGGCATTGACTTTTCTGATATTACTGCCAACTCTTTTACTGTGCACTGGATTGCTCCTC
GAGCCACCATCACTGGCTACAGGATCCGCCATCATCCCGAGCACTTCAGTGGGAGACCTCGAGAAGATCG
GGTGCCCCACTC[?]TCGGAATTCCATCACCCTCA[?]CCAACCTCACTCCAGGCAC[?]GAGTATGTGGTCAGCATC
GTTGCTCTTAATGGCAGAGAGGAAAGTCCCTTATTGATTGGCCAACAATCAACAGTTTCTGATGTTCCGA
GGGACCTGGAAGTTGTTGCTGCGACCCCCACCAGCCTACTGATCAGCTGGGATGCTCCTGCTGTCACAGT
GAGATATTACAGGATCACTTACGGAGAGACAGGAGGAAATAGCCCTGTCCAGGAGTTCACTGTGCCTGGG
AGCAAGTCTACAGCTACCATCAGCGGCCTTAAACCTGGAGTTGATTATACCATCACTGTGTATGCTGTCA
CTGGCCGTGGAGACAGCCCCGCAAGCAGCAAGCCAATTTCCATTAATTACCGAACAGAAATTGACAAACC
ATCCCAGATGCAAGTGACCGATGTTCAGGACAACAGCATTAGTGTCAAGTGGCTGCCTTCAAGTTCCCCT
GTTACTGGTTACAGAGTAACCACCACTCCCAAAAATGGACCAGGACCAACAAAAACTAAAACTGCAGGTC
CAGATCAAACAGAAATGACTATTGAAGGCTTGCAGCCCACAGTGGAGTATGTGGTTAGTGTCTATGCTCA
GAATCCAAGCGGAGAGAGTCAGCCTCTGGTTCAGACTGCAGTAACCACTATTCCTGCACCAACTGACCTG
AAGTTCACTCAGGTCACACCCACAAGCCTGAGCGCCCAGTGGACACCACCCAATGTTCAGCTCACTGGAT
ATCGAGTGCGGGTGACCCCCAAGGAGAAGACCGGACCAATGAAAGAAATCAACCTTGCTCCTGACAGCTC
ATCCGTGGTTGTATCAGGACTTATGGTGGCCACCAAATATGAAGTGAGTGTCTATGCTCTTAAGGACACT
TTGACAAGCAGACCAGCTCAGGGAGTTGTCACCACTCTGGAGAATGTCAGCCCACCAAGAAGGGCTCGTG
TGACAGATGCTACTGAGACCACCATCACCATTAGCTGGAGAACCAAGACTGAGACGATCACTGGCTTCCA
AGTTGATGCCGTTCCAGCCAATGGCCAGACTCCAATCCAGAGAACCATCAAGCCAGATGTCAGAAGCTAC
ACCATCACAGGTTTACAACCAGGCACTGACTACAAGATCTACCTGTACACCTTGAATGACAATGCTCGGA
GCTCCCCTGTGGTCATCGACGCCTCCACTGCCATTGATGCACCATCCAACCTGCGTTTCCTGGCCACCAC
ACCCAATTCCTTGCTGGTATCATGGCAGCCGCCACGTGCCAGGATTACCGGCTACATCATCAAGTATGAG
AAGCCTGGGTCTCCTCCCAGAGAAGTGGTCCCTCGGCCCCGCCCTGGTGTCACAGAGGCTACTATTACTG
GCCTGGAACCGGGAACCGAATATACAATTTATGTCATTGCCCTGAAGAATAATCAGAAGAGCGAGCCCCT
GATTGGAAGGAAAAAGACAGACGAGCTTCCCCAACTGGTAACCCTTCCACACCCCAATCTTCATGGACCA
GAGATCTTGGATGTTCCTTCCACAGTTCAAAAGACCCCTTTCGTCACCCACCCTGGGTATGACACTGGAA
ATGGTATTCAGCTTCCTGGCACTTCTGGTCAGCAACCCAGTGTTGGGCAACAAATGATCTTTGAGGAACA
TGGTTTTAGGCGGACCACACCGCCCACAACGGCCACCCCCATAAGGCATAGGCCAAGACCATACCCGCCG
AATGTAGGTGAGGAAATCCAAATTGGTCACATCCCCAGGGAAGATGTAGACTATCACCTGTACCCACACG
GTCCGGGACTCAATCCAAATGCCTCTACAGGACAAGAAGCTCTCTCTCAGACAACCATCTCATGGGCCCC
ATTCCAGGACACTTCTGAGTACATCATTTCATGTCATCCTGTTGGCACTGATGAAGAACCCTTACAGTTC
AGGCTTCCTCCAACTTCTACCACTCCCACTCTCACACCCCTCACCAGAGGTGCCACCTACAACATCATAG
TGGAGGCACTGAAAGACCAGCAGAGGCATAAGGTTCGGGAAGAGGTTGTTACCGTGGGCAACTCTGTCAA
CGAAGGCTTGAACCAACCTACGGATGACTCGTGCTTTGACCCCTACACAGTTTCCCATTATGCCGTTGGA
GATGAGTGGGAACGAATGTCTGAATCAGGCTTTAAACTGTTGTGCCAGTGCTTAGGCTTTGGAAGTGGTC
ATTTCAGATGTGATTCATCTAGATGGTGCCATGACAATGGTGTGAACTACAAGATTGGAGAGAAGTGGGA
CCGTCAGGGAGAAAATGGCCAGATGATGAGCTGCACATGTCTTGGGAACGGAAAAGGAGAATTCAAGTGT
GACCCTCATGAGGCAACGTGTTATGATGATGGGAAGACATACCACGTAGGAGAACAGTGGCAGAAGGAAT
ATCTCGGTGCCATTTGCTCCTGCACATGCTTTGGAGGCCAGCGGGGCTGGCGCTGTGACAACTGCCGCAG
ACCTGGGGGTGAACCCAGTCCCGAAGGCACTACTGGCCAGTCCTACAACCAGTATTCTCAGAGATACCAT
CAGAGAACAAACACTAATGTTAATTGCCCAATTGAGTGCTTCATGCCTTTAGATGTACAGGCTGACAGAG
AAGATTCCCGAGAGTAAATCATCTTTCCAATCCAGAGGAACAAGCATGTCTCTCTGCCAAGATCCATCTA
AACTGGAGTGATGTTAGCAGACCCAGCTTAGAGTTCTTCTTTCTTTCTTAAGCCCTTTGCTCTGGAGGAA
GTTCTCCAGCTTCAGCTCAACTCACAGCTTCTCCAAGCATCACCCTGGGAGTTTCCTGAGGGTTTTCTCA
TAAATGAGGGCTGCACATTGCCTGTTCTGCTTCGAAGTATTCAATACCGCTCAGTATTTTAAATGAAGTG
ATTCTAAGATTTGGTTTGGGATCAATAGGAAAGCATATGCAGCCAACCAAGATGCAAATGTTTTGAAATG
ATATGACCAAAATTTTAAGTAGGAAAGTCACCCAAACACTTCTGCTTTCACTTAAGTGTCTGGCCCGCAA
TACTGTAGGAACAAGCATGATCTTGTTACTGTGATATTTTAAATATCCACAGTACTCACTTTTTCCAAAT
 
GATCCTAGTAATTGCCTAGAAATATCTTTCTCTTACCTGTTATTTATCAATTTTTCCCAGTATTTTTATA
CGGAAAAAATTGTATTGAAAACACTTAGTATGCAGTTGATAAGAGGAATTTGGTATAATTATGGTGGGTG
ATTATTTTTTATACTGTATGTGCCAAAGCTTTACTACTGTGGAAAGACAACTGTTTTAATAAAAGATTTA
CATTCCACAACTTGAAGTTCATCTATTTGATATAAGACACCTTCGGGGGAAATAATTCCTGTGAATATTC
TTTTTCAATTCAGCAAACATTTGAAAATCTATGATGTGCAAGTCTAATTGTTGATTTCAGTACAAGATTT
TCTAAATCAGTTGCTACAAAAACTGATTGGTTTTTGTCACTTCATCTCTTCACTAATGGAGATAGCTTTA
CACTTTCTGCTTTAATAGATTTAAGTGGACCCCAATATTTATTAAAATTGCTAGTTTACCGTTCAGAAGT
ATAATAGAAATAATCTTTAGTTGCTCTTTTCTAACCATTGTAATTCTTCCCTTCTTCCCTCCACCTTTCC
TTCATTGAATAAACCTCTGTTCAAAGAGATTGCCTGCAAGGGAAATAAAAATGACTAAGATATTAAAAAA
AAAAAAAAAAAA
NCBI Reference Sequence⁹: NM_212474.1
   **Homo sapiens fibronectin 1 (FN1), transcript variant 6, mRNA**
   >gi|47132548|ref|NM_212474.1| Homo sapiens fibronectin 1 (FN1), transcript variant 6, mRNA 
GCCCGCGCCGGCTGTGCTGCACAGGGGGAGGAGAGGGAACCCCAGGCGCGAGCGGGAAGAGGGGACCTGC
AGCCACAACTTCTCTGGTCCTCTGCATCCCTTCTGTCCTCCACCCGTCCCCTTCCCCACCCTCTGGCCC
CCACCTTCTTGGAGGCGACAACCCCCGGGAGGCATTAGAAGGATTTTTCCCGCAGGTTGCGAAGGGAAG
CAAACTTGGTGGCAACTTGCCTCCCGGTGCGGGCGTCTCTCCCCCACCGTCTCAACATGCTTAGGGGTCC
GGGGCCCGGGCTGCTGCTGCTGGCCGTCCAGTGCCTGGGGACAGCGGTGCCCTCCACGGAGCCTCGAAG
AGCAAGAGGCAGGCTCAGCAAATGGTTCAGCCCCAGTCCCCGGTGGCTGTCAGTCAAAGCAAGCCCGGTT
GTTATGACAATGGAAAACACTATCAGATAAATCAACAGTGGGAGCGGACCTACCTAGGCAATGCGTTGGT
TTGTACTTGTTATGGAGGAAGCCGAGGTTTTAACTGCGAGAGTAAACCTGAAGCTGAAGAGACTTGCTTT
GACAAGTACACTGGGAACACTTACCGAGTGGGTGACACTTATGAGCGTCCTAAAGACTCCATGATCTGGG
ACTGTACCTGCATCGGGGCTGGGCGAGGGAGAATAAGCTGTACCATCGCAAACCGCTGCCATGAAGGGGG
TCAGTCCTACAAGATTGGTGACACCTGGAGGAGACCACATGAGACTGGTGGTTACATGTTAGAGTGTGTG
TGTCTTGGTAATGGAAAAGGAGAATGGACCTGCAAGCCCATAGCTGAGAAGTGTTTTGATCATGCTGCTG
GGACTTCCTATGTGGTCGGAGAAACGTGGGAGAAGCCCTACCAAGGCTGGATGATGGTAGATTGTACTTG
CCTGGGAGAAGGCAGCGGACGCATCACTTGCACTTCTAGAAATAGATGCAACGATCAGGACACAAGGACA
TCCTATAGAATTGGAGACACCTGGAGCAAGAAGGATAATCGAGGAAACCTGCTCCAGTGCATCTGCACAG
GCAACGGCCGAGGAGAGTGGAAGTGTGAGAGGCACACCTCTGTGCAGACCACATCGAGCGGATCTGGCCC
CTTCACCGATGTTCGTGCAGCTGTTTACCAACCGCAGCCTCACCCCCAGCCTCCTCCCTATGGCCACTGT
GTCACAGACAGTGGTGTGGTCTACTCTGTGGGGATGCAGTGGCTGAAGACACAAGGAAATAAGCAAATGC
TTTGCACGTGCCTGGGCAACGGAGTCAGCTGCCAAGAGACAGCTGTAACCCAGACTTACGGTGGCAACTC
AAATGGAGAGCCATGTGTCTTACCATTCACCTACAATGGCAGGACGTTCTACTCCTGCACCACAGAAGGG
CGACAGGACGGACATCTTTGGTGCAGCACAACTTCGAATTATGAGCAGGACCAGAAATACTCTTTCTGCA
CAGACCACACTGTTTTGGTTCAGACTCGAGGAGGAAATTCCAATGGTGCCTTGTGCCACTTCCCCTTCCT
ATACAACAACCACAATTACACTGATTGCACTTCTGAGGGCAGAAGAGACAACATGAAGTGGTGTGGGACC
ACACAGAACTATGATGCCGACCAGAAGTTTGGGTTCTGCCCCATGGCTGCCCACGAGGAAATCTGCACAA
CCAATGAAGGGGTCATGTACCGCATTGGAGATCAGTGGGATAAGCAGCATGACATGGGTCACATGATGAG
GTGCACGTGTGTTGGGAATGGTCGTGGGGAATGGACATGCATTGCCTACTCGCAGCTTCGAGATCAGTGC
ATTGTTGATGACATCACTTACAATGTGAACGACACATTCCACAAGCGTCATGAAGAGGGGCACATGCTGA
ACTGTACATGCTTCGGTCAGGGTCGGGGCAGGTGGAAGTGTGATCCCGTCGACCAATGCCAGGATTCAGA
GACTGGGACGTTTTATCAAATTGGAGATTCATGGGAGAAGTATGTGCATGGTGTCAGATACCAGTGCTAC
TGCTATGGCCGTGGCATTGGGGAGTGGCATTGCCAACCTTTACAGACCTATCCAAGCTCAAGTGGTCCTG
TCGAAGTATTTATCACTGAGACTCCGAGTCAGCCCAACTCCCACCCCATCCAGTGGAATGCACCACAGCC
ATCTCACATTTCCAAGTACATTCTCAGGTGGAGACCTAAAAATTCTGTAGGCCGTTGGAAGGAAGCTACC
ATACCAGGCCACTTAAACTCCTACACCATCAAAGGCCTGAAGCCTGGTGTGGTATACGAGGGCCAGCTCA
TCAGCATCCAGCAGTACGGCCACCAAGAAGTGACTCGCTTTGACTTCACCACCACCAGCACCAGCACACC
TGTGACCAGCAACACCGTGACAGGAGAGACGACTCCCTTTTCTCCTCTTGTGGCCACTTCTGAATCTGTG
ACCGAAATCACAGCCAGTAGCTTTGTGGTCTCCTGGGTCTCAGCTTCCGACACCGTGTCGGGATTCCGGG
TGGAATATGAGCTGAGTGAGGAGGGAGATGAGCCACAGTACCTGGATCTTCCAAGCACAGCCACTTCTGT
GAACATCCCTGACCTGCTTCCTGGCCGAAAATACATTGTAAATGTCTATCAGATATCTGAGGATGGGGAG
 
CAGAGTTTGATCCTGTCTACTTCACAAACAACAGCGCCTGATGCCCCTCCTGACCCGACTGTGGACCAAG
TTGATGACACCTCAATTGTTGTTCGCTGGAGCAGACCCCAGGCTCCCATCACAGGGTACAGAATAGTCTA
TTCGCCATCAGTAGAAGGTAGCAGCACAGAACTCAACCTTCCTGAAACTGCAAACTCCGTCACCCTCAGT
GACTTGCAACCTGGTGTTCAGTATAACATCACTATCTATGCTGTGGAAGAAAATCAAGAAAGTACACCTG
TTGTCATTCAACAAGAAACCACTGGCACCCCACGCTCAGATACAGTGCCCTCTCCCAGGGACCTGCAGTT
TGTGGAAGTGACAGACGTGAAGGTCACCATCATGTGGACACCGCCTGAGAGTGCAGTGACCGGCTACCGT
GTGGATGTGATCCCCGTCAACCTGCCTGGCGAGCACGGGCAGAGGCTGCCCATCAGCAGGAACACCTTTG
CAGAAGTCACCGGGCTGTCCCCTGGGGTCACCTATTACTTCAAAGTCTTTGCAGTGAGCCATGGGAGGGA
GAGCAAGCCTCTGACTGCTCAACAGACAACCAAACTGGATGCTCCCACTAACCTCCAGTTTGTCAATGAA
ACTGATTCTACTGTCCTGGTGAGATGGACTCCACCTCGGGCCCAGATAACAGGATACCGACTGACCGTGG
GCCTTACCCGAAGAGGACAGCCCAGGCAGTACAATGTGGGTCCCTCTGTCTCCAAGTACCCACTGAGGAA
TCTGCAGCCTGCATCTGAGTACACCGTATCCCTCGTGGCCATAAAGGGCAACCAAGAGAGCCCCAAAGCC
ACTGGAGTCTTTACCACACTGCAGCCTGGGAGCTCTATTCCACCTTACAACACCGAGGTGACTGAGACCA
CCATTGTGATCACATGGACGCCTGCTCCAAGAATTGGTTTTAAGCTGGGTGTACGACCAAGCCAGGGAGG
AGAGGCACCACGAGAAGTGACTTCAGACTCAGGAAGCATCGTTGTGTCCGGCTTGACTCCAGGAGTAGAA
TACGTCTACACCATCCAAGTCCTGAGAGATGGACAGGAAAGAGATGCGCCAATTGTAAACAAAGTGGTGA
CACCATTGTCTCCACCAACAAACTTGCATCTGGAGGCAAACCCTGACACTGGAGTGCTCACAGTCTCCTG
GGAGAGGAGCACCACCCCAGACATTACTGGTTATAGAATTACCACAACCCCTACAAACGGCCAGCAGGGA
AATTCTTTGGAAGAAGTGGTCCATGCTGATCAGAGCTCCTGCACTTTTGATAACCTGAGTCCCGGCCTGG
AGTACAATGTCAGTGTTTACACTGTCAAGGATGACAAGGAAAGTGTCCCTATCTCTGATACCATCATCCC
AGCTGTTCCTCCTCCCACTGACCTGCGATTCACCAACATTGGTCCAGACACCATGCGTGTCACCTGGGCT
CCACCCCCATCCATTGATTTAACCAACTTCCTGGTGCGTTACTCACCTGTGAAAAATGAGGAAGATGTTG
CAGAGTTGTCAATTTCTCCTTCAGACAATGCAGTGGTCTTAACAAATCTCCTGCCTGGTACAGAATATGT
AGTGAGTGTCTCCAGTGTCTACGAACAACATGAGAGCACACCTCTTAGAGGAAGACAGAAAACAGGTCTT
GATTCCCCAACTGGCATTGACTTTTCTGATATTACTGCCAACTCTTTTACTGTGCACTGGATTGCTCCTC
GAGCCACCATCACTGGCTACAGGATCCGCCATCATCCCGAGCACTTCAGTGGGAGACCTCGAGAAGATCG
GGTGCCCCACTCTCGGAATTCCATCACCCTCACCAACCTCACTCCAGGCACAGAGTATGTGGTCAGCATC
GTTGCTCTTAATGGCAGAGAGGAAAGTCCCTTATTGATTGGCCAACAATCAACAGTTTCTGATGTTCCGA
GGGACCTGGAAGTTGTTGCTGCGACCCCCACCAGCCTACTGATCAGCTGGGATGCTCCTGCTGTCACAGT
GAGATATTACAGGATCACTTACGGAGAGACAGGAGGAAATAGCCCTGTCCAGGAGTTCACTGTGCCTGGG
AGCAAGTCTACAGCTACCATCAGCGGCCTTAAACCTGGAGTTGATTATACCATCACTGTGTATGCTGTCA
CTGGCCGTGGAGACAGCCCCGCAAGCAGCAAGCCAATTTCCATTAATTACCGAACAGAAATTGACAAACC
ATCCCAGATGCAAGTGACCGATGTTCAGGACAACAGCATTAGTGTCAAGTGGCTGCCTTCAAGTTCCCCT
GTTACTGGTTACAGAGTAACCACCACTCCCAAAAATGGACCAGGACCAACAAAAACTAAAACTGCAGGTC
CAGATCAAACAGAAATGACTATTGAAGGCTTGCAGCCCACAGTGGAGTATGTGGTTAGTGTCTATGCTCA
GAATCCAAGCGGAGAGAGTCAGCCTCTGGTTCAGACTGCAGTAACCACTATTCCTGCACCAACTGACCTG
AAGTTCACTCAGGTCACACCCACAAGCCTGAGCGCCCAGTGGACACCACCCAATGTTCAGCTCACTGGAT
ATCGAGTGCGGGTGACCCCCAAGGAGAAGACCGGACCAATGAAAGAAATCAACCTTGCTCCTGACAGCTC
ATCCGTGGTTGTATCAGGACTTATGGTGGCCACCAAATATGAAGTGAGTGTCTATGCTCTTAAGGACACT
TTGACAAGCAGACCAGCTCAGGGAGTTGTCACCACTCTGGAGAATGTCAGCCCACCAAGAAGGGCTCGTG
TGACAGATGCTACTGAGACCACCATCACCATTAGCTGGAGAACCAAGACTGAGACGATCACTGGCTTCCA
AGTTGATGCCGTTCCAGCCAATGGCCAGACTCCAATCCAGAGAACCATCAAGCCAGATGTCAGAAGCTAC
ACCATCACAGGTTTACAACCAGGCACTGACTACAAGATCTACCTGTACACCTTGAATGACAATGCTCGGA
GCTCCCCTGTGGTCATCGACGCCTCCACTGCCATTGATGCACCATCCAACCTGCGTTTCCTGGCCACCAC
ACCCAATTCCTTGCTGGTATCATGGCAGCCGCCACGTGCCAGGATTACCGGCTACATCATCAAGTATGAG
AAGCCTGGGTCTCCTCCCAGAGAAGTGGTCCCTCGGCCCCGCCCTGGTGTCACAGAGGCTACTATTACTG
GCCTGGAACCGGGAACCGAATATACAATTTATGTCATTGCCCTGAAGAATAATCAGAAGAGCGAGCCCCT
GATTGGAAGGAAAAAGACAGGACAAGAAGCTCTCTCTCAGACAACCATCTCATGGGCCCCATTCCAGGAC
ACTTCTGAGTACATCATTTCATGTCATCCTGTTGGCACTGATGAAGAACCCTTACAGTTCAGGGTTCCTG
GAACTTCTACCAGTGCCACTCTGACAGGCCTCACCAGAGGTGCCACCTACAACATCATAGTGGAGGCACT
GAAAGACCAGCAGAGGCATAAGGTTCGGGAAGAGGTTGTTACCGTGGGCAACTCTGTCAACGAAGGCTTG
AACCAACCTACGGATGACTCGTGCTTTGACCCCTACACAGTTTCCCATTATGCCGTTGGAGATGAGTGGG
AACGAATGTCTGAATCAGGCTTTAAACTGTTGTGCCAGTGCTTAGGCTTTGGAAGTGGTCATTTCAGATG
TGATTCATCTAGATGGTGCCATGACAATGGTGTGAACTACAAGATTGGAGAGAAGTGGGACCGTCAGGGA
GAAAATGGCCAGATGATGAGCTGCACATGTCTTGGGAACGGAAAAGGAGAATTCAAGTGTGACCCTCATG
AGGCAACGTGTTATGATGATGGGAAGACATACCACGTAGGAGAACAGTGGCAGAAGGAATATCTCGGTGC
 
CATTTGCTCCTGCACATGCTTTGGAGGCCAGCGGGGCTGGCGCTGTGACAACTGCCGCAGACCTGGGGGT
GAACCCAGTCCCGAAGGCACTACTGGCCAGTCCTACAACCAGTATTCTCAGAGATACCATCAGAGAACAA
ACACTAATGTTAATTGCCCAATTGAGTGCTTCATGCCTTTAGATGTACAGGCTGACAGAGAAGATTCCCG
AGAGTAAATCATCTTTCCAATCCAGAGGAACAAGCATGTCTCTCTGCCAAGATCCATCTAAACTGGAGTG
ATGTTAGCAGACCCAGCTTAGAGTTCTTCTTTCTTTCTTAAGCCCTTTGCTCTGGAGGAAGTTCTCCAGC
TTCAGCTCAACTCACAGCTTCTCCAAGCATCACCCTGGGAGTTTCCTGAGGGTTTTCTCATAAATGAGGG
CTGCACATTGCCTGTTCTGCTTCGAAGTATTCAATACCGCTCAGTATTTTAAATGAAGTGATTCTAAGAT
TTGGTTTGGGATCAATAGGAAAGCATATGCAGCCAACCAAGATGCAAATGTTTTGAAATGATATGACCAA
AATTTTAAGTAGGAAAGTCACCCAAACACTTCTGCTTTCACTTAAGTGTCTGGCCCGCAATACTGTAGGA
ACAAGCATGATCTTGTTACTGTGATATTTTAAATATCCACAGTACTCACTTTTTCCAAATGATCCTAGTA
ATTGCCTAGAAATATCTTTCTCTTACCTGTTATTTATCAATTTTTCCCAGTATTTTTATACGGAAAAAAT
TGTATTGAAAACACTTAGTATGCAGTTGATAAGAGGAATTTGGTATAATTATGGTGGGTGATTATTTTTT
ATACTGTATGTGCCAAAGCTTTACTACTGTGGAAAGACAACTGTTTTAATAAAAGATTTACATTCCACAA
CTTGAAGTTCATCTATTTGATATAAGACACCTTCGGGGGAAATAATTCCTGTGAATATTCTTTTTCAATT
CAGCAAACATTTGAAAATCTATGATGTGCAAGTCTAATTGTTGATTTCAGTACAAGATTTTCTAAATCAG
TTGCTACAAAAACTGATTGGTTTTTGTCACTTCATCTCTTCACTAATGGAGATAGCTTTACACTTTCTGC
TTTAATAGATTTAAGTGGACCCCAATATTTATTAAAATTGCTAGTTTACCGTTCAGAAGTATAATAGAAA
TAATCTTTAGTTGCTCTTTTCTAACCATTGTAATTCTTCCCTTCTTCCCTCCACCTTTCCTTCATTGAAT
AAACCTCTGTTCAAAGAGATTGCCTGCAAGGGAAATAAAAATGACTAAGATATTAAAAAAAAAAAAAAAA
AA
NCBI Reference Sequence: NM_002026.2
   **Homo sapiens fibronectin 1 (FN1), transcript variant 3, mRNA**
   >gi|47132558|ref|NM_002026.2| Homo sapiens fibronectin 1 (FN1), transcript variant 3, mRNA 
GCCCGCGCCGGCTGTGCTGCACAGGGGAGGAGAGGGAACCCCAGGCGCGAGCGGGAAGAGGGGACCTGC
AGCCACAACTTCTCTGGTCCTCTGCATCCCTTCTGTCCTCCACCCGTCCCCTTCCCCACCCTCTGGCCC
CCACCTTCTTGGAGGCGACAACCCCCGGGAGGCATTAGAAGGGATTTTTCCCGCAGGTTGCGAAGGGAAG
CAAACTTGGTGGCAACTTGCCTCCCGGTGCGGGCGTCTCTCCCCCACCGTCTCAACATGCTTAGGGGTCC
GGGGCCCGGGCTGCTGCTGCTGGCCGTCCAGTGCCTGGGGACAGCGGTGCCCTCCACGGAGCCTCGAAG
AGCAAGAGGCAGGCTCAGCAAATGGTTCAGCCCCAGTCCCCGGTGGCTGTCAGTCAAAGCAAGCCCGGTT
GTTATGACAATGGAAAACACTATCAGATAAATCAACAGTGGGAGCGGACCTACCTAGGCAATGCGTTGGT
TTGTACTTGTTATGGAGGAAGCCGAGGTTTTAACTGCGAGAGTAAACCTGAAGCTGAAGAGACTTGCTTT
GACAAGTACACTGGGAACACTTACCGAGTGGGTGACACTTATGAGCGTCCTAAAGACTCCATGATCTGGG
ACTGTACCTGCATCGGGGCTGGGCGAGGGAGAATAAGCTGTACCATCGCAAACCGCTGCCATGAAGGGGG
TCAGTCCTACAAGATTGGTGACACCTGGAGGAGACCACATGAGACTGGTGGTTACATGTTAGAGTGTGTG
TGTCTTGGTAATGGAAAAGGAGAATGGACCTGCAAGCCCATAGCTGAGAAGTGTTTTGATCATGCTGCTG
GGACTTCCTATGTGGTCGGAGAAACGTGGGAGAAGCCCTACCAAGGCTGGATGATGGTAGATTGTACTTG
CCTGGGAGAAGGCAGCGGACGCATCACTTGCACTTCTAGAAATAGATGCAACGATCAGGACACAAGGACA
TCCTATAGAATTGGAGACACCTGGAGCAAGAAGGATAATCGAGGAAACCTGCTCCAGTGCATCTGCACAG
GCAACGGCCGAGGAGAGTGGAAGTGTGAGAGGCACACCTCTGTGCAGACCACATCGAGCGGATCTGGCCC
CTTCACCGATGTTCGTGCAGCTGTTTACCAACCGCAGCCTCACCCCCAGCCTCCTCCCTATGGCCACTGT
GTCACAGACAGTGGTGTGGTCTACTCTGTGGGGATGCAGTGGCTGAAGACACAAGGAAATAAGCAAATGC
TTTGCACGTGCCTGGGCAACGGAGTCAGCTGCCAAGAGACAGCTGTAACCCAGACTTACGGTGGCAACTC
AAATGGAGAGCCATGTGTCTTACCATTCACCTACAATGGCAGGACGTTCTACTCCTGCACCACAGAAGGG
CGACAGGACGGACATCTTTGGTGCAGCACAACTTCGAATTATGAGCAGGACCAGAAATACTCTTTCTGCA
CAGACCACACTGTTTTGGTTCAGACTCGAGGAGGAAATTCCAATGGTGCCTTGTGCCACTTCCCCTTCCT
ATACAACAACCACAATTACACTGATTGCACTTCTGAGGGCAGAAGAGACAACATGAAGTGGTGTGGGACC
ACACAGAACTATGATGCCGACCAGAAGTTTGGGTTCTGCCCCATGGCTGCCCACGAGGAAATCTGCACAA
CCAATGAAGGGGTCATGTACCGCATTGGAGATCAGTGGGATAAGCAGCATGACATGGGTCACATGATGAG
GTGCACGTGTGTTGGGAATGGTCGTGGGGAATGGACATGCATTGCCTACTCGCAGCTTCGAGATCAGTGC
ATTGTTGATGACATCACTTACAATGTGAACGACACATTCCACAAGCGTCATGAAGAGGGGCACATGCTGA
ACTGTACATGCTTCGGTCAGGGTCGGGGCAGGTGGAAGTGTGATCCCGTCGACCAATGCCAGGATTCAGA
 
GACTGGGACGTTTTATCAAATTGGAGATTCATGGGAGAAGTATGTGCATGGTGTCAGATACCAGTGCTAC
TGCTATGGCCGTGGCATTGGGGAGTGGCATTGCCAACCTTTACAGACCTATCCAAGCTCAAGTGGTCCTG
TCGAAGTATTTATCACTGAGACTCCGAGTCAGCCCAACTCCCACCCCATCCAGTGGAATGCACCACAGCC
ATCTCACATTTCCAAGTACATTCTCAGGTGGAGACCTAAAAATTCTGTAGGCCGTTGGAAGGAAGCTACC
ATACCAGGCCACTTAAACTCCTACACCATCAAAGGCCTGAAGCCTGGTGTGGTATACGAGGGCCAGCTCA
TCAGCATCCAGCAGTACGGCCACCAAGAAGTGACTCGCTTTGACTTCACCACCACCAGCACCAGCACACC
TGTGACCAGCAACACCGTGACAGGAGAGACGACTCCCTTTTCTCCTCTTGTGGCCACTTCTGAATCTGTG
ACCGAAATCACAGCCAGTAGCTTTGTGGTCTCCTGGGTCTCAGCTTCCGACACCGTGTCGGGATTCCGGG
TGGAATATGAGCTGAGTGAGGAGGGAGATGAGCCACAGTACCTGGATCTTCCAAGCACAGCCACTTCTGT
GAACATCCCTGACCTGCTTCCTGGCCGAAAATACATTGTAAATGTCTATCAGATATCTGAGGATGGGGAG
CAGAGTTTGATCCTGTCTACTTCACAAACAACAGCGCCTGATGCCCCTCCTGACCCGACTGTGGACCAAG
TTGATGACACCTCAATTGTTGTTCGCTGGAGCAGACCCCAGGCTCCCATCACAGGGTACAGAATAGTCTA
TTCGCCATCAGTAGAAGGTAGCAGCACAGAACTCAACCTTCCTGAAACTGCAAACTCCGTCACCCTCAGT
GACTTGCAACCTGGTGTTCAGTATAACATCACTATCTATGCTGTGGAAGAAAATCAAGAAAGTACACCTG
TTGTCATTCAACAAGAAACCACTGGCACCCCACGCTCAGATACAGTGCCCTCTCCCAGGGACCTGCAGTT
TGTGGAAGTGACAGACGTGAAGGTCACCATCATGTGGACACCGCCTGAGAGTGCAGTGACCGGCTACCGT
GTGGATGTGATCCCCGTCAACCTGCCTGGCGAGCACGGGCAGAGGCTGCCCATCAGCAGGAACACCTTTG
CAGAAGTCACCGGGCTGTCCCCTGGGGTCACCTATTACTTCAAAGTCTTTGCAGTGAGCCATGGGAGGGA
GAGCAAGCCTCTGACTGCTCAACAGACAACCAAACTGGATGCTCCCACTAACCTCCAGTTTGTCAATGAA
ACTGATTCTACTGTCCTGGTGAGATGGACTCCACCTCGGGCCCAGATAACAGGATACCGACTGACCGTGG
GCCTTACCCGAAGAGGACAGCCCAGGCAGTACAATGTGGGTCCCTCTGTCTCCAAGTACCCACTGAGGAA
TCTGCAGCCTGCATCTGAGTACACCGTATCCCTCGTGGCCATAAAGGGCAACCAAGAGAGCCCCAAAGCC
ACTGGAGTCTTTACCACACTGCAGCCTGGGAGCTCTATTCCACCTTACAACACCGAGGTGACTGAGACCA
CCATTGTGATCACATGGACGCCTGCTCCAAGAATTGGTTTTAAGCTGGGTGTACGACCAAGCCAGGGAGG
AGAGGCACCACGAGAAGTGACTTCAGACTCAGGAAGCATCGTTGTGTCCGGCTTGACTCCAGGAGTAGAA
TACGTCTACACCATCCAAGTCCTGAGAGATGGACAGGAAAGAGATGCGCCAATTGTAAACAAAGTGGTGA
CACCATTGTCTCCACCAACAAACTTGCATCTGGAGGCAAACCCTGACACTGGAGTGCTCACAGTCTCCTG
GGAGAGGAGCACCACCCCAGACATTACTGGTTATAGAATTACCACAACCCCTACAAACGGCCAGCAGGGA
AATTCTTTGGAAGAAGTGGTCCATGCTGATCAGAGCTCCTGCACTTTTGATAACCTGAGTCCCGGCCTGG
AGTACAATGTCAGTGTTTACACTGTCAAGGATGACAAGGAAAGTGTCCCTATCTCTGATACCATCATCCC
AGCTGTTCCTCCTCCCACTGACCTGCGATTCACCAACATTGGTCCAGACACCATGCGTGTCACCTGGGCT
CCACCCCCATCCATTGATTTAACCAACTTCCTGGTGCGTTACTCACCTGTGAAAAATGAGGAAGATGTTG
CAGAGTTGTCAATTTCTCCTTCAGACAATGCAGTGGTCTTAACAAATCTCCTGCCTGGTACAGAATATGT
AGTGAGTGTCTCCAGTGTCTACGAACAACATGAGAGCACACCTCTTAGAGGAAGACAGAAAACAGGTCTT
GATTCCCCAACTGGCATTGACTTTTCTGATATTACTGCCAACTCTTTTACTGTGCACTGGATTGCTCCTC
GAGCCACCATCACTGGCTACAGGATCCGCCATCATCCCGAGCACTTCAGTGGGAGACCTCGAGAAGATCG
GGTGCCCCACTCTCGGAATTCCATCACCCTCACCAACCTCACTCCAGGCACAGAGTATGTGGTCAGCATC
GTTGCTCTTAATGGCAGAGAGGAAAGTCCCTTATTGATTGGCCAACAATCAACAGTTTCTGATGTTCCGA
GGGACCTGGAAGTTGTTGCTGCGACCCCCACCAGCCTACTGATCAGCTGGGATGCTCCTGCTGTCACAGT
GAGATATTACAGGATCACTTACGGAGAGACAGGAGGAAATAGCCCTGTCCAGGAGTTCACTGTGCCTGGG
AGCAAGTCTACAGCTACCATCAGCGGCCTTAAACCTGGAGTTGATTATACCATCACTGTGTATGCTGTCA
CTGGCCGTGGAGACAGCCCCGCAAGCAGCAAGCCAATTTCCATTAATTACCGAACAGAAATTGACAAACC
ATCCCAGATGCAAGTGACCGATGTTCAGGACAACAGCATTAGTGTCAAGTGGCTGCCTTCAAGTTCCCCT
GTTACTGGTTACAGAGTAACCACCACTCCCAAAAATGGACCAGGACCAACAAAAACTAAAACTGCAGGTC
CAGATCAAACAGAAATGACTATTGAAGGCTTGCAGCCCACAGTGGAGTATGTGGTTAGTGTCTATGCTCA
GAATCCAAGCGGAGAGAGTCAGCCTCTGGTTCAGACTGCAGTAACCAACATTGATCGCCCTAAAGGACTG
GCATTCACTGATGTGGATGTCGATTCCATCAAAATTGCTTGGGAAAGCCCACAGGGGCAAGTTTCCAGGT
ACAGGGTGACCTACTCGAGCCCTGAGGATGGAATCCATGAGCTATTCCCTGCACCTGATGGTGAAGAAGA
CACTGCAGAGCTGCAAGGCCTCAGACCGGGTTCTGAGTACACAGTCAGTGTGGTTGCCTTGCACGATGAT
ATGGAGAGCCAGCCCCTGATTGGAACCCAGTCCACAGCTATTCCTGCACCAACTGACCTGAAGTTCACTC
AGGTCACACCCACAAGCCTGAGCGCCCAGTGGACACCACCCAATGTTCAGCTCACTGGATATCGAGTGCG
GGTGACCCCCAAGGAGAAGACCGGACCAATGAAAGAAATCAACCTTGCTCCTGACAGCTCATCCGTGGTT
GTATCAGGACTTATGGTGGCCACCAAATATGAAGTGAGTGTCTATGCTCTTAAGGACACTTTGACAAGCA
GACCAGCTCAGGGAGTTGTCACCACTCTGGAGAATGTCAGCCCACCAAGAAGGGCTCGTGTGACAGATGC
TACTGAGACCACCATCACCATTAGCTGGAGAACCAAGACTGAGACGATCACTGGCTTCCAAGTTGATGCC
GTTCCAGCCAATGGCCAGACTCCAATCCAGAGAACCATCAAGCCAGATGTCAGAAGCTACACCATCACAG
 
GTTTACAACCAGGCACTGACTACAAGATCTACCTGTACACCTTGAATGACAATGCTCGGAGCTCCCCTGT
GGTCATCGACGCCTCCACTGCCATTGATGCACCATCCAACCTGCGTTTCCTGGCCACCACACCCAATTCC
TTGCTGGTATCATGGCAGCCGCCACGTGCCAGGATTACCGGCTACATCATCAAGTATGAGAAGCCTGGGT
CTCCTCCCAGAGAAGTGGTCCCTCGGCCCCGCCCTGGTGTCACAGAGGCTACTATTACTGGCCTGGAACC
GGGAACCGAATATACAATTTATGTCATTGCCCTGAAGAATAATCAGAAGAGCGAGCCCCTGATTGGAAGG
AAAAAGACAGACGAGCTTCCCCAACTGGTAACCCTTCCACACCCCAATCTTCATGGACCAGAGATCTTGG
ATGTTCCTTCCACAGTTCAAAAGACCCCTTTCGTCACCCACCCTGGGTATGACACTGGAAATGGTATTCA
GCTTCCTGGCACTTCTGGTCAGCAACCCAGTGTTGGGCAACAAATGATCTTTGAGGAACATGGTTTTAGG
CGGACCACACCGCCCACAACGGCCACCCCCATAAGGCATAGGCCAAGACCATACCCGCCGAATGTAGGAC
AAGAAGCTCTCTCAGACAACCATCTCATGGGCCCATTCCAGGACACTTCTGAGTACATCATTTCATG
TCATCCTGTTGGCACTGATGAAGAACCCTTACAGTTCAGGGTTCCTGGAACTTCTACCAGTGCCACTCTG
ACAGGCCTCACCAGAGGTGCCACCTACAACATCATAGTGGAGGCACTGAAAGACCAGCAGAGGCATAAGG
TTCGGGAAGAGGTTGTTACCGTGGGCAACTCTGTCAACGAAGGCTTGAACCAACCTACGGATGACTCGTG
CTTTGACCCCTACACAGTTTCCCATTATGCCGTTGGAGATGAGTGGGAACGAATGTCTGAATCAGGCTTT
AAACTGTTGTGCCAGTGCTTAGGCTTTGGAAGTGGTCATTTCAGATGTGATTCATCTAGATGGTGCCATG
ACAATGGTGTGAACTACAAGATTGGAGAGAAGTGGGACCGTCAGGGAGAAAATGGCCAGATGATGAGCTG
CACATGTCTTGGGAACGGAAAAGGAGAATTCAAGTGTGACCCTCATGAGGCAACGTGTTATGATGATGGG
AAGACATACCACGTAGGAGAACAGTGGCAGAAGGAATATCTCGGTGCCATTTGCTCCTGCACATGCTTTG
GAGGCCAGCGGGGCTGGCGCTGTGACAACTGCCGCAGACCTGGGGGTGAACCCAGTCCCGAAGGCACTAC
TGGCCAGTCCTACAACCAGTATTCTCAGAGATACCATCAGAGAACAAACACTAATGTTAATTGCCCAATT
GAGTGCTTCATGCCTTTAGATGTACAGGCTGACAGAGAAGATTCCCGAGAGTAAATCATCTTTCCAATCC
AGAGGAACAAGCATGTCTCTCTGCCAAGATCCATCTAAACTGGAGTGATGTTAGCAGACCCAGCTTAGAG
TTCTTCTTTCTTTCTTAAGCCCTTTGCTCTGGAGGAAGTTCTCCAGCTTCAGCTCAACTCACAGCTTCTC
CAAGCATCACCCTGGGAGTTTCCTGAGGGTTTTCTCATAAATGAGGGCTGCACATTGCCTGTTCTGCTTC
GAAGTATTCAATACCGCTCAGTATTTTAAATGAAGTGATTCTAAGATTTGGTTTGGGATCAATAGGAAAG
CATATGCAGCCAACCAAGATGCAAATGTTTTGAAATGATATGACCAAAATTTTAAGTAGGAAAGTCACCC
AAACACTTCTGCTTTCACTTAAGTGTCTGGCCCGCAATACTGTAGGAACAAGCATGATCTTGTTACTGTG
ATATTTTAAATATCCACAGTACTCACTTTTTCCAAATGATCCTAGTAATTGCCTAGAAATATCTTTCTCT
TACCTGTTATTTATCAATTTTTCCCAGTATTTTTATACGGAAAAAATTGTATTGAAAACACTTAGTATGC
AGTTGATAAGAGGAATTTGGTATAATTATGGTGGGTGATTATTTTTTATACTGTATGTGCCAAAGCTTTA
CTACTGTGGAAAGACAACTGTTTTAATAAAAGATTTACATTCCACAACTTGAAGTTCATCTATTTGATAT
AAGACACCTTCGGGGGAAATAATTCCTGTGAATATTCTTTTTCAATTCAGCAAACATTTGAAAATCTATG
ATGTGCAAGTCTAATTGTTGATTTCAGTACAAGATTTTCTAAATCAGTTGCTACAAAAACTGATTGGTTT
TTGTCACTTCATCTCTTCACTAATGGAGATAGCTTTACACTTTCTGCTTTAATAGATTTAAGTGGACCCC
AATATTTATTAAAATTGCTAGTTTACCGTTCAGAAGTATAATAGAAATAATCTTTAGTTGCTCTTTTCTA
ACCATTGTAATTCTTCCCTTCTTCCCTCCACCTTTCCTTCATTGAATAAACCTCTGTTCAAAGAGATTGC
CTGCAAGGGAAATAAAAATGACTAAGATATTAAAAAAAAAAAAAAAAAA
NCBI Reference Sequence: NM_212478.1
   **Homo sapiens fibronectin 1 (FN1), transcript variant 4, mRNA**
   >gi|47132554|ref|NM_212478.1| Homo sapiens fibronectin 1 (FN1), transcript variant 4, mRNA 
GCCCGCGCCGGCTGTGCTGCACAGGGGGAGGAGAGGGAACCCCAGGCGCGAGCGGGAAGAGGGGACCTGC
AGCCACAACTTCTCTGGTCCTCTGCATCCCTTCTGTCCTCCACCCGTCCCCTTCCCCACCCTCTGGCCC
CCACCTTCTTGGAGGCGACAACCCCCGGGAGGCATTAGAAGGGATTTTTCCCGCAGGTTGCGAAGGGAAG
CAAACTTGGTGGCAACTTGCCTCCCGGTGCGGGCGTCTCTCCCCCACCGTCTCAACATGCTTAGGGGTCC
GGGGCCCGGGCTGCTGCTGCTGGCCGTCCAGTGCCTGGGGACAGCGGTGCCCTCCACGGGAGCCTCGAAG
AGCAAGAGGCAGGCTCAGCAAATGGTTCAGCCCCAGTCCCCGGTGGCTGTCAGTCAAAGCAAGCCCGGTT
GTTATGACAATGGAAAACACTATCAGATAAATCAACAGTGGGAGCGGACCTACCTAGGCAATGCGTTGGT
TTGTACTTGTTATGGAGGAAGCCGAGGTTTTAACTGCGAGAGTAAACCTGAAGCTGAAGAGACTTGCTTT
GACAAGTACACTGGGAACACTTACCGAGTGGGTGACACTTATGAGCGTCCTAAAGACTCCATGATCTGGG
ACTGTACCTGCATCGGGGCTGGGCGAGGGAGAATAAGCTGTACCATCGCAAACCGCTGCCATGAAGGGGG
TCAGTCCTACAAGATTGGTGACACCTGGAGGAGACCACATGAGACTGGTGGTTACATGTTAGAGTGTGTG
 
TGTCTTGGTAATGGAAAAGGAGAATGGACCTGCAAGCCCATAGCTGAGAAGTGTTTTGATCATGCTGCTG
GGACTTCCTATGTGGTCGGAGAAACGTGGGAGAAGCCCTACCAAGGCTGGATGATGGTAGATTGTACTTG
CCTGGGAGAAGGCAGCGGACGCATCACTTGCACTTCTAGAAATAGATGCAACGATCAGGACACAAGGACA
TCCTATAGAATTGGAGACACCTGGAGCAAGAAGGATAATCGAGGAAACCTGCTCCAGTGCATCTGCACAG
GCAACGGCCGAGGAGAGTGGAAGTGTGAGAGGCACACCTCTGTGCAGACCACATCGAGCGGATCTGGCCC
CTTCACCGATGTTCGTGCAGCTGTTTACCAACCGCAGCCTCACCCCCAGCCTCCTCCCTATGGCCACTGT
GTCACAGACAGTGGTGTGGTCTACTCTGTGGGGATGCAGTGGCTGAAGACACAAGGAAATAAGCAAATGC
TTTGCACGTGCCTGGGCAACGGAGTCAGCTGCCAAGAGACAGCTGTAACCCAGACTTACGGTGGCAACTC
AAATGGAGAGCCATGTGTCTTACCATTCACCTACAATGGCAGGACGTTCTACTCCTGCACCACAGAAGGG
CGACAGGACGGACATCTTTGGTGCAGCACAACTTCGAATTATGAGCAGGACCAGAAATACTCTTTCTGCA
CAGACCACACTGTTTTGGTTCAGACTCGAGGAGGAAATTCCAATGGTGCCTTGTGCCACTTCCCCTTCCT
ATACAACAACCACAATTACACTGATTGCACTTCTGAGGGCAGAAGAGACAACATGAAGTGGTGTGGGACC
ACACAGAACTATGATGCCGACCAGAAGTTTGGGTTCTGCCCCATGGCTGCCCACGAGGAAATCTGCACAA
CCAATGAAGGGGTCATGTACCGCATTGGAGATCAGTGGGATAAGCAGCATGACATGGGTCACATGATGAG
GTGCACGTGTGTTGGGAATGGTCGTGGGGAATGGACATGCATTGCCTACTCGCAGCTTCGAGATCAGTGC
ATTGTTGATGACATCACTTACAATGTGAACGACACATTCCACAAGCGTCATGAAGAGGGGCACATGCTGA
ACTGTACATGCTTCGGTCAGGGTCGGGGCAGGTGGAAGTGTGATCCCGTCGACCAATGCCAGGATTCAGA
GACTGGGACGTTTTATCAAATTGGAGATTCATGGGAGAAGTATGTGCATGGTGTCAGATACCAGTGCTAC
TGCTATGGCCGTGGCATTGGGGAGTGGCATTGCCAACCTTTACAGACCTATCCAAGCTCAAGTGGTCCTG
TCGAAGTATTTATCACTGAGACTCCGAGTCAGCCCAACTCCCACCCCATCCAGTGGAATGCACCACAGCC
ATCTCACATTTCCAAGTACATTCTCAGGTGGAGACCTAAAAATTCTGTAGGCCGTTGGAAGGAAGCTACC
ATACCAGGCCACTTAAACTCCTACACCATCAAAGGCCTGAAGCCTGGTGTGGTATACGAGGGCCAGCTCA
TCAGCATCCAGCAGTACGGCCACCAAGAAGTGACTCGCTTTGACTTCACCACCACCAGCACCAGCACACC
TGTGACCAGCAACACCGTGACAGGAGAGACGACTCCCTTTTCTCCTCTTGTGGCCACTTCTGAATCTGTG
ACCGAAATCACAGCCAGTAGCTTTGTGGTCTCCTGGGTCTCAGCTTCCGACACCGTGTCGGGATTCCGGG
TGGAATATGAGCTGAGTGAGGAGGGAGATGAGCCACAGTACCTGGATCTTCCAAGCACAGCCACTTCTGT
GAACATCCCTGACCTGCTTCCTGGCCGAAAATACATTGTAAATGTCTATCAGATATCTGAGGATGGGGAG
CAGAGTTTGATCCTGTCTACTTCACAAACAACAGCGCCTGATGCCCCTCCTGACCCGACTGTGGACCAAG
TTGATGACACCTCAATTGTTGTTCGCTGGAGCAGACCCCAGGCTCCCATCACAGGGTACAGAATAGTCTA
TTCGCCATCAGTAGAAGGTAGCAGCACAGAACTCAACCTTCCTGAAACTGCAAACTCCGTCACCCTCAGT
GACTTGCAACCTGGTGTTCAGTATAACATCACTATCTATGCTGTGGAAGAAAATCAAGAAAGTACACCTG
TTGTCATTCAACAAGAAACCACTGGCACCCCACGCTCAGATACAGTGCCCTCTCCCAGGGACCTGCAGTT
TGTGGAAGTGACAGACGTGAAGGTCACCATCATGTGGACACCGCCTGAGAGTGCAGTGACCGGCTACCGT
GTGGATGTGATCCCCGTCAACCTGCCTGGCGAGCACGGGCAGAGGCTGCCCATCAGCAGGAACACCTTTG
CAGAAGTCACCGGGCTGTCCCCTGGGGTCACCTATTACTTCAAAGTCTTTGCAGTGAGCCATGGGAGGGA
GAGCAAGCCTCTGACTGCTCAACAGACAACCAAACTGGATGCTCCCACTAACCTCCAGTTTGTCAATGAA
ACTGATTCTACTGTCCTGGTGAGATGGACTCCACCTCGGGCCCAGATAACAGGATACCGACTGACCGTGG
GCCTTACCCGAAGAGGACAGCCCAGGCAGTACAATGTGGGTCCCTCTGTCTCCAAGTACCCACTGAGGAA
TCTGCAGCCTGCATCTGAGTACACCGTATCCCTCGTGGCCATAAAGGGCAACCAAGAGAGCCCCAAAGCC
ACTGGAGTCTTTACCACACTGCAGCCTGGGAGCTCTATTCCACCTTACAACACCGAGGTCACTGAGACCA
CCATTGTGATCACATGGACGCCTGCTCCAAGAATTGGTTTTAAGCTGGGTGTACGACCAAGCCAGGGAGG
AGAGGCACCACGAGAAGTGACTTCAGACTCAGGAAGCATCGTTGTGTCCGGCTTGACTCCAGGAGTAGAA
TACGTCTACACCATCCAAGTCCTGAGAGATGGACAGGAAAGAGATGCGCCAATTGTAAACAAAGTGGTGA
CACCATTGTCTCCACCAACAAACTTGCATCTGGAGGCAAACCCTGACACTGGAGTGCTCACAGTCTCCTG
GGAGAGGAGCACCACCCCAGACATTACTGGTTATAGAATTACCACAACCCCTACAAACGGCCAGCAGGGA
AATTCTTTGGAAGAAGTGGTCCATGCTGATCAGAGCTCCTGCACTTTTGATAACCTGAGTCCCGGCCTGG
AGTACAATGTCAGTGTTTACACTGTCAAGGATGACAAGGAAAGTGTCCCTATCTCTGATACCATCATCCC
AGCTGTTCCTCCTCCCACTGACCTGCGATTCACCAACATTGGTCCAGACACCATGCGTGTCACCTGGGCT
CCACCCCCATCCATTGATTTAACCAACTTCCTGGTGCGTTACTCACCTGTGAAAAATGAGGAAGATGTTG
CAGAGTTGTCAATTTCTCCTTCAGACAATGCAGTGGTCTTAACAAATCTCCTGCCTGGTACAGAATATGT
AGTGAGTGTCTCCAGTGTCTACGAACAACATGAGAGCACACCTCTTAGAGGAAGACAGAAAACAGGTCTT
GATTCCCCAACTGGCATTGACTTTTCTGATATTACTGCCAACTCTTTTACTGTGCACTGGATTGCTCCTC
GAGCCACCATCACTGGCTACAGGATCCGCCATCATCCCGAGCACTTCAGTGGGAGACCTCGAGAAGATCG
GGTGCCCCACTCTCGGAATTCCATCACCCTCACCAACCTCACTCCAGGCACAGAGTATGTGGTCAGCATC
GTTGCTCTTAATGGCAGAGAGGAAAGTCCCTTATTGATTGGCCAACAATCAACAGTTTCTGATGTTCCGA
GGGACCTGGAAGTTGTTGCTGCGACCCCCACCAGCCTACTGATCAGCTGGGATGCTCCTGCTGTCACAGT
 
GAGATATTACAGGATCACTTACGGAGAGACAGGAGGAAATAGCCCTGTCCAGGAGTTCACTGTGCCTGGG
AGCAAGTCTACAGCTACCATCAGCGGCCTTAAACCTGGAGTTGATTATACCATCACTGTGTATGCTGTCA
CTGGCCGTGGAGACAGCCCCGCAAGCAGCAAGCCAATTTCCATTAATTACCGAACAGAAATTGACAAACC
ATCCCAGATGCAAGTGACCGATGTTCAGGACAACAGCATTAGTGTCAAGTGGCTGCCTTCAAGTTCCCCT
GTTACTGGTTACAGAGTAACCACCACTCCCAAAAATGGACCAGGACCAACAAAAACTAAAACTGCAGGTC
CAGATCAAACAGAAATGACTATTGAAGGCTTGCAGCCCACAGTGGAGTATGTGGTTAGTGTCTATGCTCA
GAATCCAAGCGGAGAGAGTCAGCCTCTGGTTCAGACTGCAGTAACCAACATTGATCGCCCTAAAGGACTG
GCATTCACTGATGTGGATGTCGATTCCATCAAAATTGCTTGGGAAAGCCCACAGGGGCAAGTTTCCAGGT
ACAGGGTGACCTACTCGAGCCCTGAGGATGGAATCCATGAGCTATTCCCTGCACCTGATGGTGAAGAAGA
CACTGCAGAGCTGCAAGGCCTCAGACCGGGTTCTGAGTACACAGTCAGTGTGGTTGCCTTGCACGATGAT
ATGGAGAGCCAGCCCCTGATTGGAACCCAGTCCACAGCTATTCCTGCACCAACTGACCTGAAGTTCACTC
AGGTCACACCCACAAGCCTGAGCGCCCAGTGGACACCACCCAATGTTCAGCTCACTGGATATCGAGTGCG
GGTGACCCCCAAGGAGAAGACCGGACCAATGAAAGAAATCAACCTTGCTCCTGACAGCTCATCCGTGGTT
GTATCAGGACTTATGGTGGCCACCAAATATGAAGTGAGTGTCTATGCTCTTAAGGACACTTTGACAAGCA
GACCAGCTCAGGGAGTTGTCACCACTCTGGAGAATGTCAGCCCACCAAGAAGGCTCGTGTGACAGATGC
TACTGAGACCACCATCACCATTAGCTGGAGAACCAAGACTGAGACGATCACTGGCTTCCAAGTTGATGCC
GTTCCAGCCAATGGCCAGACTCCAATCCAGAGAACCATCAAGCCAGATGTCAGAAGCTACACCATCACAG
GTTTACAACCAGGCACTGACTACAAGATCTACCTGTACACCTTGAATGACAATGCTCGGAGCTCCCCTGT
GGTCATCGACGCCTCCACTGCCATTGATGCACCATCCAACCTGCGTTTCCTGGCCACCACACCCAATTCC
TTGCTGGTATCATGGCAGCCGCCACGTGCCAGGATTACCGGCTACATCATCAAGTATGAGAAGCCTGGGT
CTCCTCCCAGAGAAGTGGTCCCTCGGCCCCGCCCTGGTGTCACAGAGGCTACTATTACTGGCCTGGAACC
GGGAACCGAATATACAATTTATGTCATTGCCCTGAAGAATAATCAGAAGAGCGAGCCCCTGATTGGAAGG
AAAAAGACAGTTCAAAAGACCCCTTTCGTCACCCACCCTGGGTATGACACTGGAAATGGTATTCAGCTTC
CTGGCACTTCTGGTCAGCAACCCAGTGTTGGGCAACAAATGATCTTTGAGGAACATGGTTTTAGGCGGAC
CACACCGCCCACAACGGCCACCCCCATAAGGCATAGGCCAAGACCATACCCGCCGAATGTAGGACAAGAA
GCTCTCTCTCAGACAACCATCTCATGGGCCCCATTCCAGGACACTTCTGAGTACATCATTTCATGTCATC
CTGTTGGCACTGATGAAGAACCCTTACAGTTCAGGGTTCCTGGAACTTCTACCAGTGCCACTCTGACAGG
CCTCACCAGAGGTGCCACCTACAACATCATAGTGGAGGCACTGAAAGACCAGCAGAGGCATAAGGTTCGG
GAAGAGGTTGTTACCGTGGGCAACTCTGTCAACGAAGGCTTGAACCAACCTACGGATGACTCGTGCTTTG
ACCCCTACACAGTTTCCCATTATGCCGTTGGAGATGAGTGGAACGAATGTCTGAATCAGGCTTTAAACT
GTTGTGCCAGTGCTTAGGCTTTGGAAGTGGTCATTTCAGATGTGATTCATCTAGATGGTGCCATGACAAT
GGTGTGAACTACAAGATTGGAGAGAAGTGGGACCGTCAGGGAGAAAATGGCCAGATGATGAGCTGCACAT
GTCTTGGGAACGGAAAAGGAGAATTCAAGTGTGACCCTCATGAGGCAACGTGTTATGATGATGGGAAGAC
ATACCACGTAGGAGAACAGTGGCAGAAGGAATATCTCGGTGCCATTTGCTCCTGCACATGCTTTGGAGGC
CAGCGGGGCTGGCGCTGTGACAACTGCCGCAGACCTGGGGGTGAACCCAGTCCCGAAGGCACTACTGGCC
AGTCCTACAACCAGTATTCTCAGAGATACCATCAGAGAACAAACACTAATGTTAATTGCCCAATTGAGTG
CTTCATGCCTTTAGATGTACAGGCTGACAGAGAAGATTCCCGAGAGTAAATCATCTTTCCAATCCAGAGG
AACAAGCATGTCTCTCTGCCAAGATCCATCTAAACTGGAGTGATGTTAGCAGACCCAGCTTAGAGTTCTT
CTTTCTTTCTTAAGCCCTTTGCTCTGGAGGAAGTTCTCCAGCTTCAGCTCAACTCACAGCTTCTCCAAGC
ATCACCCTGGGAGTTTCCTGAGGGTTTTCTCATAAATGAGGGCTGCACATTGCCTGTTCTGCTTCGAAGT
ATTCAATACCGCTCAGTATTTTAAATGAAGTGATTCTAAGATTTGGTTTGGGATCAATAGGAAAGCATAT
GCAGCCAACCAAGATGCAAATGTTTTGAAATGATATGACCAAAATTTTAAGTAGGAAAGTCACCCAAACA
CTTCTGCTTTCACTTAAGTGTCTGGCCCGCAATACTGTAGGAACAAGCATGATCTTGTTACTGTGATATT
TTAAATATCCACAGTACTCACTTTTTCCAAATGATCCTAGTAATTGCCTAGAAATATCTTTCTCTTACCT
GTTATTTATCAATTTTTCCCAGTATTTTTATACGGAAAAAATTGTATTGAAAACACTTAGTATGCAGTTG
ATAAGAGGAATTTGGTATAATTATGGTGGGTGATTATTTTTTATACTGTATGTGCCAAAGCTTTACTACT
GTGGAAAGACAACTGTTTTAATAAAAGATTTACATTCCACAACTTGAAGTTCATCTATTTGATATAAGAC
ACCTTCGGGGGAAATAATTCCTGTGAATATTCTTTTTCAATTCAGCAAACATTTGAAAATCTATGATGTG
CAAGTCTAATTGTTGATTTCAGTACAAGATTTTCTAAATCAGTTGCTACAAAAACTGATTGGTTTTTGTC
ACTTCATCTCTTCACTAATGGAGATAGCTTTACACTTTCTGCTTTAATAGATTTAAGTGGACCCCAATAT
TTATTAAAATTGCTAGTTTACCGTTCAGAAGTATAATAGAAATAATCTTTAGTTGCTCTTTTCTAACCAT
TGTAATTCTTCCCTTCTTCCCTCCACCTTTCCTTCATTGAATAAACCTCTGTTCAAAGAGATTGCCTGCA
AGGGAAATAAAAATGACTAAGATATTAAAAAAAAAAAAAAAAAA
NCBI Reference Sequence: NM_212482.1
   **Homo sapiens fibronectin 1 (FN1), transcript variant 1, mRNA**
   >gi|47132556|ref|NM_212482.1| Homo sapiens fibronectin 1 (FN1), transcript variant 1, mRNA 
GCCCGCGCCGGCTGTGCTGCACAGGGGGAGGAGAGGGAACCCCAGGCGCGAGCGGGAAGAGGGGACCTGC
AGCCACAACTTCTCTGGTCCTCTGCATCCCTTCTGTCCCTCCACCCGTCCCCTTCCCCACCCTCTGGCCC
CCACCTTCTTGGAGGCGACAACCCCCGGGAGGCATTAGAAGGGATTTTTCCCGCAGGTTGCGAAGGGAAG
CAAACTTGGTGGCAACTTGCCTCCCGGTGCGGGCGTCTCTCCCCCACCGTCTCAACATGCTTAGGGGTCC
GGGGCCCGGGCTGCTGCTGCTGGCCGTCCAGTGCCTGGGGACAGCGGTGCCCTCCACGGAGCCTCGAAG
AGCAAGAGGCAGGCTCAGCAAATGGTTCAGCCCCAGTCCCCGGTGGCTGTCAGTCAAAGCAAGCCCGGTT
GTTATGACAATGGAAAACACTATCAGATAAATCAACAGTGGGAGCGGACCTACCTAGGCAATGCGTTGGT
TTGTACTTGTTATGGAGGAAGCCGAGGTTTTAACTGCGAGAGTAAACCTGAAGCTGAAGAGACTTGCTTT
GACAAGTACACTGGGAACACTTACCGAGTGGGTGACACTTATGAGCGTCCTAAAGACTCCATGATCTGGG
ACTGTACCTGCATCGGGGCTGGGCGAGGGAGAATAAGCTGTACCATCGCAAACCGCTGCCATGAAGGGG
TCAGTCCTACAAGATTGGTGACACCTGGAGGAGACCACATGAGACTGGTGGTTACATGTTAGAGTGTGTG
TGTCTTGGTAATGGAAAAGGAGAATGGACCTGCAAGCCCATAGCTGAGAAGTGTTTTGATCATGCTGCTG
GGACTTCCTATGTGGTCGGAGAAACGTGGGAGAAGCCCTACCAAGGCTGGATGATGGTAGATTGTACTTG
CCTGGGAGAAGGCAGCGGACGCATCACTTGCACTTCTAGAAATAGATGCAACGATCAGGACACAAGGACA
TCCTATAGAATTGGAGACACCTGGAGCAAGAAGGATAATCGAGGAAACCTGCTCCAGTGCATCTGCACAG
GCAACGGCCGAGGAGAGTGGAAGTGTGAGAGGCACACCTCTGTGCAGACCACATCGAGCGGATCTGGCCC
CTTCACCGATGTTCGTGCAGCTGTTTACCAACCGCAGCCTCACCCCCAGCCTCCTCCCTATGGCCACTGT
GTCACAGACAGTGGTGTGGTCTACTCTGTGGGGATGCAGTGGCTGAAGACACAAGGAAATAAGCAAATGC
TTTGCACGTGCCTGGGCAACGGAGTCAGCTGCCAAGAGACAGCTGTAACCCAGACTTACGGTGGCAACTC
AAATGGAGAGCCATGTGTCTTACCATTCACCTACAATGGCAGGACGTTCTACTCCTGCACCACAGAAGGG
CGACAGGACGGACATCTTTGGTGCAGCACAACTTCGAATTATGAGCAGGACCAGAAATACTCTTTCTGCA
CAGACCACACTGTTTTGGTTCAGACTCGAGGAGGAAATTCCAATGGTGCCTTGTGCCACTTCCCCTTCCT
ATACAACAACCACAATTACACTGATTGCACTTCTGAGGGCAGAAGAGACAACATGAAGTGGTGTGGGACC
ACACAGAACTATGATGCCGACCAGAAGTTTGGGTTCTGCCCCATGGCTGCCCACGAGGAAATCTGCACAA
CCAATGAAGGGGTCATGTACCGCATTGGAGATCAGTGGGATAAGCAGCATGACATGGGTCACATGATGAG
GTGCACGTGTGTTGGGAATGGTCGTGGGGAATGGACATGCATTGCCTACTCGCAGCTTCGAGATCAGTGC
ATTGTTGATGACATCACTTACAATGTGAACGACACATTCCACAAGCGTCATGAAGAGGGGCACATGCTGA
ACTGTACATGCTTCGGTCAGGGTCGGGGCAGGTGGAAGTGTGATCCCGTCGACCAATGCCAGGATTCAGA
GACTGGGACGTTTTATCAAATTGGAGATTCATGGGAGAAGTATGTGCATGGTGTCAGATACCAGTGCTAC
TGCTATGGCCGTGGCATTGGGGAGTGGCATTGCCAACCTTTACAGACCTATCCAAGCTCAAGTGGTCCTG
TCGAAGTATTTATCACTGAGACTCCGAGTCAGCCCAACTCCCACCCCATCCAGTGGAATGCACCACAGCC
ATCTCACATTTCCAAGTACATTCTCAGGTGGAGACCTAAAAATTCTGTAGGCCGTTGGAAGGAAGCTACC
ATACCAGGCCACTTAAACTCCTACACCATCAAAGGCCTGAAGCCTGGTGTGGTATACGAGGGCCAGCTCA
TCAGCATCCAGCAGTACGGCCACCAAGAAGTGACTCGCTTTGACTTCACCACCACCAGCACCAGCACACC
TGTGACCAGCAACACCGTGACAGGAGAGACGACTCCCTTTTCTCCTCTTGTGGCCACTTCTGAATCTGTG
ACCGAAATCACAGCCAGTAGCTTTGTGGTCTCCTGGGTCTCAGCTTCCGACACCGTGTCGGGATTCCGGG
TGGAATATGAGCTGAGTGAGGAGGGAGATGAGCCACAGTACCTGGATCTTCCAAGCACAGCCACTTCTGT
GAACATCCCTGACCTGCTTCCTGGCCGAAAATACATTGTAAATGTCTATCAGATATCTGAGGATGGGGAG
CAGAGTTTGATCCTGTCTACTTCACAAACAACAGCGCCTGATGCCCCTCCTGACCCGACTGTGGACCAAG
TTGATGACACCTCAATTGTTGTTCGCTGGAGCAGACCCCAGGCTCCCATCACAGGGTACAGAATAGTCTA
TTCGCCATCAGTAGAAGGTAGCAGCACAGAACTCAACCTTCCTGAAACTGCAAACTCCGTCACCCTCAGT
GACTTGCAACCTGGTGTTCAGTATAACATCACTATCTATGCTGTGGAAGAAAATCAAGAAAGTACACCTG
TTGTCATTCAACAAGAAACCACTGGCACCCCACGCTCAGATACAGTGCCCTCTCCCAGGGACCTGCAGTT
TGTGGAAGTGACAGACGTGAAGGTCACCATCATGTGGACACCGCCTGAGAGTGCAGTGACCGGCTACCGT
GTGGATGTGATCCCCGTCAACCTGCCTGGCGAGCACGGGCAGAGGCTGCCCATCAGCAGGAACACCTTTG
CAGAAGTCACCGGGCTGTCCCCTGGGGTCACCTATTACTTCAAAGTCTTTGCAGTGAGCCATGGGAGGGA
GAGCAAGCCTCTGACTGCTCAACAGACAACCAAACTGGATGCTCCCACTAACCTCCAGTTTGTCAATGAA
ACTGATTCTACTGTCCTGGTGAGATGGACTCCACCTCGGGCCCAGATAACAGGATACCGACTGACCGTGG
GCCTTACCCGAAGAGGACAGCCCAGGCAGTACAATGTGGGTCCCTCTGTCTCCAAGTACCCACTGAGGAA
TCTGCAGCCTGCATCTGAGTACACCGTATCCCTCGTGGCCATAAAGGGCAACCAAGAGAGCCCCAAAGCC
 
ACTGGAGTCTTTACCACACTGCAGCCTGGGAGCTCTATTCCACCTTACAACACCGAGGTGACTGAGACCA
CCATTGTGATCACATGGACGCCTGCTCCAAGAATTGGTTTTAAGCTGGGTGTACGACCAAGCCAGGGAGG
AGAGGCACCACGAGAAGTGACTTCAGACTCAGGAAGCATCGTTGTGTCCGGCTTGACTCCAGGAGTAGAA
TACGTCTACACCATCCAAGTCCTGAGAGATGGACAGGAAAGAGATGCGCCAATTGTAAACAAAGTGGTGA
CACCATTGTCTCCACCAACAAACTTGCATCTGGAGGCAAACCCTGACACTGGAGTGCTCACAGTCTCCTG
GGAGAGGAGCACCACCCCAGACATTACTGGTTATAGAATTACCACAACCCCTACAAACGGCCAGCAGGGA
AATTCTTTGGAAGAAGTGGTCCATGCTGATCAGAGCTCCTGCACTTTTGATAACCTGAGTCCCGGCCTGG
AGTACAATGTCAGTGTTTACACTGTCAAGGATGACAAGGAAAGTGTCCCTATCTCTGATACCATCATCCC
AGAGGTGCCCCAACTCACTGACCTAAGCTTTGTTGATATAACCGATTCAAGCATCGGCCTGAGGTGGACC
CCGCTAAACTCTTCCACCATTATTGGGTACCGCATCACAGTAGTTGCGGCAGGAGAAGGTATCCCTATTT
TTGAAGATTTTGTGGACTCCTCAGTAGGATACTACACAGTCACAGGGCTGGAGCCGGGCATTGACTATGA
TATCAGCGTTATCACTCTCATTAATGGCGGCGAGAGTGCCCCTACTACACTGACACAACAAACGGCTGTT
CCTCCTCCCACTGACCTGCGATTCACCAACATTGGTCCAGACACCATGCGTGTCACCTGGGCTCCACCCC
CATCCATTGATTTAACCAACTTCCTGGTGCGTTACTCACCTGTGAAAAATGAGGAAGATGTTGCAGAGTT
GTCAATTTCTCCTTCAGACAATGCAGTGGTCTTAACAAATCTCCTGCCTGGTACAGAATATGTAGTGAGT
GTCTCCAGTGTCTACGAACAACATGAGAGCACACCTCTTAGAGGAAGACAGAAAACAGGTCTTGATTCCC
CAACTGGCATTGACTTTTCTGATATTACTGCCAACTCTTTTACTGTGCACTGGATTGCTCCTCGAGCCAC
CATCACTGGCTACAGGATCCGCCATCATCCCGAGCACTTCAGTGGGAGACCTCGAGAAGATCGGGTGCCC
CACTCTCGGAATTCCATCACCCTCACCAACCTCACTCCAGGCACAGAGTATGTGGTCAGCATCGTTGCTC
TTAATGGCAGAGAGGAAAGTCCCTTATTGATTGGCCAACAATCAACAGTTTCTGATGTTCCGAGGGACCT
GGAAGTTGTTGCTGCGACCCCCACCAGCCTACTGATCAGCTGGGATGCTCCTGCTGTCACAGTGAGATAT
TACAGGATCACTTACGGAGAGACAGGAGGAAATAGCCCTGTCCAGGAGTTCACTGTGCCTGGGAGCAAGT
CTACAGCTACCATCAGCGGCCTTAAACCTGGAGTTGATTATACCATCACTGTGTATGCTGTCACTGGCCG
TGGAGACAGCCCCGCAAGCAGCAAGCCAATTTCCATTAATTACCGAACAGAAATTGACAAACCATCCCAG
ATGCAAGTGACCGATGTTCAGGACAACAGCATTAGTGTCAAGTGGCTGCCTTCAAGTTCCCCTGTTACTG
GTTACAGAGTAACCACCACTCCCAAAAATGGACCAGGACCAACAAAAACTAAAACTGCAGGTCCAGATCA
AACAGAAATGACTATTGAAGGCTTGCAGCCCACAGTGGAGTATGTGGTTAGTGTCTATGCTCAGAATCCA
AGCGGAGAGAGTCAGCCTCTGGTTCAGACTGCAGTAACCAACATTGATCGCCCTAAAGGACTGGCATTCA
CTGATGTGGATGTCGATTCCATCAAAATTGCTTGGGAAAGCCCACAGGGGCAAGTTTCCAGGTACAGGGT
GACCTACTCGAGCCCTGAGGATGGAATCCATGAGCTATTCCCTGCACCTGATGGTGAAGAAGACACTGCA
GAGCTGCAAGGCCTCAGACCGGGTTCTGAGTACACAGTCAGTGTGGTTGCCTTGCACGATGATATGGAGA
GCCAGCCCCTGATTGGAACCCAGTCCACAGCTATTCCTGCACCAACTGACCTGAAGTTCACTCAGGTCAC
ACCCACAAGCCTGAGCGCCCAGTGGACACCACCCAATGTTCAGCTCACTGGATATCGAGTGCGGGTGACC
CCCAAGGAGAAGACCGGACCAATGAAAGAAATCAACCTTGCTCCTGACAGCTCATCCGTGGTTGTATCAG
GACTTATGGTGGCCACCAAATATGAAGTGAGTGTCTATGCTCTTAAGGACACTTTGACAAGCAGACCAGC
TCAGGGAGTTGTCACCACTCTGGAGAATGTCAGCCCACCAAGAAGGGCTCGTGTGACAGATGCTACTGAG
ACCACCATCACCATTAGCTGGAGAACCAAGACTGAGACGATCACTGGCTTCCAAGTTGATGCCGTTCCAG
CCAATGGCCAGACTCCAATCCAGAGAACCATCAAGCCAGATGTCAGAAGCTACACCATCACAGGTTTACA
ACCAGGCACTGACTACAAGATCTACCTGTACACCTTGAATGACAATGCTCGGAGCTCCCCTGTGGTCATC
GACGCCTCCACTGCCATTCATCCACCATCCAACCTCCCTTTCCTCCCCACCACACCCAATTCCTTGCTGG
TATCATGGCAGCCGCCACGTGCCAGGATTACCGGCTACATCATCAAGTATGAGAAGCCTGGGTCTCCTCC
CAGAGAAGTGGTCCCTCGGCCCCGCCCTGGTGTCACAGAGGCTACTATTACTGGCCTGGAACCGGGAACC
GAATATACAATTTATGTCATTGCCCTGAAGAATAATCAGAAGAGCGAGCCCCTGATTGGAAGGAAAAAGA
CAGACGAGCTTCCCCAACTGGTAACCCTTCCACACCCCAATCTTCATGGACCAGAGATCTTGGATGTTCC
TTCCACAGTTCAAAAGACCCCTTTCGTCACCCACCCTGGGTATGACACTGGAAATGGTATTCAGCTTCCT
GGCACTTCTGGTCAGCAACCCAGTGTTGGGCAACAAATGATCTTTGAGGAACATGGTTTTAGGCGGACCA
CACCGCCCACAACGGCCACCCCCATAAGGCATAGGCCAAGACCATACCCGCCGAATGTAGGTGAGGAAAT
CCAAATTGGTCACATCCCCAGGGAAGATGTAGACTATCACCTGTACCCACACGGTCCGGGACTCAATCCA
AATGCCTCTACAGGACAAGAAGCTCTCTCTCAGACAACCATCTCATGGGCCCCATTCCAGGACACTTCTG
AGTACATCATTTCATGTCATCCTGTTGGCACTGATGAAGAACCCTTACAGTTCAGGGTTCCTGGAACTTC
TACCAGTGCCACTCTGACAGGCCTCACCAGAGGTGCCACCTACAACATCATAGTGGAGGCACTGAAAGAC
CAGCAGAGGCATAAGGTTCGGGAAGAGGTTGTTACCGTGGGCAACTCTGTCAACGAAGGCTTGAACCAAC
CTACGGATGACTCGTGCTTTGACCCCTACACAGTTTCCCATTATGCCGTTGGAGATGAGTGGGAACGAAT
GTCTGAATCAGGCTTTAAACTGTTGTGCCAGTGCTTAGGCTTTGGAAGTGGTCATTTCAGATGTGATTCA
TCTAGATGGTGCCATGACAATGGTGTGAACTACAAGATTGGAGAGAAGTGGGACCGTCAGGGAGAAAATG
GCCAGATGATGAGCTGCACATGTCTTGGGAACGGAAAAGGAGAATTCAAGTGTGACCCTCATGAGGCAAC
 
GTGTTATGATGATGGGAAGACATACCACGTAGGAGAACAGTGGCAGAAGGAATATCTCGGTGCCATTTGC
TCCTGCACATGCTTTGGAGGCCAGCGGGGCTGGCGCTGTGACAACTGCCGCAGACCTGGGGGTGAACCCA
GTCCCGAAGGCACTACTGGCCAGTCCTACAACCAGTATTCTCAGAGATACCATCAGAGAACAAACACTAA
TGTTAATTGCCCAATTGAGTGCTTCATGCCTTTAGATGTACAGGCTGACAGAGAAGATTCCCGAGAGTAA
ATCATCTTTCCAATCCAGAGGAACAAGCATGTCTCTCTGCCAAGATCCATCTAAACTGGAGTGATGTTAG
CAGACCCAGCTTAGAGTTCTTCTTTCTTTCTTAAGCCCTTTGCTCTGGAGGAAGTTCTCCAGCTTCAGCT
CAACTCACAGCTTCTCCAAGCATCACCCTGGGAGTTTCCTGAGGGTTTCTCATAAATGAGGGCTGCACA
TTGCCTGTTCTGCTTCGAAGTATTCAATACCGCTCAGTATTTTAAATGAAGTGATTCTAAGATTTGGTTT
GGGATCAATAGGAAAGCATATGCAGCCAACCAAGATGCAAATGTTTTGAAATGATATGACCAAAATTTTA
AGTAGGAAAGTCACCCAAACACTTCTGCTTTCACTTAAGTGTCTGGCCCGCAATACTGTAGGAACAAGCA
TGATCTTGTTACTGTGATATTTTAAATATCCACAGTACTCACTTTTTCCAAATGATCCTAGTAATTGCCT
AGAAATATCTTTCTCTTACCTGTTATTTATCAATTTTTCCCAGTATTTTTATACGGAAAAAATTGTATTG
AAAACACTTAGTATGCAGTTGATAAGAGGAATTTGGTATAATTATGGTGGGTGATTATTTTTTATACTGT
ATGTGCCAAAGCTTTACTACTGTGGAAAGACAACTGTTTTAATAAAAGATTTACATTCCACAACTTGAAG
TTCATCTATTTGATATAAGACACCTTCGGGGGAAATAATTCCTGTGAATATTCTTTTTCAATTCAGCAAA
CATTTGAAAATCTATGATGTGCAAGTCTAATTGTTGATTTCAGTACAAGATTTTCTAAATCAGTTGCTAC
AAAAACTGATTGGTTTTTGTCACTTCATCTCTTCACTAATGGAGATAGCTTTACACTTTCTGCTTTAATA
GATTTAAGTGGACCCCAATATTTATTAAAATTGCTAGTTTACCGTTCAGAAGTATAATAGAAATAATCTT
TAGTTGCTCTTTTCTAACCATTGTAATTCTTCCCTTCTTCCCTCCACCTTTCCTTCATTGAATAAACCTC
TGTTCAAAGAGATTGCCTGCAAGGGAAATAAAAATGACTAAGATATTAAAAAAAAAAAAAAAAAA
NCBI Reference Sequence: NM_212475.1
   **Homo sapiens fibronectin 1 (FN1), transcript variant 2, mRNA**
   >gi|47132550|ref|NM_212475.1| Homo sapiens fibronectin 1 (FN1), transcript variant 2, mRNA 
GCCCGCGCCGGCTGTGCTGCACAGGGGAGGAGAGGGAACCCAGGCGCGAGCGGAAGAGGGGACCTGC
AGCCACAACTTCTCTGGTCCTCTGCATCCCTTCTGTCCTCCACCCGTCCCCTTCCCCACCCTCTGGCCC
CCACCTTCTTGGAGGCGACAACCCCCGGGAGGCATTAGAAGGGATTTTTCCCGCAGGTTGCGAAGGGAAG
CAAACTTGGTGGCAACTTGCCTCCCGGTGCGGGCGTCTCTCCCCCACCGTCTCAACATGCTTAGGGGTCC
GGGGCCCGGGCTGCTGCTGCTGGCCGTCCAGTGCCTGGGGACAGCGGTGCCCTCCACGGGAGCCTCGAAG
AGCAAGAGGCAGGCTCAGCAAATGGTTCAGCCCCAGTCCCCGGTGGCTGTCAGTCAAAGCAAGCCCGGTT
GTTATGACAATGGAAAACACTATCAGATAAATCAACAGTGGGAGCGGACCTACCTAGGCAATGCGTTGGT
TTGTACTTGTTATGGAGGAAGCCGAGGTTTTAACTGCGAGAGTAAACCTGAAGCTGAAGAGACTTGCTTT
GACAAGTACACTGGGAACACTTACCGAGTGGGTGACACTTATGAGCGTCCTAAAGACTCCATGATCTGGG
ACTGTACCTGCATCGGGGCTGGGCGAGGGAGAATAAGCTGTACCATCGCAAACCGCTGCCATGAAGGGGG
TCAGTCCTACAAGATTGGTGACACCTGGAGGAGACCACATGAGACTGGTGGTTACATGTTAGAGTGTGTG
TGTCTTGGTAATGGAAAAGGAGAATGGACCTGCAAGCCCATAGCTGAGAAGTGTTTTGATCATGCTGCTG
GGACTTCCTATGTGGTCGGAGAAACGTGGGAGAAGCCCTACCAAGGCTGGATGATGGTAGATTGTACTTG
CCTGGGAGAAGGCAGCGGACGCATCACTTGCACTTCTAGAAATAGATGCAACGATCAGGACACAAGGACA
TCCTATAGAATTGGAGACACCTGGAGCAAGAAGGATAATCGAGGAAACCTGCTCCAGTGCATCTGCACAG
GCAACGGCCGAGGAGAGTGGAAGTGTGAGAGGCACACCTCTGTGCAGACCACATCGAGCGGATCTGGCCC
CTTCACCGATGTTCGTGCAGCTGTTTACCAACCGCAGCCTCACCCCCAGCCTCCTCCCTATGGCCACTGT
GTCACAGACAGTGGTGTGGTCTACTCTGTGGGGATGCAGTGGCTGAAGACACAAGGAAATAAGCAAATGC
TTTGCACGTGCCTGGGCAACGGAGTCAGCTGCCAAGAGACAGCTGTAACCCAGACTTACGGTGGCAACTC
AAATGGAGAGCCATGTGTCTTACCATTCACCTACAATGGCAGGACGTTCTACTCCTGCACCACAGAAGGG
CGACAGGACGGACATCTTTGGTGCAGCACAACTTCGAATTATGAGCAGGACCAGAAATACTCTTTCTGCA
CAGACCACACTGTTTTGGTTCAGACTCGAGGAGGAAATTCCAATGGTGCCTTGTGCCACTTCCCCTTCCT
ATACAACAACCACAATTACACTGATTGCACTTCTGAGGGCAGAAGAGACAACATGAAGTGGTGTGGGACC
ACACAGAACTATGATGCCGACCAGAAGTTTGGGTTCTGCCCCATGGCTGCCCACGAGGAAATCTGCACAA
CCAATGAAGGGGTCATGTACCGCATTGGAGATCAGTGGGATAAGCAGCATGACATGGGTCACATGATGAG
GTGCACGTGTGTTGGGAATGGTCGTGGGGAATGGACATGCATTGCCTACTCGCAGCTTCGAGATCAGTGC
ATTGTTGATGACATCACTTACAATGTGAACGACACATTCCACAAGCGTCATGAAGAGGGGCACATGCTGA
 
ACTGTACATGCTTCGGTCAGGGTCGGGGCAGGTGGAAGTGTGATCCCGTCGACCAATGCCAGGATTCAGA
GACTGGGACGTTTTATCAAATTGGAGATTCATGGGAGAAGTATGTGCATGGTGTCAGATACCAGTGCTAC
TGCTATGGCCGTGGCATTGGGGAGTGGCATTGCCAACCTTTACAGACCTATCCAAGCTCAAGTGGTCCTG
TCGAAGTATTTATCACTGAGACTCCGAGTCAGCCCAACTCCCACCCCATCCAGTGGAATGCACCACAGCC
ATCTCACATTTCCAAGTACATTCTCAGGTGGAGACCTAAAAATTCTGTAGGCCGTTGGAAGGAAGCTACC
ATACCAGGCCACTTAAACTCCTACACCATCAAAGGCCTGAAGCCTGGTGTGGTATACGAGGGCCAGCTCA
TCAGCATCCAGCAGTACGGCCACCAAGAAGTGACTCGCTTTGACTTCACCACCACCAGCACCAGCACACC
TGTGACCAGCAACACCGTGACAGGAGAGACGACTCCCTTTTCTCCTCTTGTGGCCACTTCTGAATCTGTG
ACCGAAATCACAGCCAGTAGCTTTGTGGTCTCCTGGGTCTCAGCTTCCGACACCGTGTCGGGATTCCGGG
TGGAATATGAGCTGAGTGAGGAGGGAGATGAGCCACAGTACCTGGATCTTCCAAGCACAGCCACTTCTGT
GAACATCCCTGACCTGCTTCCTGGCCGAAAATACATTGTAAATGTCTATCAGATATCTGAGGATGGGGAG
CAGAGTTTGATCCTGTCTACTTCACAAACAACAGCGCCTGATGCCCCTCCTGACCCGACTGTGGACCAAG
TTGATGACACCTCAATTGTTGTTCGCTGGAGCAGACCCCAGGCTCCCATCACAGGGTACAGAATAGTCTA
TTCGCCATCAGTAGAAGGTAGCAGCACAGAACTCAACCTTCCTGAAACTGCAAACTCCGTCACCCTCAGT
GACTTGCAACCTGGTGTTCAGTATAACATCACTATCTATGCTGTGGAAGAAAATCAAGAAAGTACACCTG
TTGTCATTCAACAAGAAACCACTGGCACCCCACGCTCAGATACAGTGCCCTCTCCCAGGGACCTGCAGTT
TGTGGAAGTGACAGACGTGAAGGTCACCATCATGTGGACACCGCCTGAGAGTGCAGTGACCGGCTACCGT
GTGGATGTGATCCCCGTCAACCTGCCTGGCGAGCACGGGCAGAGGCTGCCCATCAGCAGGAACACCTTTG
CAGAAGTCACCGGGCTGTCCCCTGGGGTCACCTATTACTTCAAAGTCTTTGCAGTGAGCCATGGGAGGGA
GAGCAAGCCTCTGACTGCTCAACAGACAACCAAACTGGATGCTCCCACTAACCTCCAGTTTGTCAATGAA
ACTGATTCTACTGTCCTGGTGAGATGGACTCCACCTCGGGCCCAGATAACAGGATACCGACTGACCGTGG
GCCTTACCCGAAGAGGACAGCCCAGGCAGTACAATGTGGGTCCCTCTGTCTCCAAGTACCCACTGAGGAA
TCTGCAGCCTGCATCTGAGTACACCGTATCCCTCGTGGCCATAAAGGGCAACCAAGAGAGCCCCAAAGCC
ACTGGAGTCTTTACCACACTGCAGCCTGGGAGCTCTATTCCACCTTACAACACCGAGGTGACTGAGACCA
CCATTGTGATCACATGGACGCCTGCTCCAAGAATTGGTTTTAAGCTGGGTGTACGACCAAGCCAGGGAGG
AGAGGCACCACGAGAAGTGACTTCAGACTCAGGAAGCATCGTTGTGTCCGGCTTGACTCCAGGAGTAGAA
TACGTCTACACCATCCAAGTCCTGAGAGATGGACAGGAAAGAGATGCGCCAATTGTAAACAAAGTGGTGA
CACCATTGTCTCCACCAACAAACTTGCATCTGGAGGCAAACCCTGACACTGGAGTGCTCACAGTCTCCTG
GGAGAGGAGCACCACCCCAGACATTACTGGTTATAGAATTACCACAACCCCTACAAACGGCCAGCAGGGA
AATTCTTTGGAAGAAGTGGTCCATGCTGATCAGAGCTCCTGCACTTTTGATAACCTGAGTCCCGGCCTGG
AGTACAATGTCAGTGTTTACACTGTCAAGGATGACAAGGAAAGTGTCCCTATCTCTGATACCATCATCCC
AGAGGTGCCCCAACTCACTGACCTAAGCTTTGTTGATATAACCGATTCAAGCATCGGCCTGAGGTGGACC
CCGCTAAACTCTTCCACCATTATTGGGTACCGCATCACAGTAGTTGCGGCAGGAGAAGGTATCCCTATTT
TTGAAGATTTTGTGGACTCCTCAGTAGGATACTACACAGTCACAGGGCTGGAGCCGGGCATTGACTATGA
TATCAGCGTTATCACTCTCATTAATGGCGGCGAGAGTGCCCCTACTACACTGACACAACAAACGGCTGTT
CCTCCTCCCACTGACCTGCGATTCACCAACATTGGTCCAGACACCATGCGTGTCACCTGGGCTCCACCCC
CATCCATTGATTTAACCAACTTCCTGGTGCGTTACTCACCTGTGAAAAATGAGGAAGATGTTGCAGAGTT
GTCAATTTCTCCTTCAGACAATGCAGTGGTCTTAACAAATCTCCTGCCTGGTACAGAATATGTAGTGAGT
GTCTCCAGTGTCTACGAACAACATGAGAGCACACCTCTTAGAGGAAGACAGAAAACAGGTCTTGATTCCC
CAACTGGCATTGACTTTTCTGATATTACTGCCAACTCTTTTACTGTGCACTGGATTGCTCCTCGAGCCAC
CATCACTGGCTACAGGATCCGCCATCATCCCGAGCACTTCAGTGGGAGACCTCGAGAAGATCGGGTGCCC
CACTCTCGGAATTCCATCACCCTCACCAACCTCACTCCAGGCACAGAGTATGTGGTCAGCATCGTTGCTC
TTAATGGCAGAGAGGAAAGTCCCTTATTGATTGGCCAACAATCAACAGTTTCTGATGTTCCGAGGGACCT
GGAAGTTGTTGCTGCGACCCCCACCAGCCTACTGATCAGCTGGGATGCTCCTGCTGTCACAGTGAGATAT
TACAGGATCACTTACGGAGAGACAGGAGGAAATAGCCCTGTCCAGGAGTTCACTGTGCCTGGGAGCAAGT
CTACAGCTACCATCAGCGGCCTTAAACCTGGAGTTGATTATACCATCACTGTGTATGCTGTCACTGGCCG
TGGAGACAGCCCCGCAAGCAGCAAGCCAATTTCCATTAATTACCGAACAGAAATTGACAAACCATCCCAG
ATGCAAGTGACCGATGTTCAGGACAACAGCATTAGTGTCAAGTGGCTGCCTTCAAGTTCCCCTGTTACTG
GTTACAGAGTAACCACCACTCCCAAAAATGGACCAGGACCAACAAAAACTAAAACTGCAGGTCCAGATCA
AACAGAAATGACTATTGAAGGCTTGCAGCCCACAGTGGAGTATGTGGTTAGTGTCTATGCTCAGAATCCA
AGCGGAGAGAGTCAGCCTCTGGTTCAGACTGCAGTAACCAACATTGATCGCCCTAAAGGACTGGCATTCA
CTGATGTGGATGTCGATTCCATCAAAATTGCTTGGGAAAGCCCACAGGGGCAAGTTTCCAGGTACAGGGT
GACCTACTCGAGCCCTGAGGATGGAATCCATGAGCTATTCCCTGCACCTGATGGTGAAGAAGACACTGCA
GAGCTGCAAGGCCTCAGACCGGGTTCTGAGTACACAGTCAGTGTGGTTGCCTTGCACGATGATATGGAGA
GCCAGCCCCTGATTGGAACCCAGTCCACAGCTATTCCTGCACCAACTGACCTGAAGTTCACTCAGGTCAC
ACCCACAAGCCTGAGCGCCCAGTGGACACCACCCAATGTTCAGCTCACTGGATATCGAGTGCGGGTGACC
 
CCCAAGGAGAAGACCGGACCAATGAAAGAAATCAACCTTGCTCCTGACAGCTCATCCGTGGTTGTATCAG
GACTTATGGTGGCCACCAAATATGAAGTGAGTGTCTATGCTCTTAAGGACACTTTGACAAGCAGACCAGC
TCAGGGAGTTGTCACCACTCTGGAGAATGTCAGCCCACCAAGAAGGGCTCGTGTGACAGATGCTACTGAG
ACCACCATCACCATTAGCTGGAGAACCAAGACTGAGACGATCACTGGCTTCCAAGTTGATGCCGTTCCAG
CCAATGGCCAGACTCCAATCCAGAGAACCATCAAGCCAGATGTCAGAAGCTACACCATCACAGGTTTACA
ACCAGGCACTGACTACAAGATCTACCTGTACACCTTGAATGACAATGCTCGGAGCTCCCCTGTGGTCATC
GACGCCTCCACTGCCATTGATGCACCATCCAACCTGCGTTTCCTGGCCACCACACCCAATTCCTTGCTGG
TATCATGGCAGCCGCCACGTGCCAGGATTACCGGCTACATCATCAAGTATGAGAAGCCTGGGTCTCCTCC
CAGAGAAGTGGTCCCTCGGCCCCGCCCTGGTGTCACAGAGGCTACTATTACTGGCCTGGAACCGGGAACC
GAATATACAATTTATGTCATTGCCCTGAAGAATAATCAGAAGAGCGAGCCCCTGATTGGAAGGAAAAAGA
CAGTTCAAAAGACCCCTTTCGTCACCCACCCTGGGTATGACACTGGAAATGGTATTCAGCTTCCTGGCAC
TTCTGGTCAGCAACCCAGTGTTGGGCAACAAATGATCTTTGAGGAACATGGTTTAGGCGGACCACACCG
CCCACAACGGCCACCCCCATAAGGCATAGGCCAAGACCATACCCGCCGAATGTAGGACAAGAAGCTCTCT
CTCAGACAACCATCTCATGGCCCCATTCCAGGACACTTCTGAGTACATCATTTCATGTCATCCTGTTGG
CACTGATGAAGAACCCTTACAGTTCAGGGTTCCTGGAACTTCTACCAGTGCCACTCTGACAGGCCTCACC
AGAGGTGCCACCTACAACATCATAGTGGAGGCACTGAAAGACCAGCAGAGGCATAAGGTTCGGGAAGAGG
TTGTTACCGTGGGCAACTCTGTCAACGAAGGCTTGAACCAACCTACGGATGACTCGTGCTTTGACCCCTA
CACAGTTTCCCATTATGCCGTTGGAGATGAGTGGAACGAATGTCTGAATCAGGCTTTAAACTGTTGTGC
CAGTGCTTAGGCTTTGGAAGTGGTCATTTCAGATGTGATTCATCTAGATGGTGCCATGACAATGGTGTGA
ACTACAAGATTGGAGAGAAGTGGGACCGTCAGGGAGAAAATGGCCAGATGATGAGCTGCACATGTCTTGG
GAACGGAAAAGGAGAATTCAAGTGTGACCCTCATGAGGCAACGTGTTATGATGATGGAAGACATACCAC
GTAGGAGAACAGTGGCAGAAGGAATATCTCGGTGCCATTTGCTCCTGCACATGCTTTGGAGGCCAGCGGG
GCTGGCGCTGTGACAACTGCCGCAGACCTGGGGGTGAACCCAGTCCCGAAGGCACTACTGGCCAGTCCTA
CAACCAGTATTCTCAGAGATACCATCAGAGAACAAACACTAATGTTAATTGCCCAATTGAGTGCTTCATG
CCTTTAGATGTACAGGCTGACAGAGAAGATTCCCGAGAGTAAATCATCTTTCCAATCCAGAGGAACAAGC
ATGTCTCTCTGCCAAGATCCATCTAAACTGGAGTGATGTTAGCAGACCCAGCTTAGAGTTCTTCTTTCTT
TCTTAAGCCCTTTGCTCTGGAGGAAGTTCTCCAGCTTCAGCTCAACTCACAGCTTCTCCAAGCATCACCC
TGGGAGTTTCCTGAGGGTTTTCTCATAAATGAGGGCTGCACATTGCCTGTTCTGCTTCGAAGTATTCAAT
ACCGCTCAGTATTTTAAATGAAGTGATTCTAAGATTTGGTTTGGGATCAATAGGAAAGCATATGCAGCCA
ACCAAGATGCAAATGTTTTGAAATGATATGACCAAAATTTTAAGTAGGAAAGTCACCCAAACACTTCTGC
TTTCACTTAAGTGTCTGGCCCGCAATACTGTAGGAACAAGCATGATCTTGTTACTGTGATATTTTAAATA
TCCACAGTACTCACTTTTTCCAAATGATCCTAGTAATTGCCTAGAAATATCTTTCTCTTACCTGTTATTT
ATCAATTTTTCCCAGTATTTTTATACGGAAAAAATTGTATTGAAAACACTTAGTATGCAGTTGATAAGAG
GAATTTGGTATAATTATGGTGGGTGATTATTTTTTATACTGTATGTGCCAAAGCTTTACTACTGTGGAAA
GACAACTGTTTTAATAAAAGATTTACATTCCACAACTTGAAGTTCATCTATTTGATATAAGACACCTTCG
GGGGAAATAATTCCTGTGAATATTCTTTTTCAATTCAGCAAACATTTGAAAATCTATGATGTGCAAGTCT
AATTGTTGATTTCAGTACAAGATTTTCTAAATCAGTTGCTACAAAAACTGATTGGTTTTTGTCACTTCAT
CTCTTCACTAATGGAGATAGCTTTACACTTTCTGCTTTAATAGATTTAAGTGGACCCCAATATTTATTAA
AATTGCTAGTTTACCGTTCAGAAGTATAATAGAAATAATCTTTAGTTGCTCTTTTCTAACCATTGTAATT
CTTCCCTTCTTCCCTCCACCTTTCCTTCATTGAATAAACCTCTGTTCAAAGAGATTGCCTGCAAGGGAAA
TAAAAATGACTAAGATATTAAAAAAAAAAAAAAAAA
NCBI Reference Sequence: NM_054034.2
   **Homo sapiens fibronectin 1 (FN1), transcript variant 7, mRNA**
   >gi|47132546|ref|NM_054034.2| Homo sapiens fibronectin 1 (FN1), transcript variant 7, mRNA 
GCCCGCGCCGGCTGTGCTGCACAGGGGGAGGAGAGGGAACCCCAGGCGCGAGCGGGAAGAGGGGACCTGC
AGCCACAACTTCTCTGGTCCTCTGCATCCCTTCTGTCCTCCACCCGTCCCCTTCCCCACCCTCTGGCCC
CCACCTTCTTGGAGGCGACAACCCCCGGGAGGCATTAGAAGGGATTTTTCCCGCAGGTTGCGAAGGGAAG
CAAACTTGGTGGCAACTTGCCTCCCGGTGCGGGCGTCTCTCCCCCACCGTCTCAACATGCTTAGGGGTCC
GGGGCCCGGGCTGCTGCTGCTGGCCGTCCAGTGCCTGGGGACAGCGGTGCCCTCCACGGGAGCCTCGAAG
AGCAAGAGGCAGGCTCAGCAAATGGTTCAGCCCCAGTCCCCGGTGGCTGTCAGTCAAAGCAAGCCCGGTT
GTTATGACAATGGAAAACACTATCAGATAAATCAACAGTGGGAGCGGACCTACCTAGGCAATGCGTTGGT
 
TTGTACTTGTTATGGAGGAAGCCGAGGTTTTAACTGCGAGAGTAAACCTGAAGCTGAAGAGACTTGCTTT
GACAAGTACACTGGGAACACTTACCGAGTGGGTGACACTTATGAGCGTCCTAAAGACTCCATGATCTGGG
ACTGTACCTGCATCGGGGCTGGGCGAGGGAGAATAAGCTGTACCATCGCAAACCGCTGCCATGAAGGGGG
TCAGTCCTACAAGATTGGTGACACCTGGAGGAGACCACATGAGACTGGTGGTTACATGTTAGAGTGTGTG
TGTCTTGGTAATGGAAAAGGAGAATGGACCTGCAAGCCCATAGCTGAGAAGTGTTTTGATCATGCTGCTG
GGACTTCCTATGTGGTCGGAGAAACGTGGGAGAAGCCCTACCAAGGCTGGATGATGGTAGATTGTACTTG
CCTGGGAGAAGGCAGCGGACGCATCACTTGCACTTCTAGAAATAGATGCAACGATCAGGACACAAGGACA
TCCTATAGAATTGGAGACACCTGGAGCAAGAAGGATAATCGAGGAAACCTGCTCCAGTGCATCTGCACAG
GCAACGGCCGAGGAGAGTGGAAGTGTGAGAGGCACACCTCTGTGCAGACCACATCGAGCGGATCTGGCCC
CTTCACCGATGTTCGTGCAGCTGTTTACCAACCGCAGCCTCACCCCCAGCCTCCTCCCTATGGCCACTGT
GTCACAGACAGTGGTGTGGTCTACTCTGTGGGGATGCAGTGGCTGAAGACACAAGGAAATAAGCAAATGC
TTTGCACGTGCCTGGGCAACGGAGTCAGCTGCCAAGAGACAGCTGTAACCCAGACTTACGGTGGCAACTC
AAATGGAGAGCCATGTGTCTTACCATTCACCTACAATGGCAGGACGTTCTACTCCTGCACCACAGAAGGG
CGACAGGACGGACATCTTTGGTGCAGCACAACTTCGAATTATGAGCAGGACCAGAAATACTCTTTCTGCA
CAGACCACACTGTTTTGGTTCAGACTCGAGGAGGAAATTCCAATGGTGCCTTGTGCCACTTCCCCTTCCT
ATACAACAACCACAATTACACTGATTGCACTTCTGAGGGCAGAAGAGACAACATGAAGTGGTGTGGGACC
ACACAGAACTATGATGCCGACCAGAAGTTTGGGTTCTGCCCCATGGCTGCCCACGAGGAAATCTGCACAA
CCAATGAAGGGGTCATGTACCGCATTGGAGATCAGTGGGATAAGCAGCATGACATGGGTCACATGATGAG
GTGCACGTGTGTTGGGAATGGTCGTGGGGAATGGACATGCATTGCCTACTCGCAGCTTCGAGATCAGTGC
ATTGTTGATGACATCACTTACAATGTGAACGACACATTCCACAAGCGTCATGAAGAGGGGCACATGCTGA
ACTGTACATGCTTCGGTCAGGGTCGGGGCAGGTGGAAGTGTGATCCCGTCGACCAATGCCAGGATTCAGA
GACTGGGACGTTTTATCAAATTGGAGATTCATGGGAGAAGTATGTGCATGGTGTCAGATACCAGTGCTAC
TGCTATGGCCGTGGCATTGGGGAGTGGCATTGCCAACCTTTACAGACCTATCCAAGCTCAAGTGGTCCTG
TCGAAGTATTTATCACTGAGACTCCGAGTCAGCCCAACTCCCACCCCATCCAGTGGAATGCACCACAGCC
ATCTCACATTTCCAAGTACATTCTCAGGTGGAGACCTGTGAGTATCCCACCCAGAAACCTTGGATACTGA
GTCTCCTAATCTTATCAATTCTGATGGTTTCTTTTTTTCCCAGCTTTTGAGCCAACAACTCTGATTAACT
ATTCCTATAGCATTTACTATATTTGTTTAGTGAACAAACAATATGTGGTCAATTAAATTGACTTGTAGAC
TGAAAAAAAAAAAAAAAAAAA
NCBI Reference Sequence: NM_002318.2
   **Homo sapiens lysyl oxidase-like 2 (LOXL2), mRNA**
   >gi|67782347|ref|NM_002318.2| Homo sapiens lysyl oxidase-like 2 (LOXL2), mRNA 
GCGGCGGCCGCCTTCGGGAAGGGGGTGGTCCGGGGAGGGGTTTGCCATCCTCCTCTAGTTAAAAGTAA
GGGGGAAAAGAGTAAACGCGCGACTCCAGCGCGCGGCTACCTACGCTTGGTGCTTGCTTTCTCCAGCCAT
CGGAGACCAGAGCCGCCCCCTCTGCTCGAGAAAGGGGCTCAGCGGCGGCGGAAGCGGAGGGGGACCACCG
TGGAGAGCGCGGTCCCAGCCCGGCCACTGCGGATCCCTGAAACCAAAAAGCTCCTGCTGCTTCTGTACCC
CGCCTGTCCCTCCCAGCTGCGCAGGGCCCCTTCGTGGGATCATCAGCCCGAAGACAGGGATGGAGAGGCC
TCTGTGCTCCCACCTCTGCAGCTGCCTGGCTATGCTGGCCCTCCTGTCCCCCCTGAGCCTGGCACAGTAT
GACAGCTGGCCCCATTACCCCGAGTACTTCCAGCAACCGGCTCCTGAGTATCACCAGCCCCAGGCCCCCG
CCAACGTGGCCAAGATTCAGCTGCGCCTGGCTGGGCAGAAGAGGAAGCACAGCGAGGGCCGGGTGGAGGT
GTACTATGATGGCCAGTGGGGCACCGTGTGCGATGACGACTTCTCCATCCACGCTGCCCACGTCGTCTGC
CGGGAGCTGGGCTACGTGGAGGCCAAGTCCTGGACTGCCAGCTCCTCCTACGGCAAGGGAGAAGGCCCCA
TCTGGTTAGACAATCTCCACTGTACTGGCAACGAGGCGACCCTTGCAGCATGCACCTCCAATGGCTGGGG
CGTCACTGACTGCAAGCACACGGAGGATGTCGGTGTGGTGTGCAGCGACAAAAGGATTCCTGGGTTCAAA
TTTGACAATTCGTTGATCAACCAGATAGAGAACCTGAATATCCAGGTGGAGGACATTCGGATTCGAGCCA
TCCTCTCAACCTACCGCAAGCGCACCCCAGTGATGGAGGGCTACGTGGAGGTGAAGGAGGGCAAGACCTG
GAAGCAGATCTGTGACAAGCACTGGACGGCCAAGAATTCCCGCGTGGTCTGCGGCATGTTTGGCTTCCCT
GGGGAGAGGACATACAATACCAAAGTGTACAAAATGTTTGCCTCACGGAGGAAGCAGCGCTACTGGCCAT
TCTCCATGGACTGCACCGGCACAGAGGCCCACATCTCCAGCTGCAAGCTGGGCCCCCAGGTGTCACTGGA
CCCCATGAAGAATGTCACCTGCGAGAATGGGCTACCGGCCGTGGTGAGTTGTGTGCCTGGGCAGGTCTTC
AGCCCTGACGGACCCTCAAGATTCCGGAAAGCGTACAAGCCAGAGCAACCCCTGGTGCGACTGAGAGGCG
 
GTGCCTACATCGGGGAGGGCCGCGTGGAGGTGCTCAAAAATGGAGAATGGGGGACCGTCTGCGACGACAA
GTGGGACCTGGTGTCGGCCAGTGTGGTCTGCAGAGAGCTGGGCTTTGGGAGTGCCAAAGAGGCAGTCACT
GGCTCCCGACTGGGGCAAGGGATCGGACCCATCCACCTCAACGAGATCCAGTGCACAGGCAATGAGAAGT
CCATTATAGACTGCAAGTTCAATGCCGAGTCTCAGGGCTGCAACCACGAGGAGGATGCTGGTGTGAGATG
CAACACCCCTGCCATGGGCTTGCAGAAGAAGCTGCGCCTGAACGGCGGCCGCAATCCCTACGAGGGCCGA
GTGGAGGTGCTGGTGGAGAGAAACGGGTCCCTTGTGTGGGGGATGGTGTGTGGCCAAAACTGGGGCATCG
TGGAGGCCATGGTGGTCTGCCGCCAGCTGGGCCTGGGATTCGCCAGCAACGCCTTCCAGGAGACCTGGTA
TTGGCACGGAGATGTCAACAGCAACAAAGTGGTCATGAGTGGAGTGAAGTGCTCGGGAACGGAGCTGTCC
CTGGCGCACTGCCGCCACGACGGGGAGGACGTGGCCTGCCCCCAGGGCGGAGTGCAGTACGGGGCCGGAG
TTGCCTGCTCAGAAACCGCCCCTGACCTGGTCCTCAATGCGGAGATGGTGCAGCAGACCACCTACCTGGA
GGACCGGCCCATGTTCATGCTGCAGTGTGCCATGGAGGAGAACTGCCTCTCGGCCTCAGCCGCGCAGACC
GACCCCACCACGGGCTACCGCCGGCTCCTGCGCTTCTCCTCCCAGATCCACAACAATGGCCAGTCCGACT
TCCGGCCCAAGAACGGCCGCCACGCGTGGATCTGGCACGACTGTCACAGGCACTACCACAGCATGGAGGT
GTTCACCCACTATGACCTGCTGAACCTCAATGGCACCAAGGTGGCAGAGGGCCACAAGGCCAGCTTCTGC
TTGGAGGACACAGAATGTGAAGGAGACATCCAGAAGAATTACGAGTGTGCCAACTTCGGCGATCAGGGCA
TCACCATGGGCTGCTGGGACATGTACCGCCATGACATCGACTGCCAGTGGGTTGACATCACTGACGTGCC
CCCTGGAGACTACCTGTTCCAGGTTGTTATTAACCCCAACTTCGAGGTTGCAGAATCCGATTACTCCAAC
AACATCATGAAATGCAGGAGCCGCTATGACGGCCACCGCATCTGGATGTACAACTGCCACATAGGTGGTT
CCTTCAGCGAAGAGACGGAAAAAAAGTTTGAGCACTTCAGCGGGCTCTTAAACAACCAGCTGTCCCCGCA
GTAAAGAAGCCTGCGTGGTCAACTCCTGTCTTCAGGCCACACCACATCTTCCATGGGACTTCCCCCCAAC
AACTGAGTCTGAACGAATGCCACGTGCCCTCACCCAGCCCGGCCCCCACCCTGTCCAGACCCCTACAGCT
GTGTCTAAGCTCAGGAGGAAAGGGACCCTCCCATCATTCATGGGGGGCTGCTACCTGACCCTTGGGGCCT
GAGAAGGCCTTGCGGGGGTGGGGTTTGTCCACAGAGCTGCTGGAGCAGCACCAAGAGCCAGTCTTGACCG
GGATGAGGCCCACAGACAGGTTGTCATCAGCTTGTCCCATTCAAGCCACCGAGCTCACCACAGACACAGT
GGAGCCGCGCTCTTCTCCAGTGACACGTGGACAAATGCGGGCTCATCAGCCCCCCCAGAGAGGGTCAGGC
CGAACCCCATTTCTCCTCCTCTTAGGTCATTTTCAGCAAACTTGAATATCTAGACCTCTCTTCCAATGAA
ACCCTCCAGTCTATTATAGTCACATAGATAATGGTGCCACGTGTTTTCTGATTTGGTGAGCTCAGACTTG
GTGCTTCCCTATCCACAGCCCCCACCCCTTGTTTTTCAAGATACTATTATTATATTTTCACAGACTTTTG
AAGCACAAATTTATTGGCATTTAATATTGGACATCTGGGCCCTTGGAAGTACAAATCTAAGGAAAAACCA
ACCCACTGTGTAAGTGACTCATCTTCCTGTTGTTCCAATTCTGTGGGTTTTTGATTCAACGGTGCTATAA
CCAGGGTCCTGGGTGACAGGGCGCTCACTGAGCACCATGTGTCATCACAGACACTTACACATACTTGAAA
CTTGGAATAAAAGAAAGATTTATGAAACGTGTCTGTGTTTCCTTTGACCCACAGCACCTGGGCCCTGAGC
AGCAGGCTTCCTATGTTCAGTGGCCAGAAGCAGAGCTTCAGGTACATTCGTGGTTTTCTCCGGTGGACAT
GGGTCCTCAGATCCCCTCCAGCCCAGTGTGGCCACCAGGGCACCTCCTTCAATAGACTCCAAAAGGGGCA
GCTCCTACCATCTGGGAGAAGCAATCTAAGGAGATCACAAAAAGTAACGGAACAGGAGTCATAATCTTTC
TTGAACTCCTGTGGTTTTTACTGAAACTTG
NCBI Reference Sequence: NM_000090.3
   **Homo sapiens collagen, type III, alpha 1 (COL3A1), mRNA**
   >gi|110224482|ref|NM_000090.3| Homo sapiens collagen, type III, alpha 1 (COL3A1), mRNA 
GGCTGAGTTTTATGACGGGCCCGGTGCTGAAGGGCAGGGAACAACTTGATGGTGCTACTTTGAACTGCTT
TTCTTTTCTCCTTTTTGCACAAAGAGTCTCATGTCTGATATTTAGACATGATGAGCTTTGTGCAAAAGGG
GAGCTGGCTACTTCTCGCTCTGCTTCATCCCACTATTATTTGGCACAACAGGAAGCTGTTGAAGGAGGA
TGTTCCCATCTTGGTCAGTCCTATGCGGATAGAGATGTCTGGAAGCCAGAACCATGCCAAATATGTGTCT
GTGACTCAGGATCCGTTCTCTGCGATGACATAATATGTGACGATCAAGAATTAGACTGCCCCAACCCAGA
AATTCCATTTGGAGAATGTTGTGCAGTTTGCCCACAGCCTCCAACTGCTCCTACTCGCCCTCCTAATGGT
CAAGGACCTCAAGGCCCCAAGGGAGATCCAGGCCCTCCTGGTATTCCTGGGAGAAATGGTGACCCTGGTA
TTCCAGGACAACCAGGGTCCCCTGGTTCTCCTGGCCCCCCTGGAATCTGTGAATCATGCCCTACTGGTCC
TCAGAACTATTCTCCCCAGTATGATTCATATGATGTCAAGTCTGGAGTAGCAGTAGGAGGACTCGCAGGC
TATCCTGGACCAGCTGGCCCCCCAGGCCCTCCCGGTCCCCCTGGTACATCTGGTCATCCTGGTTCCCCTG
GATCTCCAGGATACCAAGGACCCCCTGGTGAACCTGGGCAAGCTGGTCCTTCAGGCCCTCCAGGACCTCC
 
TGGTGCTATAGGTCCATCTGGTCCTGCTGGAAAAGATGGAGAATCAGGTAGACCCGGACGACCTGGAGAG
CGAGGATTGCCTGGACCTCCAGGTATCAAAGGTCCAGCTGGGATACCTGGATTCCCTGGTATGAAAGGAC
ACAGAGGCTTCGATGGACGAAATGGAGAAAAGGGTGAAACAGGTGCTCCTGGATTAAAGGGTGAAAATGG
TCTTCCAGGCGAAAATGGAGCTCCTGGACCCATGGGTCCAAGAGGGGCTCCTGGTGAGCGAGGACGGCCA
GGACTTCCTGGGGCTGCAGGTGCTCGGGGTAATGACGGTGCTCGAGGCAGTGATGGTCAACCAGGCCCTC
CTGGTCCTCCTGGAACTGCCGGATTCCCTGGATCCCCTGGTGCTAAGGGTGAAGTTGGACCTGCAGGGTC
TCCTGGTTCAAATGGTGCCCCTGGACAAAGAGGAGAACCTGGACCTCAGGGACACGCTGGTGCTCAAGGT
CCTCCTGGCCCTCCTGGGATTAATGGTAGTCCTGGTGGTAAAGGCGAAATGGGTCCCGCTGGCATTCCTG
GAGCTCCTGGACTGATGGGAGCCCGGGGTCCTCCAGGACCAGCCGGTGCTAATGGTGCTCCTGGACTGCG
AGGTGGTGCAGGTGAGCCTGGTAAGAATGGTGCCAAAGGAGAGCCCGGACCACGTGGTGAACGCGGTGAG
GCTGGTATTCCAGGTGTTCCAGGAGCTAAAGGCGAAGATGGCAAGGATGGATCACCTGGAGAACCTGGTG
CAAATGGGCTTCCAGGAGCTGCAGGAGAAAGGGGTGCCCCTGGGTTCCGAGGACCTGCTGGACCAAATGG
CATCCCAGGAGAAAAGGGTCCTGCTGGAGAGCGTGGTGCTCCAGGCCCTGCAGGGCCCAGAGGAGCTGCT
GGAGAACCTGGCAGAGATGGCGTCCCTGGAGGTCCAGGAATGAGGGGCATGCCCGGAAGTCCAGGAGGAC
CAGGAAGTGATGGGAAACCAGGGCCTCCCGGAAGTCAAGGAGAAAGTGGTCGACCAGGTCCTCCTGGGCC
ATCTGGTCCCCGAGGTCAGCCTGGTGTCATGGGCTTCCCCGGTCCTAAAGGAAATGATGGTGCTCCTGGT
AAGAATGGAGAACGAGGTGGCCCTGGAGGACCTGGCCCTCAGGGTCCTCCTGGAAAGAATGGTGAAACTG
GACCTCAGGGACCCCCAGGGCCTACTGGGCCTGGTGGTGACAAAGGAGACACAGGACCCCCTGGTCCACA
AGGATTACAAGGCTTGCCTGGTACAGGTGGTCCTCCAGGAGAAAATGGAAAACCTGGGGAACCAGGTCCA
AAGGGTGATGCCGGTGCACCTGGAGCTCCAGGAGGCAAGGGTGATGCTGGTGCCCCTGGTGAACGTGGAC
CTCCTGGATTGGCAGGGGCCCCAGGACTTAGAGGTGGAGCTGGTCCCCCTGGTCCCGAAGGAGGAAAGGG
TGCTGCTGGTCCTCCTGGGCCACCTGGTGCTGCTGGTACTCCTGGTCTGCAAGGAATGCCTGGAGAAAGA
GGAGGTCTTGGAAGTCCTGGTCCAAAGGGTGACAAGGGTGAACCAGGCGGTCCAGGTGCTGATGGTGTCC
CAGGGAAAGATGGCCCAAGGGGTCCTACTGGTCCTATTGGTCCTCCTGGCCCAGCTGGCCAGCCTGGAGA
TAAGGGTGAAGGTGGTGCCCCCGGACTTCCAGGTATAGCTGGACCTCGTGGTAGCCCTGGTGAGAGAGGT
GAAACTGGCCCTCCAGGACCTGCTGGTTTCCCTGGTGCTCCTGGACAGAATGGTGAACCTGGTGGTAAAG
GAGAAAGAGGGGCTCCGGGTGAGAAAGGTGAAGGAGGCCCTCCTGGAGTTGCAGGACCCCCTGGAGGTTC
TGGACCTGCTGGTCCTCCTGGTCCCCAAGGTGTCAAAGGTGAACGTGGCAGTCCTGGTGGACCTGGTGCT
GCTGGCTTCCCTGGTGCTCGTGGTCTTCCTGGTCCTCCTGGTAGTAATGGTAACCCAGGACCCCCAGGTC
CCAGCGGTTCTCCAGGCAAGGATGGGCCCCCAGGTCCTGCGGGTAACACTGGTGCTCCTGGCAGCCCTGG
AGTGTCTGGACCAAAAGGTGATGCTGGCCAACCAGGAGAGAAGGGATCGCCTGGTGCCCAGGGCCCACCA
GGAGCTCCAGGCCCACTTGGGATTGCTGGGATCACTGGAGCACGGGGTCTTGCAGGACCACCAGGCATGC
CAGGTCCTAGGGGAAGCCCTGGCCCTCAGGGTGTCAAGGGTGAAAGTGGGAAACCAGGAGCTAACGGTCT
CAGTGGAGAACGTGGTCCCCCTGGACCCCAGGGTCTTCCTGGTCTGGCTGGTACAGCTGGTGAACCTGGA
AGAGATGGAAACCCTGGATCAGATGGTCTTCCAGGCCGAGATGGATCTCCTGGTGGCAAGGGTGATCGTG
GTGAAAATGGCTCTCCTGGTGCCCCTGGCGCTCCTGGTCATCCAGGCCCACCTGGTCCTGTCGGTCCAGC
TGGAAAGAGTGGTGACAGAGGAGAAAGTGGCCCTGCTGGCCCTGCTGGTGCTCCCGGTCCTGCTGGTTCC
CGAGGTGCTCCTGGTCCTCAAGGCCCACGTGGTGACAAAGGTGAAACAGGTGAACGTGGAGCTGCTGGCA
TCAAAGGACATCGAGGATTCCCTGGTAATCCAGGTGCCCCAGGTTCTCCAGGCCCTGCTGGTCAGCAGGG
TGCAATCGGCAGTCCAGGACCTGCAGGCCCCAGAGGACCTGTTGGACCCAGTGGACCTCCTGGCAAAGAT
GGAACCAGTGGACATCCAGGTCCCATTGGACCACCAGGGCCTCGAGGTAACAGAGGTGAAAGAGGATCTG
AGGGCTCCCCAGGCCACCCAGGGCAACCAGGCCCTCCTGGACCTCCTGGTGCCCCTGGTCCTTGCTGTGG
TGGTGTTGGAGCCGCTGCCATTGCTGGGATTGGAGGTGAAAAAGCTGGCGGTTTTGCCCCGTATTATGGA
GATGAACCAATGGATTTCAAAATCAACACCGATGAGATTATGACTTCACTCAAGTCTGTTAATGGACAAA
TAGAAAGCCTCATTAGTCCTGATGGTTCTCGTAAAAACCCCGCTAGAAACTGCAGAGACCTGAAATTCTG
CCATCCTGAACTCAAGAGTGGAGAATACTGGGTTGACCCTAACCAAGGATGCAAATTGGATGCTATCAAG
GTATTCTGTAATATGGAAACTGGGGAAACATGCATAAGTGCCAATCCTTTGAATGTTCCACGGAAACACT
GGTGGACAGATTCTAGTGCTGAGAAGAAACACGTTTGGTTTGGAGAGTCCATGGATGGTGGTTTTCAGTT
TAGCTACGGCAATCCTGAACTTCCTGAAGATGTCCTTGATGTGCAGCTGGCATTCCTTCGACTTCTCTCC
AGCCGAGCTTCCCAGAACATCACATATCACTGCAAAAATAGCATTGCATACATGGATCAGGCCAGTGGAA
ATGTAAAGAAGGCCCTGAAGCTGATGGGGTCAAATGAAGGTGAATTCAAGGCTGAAGGAAATAGCAAATT
CACCTACACAGTTCTGGAGGATGGTTGCACGAAACACACTGGGGAATGGAGCAAAACAGTCTTTGAATAT
CGAACACGCAAGGCTGTGAGACTACCTATTGTAGATATTGCACCCTATGACATTGGTGGTCCTGATCAAG
AATTTGGTGTGGACGTTGGCCCTGTTTGCTTTTTATAAACCAAACTCTATCTGAAATCCCAACAAAAAAA
ATTTAACTCCATATGTGTTCCTCTTGTTCTAATCTTGTCAACCAGTGCAAGTGACCGACAAAATTCCAGT
TATTTATTTCCAAAATGTTTGGAAACAGTATAATTTGACAAAGAAAAATGATACTTCTCTTTTTTTGCTG
 
TTCCACCAAATACAATTCAAATGCTTTTGTTTATTTTTTACCAATTCCAATTTCAAAATGTCTCAAT
GGTGCTATAATAAATAAACTTCAACACTCTTTATGATAACAACACTGTGTTATATTCTTTGAATCCTAGC
CCATCTGCAGAGCAATGACTGTGCTCACCAGTAAAAGATAACCTTTCTTTCTGAAATAGTCAAATACGAA
ATTAGAAAAGCCCTCCCTATTTTAACTACCTCAACTGGTCAGAAACACAGATTGTATTCTATGAGTCCCA
GAAGATGAAAAAAATTTTATACGTTGATAAAACTTATAAATTTCATTGATTAATCTCCTGGAAGATTGGT
TTAAAAAGAAAAGTGTAATGCAAGAATTTAAAGAAATATTTTTAAAGCCACAATTATTTTAATATTGGAT
ATCAACTGCTTGTAAAGGTGCTCCTCTTTTTTCTTGTCATTGCTGGTCAAGATTACTAATATTTGGGAAG
GCTTTAAAGACGCATGTTATGGTGCTAATGTACTTTCACTTTTAAACTCTAGATCAGAATTGTTGACTTG
CATTCAGAACATAAATGCACAAAATCTGTACATGTCTCCCATCAGAAAGATTCATTGGCATGCCACAGGG
GATTCTCCTCCTTCATCCTGTAAAGGTCAACAATAAAAACCAAATTATGGGGCTGCTTTTGTCACACTAG
CATAGAGAATGTGTTGAAATTTAACTTTGTAAGCTTGTATGTGGTTGTTGATCTTTTTTTTCCTTACAGA
CACCCATAATAAAATATCATATTAAAATTC
NCBI Reference Sequence: NM_000088.3
   **Homo sapiens collagen, type I, alpha 1 (COL1A1), mRNA**
   >gi|110349771|ref|NM_000088.3| Homo sapiens collagen, type I, alpha 1 (COL1A1), mRNA 
TCGTCGGAGCAGACGGGAGTTTCTCCTCGGGGTCGGAGCAGGAGGCACGCGGAGTGTGAGGCCACGCATG
AGCGGACGCTAACCCCCTCCCCAGCCACAAAGAGTCTACATGTCTAGGGTCTAGACATGTTCAGCTTTGT
GGACCTCCGGCTCCTGCTCCTCTTAGCGGCCACCGCCCTCCTGACGCACGGCCAAGAGGAAGGCCAAGTC
GAGGGCCAAGACGAAGACATCCCACCAATCACCTGCGTACAGAACGGCCTCAGGTACCATGACCGAGACG
TGTGGAAACCCGAGCCCTGCCGGATCTGCGTCTGCGACAACGGCAAGGTGTTGTGCGATGACGTGATCTG
TGACGAGACCAAGAACTGCCCCGGCGCCGAAGTCCCCGAGGGCGAGTGCTGTCCCGTCTGCCCCGACGGC
TCAGAGTCACCCACCGACCAAGAAACCACCGGCGTCGAGGGACCCAAGGGAGACACTGGCCCCCGAGGCC
CAAGGGGACCCGCAGGCCCCCCTGGCCGAGATGGCATCCCTGGACAGCCTGGACTTCCCGGACCCCCCGG
ACCCCCCGGACCTCCCGGACCCCCTGGCCTCGGAGGAAACTTTGCTCCCCAGCTGTCTTATGGCTATGAT
GAGAAATCAACCGGAGGAATTTCCGTGCCTGGCCCCATGGGTCCCTCTGGTCCTCGTGGTCTCCCTGGCC
CCCCTGGTGCACCTGGTCCCCAAGGCTTCCAAGGTCCCCCTGGTGAGCCTGGCGAGCCTGGAGCTTCAGG
TCCCATGGGTCCCCGAGGTCCCCCAGGTCCCCCTGGAAAGAATGGAGATGATGGGGAAGCTGGAAAACCT
GGTCGTCCTGGTGAGCGTGGGCCTCCTGGGCCTCAGGGTGCTCGAGGATTGCCCGGAACAGCTGGCCTCC
CTGGAATGAAGGGACACAGAGGTTTCAGTGGTTTGGATGGTGCCAAGGGAGATGCTGGTCCTGCTGGTCC
TAAGGGTGAGCCTGGCAGCCCTGGTGAAAATGGAGCTCCTGGTCAGATGGGCCCCCGTGGCCTGCCTGGT
GAGAGAGGTCGCCCTGGAGCCCCTGGCCCTGCTGGTGCTCGTGGAAATGATGGTGCTACTGGTGCTGCCG
GGCCCCCTGGTCCCACCGGCCCCGCTGGTCCTCCTGGCTTCCCTGGTGCTGTTGGTGCTAAGGGTGAAGC
TGGTCCCCAAGGGCCCCGAGGCTCTGAAGGTCCCCAGGGTGTGCGTGGTGAGCCTGGCCCCCCTGGCCCT
GCTGGTGCTGCTGGCCCTGCTGGAAACCCTGGTGCTGATGGACAGCCTGGTGCTAAAGGTGCCAATGGTG
CTCCTCCTATTCCTCCTCCTCCTGGCTTCCCTGGTGCCCGAGGCCC[?][?][?]TGGACCCCAGGGCCCCGGCGG
CCCTCCTGGTCCCAAGGGTAACAGCGGTGAACCTGGTGCTCCTGGCAGCAAAGGAGACACTGGTGCTAAG
GGAGAGCCTGGCCCTGTTGGTGTTCAAGGACCCCCTGGCCCTGCTGGAGAGGAAGGAAAGCGAGGAGCTC
GAGGTGAACCCGGACCCACTGGCCTGCCCGGACCCCCTGGCGAGCGTGGTGGACCTGGTAGCCGTGGTTT
CCCTGGCGCAGATGGTGTTGCTGGTCCCAAGGGTCCCGCTGGTGAACGTGGTTCTCCTGGCCCTGCTGGC
CCCAAAGGATCTCCTGGTGAAGCTGGTCGTCCCGGTGAAGCTGGTCTGCCTGGTGCCAAGGGTCTGACTG
GAAGCCCTGGCAGCCCTGGTCCTGATGGCAAAACTGGCCCCCCTGGTCCCGCCGGTCAAGATGGTCGCCC
CGGACCCCCAGGCCCACCTGGTGCCCGTGGTCAGGCTGGTGTGATGGGATTCCCTGGACCTAAAGGTGCT
GCTGGAGAGCCCGGCAAGGCTGGAGAGCGAGGTGTTCCCGGACCCCCTGGCGCTGTCGGTCCTGCTGGCA
AAGATGGAGAGGCTGGAGCTCAGGGACCCCCTGGCCCTGCTGGTCCCGCTGGCGAGAGAGGTGAACAAGG
CCCTGCTGGCTCCCCCGGATTCCAGGGTCTCCCTGGTCCTGCTGGTCCTCCAGGTGAAGCAGGCAAACCT
GGTGAACAGGGTGTTCCTGGAGACCTTGGCGCCCCTGGCCCCTCTGGAGCAAGAGGCGAGAGAGGTTTCC
CTGGCGAGCGTGGTGTGCAAGGTCCCCCTGGTCCTGCTGGTCCCCGAGGGGCCAACGGTGCTCCCGGCAA
CGATGGTGCTAAGGGTGATGCTGGTGCCCCTGGAGCTCCCGGTAGCCAGGGCGCCCCTGGCCTTCAGGGA
ATGCCTGGTGAACGTGGTGCAGCTGGTCTTCCAGGGCCTAAGGGTGACAGAGGTGATGCTGGTCCCAAAG
GTGCTGATGGCTCTCCTGGCAAAGATGGCGTCCGTGGTCTGACTGGCCCCATTGGTCCTCCTGGCCCTGC
TGGTGCCCCTGGTGACAAGGGTGAAAGTGGTCCCAGCGGCCCTGCTGGTCCCACTGGAGCTCGTGGTGCC
 
CCCGGAGACCGTGGTGAGCCTGGTCCCCCGGCCCTGCTGGCTTTGCTGGCCCCCTGGTGCTGACGGCC
AACCTGGTGCTAAAGGCGAACCTGGTGATGCTGGTGCTAAAGGCGATGCTGGTCCCCCTGGCCCTGCCGG
ACCCGCTGGACCCCCTGGCCCCATTGGTAATGTTGGTGCTCCTGGAGCCAAAGGTGCTCGCGGCAGCGCT
GGTCCCCCTGGTGCTACTGGTTTCCCTGGTGCTGCTGGCCGAGTCGGTCCTCCTGGCCCCTCTGGAAATG
CTGGACCCCCTGGCCCTCCTGGTCCTGCTGGCAAAGAAGGCGGCAAAGGTCCCCGTGGTGAGACTGGCCC
TGCTGGACGTCCTGGTGAAGTTGGTCCCCCTGGTCCCCCTGGCCCTGCTGGCGAGAAAGGATCCCCTGGT
GCTGATGGTCCTGCTGGTGCTCCTGGTACTCCCGGCCTCAAGGTATTGCTGGACAGCGTGGTGTGGTCG
GCCTGCCTGGTCAGAGAGGAGAGAGAGGCTTCCCTGGTCTTCCTGGCCCCTCTGGTGAACCTGGCAAACA
AGGTCCCTCTGGAGCAAGTGGTGAACGTGGTCCCCCTGGTCCCATGGGCCCCCCTGGATTGGCTGGACCC
CCTGGTGAATCTGGACGTGAGGGGGCTCCTGGTGCCGAAGGTTCCCCTGGACGAGACGGTTCTCCTGGCG
CCAAGGGTGACCGTGGTGAGACCGGCCCCGCTGGACCCCCTGGTGCTCCTGGTGCTCCTGGTGCCCCTGG
CCCCGTTGGCCCTGCTGGCAAGAGTGGTGATCGTGGTGAGACTGGTCCTGCTGGTCCCGCCGGTCCTGTC
GGCCCTGTTGGCGCCCGTGGCCCCGCCGGACCCCAAGGCCCCCGTGGTGACAAGGGTGAGACAGGCGAAC
AGGGCGACAGAGGCATAAAGGGTCACCGTGGCTTCTCTGGCCTCCAGGGTCCCCCTGGCCCTCCTGGCTC
TCCTGGTGAACAAGGTCCCTCTGGAGCCTCTGGTCCTGCTGGTCCCCGAGGTCCCCCTGGCTCTGCTGGT
GCTCCTGGCAAAGATGGACTCAACGGTCTCCCTGGCCCCATTGGGCCCCCTGGTCCTCGCGGTCGCACTG
GTGATGCTGGTCCTGTTGGTCCCCCCGGCCCTCCTGGACCTCCTGGTCCCCCTGGTCCTCCCAGCGCTGG
TTTCGACTTCAGCTTCCTGCCCCAGCCACCTCAAGAGAAGGCTCACGATGGTGGCCGCTACTACCGGGCT
GATGATGCCAATGTGGTTCGTGACCGTGACCTCGAGGTGGACACCACCCTCAAGAGCCTGAGCCAGCAGA
TCGAGAACATCCGGAGCCCAGAGGGCAGCCGCAAGAACCCCGCCCGCACCTGCCGTGACCTCAAGATGTG
CCACTCTGACTGGAAGAGTGGAGAGTACTGGATTGACCCCAACCAAGGCTGCAACCTGGATGCCATCAAA
GTCTTCTGCAACATGGAGACTGGTGAGACCTGCGTGTACCCCACTCAGCCCAGTGTGGCCCAGAAGAACT
GGTACATCAGCAAGAACCCCAAGGACAAGAGGCATGTCTGGTTCGGCGAGAGCATGACCGATGGATTCCA
GTTCGAGTATGGCGGCCAGGGCTCCGACCCTGCCGATGTGGCCATCCAGCTGACCTTCCTGCGCCTGATG
TCCACCGAGGCCTCCCAGAACATCACCTACCACTGCAAGAACAGCGTGGCCTACATGGACCAGCAGACTG
GCAACCTCAAGAAGGCCCTGCTCCTCCAGGGCTCCAACGAGATCGAGATCCGCGCCGAGGGCAACAGCCG
CTTCACCTACAGCGTCACTGTCGATGGCTGCACGAGTCACACCGGAGCCTGGGGCAAGACAGTGATTGAA
TACAAAACCACCAAGACCTCCCGCCTGCCCATCATCGATGTGGCCCCCTTGGACGTTGGTGCCCCAGACC
AGGAATTCGGCTTCGACGTTGGCCCTGTCTGCTTCCTGTAAACTCCCTCCATCCCAACCTGGCTCCCTCC
CACCCAACCAACTTTCCCCCCAACCCGGAAACAGACAAGCAACCCAAACTGAACCCCCTCAAAAGCCAAA
AAATGGGAGACAATTTCACATGGACTTTGGAAAATATTTTTTTCCTTTGCATTCATCTCTCAAACTTAGT
TTTTATCTTTGACCAACCGAACATGACCAAAAACCAAAAGTGCATTCAACCTTACCAAAAAAAAAAAAAA
AAAAAGAATAAATAAATAACTTTTTAAAAAAGGAAGCTTGGTCCACTTGCTTGAAGACCCATGCGGGGGT
AAGTCCCTTTCTGCCCGTTGGGCTTATGAAACCCCAATGCTGCCCTTTCTGCTCCTTTCTCCACACCCCC
CTTGGGGCCTCCCCTCCACTCCTTCCCAAATCTGTCTCCCCAGAAGACACAGGAAACAATGTATTGTCTG
CCCAGCAATCAAAGGCAATGCTCAAACACCCAAGTGGCCCCCACCCTCAGCCCGCTCCTGCCCGCCCAGC
ACCCCCAGGCCCTGGGGGACCTGGGGTTCTCAGACTGCCAAAGAAGCCTTGCCATCTGGCGCTCCCATGG
CTCTTGCAACATCTCCCCTTCGTTTTTGAGGGGGTCATGCCGGGGGAGCCACCAGCCCCTCACTGGGTTC
GGAGGAGAGTCAGGAAGGGCCACGACAAAGCAGAAACATCGGATTTGGGGAACGCGTGTCAATCCCTTGT
GCCGCAGGGCTGGGCGGGAGAGACTGTTCTGTTCCTTGTGTAACTGTGTTGCTGAAAGACTACCTCGTTC
TTGTCTTGATGTGTCACCGGGGCAACTGCCTGGGGGCGGGGATGGGGGCAGGGTGGAAGCGGCTCCCCAT
TTTATACCAAAGGTGCTACATCTATGTGATGGGTGGGGTGGGGAGGGAATCACTGGTGCTATAGAAATTG
AGATGCCCCCCCAGGCCAGCAAATGTTCCTTTTTGTTCAAAGTCTATTTTTATTCCTTGATATTTTTCTT
TTTTTTTTTTTTTTTTTGTGGATGGGGACTTGTGAATTTTTCTAAAGGTGCTATTTAACATGGGAGGAGA
GCGTGTGCGGCTCCAGCCCAGCCCGCTGCTCACTTTCCACCCTCTCTCCACCTGCCTCTGGCTTCTCAGG
CCTCTGCTCTCCGACCTCTCTCCTCTGAAACCCTCCTCCACAGCTGCAGCCCATCCTCCCGGCTCCCTCC
TAGTCTGTCCTGCGTCCTCTGTCCCCGGGTTTCAGAGACAACTTCCCAAAGCACAAAGCAGTTTTTCCCC
CTAGGGGTGGGAGGAAGCAAAAGACTCTGTACCTATTTTGTATGTGTATAATAATTTGAGATGTTTTTAA
TTATTTTGATTGCTGGAATAAAGCATGTGGAAATGACCCAAACATAA
NCBI Reference Sequence: NM_001081754.1
   **Homo sapiens elastin (ELN), transcript variant 4, mRNA**
   >gi|126352445|ref|NM_001081754.1| Homo sapiens elastin (ELN), transcript variant 4, mRNA 
CTCCCTCTTTCCCTCACAGCCGACGAGGCAACAATTAGGCTTTGGGGATAAAACGAGGTGCGGAGAGCGG
GCTGGGGCATTTCTCCCCGAGATGGCGGGTCTGACGGCGGCGGCCCCGCGGCCCGGAGTCCTCCTGCTCC
TGCTGTCCATCCTCCACCCCTCTCGGCCTGGAGGGGTCCCTGGGGCCATTCCTGGTGGAGTTCCTGGAGG
AGTCTTTTATCCAGGGGCTGGTCTCGGAGCCCTTGGAGGAGGAGCGCTGGGGCCTGGAGGCAAACCTCTT
AAGCCAGTTCCCGGAGGGCTTGCGGGTGCTGGCCTTGGGGCAGGGCTCGGCGCCTTCCCCGCAGTTACCT
TTCCGGGGGCTCTGGTGCCTGGTGGAGTGGCTGACGCTGCTGCAGCCTATAAAGCTGCTAAGGCTGGCGC
TGGGCTTGGTGGTGTCCCAGGAGTTGGTGGCTTAGGAGTGTCTGCAGCCCCTTCTGTGCCAGGTGCGGTG
GTTCCTCAGCCTGGAGCCGGAGTGAAGCCTGGGAAAGTGCCGGGTGTGGGGCTGCCAGGTGTATACCCAG
GTGGCGTGCTCCCAGGAGCTCGGTTCCCCGGTGTGGGGGTGCTCCCTGGAGTTCCCACTGGAGCAGGAGT
TAAGCCCAAGGCTCCAGGTGTAGGTGGAGCTTTTGCTGGAATCCCAGGAGTTGGACCCTTTGGGGGACCG
CAACCTGGAGTCCCACTGGGGTATCCCATCAAGGCCCCCAAGCTGCCTGGTGGCTATGGACTGCCCTACA
CCACAGGGAAACTGCCCTATGGCTATGGGCCCGGAGGAGTGGCTGGTGCAGCGGGCAAGGCTGGTTACCC
AACAGGGACAGGGGTTGGCCCCCAGGCAGCAGCAGCGGCAGCTAAAGCAGCAGCAAAGTTCGGTGCT
GGAGCAGCCGGAGTCCTCCCTGGTGTTGGAGGGGCTGGTGTTCCTGGCGTGCCTGGGGCAATTCCTGGAA
TTGGAGGCATCGCAGGCGTTGGGACTCCAGCTGCAGCTGCAGCTGCAGCAGCAGCCGCTAAGGCAGCCAA
GTATGGAGCTGCTGCAGGCTTAGTGCCTGGTGGGCCAGGCTTTGGCCCGGGAGTAGTTGGTGTCCCAGGA
GCTGGCGTTCCAGGTGTTGGTGTCCCAGGAGCTGGGATTCCAGTTGTCCCAGGTGCTGGGATCCCAGGTG
CTGCGGTTCCAGGGGTTGTGTCACCAGAAGCAGCTGCTAAGGCAGCTGCAAAGGCAGCCAAATACGGGGC
CAGGCCCGGAGTCGGAGTTGGAGGCATTCCTACTTACGGGGTTGGAGCTGGGGGCTTTCCCGGCTTTGGT
GTCGGAGTCGGAGGTATCCCTGGAGTCGCAGGTGTCCCTGGTGTCGGAGGTGTTCCCGGAGTCGGAGGTG
TCCCGGGAGTTGGCATTTCCCCCGAAGCTCAGGCAGCAGCTGCCGCCAAGGCTGCCAAGTACGGAGTGGG
GACCCCAGCAGCTGCAGCTGCTAAAGCAGCCGCCAAAGCCGCCCAGTTTGGGTTAGTTCCTGGTGTCGGC
GTGGCTCCTGGAGTTGGCGTGGCTCCTGGTGTCGGTGTGGCTCCTGGAGTTGGCTTGGCTCCTGGAGTTG
GCGTGGCTCCTGGAGTTGGTGTGGCTCCTGGCGTTGGCGTGGCTCCCGGCATTGGCCCTGGTGGAGTTGC
AGCTGCAGCAAAATCCGCTGCCAAGGTGGCTGCCAAAGCCCAGCTCCGAGCTGCAGCTGGGCTTGGTGCT
GGCATCCCTGGACTTGGAGTTGGTGTCGGCGTCCCTGGACTTGGAGTTGGTGCTGGTGTTCCTGGACTTG
GAGTTGGTGCTGGTGTTCCTGGCTTCGGGGCAGTACCTGGAGCCCTGGCTGCCGCTAAAGCAGCCAAATA
TGGAGCAGCAGTGCCTGGGGTCCTTGGAGGGCTCGGGGCTCTCGGTGGAGTAGGCATCCCAGGCGGTGTG
GTGGGAGCCGGACCCGCCGCCGCCGCTGCCGCAGCCAAAGCTGCTGCCAAAGCCGCCCAGTTTGGCCTAG
TGGGAGCCGCTGGGCTCGGAGGACTCGGAGTCGGAGGGCTTGGAGTTCCAGGTGTTGGGGGCCTTGGAGG
TATACCTCCAGCTGCAGCCGCTAAAGCAGCTAAATACGGAGTGGCAGCAAGACCTGGCTTCGGATTGTCT
CCCATTTTCCCAGGTGGGGCCTGCCTGGGGAAAGCTTGTGGCCGGAAGAGAAAATGAGCTTCCTAGGACC
CCTGACTCACGACCTCATCAACGTTGGTGCTACTGCTTGGTGGAGAATGTAAACCCTTTGTAACCCCATC
CCATGCCCCTCCGACTCCCCACCCCAGGAGGGAACGGCAGGCCGGGCGGCCTTGCAGATCCACAGGGCA
AGGAAACAAGAGGGGAGCGGCCAAGTGCCCCGACCAGGAGGCCCCCTACTTCAGAGGCAAGGGCCATGTG
GTCCTGGCCCCCCACCCCATCCCTTCCCACCTAGGAGCTCCCCCTCCACACAGCCTCCATCTCCAGGGGA
ACTTGGTGCTACACGCTGGTGCTCTTATCTTCCTGGGGGGAGGGAGGAGGGAAGGGTGGCCCCTCGGGGA
ACCCCCTACCTGGGGCTCCTCTAAAGATGGTGCAGACACTTCCTGGGCAGTCCCAGCTCCCCCTCCCCAC
CAGGACCCACCGTTGGCTGCCATCCAGTTGGTACCCAAGCACCTGAAGCCTCAAAGCTGGATTCGCTCTA
GCATCCCTCCTCTCCTGGGTCCACTTGGCCGTCTCCTCCCCACCGATCGCTGTTCCCCACATCTGGGGCG
CTTTTGGGTTGGAAAACCACCCCACACTGGGAATAGCCACCTTGCCCTTGTAGAATCCATCCGCCCATCC
GTCCATTCATCCATCGGTCCGTCCATCCATGTCCCCAGTTGACCGCCCGGCACCACTAGCTGGCTGGGTG
CACCCACCATCAACCTGGTTGACCTGTCATGGCCGCCTGTGCCCTGCCTCCACCCCCATCCTACACTCCC
CCAGGGCGTGCGGGGCTGTGCAGACTGGGGTGCCAGGCATCTCCTCCCCACCCGGGGTGTCCCCACATGC
AGTACTGTATACCCCCCATCCCTCCCTCGGTCCACTGAACTTCAGAGCAGTTCCCATTCCTGCCCCGCCC
ATCTTTTTGTGTCTCGCTGTGATAGATCAATAAATATTTTATTTTTTGTCCTGGATATTTGGGGATTATT
TTTGATTGTTGATATTCTCTTTTGGTTTTATTGTTGTGGTTCATTGAAAAAAAAAGATAATTTTTTTTTC
TGATCCGGGGAGCTGTATCCCCAGTAGAAAAAACATTTTAATCACTCTAATATAACTCTGGATGAAACAC
ACCTTTTTTTTTAATAAGAAAAGAGAATTAACTGCTTCAGAAATGACTAATAAATGAAAAACCTTTAAAG
GAAAAAAAAAA
NCBI Reference Sequence: NM_001081753.1
   **Homo sapiens elastin (ELN), transcript variant 3, mRNA**
   >gi|126352321|ref|NM_001081753.1| Homo sapiens elastin (ELN), transcript variant 3, mRNA 
CTCCCTCTTTCCCTCACAGCCGACGAGGCAACAATTAGGCTTTGGGGATAAAACGAGGTGCGGAGAGCGG
GCTGGGGCATTTCTCCCCGAGATGGCGGGTCTGACGGCGGCGGCCCCGCGGCCCGGAGTCCTCCTGCTCC
TGCTGTCCATCCTCCACCCCTCTCGGCCTGGAGGGGTCCCTGGGGCCATTCCTGGTGGAGTTCCTGGAGG
AGTCTTTATCCAGGGGCTGGTCTCGGAGCCCTTGGAGGAGGAGCGCTGGGGCCTGGAGGCAAACCTCTT
AAGCCAGTTCCCGGAGGGCTTGCGGGTGCTGGCCTTGGGGCAGGGCTCGGCGCCTTCCCCGCAGTTACCT
TTCCGGGGGCTCTGGTGCCTGGTGGAGTGGCTGACGCTGCTGCAGCCTATAAAGCTGCTAAGGCTGGCGC
TGGGCTTGGTGGTGTCCCAGGAGTTGGTGGCTTAGGAGTGTCTGCAGCCCCTTCTGTGCCAGGTGCGGTG
GTTCCTCAGCCTGGAGCCGGAGTGAAGCCTGGGAAAGTGCCGGGTGTGGGGCTGCCAGGTGTATACCCAG
GTGGCGTGCTCCCAGGAGCTCGGTTCCCCGGTGTGGGGGTGCTCCCTGGAGTTCCCACTGGAGCAGGAGT
TAAGCCCAAGGCTCCAGGTGTAGGTGGAGCTTTTGCTGGAATCCCAGGAGTTGGACCCTTTGGGGGACCG
CAACCTGGAGTCCCACTGGGGTATCCCATCAAGGCCCCCAAGCTGCCTGGTGGCTATGGACTGCCCTACA
CCACAGGGAAACTGCCCTATGGCTATGGGCCCGGAGGAGTGGCTGGTGCAGCGGGCAAGGCTGGTTACCC
AACAGGGACAGGGGTTGGCCCCCAGGCAGCAGCAGCAGCGGCAGCTAAAGCAGCAGCAAAGTTCGGTGCT
GGAGCAGCCGGAGTCCTCCCTGGTGTTGGAGGGGCTGGTGTTCCTGGCGTGCCTGGGGCAATTCCTGGAA
TTGGAGGCATCGCAGGCGTTGGGACTCCAGCTGCAGCTGCAGCTGCAGCAGCAGCCGCTAAGGCAGCCAA
GTATGGAGCTGCTGCAGGCTTAGTGCCTGGTGGGCCAGGCTTTGGCCCGGGAGTAGTTGGTGTCCCAGGA
GCTGGCGTTCCAGGTGTTGGTGTCCCAGGAGCTGGGATTCCAGTTGTCCCAGGTGCTGGGATCCCAGGTG
CTGCGGTTCCAGGGGTTGTGTCACCAGAAGCAGCTGCTAAGGCAGCTGCAAAGGCAGCCAAATACGGGGC
CAGGCCCGGAGTCGGAGTTGGAGGCATTCCTACTTACGGGGTTGGAGCTGGGGGCTTTCCCGGCTTTGGT
GTCGGAGTCGGAGGTATCCCTGGAGTCGCAGGTGTCCCTGGTGTCGGAGGTGTTCCCGGAGTCGGAGGTG
TCCCGGGAGTTGGCATTTCCCCCGAAGCTCAGGCAGCAGCTGCCGCCAAGGCTGCCAAGTACGGTTAGT
TCCTGGTGTCGGCGTGGCTCCTGGAGTTGGCGTGGCTCCTGGTGTCGGTGTGGCTCCTGGAGTTGGCTTG
GCTCCTGGAGTTGGCGTGGCTCCTGGAGTTGGTGTGGCTCCTGGCGTTGGCGTGGCTCCCGGCATTGGCC
CTGGTGGAGTTGCAGCTGCAGCAAAATCCGCTGCCAAGGTGGCTGCCAAAGCCCAGCTCCGAGCTGCAGC
TGGGCTTGGTGCTGGCATCCCTGGACTTGGAGTTGGTGTCGGCGTCCCTGGACTTGGAGTTGGTGCTGGT
GTTCCTGGACTTGGAGTTGGTGCTGGTGTTCCTGGCTTCGGGGCAGTACCTGGAGCCCTGGCTGCCGCTA
AAGCAGCCAAATATGGAGCAGCAGTGCCTGGGGTCCTTGGAGGGCTCGGGGCTCTCGGTGGAGTAGGCAT
CCCAGGCGGTGTGGTGGGAGCCGGACCCGCCGCCGCCGCTGCCGCAGCCAAAGCTGCTGCCAAAGCCGCC
CAGTTTGGCCTAGTGGGAGCCGCTGGGCTCGGAGGACTCGGAGTCGGAGGGCTTGGAGTTCCAGGTGTTG
GGGGCCTTGGAGGTATACCTCCAGCTGCAGCCGCTAAAGCAGCTAAATACGGAGTGGCAGCAAGACCTGG
CTTCGGATTGTCTCCCATTTCCCAGGTGGGGCCTGCCTGGGGAAAGCTTGTGGCCGGAAGAGAAAATGA
GCTTCCTAGGACCCCTGACTCACGACCTCATCAACGTTGGTGCTACTGCTTGGTGGAGAATGTAAACCCT
TTGTAACCCCATCCCATGCCCCTCCGACTCCCCACCCCAGGAGGGAACGGGCAGGCCGGGCGGCCTTGCA
GATCCACAGGGCAAGGAAACAAGAGGGGAGCGGCCAAGTGCCCCGACCAGGAGGCCCCCTACTTCAGAGG
CAAGGGCCATGTGGTCCTGGCCCCCCACCCCATCCCTTCCCACCTAGGAGCTCCCCCTCCACACAGCCTC
CATCTCCAGGGGAACTTGGTGCTACACGCTGGTGCTCTTATCTTCCTGGGGGGAGGGAGGAGGGAAGGGT
GGCCCCTCGGGGAACCCCCTACCTGGGGCTCCTCTAAAGATGGTGCAGACACTTCCTGGCAGTCCCAGC
TCCCCCTGCCCACCAGGACCCACCGTTGGCTGCCATCCAGTTGGTACCCAAGCACCTGAAGCCTCAAAGC
TGGATTCGCTCTAGCATCCCTCCTCTCCTGGGTCCACTTGGCCGTCTCCTCCCCACCGATCGCTGTTCCC
CACATCTGGGGCGCTTTTGGGTTGGAAAACCACCCCACACTGGGAATAGCCACCTTGCCCTTGTAGAATC
CATCCGCCCATCCGTCCATTCATCCATCGGTCCGTCCATCCATGTCCCCAGTTGACCGCCCGGCACCACT
AGCTGGCTGGGTGCACCCACCATCAACCTGGTTGACCTGTCATGGCCGCCTGTGCCCTGCCTCCACCCCC
ATCCTACACTCCCCCAGGGCGTGCGGGGCTGTGCAGACTGGGGTGCCAGGCATCTCCTCCCCACCCGGGG
TGTCCCCACATGCAGTACTGTATACCCCCCATCCCTCCCTCGGTCCACTGAACTTCAGAGCAGTTCCCAT
TCCTGCCCCGCCCATCTTTTTGTGTCTCGCTGTGATAGATCAATAAATATTTTATTTTTTGTCCTGGATA
TTTGGGGATTATTTTTGATTGTTGATATTCTCTTTTGGTTTTATTGTTGTGGTTCATTGAAAAAAAAAGA
TAATTTTTTTTTCTGATCCGGGGAGCTGTATCCCCAGTAGAAAAACATTTAATCACTCTAATATAACT
CTGGATGAAACACACCTTTTTTTTTAATAAGAAAAGAGAATTAACTGCTTCAGAAATGACTAATAAATGA
AAAACCTTTAAAGGAAAAAAAAAA
NCBI Reference Sequence: NM_000501.2
   **Homo sapiens elastin (ELN), transcript variant 1, mRNA**
   >gi|126352439|ref|NM_000501.2| Homo sapiens elastin (ELN), transcript variant 1, mRNA 
CTCCCTCTTTCCCTCACAGCCGACGAGGCAACAATTAGGCTTTGGGGATAAAACGAGGTGCGGAGAGCGG
GCTGGGGCATTTCTCCCCGAGATGGCGGGTCTGACGGCGGCGGCCCCGCGGCCCGGAGTCCTCCTGCTCC
TGCTGTCCATCCTCCACCCCTCTCGGCCTGGAGGGGTCCCTGGGGCCATTCCTGGTGGAGTTCCTGGAGG
AGTCTTTTATCCAGGGGCTGGTCTCGGAGCCCTTGGAGGAGGAGCGCTGGGGCCTGGAGGCAAACCTCTT
AAGCCAGTTCCCGGAGGGCTTGCGGGTGCTGGCCTTGGGGCAGGGCTCGGCGCCTTCCCCGCAGTTACCT
TTCCGGGGGCTCTGGTGCCTGGTGGAGTGGCTGACGCTGCTGCAGCCTATAAAGCTGCTAAGGCTGGCGC
TGGGCTTGGTGGTGTCCCAGGAGTTGGTGGCTTAGGAGTGTCTGCAGGTGCGGTGGTTCCTCAGCCTGGA
GCCGGAGTGAAGCCTGGGAAAGTGCCGGGTGTGGGGCTGCCAGGTGTATACCCAGGTGGCGTGCTCCCAG
GAGCTCGGTTCCCCGGTGTGGGGGTGCTCCCTGGAGTTCCCACTGGAGCAGGAGTTAAGCCCAAGGCTCC
AGGTGTAGGTGGAGCTTTTGCTGGAATCCCAGGAGTTGGACCCTTTGGGGGACCGCAACCTGGAGTCCCA
CTGGGGTATCCCATCAAGGCCCCCAAGCTGCCTGGTGGCTATGGACTGCCCTACACCACAGGGAAACTGC
CCTATGGCTATGGGCCCGGAGGAGTGGCTGGTGCAGCGGGCAAGGCTGGTTACCCAACAGGGACAGGGGT
TGGCCCCCAGGCAGCAGCAGCAGCGGCAGCTAAAGCAGCAGCAAAGTTCGGTGCTGGAGCAGCCGGAGTC
CTCCCTGGTGTTGGAGGGGCTGGTGTTCCTGGCGTGCCTGGGGCAATTCCTGGAATTGGAGGCATCGCAG
GCGTTGGGACTCCAGCTGCAGCTGCAGCTGCAGCAGCAGCCGCTAAGGCAGCCAAGTATGGAGCTGCTGC
AGGCTTAGTGCCTGGTGGGCCAGGCTTTGGCCCGGGAGTAGTTGGTGTCCCAGGAGCTGGCGTTCCAGGT
GTTGGTGTCCCAGGAGCTGGGATTCCAGTTGTCCCAGGTGCTGGGATCCCAGGTGCTGCGGTTCCAGGGG
TTGTGTCACCAGAAGCAGCTGCTAAGGCAGCTGCAAAGGCAGCCAAATACGGGGCCAGGCCCGGAGTCGG
AGTTGGAGGCATTCCTACTTACGGGGTTGGAGCTGGGGGCTTTCCCGGCTTTGGTGTCGGAGTCGGAGGT
ATCCCTGGAGTCGCAGGTGTCCCTGGTGTCGGAGGTGTTCCCGGAGTCGGAGGTGTCCCGGGAGTTGGCA
TTTCCCCCGAAGCTCAGGCAGCAGCTGCCGCCAAGGCTGCCAAGTACGGAGTGGGGACCCCAGCAGCTGC
AGCTGCTAAAGCAGCCGCCAAAGCCGCCCAGTTTGGGTTAGTTCCTGGTGTCGGCGTGGCTCCTGGAGTT
GGCGTGGCTCCTGGTGTCGGTGTGGCTCCTGGAGTTGGCTTGGCTCCTGGAGTTGGCGTGGCTCCTGGAG
TTGGTGTGGCTCCTGGCGTTGGCGTGGCTCCCGGCATTGGCCCTGGTGGAGTTGCAGCTGCAGCAAAATC
CGCTGCCAAGGTGGCTGCCAAAGCCCAGCTCCGAGCTGCAGCTGGGCTTGGTGCTGGCATCCCTGGACTT
GGAGTTGGTGTCGGCGTCCCTGGACTTGGAGTTGGTGCTGGTGTTCCTGGACTTGGAGTTGGTGCTGGTG
TTCCTGGCTTCGGGGCAGTACCTGGAGCCCTGGCTGCCGCTAAAGCAGCCAAATATGGAGCAGCAGTGCC
TGGGGTCCTTGGAGGGCTCGGGGCTCTCGGTGGAGTAGGCATCCCAGGCGGTGTGGTGGGAGCCGGACCC
GCCGCCGCCGCTGCCGCAGCCAAAGCTGCTGCCAAAGCCGCCCAGTTTGGCCTAGTGGGAGCCGCTGGGC
TCGGAGGACTCGGAGTCGGAGGGCTTGGAGTTCCAGGTGTTGGGGGCCTTGGAGGTATACCTCCAGCTGC
AGCCGCTAAAGCAGCTAAATACGGTGCTGCTGGCCTTGGAGGTGTCCTAGGGGGTGCCGGGCAGTTCCCA
CTTGGAGGAGTGGCAGCAAGACCTGGCTTCGGATTGTCTCCCATTTTCCCAGGTGGGGCCTGCCTGGGGA
AAGCTTGTGGCCGGAAGAGAAAATGAGCTTCCTAGGACCCCTGACTCACGACCTCATCAACGTTGGTGCT
ACTGCTTGGTGGAGAATGTAAACCCTTTGTAACCCCATCCCATGCCCCTCCGACTCCCCACCCCAGGAGG
GAACGGGCAGGCCGGGCGGCCTTGCAGATCCACAGGGCAAGGAAACAAGAGGGGAGCGGCCAAGTGCCCC
GACCAGGAGGCCCCCTACTTCAGAGGCAAGGGCCATGTGGTCCTGGCCCCCCACCCCATCCCTTCCCACC
TAGGAGCTCCCCCTCCACACAGCCTCCATCTCCAGGGGAACTTGGTGCTACACGCTGGTGCTCTTATCTT
CCTGGGGGGAGGGAGGAGGGAAGGGTGGCCCCTCGGGGAACCCCCTACCTGGGGCTCCTCTAAAGATGGT
GCAGACACTTCCTGGGCAGTCCCAGCTCCCCCTGCCCACCAGGACCCACCGTTGGCTGCCATCCAGTTGG
TACCCAAGCACCTGAAGCCTCAAAGCTGGATTCGCTCTAGCATCCCTCCTCTCCTGGGTCCACTTGGCCG
TCTCCTCCCCACCGATCGCTGTTCCCCACATCTGGGGCGCTTTTGGGTTGGAAAACCACCCCACACTGGG
AATAGCCACCTTGCCCTTGTAGAATCCATCCGCCCATCCGTCCATTCATCCATCGGTCCGTCCATCCATG
TCCCCAGTTGACCGCCCGGCACCACTAGCTGGCTGGGTGCACCCACCATCAACCTGGTTGACCTGTCATG
GCCGCCTGTGCCCTGCCTCCACCCCCATCCTACACTCCCCCAGGGCGTGCGGGGCTGTGCAGACTGGGGT
GCCAGGCATCTCCTCCCCACCCGGGGTGTCCCCACATGCAGTACTGTATACCCCCCATCCCTCCCTCGGT
CCACTGAACTTCAGAGCAGTTCCCATTCCTGCCCCGCCCATCTTTTTGTGTCTCGCTGTGATAGATCAAT
AAATATTTATTTTTTGTCCTGGATATTTGGGGATTATTTTGATTGTTGATATTCTCTTTTGGTTTTAT
TGTTGTGGTTCATTGAAAAAAAAAGATAATTTTTTTTTCTGATCCGGGGAGCTGTATCCCCAGTAGAAAA
AACATTTTAATCACTCTAATATAACTCTGGATGAAACACACCTTTTTTTTAATAAGAAAAGAGAATTAA
CTGCTTCAGAAATGACTAATAAATGAAAAACCTTTAAAGGAAAAAAAAAA
NCBI Reference Sequence: NM_001081755.1
   **Homo sapiens elastin (ELN), transcript variant 5, mRNA**
   >gi|126352607|ref|NM_001081755.1| Homo sapiens elastin (ELN), transcript variant 5, mRNA 
CTCCCTCTTTCCCTCACAGCCGACGAGGCAACAATTAGGCTTTGGGGATAAAACGAGGTGCGGAGAGCGG
GCTGGGGCATTTCTCCCCGAGATGGCGGGTCTGACGGCGGCGGCCCCGCGGCCCGGAGTCCTCCTGCTCC
TGCTGTCCATCCTCCACCCCTCTCGGCCTGGAGGGGTCCCTGGGGCCATTCCTGGTGGAGTTCCTGGAGG
AGTCTTTATCCAGGGGCTGGTCTCGGAGCCCTTGGAGGAGGAGCGCTGGGGCCTGGAGGCAAACCTCTT
AAGCCAGTTCCCGGAGGGCTTGCGGGTGCTGGCCTTGGGGCAGGGCTCGGCGCCTTCCCCGCAGTTACCT
TTCCGGGGGCTCTGGTGCCTGGTGGAGTGGCTGACGCTGCTGCAGCCTATAAAGCTGCTAAGGCTGGCGC
TGGGCTTGGTGGTGTCCCAGGAGTTGGTGGCTTAGGAGTGTCTGCAGGTGCGGTGGTTCCTCAGCCTGGA
GCCGGAGTGAAGCCTGGGAAAGTGCCGGGTGTGGGGCTGCCAGGTGTATACCCAGGTGGCGTGCTCCCAG
GAGCTCGGTTCCCCGGTGTGGGGGTGCTCCCTGGAGTTCCCACTGGAGCAGGAGTTAAGCCCAAGGCTCC
AGGTGTAGGTGGAGCTTTTGCTGGAATCCCAGGAGTTGGACCCTTTGGGGGACCGCAACCTGGAGTCCCA
CTGGGGTATCCCATCAAGGCCCCCAAGCTGCCTGGTGGCTATGGACTGCCCTACACCACAGGGAAACTGC
CCTATGGCTATGGGCCCGGAGGAGTGGCTGGTGCAGCGGGCAAGGCTGGTTACCCAACAGGGACAGGGGT
TGGCCCCCAGGCAGCAGCAGCAGCGGCAGCTAAAGCAGCAGCAAAGTTCGGTGCTGGAGCAGCCGGAGTC
CTCCCTGGTGTTGGAGGGGCTGGTGTTCCTGGCGTGCCTGGGGCAATTCCTGGAATTGGAGGCATCGCAG
GCGTTGGGACTCCAGCTGCAGCTGCAGCTGCAGCAGCAGCCGCTAAGGCAGCCAAGTATGGAGCTGCTGC
AGGCTTAGTGCCTGGTGGGCCAGGCTTTGGCCCGGGAGTAGTTGGTGTCCCAGGAGCTGGCGTTCCAGGT
GTTGGTGTCCCAGGAGCTGGGATTCCAGTTGTCCCAGGTGCTGGGATCCCAGGTGCTGCGGTTCCAGGGG
TTGTGTCACCAGAAGCAGCTGCTAAGGCAGCTGCAAAGGCAGCCAAATACGGGGCCAGGCCCGGAGTCGG
AGTTGGAGGCATTCCTACTTACGGGGTTGGAGCTGGGGGCTTTCCCGGCTTTGGTGTCGGAGTCGGAGGT
ATCCCTGGAGTCGCAGGTGTCCCTGGTGTCGGAGGTGTTCCCGGAGTCGGAGGTGTCCCGGGAGTTGGCA
TTTCCCCCGAAGCTCAGGCAGCAGCTGCCGCCAAGGCTGCCAAGTACGGGTTAGTTCCTGGTGTCGGCGT
GGCTCCTGGAGTTGGCGTGGCTCCTGGTGTCGGTGTGGCTCCTGGAGTTGGCTTGGCTCCTGGAGTTGGC
GTGGCTCCTGGAGTTGGTGTGGCTCCTGGCGTTGGCGTGGCTCCCGGCATTGGCCCTGGTGGAGTTGCAG
CTGCAGCAAAATCCGCTGCCAAGGTGGCTGCCAAAGCCCAGCTCCGAGCTGCAGCTGGGCTTGGTGCTGG
CATCCCTGGACTTGGAGTTGGTGTCGGCGTCCCTGGACTTGGAGTTGGTGCTGGTGTTCCTGGACTTGGA
GTTGGTGCTGGTGTTCCTGGCTTCGGGGCAGTACCTGGAGCCCTGGCTGCCGCTAAAGCAGCCAAATATG
GAGCAGCAGTGCCTGGGGTCCTTGGAGGGCTCGGGGCTCTCGGTGGAGTAGGCATCCCAGGCGGTGTGGT
GGGAGCCGGACCCGCCGCCGCCGCTGCCGCAGCCAAAGCTGCTGCCAAAGCCGCCCAGTTTGGCCTAGTG
GGAGCCGCTGGGCTCGGAGGACTCGGAGTCGGAGGGCTTGGAGTTCCAGGTGTTGGGGGCCTTGGAGGTA
TACCTCCAGCTGCAGCCGCTAAAGCAGCTAAATACGGTGCTGCTGGCCTTGGAGGTGTCCTAGGGGGTGC
CGGGCAGTTCCCACTTGGAGGAGTGGCAGCAAGACCTGGCTTCGGATTGTCTCCCATTTTCCCAGGTGGG
GCCTGCCTGGGGAAAGCTTGTGGCCGGAAGAGAAAATGAGCTTCCTAGGACCCCTGACTCACGACCTCAT
CAACGTTGGTGCTACTGCTTGGTGGAGAATGTAAACCCTTTGTAACCCATCCCATGCCCCTCCGACTCC
CCACCCCAGGAGGGAACGGCAGGCCGGGCGGCCTTGCAGATCCACAGGGCAAGGAAACAAGAGGGGAGC
GGCCAAGTGCCCCGACCAGGAGGCCCCCTACTTCAGAGGCAAGGGCCATGTGGTCCTGGCCCCCCACCCC
ATCCCTTCCCACCTAGGAGCTCCCCCTCCACACAGCCTCCATCTCCAGGGGAACTTGGTGCTACACGCTG
GTGCTCTTATCTTCCTGGGGGGAGGAGGAGGGAAGGGTGGCCCCTCGGGGAACCCCCTACCTGGGGCTC
CTCTAAAGATGGTGCAGACACTTCCTGGCAGTCCCAGCTCCCCCTGCCCACCAGGACCCACCGTTGGCT
GCCATCCAGTTGGTACCCAAGCACCTGAAGCCTCAAAGCTGGATTCGCTCTAGCATCCCTCCTCTCCTGG
GTCCACTTGGCCGTCTCCTCCCCACCGATCGCTGTTCCCCACATCTGGGGCGCTTTTGGGTTGGAAAACC
ACCCCACACTGGGAATAGCCACCTTGCCCTTGTAGAATCCATCCGCCCATCCGTCCATTCATCCATCGGT
CCGTCCATCCATGTCCCCAGTTGACCGCCCGGCACCACTAGCTGGCTGGGTGCACCCACCATCAACCTGG
TTGACCTGTCATGGCCGCCTGTGCCCTGCCTCCACCCCCATCCTACACTCCCCCAGGGCGTGCGGGGCTG
TGCAGACTGGGGTGCCAGGCATCTCCTCCCCACCCGGGGTGTCCCCACATGCAGTACTGTATACCCCCCA
TCCCTCCCTCGGTCCACTGAACTTCAGAGCAGTTCCCATTCCTGCCCCGCCCATCTTTTTGTGTCTCGCT
GTGATAGATCAATAAATATTTTATTTTTTGTCCTGGATATTTGGGGATTATTTTTGATTGTTGATATTCT
CTTTTGGTTTTATTGTTGTGGTTCATTGAAAAAAAAAGATAATTTTTTTTTCTGATCCGGGGAGCTGTAT
CCCCAGTAGAAAAAACATTTTAATCACTCTAATATAACTCTGGATGAAACACACCTTTTTTTTTAATAAG
 AAAAGAGAATTAACTGCTTCAGAAATGACTAATAAATGAAAAACCTTTAAAGGAAAAAAAAA
NCBI Reference Sequence: NM_001081752.1
   **Homo sapiens elastin (ELN), transcript variant 2, mRNA**
   >gi|126352699|ref|NM_001081752.1| Homo sapiens elastin (ELN), transcript variant 2, mRNA 
CTCCCTCTTTCCCTCACAGCCGACGAGGCAACAATTAGGCTTTGGGGATAAAACGAGGTGCGGAGAGCGG
GCTGGGGCATTTCTCCCCGAGATGGCGGGTCTGACGGCGGCGGCCCCGCGGCCCGGAGTCCTCCTGCTCC
TGCTGTCCATCCTCCACCCCTCTCGGCCTGGAGGGGTCCCTGGGGCCATTCCTGGTGGAGTTCCTGGAGG
AGTCTTTATCCAGCGCTGGGGCCTGGAGGCAAACCTCTTAAGCCAGTTCCCGGAGGCTTGCGGGTGCT
GGCCTTGGGGCAGGGCTCGGCGCCTTCCCCGCAGTTACCTTTCCGGGGGCTCTGGTGCCTGGTGGAGTGG
CTGACGCTGCTGCAGCCTATAAAGCTGCTAAGGCTGGCGCTGGGCTTGGTGGTGTCCCAGGAGTTGGTGG
CTTAGGAGTGTCTGCAGGTGCGGTGGTTCCTCAGCCTGGAGCCGGAGTGAAGCCTGGGAAAGTGCCGGGT
GTGGGGCTGCCAGGTGTATACCCAGGTGGCGTGCTCCCAGGAGCTCGGTTCCCCGGTGTGGGGGTGCTCC
CTGGAGTTCCCACTGGAGCAGGAGTTAAGCCCAAGGCTCCAGGTGTAGGTGGAGCTTTTGCTGGAATCCC
AGGAGTTGGACCCTTTGGGGGACCGCAACCTGGAGTCCCACTGGGGTATCCCATCAAGGCCCCCAAGCTG
CCTGGTGGCTATGGACTGCCCTACACCACAGGGAAACTGCCCTATGGCTATGGGCCCGGAGGAGTGGCTG
GTGCAGCGGGCAAGGCTGGTTACCCAACAGGGACAGGGGTTGGCCCCCAGGCAGCAGCAGCAGCGGCAGC
TAAAGCAGCAGCAAAGTTCGGTGCTGGAGCAGCCGGAGTCCTCCCTGGTGTTGGAGGGGCTGGTGTTCCT
GGCGTGCCTGGGGCAATTCCTGGAATTGGAGGCATCGCAGGCGTTGGGACTCCAGCTGCAGCTGCAGCTG
CAGCAGCAGCCGCTAAGGCAGCCAAGTATGGAGCTGCTGCAGGCTTAGTGCCTGGTGGGCCAGGCTTTGG
CCCGGGAGTAGTTGGTGTCCCAGGAGCTGGCGTTCCAGGTGTTGGTGTCCCAGGAGCTGGGATTCCAGTT
GTCCCAGGTGCTGGGATCCCAGGTGCTGCGGTTCCAGGGGTTGTGTCACCAGAAGCAGCTGCTAAGGCAG
CTGCAAAGGCAGCCAAATACGGGGCCAGGCCCGGAGTCGGAGTTGGAGGCATTCCTACTTACGGGGTTGG
AGCTGGGGGCTTTCCCGGCTTTGGTGTCGGAGTCGGAGGTATCCCTGGAGTCGCAGGTGTCCCTGGTGTC
GGAGGTGTTCCCGGAGTCGGAGGTGTCCCGGGAGTTGGCATTTCCCCCGAAGCTCAGGCAGCAGCTGCCG
CCAAGGCTGCCAAGTACGGGTTAGTTCCTGGTGTCGGCGTGGCTCCTGGAGTTGGCGTGGCTCCTGGTGT
CGGTGTGGCTCCTGGAGTTGGCTTGGCTCCTGGAGTTGGCGTGGCTCCTGGAGTTGGTGTGGCTCCTGGC
GTTGGCGTGGCTCCCGGCATTGGCCCTGGTGGAGTTGCAGCTGCAGCAAAATCCGCTGCCAAGGTGGCTG
CCAAAGCCCAGCTCCGAGCTGCAGCTGGGCTTGGTGCTGGCATCCCTGGACTTGGAGTTGGTGTCGGCGT
CCCTGGACTTGGAGTTGGTGCTGGTGTTCCTGGACTTGGAGTTGGTGCTGGTGTTCCTGGCTTCGGGGCA
GTACCTGGAGCCCTGGCTGCCGCTAAAGCAGCCAAATATGGAGCAGCAGTGCCTGGGGTCCTTGGAGGGC
TCGGGGCTCTCGGTGGAGTAGGCATCCCAGGCGGTGTGGTGGGAGCCGGACCCGCCGCCGCCGCTGCCGC
AGCCAAAGCTGCTGCCAAAGCCGCCCAGTTTGGCCTAGTGGGAGCCGCTGGGCTCGGAGGACTCGGAGTC
GGAGGGCTTGGAGTTCCAGGTGTTGGGGGCCTTGGAGGTATACCTCCAGCTGCAGCCGCTAAAGCAGCTA
AATACGGAGTGGCAGCAAGACCTGGCTTCGGATTGTCTCCCATTTTCCCAGGTGGGGCCTGCCTGGGGAA
AGCTTGTGGCCGGAAGAGAAAATGAGCTTCCTAGGACCCCTGACTCACGACCTCATCAACGTTGGTGCTA
CTGCTTGGTGGAGAATGTAAACCCTTTGTAACCCCATCCCATGCCCCTCCGACTCCCCACCCCAGGAGGG
AACGGGCAGGCCGGGCGGCCTTGCAGATCCACAGGGCAAGGAAACAAGAGGGGAGCGGCCAAGTGCCCCG
ACCAGGAGGCCCCCTACTTCAGAGGCAAGGGCCATGTGGTCCTGGCCCCCCACCCCATCCCTTCCCACCT
AGGAGCTCCCCCTCCACACAGCCTCCATCTCCAGGGGAACTTGGTGCTACACGCTGGTGCTCTTATCTTC
CTGGGGGGAGGAGGAGGGAAGGGTGGCCCCTCGGGGAACCCCCTACCTGGGGCTCCTCTAAAGATGGTG
CAGACACTTCCTGGGCAGTCCCAGCTCCCCCTGCCCACCAGGACCCACCGTTGGCTGCCATCCAGTTGGT
ACCCAAGCACCTGAAGCCTCAAAGCTGGATTCGCTCTAGCATCCCTCCTCTCCTGGGTCCACTTGGCCGT
CTCCTCCCCACCGATCGCTGTTCCCCACATCTGGGGCGCTTTTGGGTTGGAAAACCACCCCACACTGGGA
ATAGCCACCTTGCCCTTGTAGAATCCATCCGCCCATCCGTCCATTCATCCATCGGTCCGTCCATCCATGT
CCCCAGTTGACCGCCCGGCACCACTAGCTGGCTGGGTGCACCCACCATCAACCTGGTTGACCTGTCATGG
CCGCCTGTGCCCTGCCTCCACCCCCATCCTACACTCCCCCAGGGCGTGCGGGGCTGTGCAGACTGGGGTG
CCAGGCATCTCCTCCCCACCCGGGGTGTCCCCACATGCAGTACTGTATACCCCCCATCCCTCCCTCGGTC
CACTGAACTTCAGAGCAGTTCCCATTCCTGCCCCGCCCATCTTTTTGTGTCTCGCTGTGATAGATCAATA
AATATTTTATTTTTTGTCCTGGATATTTGGGGATTATTTTTGATTGTTGATATTCTCTTTTGGTTTTATT
GTTGTGGTTCATTGAAAAAAAAAGATAATTTTTTTTTCTGATCCGGGGAGCTGTATCCCCAGTAGAAAAA
 
ACATTTTAATCACTCTAATATAACTCTGGATGAAACACACCTTTTTTTTAATAAGAAAAGAGAATTAAC
TGCTTCAGAAATGACTAATAAATGAAAAACCTTTAAAGGAAAAAAAAA
NCBI Reference Sequence: NM_005576.2
   **Homo sapiens lysyl oxidase-like 1 (LOXL1), mRNA**
   >gi|67782345|ref|NM_005576.2| Homo sapiens lysyl oxidase-like 1 (LOXL1), mRNA 
CCTGTTGCTTATTCATTCAGAGTGGAAAGCGCCAGCCGAGCGGCCAGCCAGTGCGGGCTGGCCATGTA
AGGCCCACAGGCGGTCCTGCCCGCCCGGTGCCCTGCGGAGAGCCTCGTGCAGCCCTGGGCACCGCCCCTG
CCCTGCCCTGACCCCTTGGCCTTGAAATGCTGTCATCGGAGGAGCCGTCCCGCTCGGGACAAGGCCAGCA
TGGACAAAGCTAGAGCTGGGGCAAGCAAGGAGCCTTCCTGTCCTCGAGGCCGTGGAAGAGAAGCACGCC
CAGGGGGCCACTCCTGAGAGCCTCTCTGTCCACCAGGCCTCTGCAGAGGGGTCACCATGGCTCTGGCCCG
AGGCAGCCGGCAGCTGGGGGCCCTGGTGTGGGGCGCCTGCCTGTGCGTGCTGGTGCACGGGCAGCAGGCG
CAGCCCGGGCAGGGCTCGGACCCCGCCCGCTGGCGGCAGCTGATCCAGTGGGAGAACAACGGGCAGGTGT
ACAGCTTGCTCAACTCGGGCTCAGAGTACGTGCCGGCCGGACCTCAGCGCTCCGAGAGTAGCTCCCGGGT
GCTGCTGGCCGGCGCGCCCCAGGCCCAGCAGCGGCGCAGCCACGGGAGCCCCCGGCGTCGGCAGGCGCCG
TCCCTGCCCCTGCCGGGCGCGTGGGCTCGGACACCGTGCGCGGCCAGGCGCGGCACCCATTCGGCTTTG
GCCAGGTGCCCGACAACTGGCGCGAGGTGGCCGTCGGGGACAGCACGGGCATGGCCCGGGCCCGCACCTC
CGTCTCCCAGCAACGGCACGGGGGCTCCGCCTCCTCGGTCTCGGCTTCGGCCTTCGCCAGCACCTACCGC
CAGCAGCCCTCCTACCCGCAGCAGTTCCCCTACCCGCAGGCGCCCTTCGTCAGCCAGTACGAGAACTACG
ACCCCGCGTCGCGGACCTACGACCAGGGTTTCGTGTACTACCGGCCCGCGGGCGGCGGCGTGGGCGCGGG
GGCGGCGGCCGTGGCCTCGGCGGGGGTCATCTACCCCTACCAGCCCCGGGCGCGCTACGAGGAGTACGGC
GGCGGCGAAGAGCTGCCCGAGTACCCGCCTCAGGGCTTCTACCCGGCCCCCGAGAGGCCCTACGTGCCGC
CGCCGCCGCCGCCCCCCGACGGCCTGGACCGCCGCTACTCGCACAGTCTGTACAGCGAGGGCACCCCCGG
CTTCGAGCAGGCCTACCCTGACCCCGGTCCCGAGGCGGCGCAGGCCCATGGCGGAGACCCACGCCTGGGC
TGGTACCCGCCCTACGCCAACCCGCCGCCCGAGGCGTACGGCCGCCGCGCGCTGGAGCCGCCCTACC
TGCCGGTGCGCAGCTCCGACACGCCCCCGCCGGGTGGGGAGCGGAACGGCGCGCAGCAGGGCCGCCTCAG
CGTGGGCAGCGTGTACCGGCCCAACCAGAACGGCCGCGGTCTCCCTGACTTGGTCCCAGACCCCAACTAT
GTGCAAGCATCCACTTATGTGCAGAGAGCCCACCTGTACTCCCTGCGCTGTGCTGCGGAGGAGAAGTGTC
TGGCCAGCACAGCCTATGCCCCTGAGGCCACCGACTACGATGTGCGGGTGCTACTGCGCTTCCCCCAGCG
CGTGAAGAACCAGGGCACAGCAGACTTCCTCCCCAACCGGCCACGGCACACCTGGGAGTGGCACAGCTGC
CACCAGCATTACCACAGCATGGACGAGTTCAGCCACTACGACCTACTGGATGCAGCCACAGGCAAGAAGG
TGGCCGAGGGCCACAAGGCCAGTTTCTGCCTGGAGGACAGCACCTGTGACTTCGGCAACCTCAAGCGCTA
TGCATGCACCTCTCATACCCAGGGCCTGAGCCCAGGCTGCTATGACACCTACAATGCGGACATCGACTGC
CAGTGGATCGACATAACCGACGTGCAGCCTGGGAACTACATCCTCAAGGTGCACGTGAACCCAAAGTATA
TTGTTTTGGAGTCTGACTTCACCAACAACGTGGTGAGATGCAACATTCACTACACAGGTCGCTACGTTTC
TGCAACAAACTGCAAAATTGTCCAATCCTGATCTCCGGGAGGGACAGATGGCCAATCTCTCCCCTTCCAA
AGCAGGCCCTGCTCCCCGGGCAGCCTCCCGCCGAGGGGCCCAGCCCCCAACCCACAGGCACGGAGGGGCA
TCCCTCCCTGCCGGCCTCAGGGAGCGAACGTGGATGAAAACCACAGGGATTCCGGACGCCAGACCCCATT
TTATACTTCACTTTTCTCTACAGTGTTGTTTTGTTGTTGTTGGTTTTTATTTTTTATACTTTGGCCATAC
CACAGAGCTAGATTGCCCAGGTCTGGGCTGAATAAAACAAGGTTTTTCTAC
NCBI Reference Sequence: NM_003255.4
   **Homo sapiens TIMP metallopeptidase inhibitor 2 (TIMP2), mRNA**
   >gi|73858577|ref|NM_003255.4| Homo sapiens TIMP metallopeptidase inhibitor 2 (TIMP2), mRNA 
CGCAGCAAACACATCCGTAGAAGGCAGCGCGGCCGCCGAGAACCGCAGCGCCGCTCGCCCGCCGCCCCC
ACCCCGCCGCCCCGCCCGGCGAATTGCGCCCCGCGCCCCTCCCCTCGCGCCCCCGAGACAAAGAGGAGAG
 
AAAGTTTGCGCGGCCGAGCGGGGCAGGTGAGGAGGGTGAGCCGCGCGGGAGGGCCCGCCTCGGCCCCGG
CTCAGCCCCCGCCCGCGCCCCCAGCCCGCCGCCGCGAGCAGCGCCCGGACCCCCCAGCGGCGGCCCCCGC
CCGCCCAGCCCCCCGGCCCGCCATGGGCGCCGCGGCCCGCACCCTGCGGCTGGCGCTCGGCCTCCTGCTG
CTGGCGACGCTGCTTCGCCCGGCCGACGCCTGCAGCTGCTCCCCGGTGCACCCGCAACAGGCGTTTTGCA
ATGCAGATGTAGTGATCAGGGCCAAAGCGGTCAGTGAGAAGGAAGTGGACTCTGGAAACGACATTTATGG
CAACCCTATCAAGAGGATCCAGTATGAGATCAAGCAGATAAAGATGTTCAAAGGGCCTGAGAAGGATATA
GAGTTTATCTACACGGCCCCCTCCTCGGCAGTGTGTGGGGTCTCGCTGGACGTTGGAGGAAAGAAGGAAT
ATCTCATTGCAGGAAAGGCCGAGGGGGACGGCAAGATGCACATCACCCTCTGTGACTTCATCGTGCCCTG
GGACACCCTGAGCACCACCCAGAAGAAGAGCCTGAACCACAGGTACCAGATGGGCTGCGAGTGCAAGATC
ACGCGCTGCCCCATGATCCCGTGCTACATCTCCTCCCCGGACGAGTGCCTCTGGATGGACTGGGTCACAG
AGAAGAACATCAACGGGCACCAGGCCAAGTTCTTCGCCTGCATCAAGAGAAGTGACGGCTCCTGTGCGTG
GTACCGCGGCGCGGCGCCCCCCAAGCAGGAGTTTCTCGACATCGAGGACCCATAAGCAGGCCTCCAACGC
CCCTGTGGCCAACTGCAAAAAAAGCCTCCAAGGGTTTCGACTGGTCCAGCTCTGACATCCCTTCCTGGAA
ACAGCATGAATAAAACACTCATCCCATGGGTCCAAATTAATATGATTCTGCTCCCCCCTTCTCCTTTTAG
ACATGGTTGTGGGTCTGGAGGGAGACGTGGGTCCAAGGTCCTCATCCCATCCTCCCTCTGCCAGGCACTA
TGTGTCTGGGGCTTCGATCCTTGGGTGCAGGCAGGGCTGGGACACGCGGCTTCCCTCCCAGTCCCTGCCT
TGGCACCGTCACAGATGCCAAGCAGGCAGCACTTAGGGATCTCCCAGCTGGGTTAGGGCAGGGCCTGGAA
ATGTGCATTTTGCAGAAACTTTTGAGGGTCGTTGCAAGACTGTGTAGCAGGCCTACCAGGTCCCTTTCAT
CTTGAGAGGGACATGGCCCTTGTTTTCTGCAGCTTCCACGCCTCTGCACTCCCTGCCCCTGGCAAGTGCT
CCCATCGCCCCGGTGCCCACCATGAGCTCCCAGCACCTGACTCCCCCACATCCAAGGGCAGCCTGGAAC
CAGTGGCTAGTTCTTGAAGGAGCCCCATCAATCCTATTAATCCTCAGAATTCCAGTGGGAGCCTCCCTCT
GAGCCTTGTAGAAATGGGAGCGAGAAACCCCAGCTGAGCTGCGTTCCAGCCTCAGCTGAGTCTTTTTGGT
CTGCACCCACCCCCCCACCCCCCCCCCCCGCCCACATGCTCCCCAGCTTGCAGGAGGAATCGGTGAGGT
CCTGTCCTGAGGCTGCTGTCCGGGGCCGGTGGCTGCCCTCAAGGTCCCTTCCCTAGCTGCTGCGGTTGCC
ATTGCTTCTTGCCTGTTCTGGCATCAGGCACCTGGATTGAGTTGCACAGCTTTGCTTTATCCGGGCTTGT
GTGCAGGGCCCGGCTGGGCTCCCCATCTGCACATCCTGAGGACAGAAAAAGCTGGGTCTTGCTGTGCCCT
CCCAGGCTTAGTGTTCCCTCCCTCAAAGACTGACAGCCATCGTTCTGCACGGGGCTTTCTGCATGTGACG
CCAGCTAAGCATAGTAAGAAGTCCAGCCTAGGAAGGGAAGGATTTTGGAGGTAGGTGGCTTTGGTGACAC
ACTCACTTCTTTCTCAGCCTCCAGGACACTATGGCCTGTTTTAAGAGACATCTTATTTTTCTAAAGGTGA
ATTCTCAGATGATAGGTGAACCTGAGTTGCAGATATACCAACTTCTGCTTGTATTTCTTAAATGACAAAG
ATTACCTAGCTAAGAAACTTCCTAGGGAACTAGGGAACCTATGTGTTCCCTCAGTGTGGTTTCCTGAAGC
CAGTGATATGGGGGTTAGGATAGGAAGAACTTTCTCGGTAATGATAAGGAGAATCTCTTGTTTCCTCCCA
CCTGTGTTGTAAAGATAAACTGACGATATACAGGCACATTATGTAAACATACACACGCAATGAAACCGAA
GCTTGGCGGCCTGGGCGTGGTCTTGCAAAATGCTTCCAAAGCCACCTTAGCCTGTTCTATTCAGCGGCAA
CCCCAAAGCACCTGTTAAGACTCCTGACCCCCAAGTGGCATGCAGCCCCCATGCCCACCGGGACCTGGTC
AGCACAGATCTTGATGACTTCCCTTTCTAGGGCAGACTGGGAGGGTATCCAGGAATCGGCCCCTGCCCCA
CGGGCGTTTTCATGCTGTACAGTGACCTAAAGTTGGTAAGATGTCATAATGGACCAGTCCATGTGATTTC
AGTATATACAACTCCACCAGACCCCTCCAACCCATATAACACCCCACCCCTGTTCGCTTCCTGTATGGTG
ATATCATATGTAACATTTACTCCTGTTTCTGCTGATTGTTTTTTAATGTTTTGGTTTGTTTTTGACATC
AGCTGTAATCATTCCTGTGCTGTGTTTTTTATTACCCTTGGTAGGTATTAGACTTGCACTTTTTTAAAAA
AAGGTTTCTGCATCGTGGAAGCATTTGACCCAGAGTGGAACGCGTGGCCTATGCAGGTGGATTCCTTCAG
GTCTTTCCTTTGGTTCTTTGAGCATCTTTGCTTTCATTCGTCTCCCGTCTTTGGTTCTCCAGTTCAAATT
ATTGCAAAGTAAAGGATCTTTGAGTAGGTTCGGTCTGAAAGGTGTGGCCTTTATATTTGATCCACACACG
TTGGTCTTTTAACCGTGCTGAGCAGAAAACAAAACAGGTTAAGAAGAGCCGGGTGGCAGCTGACAGAGGA
AGCCGCTCAAATACCTTCACAATAAATAGTGGCAATATATATATAGTTTAAGAAGGCTCTCCATTTGGCA
TCGTTTAATTTATATGTTATGTTCTAAGCACAGCTCTCTTCTCCTATTTTCATCCTGCAAGCAACTCAAA
ATATTTAAAATAAAGTTTACATTGTAGTTATTTTCAAATCTTTGCTTGATAAGTATTAAGAAATATTGGA
CTTGCTGCCGTAATTTAAAGCTCTGTTGATTTTGTTTCCGTTTGGATTTTTGGGGGAGGGGAGCACTGTG
TTTATGCTGGAATATGAAGTCTGAGACCTTCCGGTGCTGGGAACACACAAGAGTTGTTGAAAGTTGACAA
GCAGACTGCGCATGTCTCTGATGCTTTGTATCATTCTTGAGCAATCGCTCGGTCCGTGGACAATAAACAG
TATTATCAAAGAGAAAAAAAAAAAAAAAAA
NCBI Reference Sequence: NM_000575.3
   **Homo sapiens interleukin 1, alpha (IL1A), mRNA**
   >gi|27894329|ref|NM_000575.3| Homo sapiens interleukin 1, alpha (IL1A), mRNA 
ACCAGGCAACACCATTGAAGGCTCATATGTAAAAATCCATGCCTTCCTTTCTCCCAATCTCCATTCCCAA
ACTTAGCCACTGGCTTCTGGCTGAGGCCTTACGCATACCTCCCGGGGCTTGCACACACCTTCTTCTACAG
AAGACACACCTTGGGCATATCCTACAGAAGACCAGGCTTCTCTCTGGTCCTTGGTAGAGGGCTACTTTAC
TGTAACAGGGCCAGGGTGGAGAGTTCTCTCCTGAAGCTCCATCCCCTCTATAGGAAATGTGTTGACAATA
TTCAGAAGAGTAAGAGGATCAAGACTTCTTTGTGCTCAAATACCACTGTTCTCTTCTCTACCCTGCCCTA
ACCAGGAGCTTGTCACCCCAAACTCTGAGGTGATTTATGCCTTAATCAAGCAAACTTCCCTCTTCAGAAA
AGATGGCTCATTTTCCCTCAAAAGTTGCCAGGAGCTGCCAAGTATTCTGCCAATTCACCCTGGAGCACAA
TCAACAAATTCAGCCAGAACACAACTACAGCTACTATTAGAACTATTATTATTAATAAATTCCTCTCCAA
ATCTAGCCCCTTGACTTCGGATTTCACGATTTCTCCCTTCCTCCTAGAAACTTGATAAGTTTCCCGCGCT
TCCCTTTTTCTAAGACTACATGTTTGTCATCTTATAAAGCAAAGGGGTGAATAAATGAACCAAATCAATA
ACTTCTGGAATATCTGCAAACAACAATAATATCAGCTATGCCATCTTTCACTATTTAGCCAGTATCGAG
TTGAATGAACATAGAAAAATACAAAACTGAATTCTTCCCTGTAAATTCCCCGTTTTGACGACGCACTTGT
AGCCACGTAGCCACGCCTACTTAAGACAATTACAAAAGGCGAAGAAGACTGACTCAGGCTTAAGCTGCCA
GCCAGAGAGGGAGTCATTTCATTGGCGTTTGAGTCAGCAAAGAAGTCAAGATGGCCAAAGTTCCAGACAT
GTTTGAAGACCTGAAGAACTGTTACAGTGAAAATGAAGAAGACAGTTCCTCCATTGATCATCTGTCTCTG
AATCAGAAATCCTTCTATCATGTAAGCTATGGCCCACTCCATGAAGGCTGCATGGATCAATCTGTGTCTC
TGAGTATCTCTGAAACCTCTAAAACATCCAAGCTTACCTTCAAGGAGAGCATGGTGGTAGTAGCAACCAA
CGGGAAGGTTCTGAAGAAGAGACGGTTGAGTTTAAGCCAATCCATCACTGATGATGACCTGGAGGCCATC
GCCAATGACTCAGAGGAAGAAATCATCAAGCCTAGGTCAGCACCTTTTAGCTTCCTGAGCAATGTGAAAT
ACAACTTTATGAGGATCATCAAATACGAATTCATCCTGAATGACGCCCTCAATCAAAGTATAATTCGAGC
CAATGATCAGTACCTCACGGCTGCTGCATTACATAATCTGGATGAAGCAGTGAAATTTGACATGGGTGCT
TATAAGTCATCAAAGGATGATGCTAAAATTACCGTGATTCTAAGAATCTCAAAAACTCAATTGTATGTGA
CTGCCCAAGATGAAGACCAACCAGTGCTGCTGAAGGAGATGCCTGAGATACCCAAAACCATCACAGGTAG
TGAGACCAACCTCCTCTTCTTCTGGAAACTCACGGCACTAAGAACTATTTCACATCAGTTGCCCATCCA
AACTTGTTTATTGCCACAAAGCAAGACTACTGGGTGTGCTTGGCAGGGGGCCACCCTCTATCACTGACT
TTCAGATACTGGAAAACCAGGCGTAGGTCTGGAGTCTCACTTGTCTCACTTGTGCAGTGTTGACAGTTCA
TATGTACCATGTACATGAAGAAGCTAAATCCTTTACTGTTAGTCATTTGCTGAGCATGTACTGAGCCTTG
TAATTCTAAATGAATGTTTACACTCTTTGTAAGAGTGGAACCAACACTAACATATAATGTTGTTATTTAA
AGAACACCCTATATTTTGCATAGTACCAATCATTTTAATTATTATTCTTCATAACAATTTTAGGAGGACC
AGAGCTACTGACTATGGCTACCAAAAAGACTCTACCCATATTACAGATGGGCAAATTAAGGCATAAGAAA
ACTAAGAAATATGCACAATAGCAGTTGAAACAAGAAGCCACAGACCTAGGATTTCATGATTTCATTTCAA
CTGTTTGCCTTCTACTTTTAAGTTGCTGATGAACTCTTAATCAAATAGCATAAGTTTCTGGGACCTCAGT
TTTATCATTTTCAAAATGGAGGGAATAATACCTAAGCCTTCCTGCCGCAACAGTTTTTATGCTAATCAG
GGAGGTCATTTTGGTAAAATACTTCTTGAAGCCGAGCCTCAAGATGAAGGCAAAGCACGAAATGTTATTT
TTTAATTATTATTTATATATGTATTTATAAATATATTTAAGATAATTATAATATACTATATTTATGGAA
CCCCTTCATCCTCTGAGTGTGACCAGGCATCCTCCACAATAGCAGACAGTGTTTTCTGGATAAGTAAGT
TTGATTTCATTAATACAGGGCATTTTGGTCCAAGTTGTGCTTATCCCATAGCCAGGAAACTCTGCATTCT
AGTACTTGGGAGACCTGTAATCATATAATAAATGTACATTAATTACCTTGAGCCAGTAATTGGTCCGATC
TTTGACTCTTTTGCCATTAAACTTACCTGGGCATTCTTGTTTCAATTCCACCTGCAATCAAGTCCTACAA
GCTAAAATTAGATGAACTCAACTTTGACAACCATGAGACCACTGTTATCAAAACTTTCTTTTCTGGAATG
TAATCAATGTTTCTTCTAGGTTCTAAAAATTGTGATCAGACCATAATGTTACATTATTATCAACAATAGT
GATTGATAGAGTGTTATCAGTCATAACTAAATAAAGCTTGCAACAAAATTCTCTGACAAAAAAAAAAAAA
AAA
NCBI Reference Sequence: NM_003254.2
   **Homo sapiens TIMP metallopeptidase inhibitor 1 (TIMP1), mRNA**
   >gi|73858576|ref|NM_003254.2| Homo sapiens TIMP metallopeptidase inhibitor 1 (TIMP1), mRNA TTTCGTCGGCCCGCCCCTTGGCTTCTGCACTGATGGTGGTGGATGAGTAATGCATCCAGGAAGCCTGGA 
GGCCTGTGGTTTCCGCACCCGCTGCCACCCCCGCCCCTAGCGTGGACATTTATCCTCTAGCGCTCAGGCC
CTGCCGCCATCGCCGCAGATCCAGCGCCCAGAGAGACACCAGAGAACCCACCATGGCCCCCTTTGAGCCC
CTGGCTTCTGGCATCCTGTTGTTGCTGTGGCTGATAGCCCCCAGCAGGGCCTGCACCTGTGTCCCACCCC
ACCCACAGACGGCCTTCTGCAATTCCGACCTCGTCATCAGGGCCAAGTTCGTGGGGACACCAGAAGTCAA
CCAGACCACCTTATACCAGCGTTATGAGATCAAGATGACCAAGATGTATAAAGGGTTCCAAGCCTTAGGG
GATGCCGCTGACATCCGGTTCGTCTACACCCCCGCCATGGAGAGTGTCTGCGGATACTTCCACAGGTCCC
ACAACCGCAGCGAGGAGTTTCTCATTGCTGGAAAACTGCAGGATGGACTCTTGCACATCACTACCTGCAG
TTTTGTGGCTCCCTGGAACAGCCTGAGCTTAGCTCAGCGCCGGGGCTTCACCAAGACCTACACTGTTGGC
TGTGAGGAATGCACAGTGTTTCCCTGTTTATCCATCCCCTGCAAACTGCAGAGTGGCACTCATTGCTTGT
GGACGGACCAGCTCCTCCAAGGCTCTGAAAAGGGCTTCCAGTCCCGTCACCTTGCCTGCCTGCCTCGGGA
GCCAGGGCTGTGCACCTGGCAGTCCCTGCGGTCCCAGATAGCCTGAATCCTGCCCGGAGTGGAAGCTGAA
GCCTGCACAGTGTCCACCCTGTTCCCACTCCCATCTTTCTTCCGGACAATGAAATAAAGAGTTACCACCC
AGCAGAAAAAAAAAAAAAAAA
NCBI Reference Sequence: NM_000594.2
   **Homo sapiens tumor necrosis factor (TNF superfamily, member 2) (TNF), mRNA**
   >gi|25952110|ref|NM_000594.2| Homo sapiens tumor necrosis factor (TNF superfamily, member 2) (TNF), mRNA 
CTCCCTCAGCAAGGACAGCAGAGGACCAGCTAAGAGGGAGAGAAGCAACTACAGACCCCCCCTGAAAACA
ACCCTCAGACGCCACATCCCCTGACAAGCTGCCAGGCAGGTTCTCTTCCTCTCACATACTGACCCACGGC
TCCACCCTCTCTCCCCTGGAAAGGACACCATGAGCACTGAAAGCATGATCCGGGACGTGGAGCTGGCCGA
GGAGGCGCTCCCCAAGAAGACAGGGGGGCCCCAGGGCTCCAGGCGGTGCTTGTTCCTCAGCCTCTTCTCC
TTCCTGATCGTGGCAGGCGCCACCACGCTCTTCTGCCTGCTGCACTTTGGAGTGATCGGCCCCCAGAGGG
AAGAGTTCCCCAGGGACCTCTCTCTAATCAGCCCTCTGGCCCAGGCAGTCAGATCATCTTCTCGAACCCC
GAGTGACAAGCCTGTAGCCCATGTTGTAGCAAACCCTCAAGCTGAGGGGCAGCTCCAGTGGCTGAACCGC
CGGGCCAATGCCCTCCTGGCCAATGGCGTGGAGCTGAGAGATAACCAGCTGGTGGTGCCATCAGAGGGCC
TGTACCTCATCTACTCCCAGGTCCTCTTCAAGGGCCAAGGCTGCCCCTCCACCCATGTGCTCCTCACCCA
CACCATCAGCCGCATCGCCGTCTCCTACCAGACCAAGGTCAACCTCCTCTCTGCCATCAAGAGCCCCTGC
CAGAGGGAGACCCCAGAGGGGGCTGAGGCCAAGCCCTGGTATGAGCCCATCTATCTGGGAGGGGTCTTCC
AGCTGGAGAAGGGTGACCGACTCAGCGCTGAGATCAATCGGCCCGACTATCTCGACTTTGCCGAGTCTGG
GCAGGTCTACTTTGGGATCATTGCCCTGTGAGGAGGACGAACATCCAACCTTCCCAAACGCCTCCCCTGC
CCCAATCCCTTTATTACCCCCTCCTTCAGACACCCTCAACCTCTTCTGGCTCAAAAAGAGAATTGGGGGC
TTAGGGTCGGAACCCAAGCTTAGAACTTTAAGCAACAAGACCACCACTTCGAAACCTGGGATTCAGGAAT
GTGTGGCCTGCACAGTGAAGTGCTGGCAACCACTAAGAATTCAAACTGGGGCCTCCAGAACTCACTGGGG
CCTACAGCTTTGATCCCTGACATCTGGAATCTGGAGACCAGGGAGCCTTTGGTTCTGGCCAGAATGCTGC
AGGACTTGAGAAGACCTCACCTAGAAATTGACACAAGTGGACCTTAGGCCTTCCTCTCTCCAGATGTTTC
CAGACTTCCTTGAGACACGGAGCCCAGCCCTCCCCATGGAGCCAGCTCCCTCTATTTATGTTTGCACTTG
TGATTATTTATTATTTATTTATTATTTATTTATTTACAGATGAATGTATTTATTTGGGAGACCGGGGTAT
CCTGGGGGACCCAATGTAGGAGCTGCCTTGGCTCAGACATGTTTTCCGTGAAAACGGAGCTGAACAATAG
GCTGTTCCCATGTAGCCCCCTGGCCTCTGTGCCTTCTTTTGATTATGTTTTTTAAAATATTTATCTGATT
AAGTTGTCTAAACAATGCTGATTTGGTGACCAACTGTCACTCATTGCTGAGCCTCTGCTCCCCAGGGGAG
TTGTGTCTGTAATCGCCCTACTATTCAGTGGCGAGAAATAAAGTTTGCTTAGAAAAGAA
NCBI Reference Sequence: NM_002421.2
   **Homo sapiens matrix metallopeptidase 1 (interstitial collagenase) (MMP1), mRNA**
   >gi|13027798|ref|NM_002421.2| Homo sapiens matrix metallopeptidase 1 (interstitial collagenase) (MMP1), mRNA 
GGGATATTGGAGTAGCAAGAGGCTGGGAAGCCATCACTTACCTTGCACTGAGAAAGAAGACAAAGGCCAG
TATGCACAGCTTTCCTCCACTGCTGCTGCTGCTGTTCTGGGGTGTGGTGTCTCACAGCTTCCCAGCGACT
CTAGAAACACAAGAGCAAGATGTGGACTTAGTCCAGAAATACCTGGAAAAATACTACAACCTGAAGAATG
ATGGGAGGCAAGTTGAAAAGCGGAGAAATAGTGGCCCAGTGGTTGAAAAATTGAAGCAAATGCAGGAATT
CTTTGGGCTGAAAGTGACTGGGAAACCAGATGCTGAAACCCTGAAGGTGATGAAGCAGCCCAGATGTGGA
GTGCCTGATGTGGCTCAGTTTGTCCTCACTGAGGGGAACCCTCGCTGGGAGCAAACACATCTGACCTACA
GGATTGAAAATTACACGCCAGATTTGCCAAGAGCAGATGTGGACCATGCCATTGAGAAAGCCTTCCAACT
CTGGAGTAATGTCACACCTCTGACATTCACCAAGGTCTCTGAGGGTCAAGCAGACATCATGATATCTTTT
GTCAGGGGAGATCATCGGGACAACTCTCCTTTTGATGGACCTGGAGGAAATCTTGCTCATGCTTTTCAAC
CAGGCCAGGTATTGGAGGGGATGCTCATTTTGATGAAGATGAAAGGTGGACCAACAATTTCAGAGAGTA
CAACTTACATCGTGTTGCGGCTCATGAACTCGGCCATTCTCTTGGACTCTCCCATTCTACTGATATCGGG
GCTTTGATGTACCCTAGCTACACCTTCAGTGGTGATGTTCAGCTAGCTCAGGATGACATTGATGGCATCC
AAGCCATATATGGACGTTCCCAAAATCCTGTCCAGCCCATCGGCCCACAAACCCCAAAAGCGTGTGACAG
TAAGCTAACCTTTGATGCTATAACTACGATTCGGGGAGAAGTGATGTTCTTTAAAGACAGATTCTACATG
CGCACAAATCCCTTCTACCCGGAAGTTGAGCTCAATTTCATTTCTGTTTTCTGGCCACAACTGCCAAATG
GGCTTGAAGCTGCTTACGAATTTGCCGACAGAGATGAAGTCCGGTTTTTCAAAGGGAATAAGTACTGGGC
TGTTCAGGGACAGAATGTGCTACACGGATACCCCAAGGACATCTACAGCTCCTTTGGCTTCCCTAGAACT
GTGAAGCATATCGATGCTGCTCTTTCTGAGGAAAACACTGGAAAAACCTACTTCTTTGTTGCTAACAAAT
ACTGGAGGTATGATGAATATAAACGATCTATGGATCCAGGTTATCCCAAAATGATAGCACATGACTTTCC
TGGAATTGGCCACAAAGTTGATGCAGTTTTCATGAAAGATGGATTTTTCTATTTCTTTCATGGAACAAGA
CAATACAAATTTGATCCTAAAACGAAGAGAATTTTGACTCTCCAGAAAGCTAATAGCTGGTTCAACTGCA
GGAAAAATTGAACATTACTAATTTGAATGGAAAACACATGGTGTGAGTCCAAAGAAGGTGTTTTCCTGAA
GAACTGTCTATTTTCTCAGTCATTTTTAACCTCTAGAGTCACTGATACACAGAATATAATCTTATTTATA
CCTCAGTTTGCATATTTTTTTACTATTTAGAATGTAGCCCTTTTTGTACTGATATAATTTAGTTCCACAA
ATGGTGGGTACAAAAAGTCAAGTTTGTGGCTTATGGATTCATATAGGCCAGAGTTGCAAAGATCTTTTCC
AGAGTATGCAACTCTGACGTTGATCCCAGAGAGCAGCTTCAGTGACAAACATATCCTTTCAAGACAGAAA
GAGACAGGAGACATGAGTCTTTGCCGGAGGAAAAGCAGCTCAAGAACACATGTGCAGTCACTGGTGTCAC
CCTGGATAGGCAAGGGATAACTCTTCTAACACAAAATAAGTGTTTTATGTTTGGAATAAAGTCAACCTTG
TTTCTACTGTTTT
NCBI Reference Sequence: NM_004994.2
   **Homo sapiens matrix metallopeptidase 9 (gelatinase B, 92kDa gelatinase, 92kDa type IV collagenase) (MMP9), mRNA**
   >gi|74272286|ref|NM_004994.2| Homo sapiens matrix metallopeptidase 9 (gelatinase B, 92kDa gelatinase, 92kDa type IV collagenase) (MMP9), mRNA 
AGACACCTCTGCCCTCACCATGAGCCTCTGGCAGCCCCTGGTCCTGGTGCTCCTGGTGCTGGGCTGCTGC
TTTGCTGCCCCCAGACAGCGCCAGTCCACCCTTGTGCTCTTCCCTGGAGACCTGAGAACCAATCTCACCG
ACAGGCAGCTGGCAGAGGAATACCTGTACCGCTATGGTTACACTCGGGTGGCAGAGATGCGTGGAGAGTC
GAAATCTCTGGGGCCTGCGCTGCTGCTTCTCCAGAAGCAACTGTCCCTGCCCGAGACCGGTGAGCTGGAT
AGCGCCACGCTGAAGGCCATGCGAACCCCACGGTGCGGGGTCCCAGACCTGGGCAGATTCCAAACCTTTG
AGGGCGACCTCAAGTGGCACCACCACAACATCACCTATTGGATCCAAAACTACTCGGAAGACTTGCCGCG
GGCGGTGATTGACGACGCCTTTGCCCGCGCCTTCGCACTGTGGAGCGCGGTGACGCCGCTCACCTTCACT
CGCGTGTACAGCCGGGACGCAGACATCGTCATCCAGTTTGGTGTCGCGGAGCACGGAGACGGGTATCCCT
TCGACGGGAAGGACGGGCTCCTGGCACACGCCTTTCCTCCTGGCCCCGGCATTCAGGGAGACGCCCATTT
CGACGATGACGAGTTGTGGTCCCTGGGCAAGGGCGTCGTGGTTCCAACTCGGTTTGGAAACGCAGATGGC
GCGGCCTGCCACTTCCCCTTCATCTTCGAGGGCCGCTCCTACTCTGCCTGCACCACCGACGGTCGCTCCG
ACGGCTTGCCCTGGTGCAGTACCACGGCCAACTACGACACCGACGACCGGTTTGGCTTCTGCCCCAGCGA
GAGACTCTACACCCAGGACGGCAATGCTGATGGGAAACCCTGCCAGTTTCCATTCATCTTCCAAGGCCAA
TCCTACTCCGCCTGCACCACGGACGGTCGCTCCGACGGCTACCGCTGGTGCGCCACCACCGCCAACTACG
 
ACCGGGACAAGCTCTTCGGCTTCTGCCCGACCCGAGCTGACTCGACGGTGATGGGGGCAACTCGGCGGG
GGAGCTGTGCGTCTTCCCCTTCACTTTCCTGGGTAAGGAGTACTCGACCTGTACCAGCGAGGGCCGCGGA
GATGGGCGCCTCTGGTGCGCTACCACCTCGAACTTTGACAGCGACAAGAAGTGGGGCTTCTGCCCGGACC
AAGGATACAGTTTGTTCCTCGTGGCGGCGCATGAGTTCGGCCACGCGCTGGGCTTAGATCATTCCTCAGT
GCCGGAGGCGCTCATGTACCCTATGTACCGCTTCACTGAGGGGCCCCCCTTGCATAAGGACGACGTGAAT
GGCATCCGGCACCTCTATGGTCCTCGCCCTGAACCTGAGCCACGGCCTCCAACCACCACCACACCGCAGC
CCACGGCTCCCCCGACGGTCTGCCCCACCGGACCCCCCACTGTCCACCCCTCAGAGCGCCCCACAGCTGG
CCCCACAGGTCCCCCCTCAGCTGGCCCCACAGGTCCCCCCACTGCTGGCCCTTCTACGGCCACTACTGTG
CCTTTGAGTCCGGTGGACGATGCCTGCAACGTGAACATCTTCGACGCCATCGCGGAGATTGGGAACCAGC
TGTATTTGTTCAAGGATGGGAAGTACTGGCGATTCTCTGAGGGCAGGGGGAGCCGGCCGCAGGGCCCCTT
CCTTATCGCCGACAAGTGGCCCGCGCTGCCCCGCAAGCTGGACTCGGTCTTTGAGGAGCGGCTCTCCAAG
AAGCTTTTCTTCTTCTCTGGGCGCCAGGTGTGGGTGTACACAGGCGCGTCGGTGCTGGGCCCGAGGCGTC
TGGACAAGCTGGGCCTGGGAGCCGACGTGGCCCAGGTGACCGGGGCCCTCCGGAGTGGCAGGGGGAAGAT
GCTGCTGTTCAGCGGGCGGCGCCTCTGGAGGTTCGACGTGAAGGCGCAGATGGTGGATCCCCGGAGCGCC
AGCGAGGTGGACCGGATGTTCCCCGGGGTGCCTTTGGACACGCACGACGTCTTCCAGTACCGAGAGAAAG
CCTATTTCTGCCAGGACCGCTTCTACTGGCGCGTGAGTTCCCGGAGTGAGTTGAACCAGGTGGACCAAGT
GGGCTACGTGACCTATGACATCCTGCAGTGCCCTGAGGACTAGGGCTCCCGTCCTGCTTTGGCAGTGCCA
TGTAAATCCCCACTGGGACCAACCCTGGGGAAGGAGCCAGTTTGCCGGATACAAACTGGTATTCTGTTCT
GGAGGAAAGGGAGGAGTGGAGGTGGGCTGGGCCCTCTCTTCTCACCTTTGTTTTTTGTTGGAGTGTTTCT
AATAAACTTGGATTCTCTAACCTTTAAAAAAAAAAAAAAAAAAAAAAAAAAAAAAAAAAAAAAAAAAAAA
AAAAAAA
NCBI Reference Sequence: NM_002422.3
   **Homo sapiens matrix metallopeptidase 3 (stromelysin 1, progelatinase) (MMP3), mRNA**
   >gi|73808272|ref|NM_002422.3| Homo sapiens matrix metallopeptidase 3 (stromelysin 1, progelatinase) (MMP3), mRNA 
CTACAAGGAGGCAGGCAAGACAGCAAGGCATAGAGACAACATAGAGCTAAGTAAAGCCAGTGGAAATGAA
GAGTCTTCCAATCCTACTGTTGCTGTGCGTGGCAGTTTGCTCAGCCTATCCATTGGATGGAGCTGCAAGG
GGTGAGGACACCAGCATGAACCTTGTTCAGAAATATCTAGAAAACTACTACGACCTCAAAAAAGATGTGA
AACAGTTTGTTAGGAGAAAGGACAGTGGTCCTGTTGTTAAAAAAATCCGAGAAATGCAGAAGTTCCTTGG
ATTGGAGGTGACGGGGAAGCTGGACTCCGACACTCTGGAGGTGATGCGCAAGCCCAGGTGTGGAGTTCCT
GATGTTGGTCACTTCAGAACCTTTCCTGGCATCCCGAAGTGGAGGAAAACCCACCTTACATACAGGATTG
TGAATTATACACCAGATTTGCCAAAAGATGCTGTTGATTCTGCTGTTGAGAAAGCTCTGAAAGTCTGGGA
AGAGGTGACTCCACTCACATTCTCCAGGCTGTATGAAGGAGAGGCTGATATAATGATCTCTTTTGCAGTT
AGAGAACATGGAGACTTTTACCCTTTTGATGGACCTGGAAATGTTTTGGCCCATGCCTATGCCCCTGGGC
CAGGGATTAATGGAGATGCCCACTTTGATGATGATGAACAATGGACAAAGGATACAACAGGGACCAATTT
ATTTCTCGTTGCTGCTCATGAAATTGGCCACTCCCTGGGTCTCTTTCACTCAGCCAACACTGAAGCTTTG
ATGTACCCACTCTATCACTCACTCACAGACCTGACTCGGTTCCGCCTGTCTCAAGATGATATAAATGGCA
TTCAGTCCCTCTATGGACCTCCCCCTGACTCCCCTGAGACCCCCCTGGTACCCACGGAACCTGTCCCTCC
AGAACCTGGGACGCCAGCCAACTGTGATCCTGCTTTGTCCTTTGATGCTGTCAGCACTCTGAGGGGAGAA
ATCCTGATCTTTAAAGACAGGCACTTTTGGCGCAAATCCCTCAGGAAGCTTGAACCTGAATTGCATTTGA
TCTCTTCATTTTGGCCATCTCTTCCTTCAGGCGTGGATGCCGCATATGAAGTTACTAGCAAGGACCTCGT
TTTCATTTTTAAAGGAAATCAATTCTGGGCTATCAGAGGAAATGAGGTACGAGCTGGATACCCAAGAGGC
ATCCACACCCTAGGTTTCCCTCCAACCGTGAGGAAAATCGATGCAGCCATTTCTGATAAGGAAAAGAACA
AAACATATTTCTTTGTAGAGGACAAATACTGGAGATTTGATGAGAAGAGAAATTCCATGGAGCCAGGCTT
TCCCAAGCAAATAGCTGAAGACTTTCCAGGGATTGACTCAAAGATTGATGCTGTTTTTGAAGAATTTGGG
TTCTTTTATTTCTTTACTGGATCTTCACAGTTGGAGTTTGACCCAAATGCAAAGAAAGTGACACACACTT
TGAAGAGTAACAGCTGGCTTAATTGTTGAAAGAGATATGTAGAAGGCACAATATGGGCACTTTAAATGAA
GCTAATAATTCTTCACCTAAGTCTCTGTGAATTGAAATGTTCGTTTTCTCCTGCCTGTGCTGTGACTCGA
GTCACACTCAAGGGAACTTGAGCGTGAATCTGTATCTTGCCGGTCATTTTTATGTTATTACAGGGCATTC
AAATGGGCTGCTGCTTAGCTTGCACCTTGTCACATAGAGTGATCTTTCCCAAGAGAAGGGGAAGCACTCG
 
TGTGCAACAGACAAGTGACTGTATCTGTGTAGACTATTTGCTTATTTAATAAAGACGATTTGTCAGTTAT
TTTATCTT
NCBI Reference Sequence: NM_001024466.1
   **Homo sapiens superoxide dismutase 2, mitochondrial (SOD2), nuclear gene encoding mitochondrial protein, transcript variant 3, mRNA**
   >gi|67782308|ref|NM_001024466.1| Homo sapiens superoxide dismutase 2, mitochondrial (SOD2), nuclear gene encoding mitochondrial protein, transcript variant 3, mRNA 
GCGGTGCCCTTGCGGCGCAGCTGGGGTCGCGGCCCTGCTCCCGCGCTTTCTTAAGGCCGCGGGCGGCG
CAGGAGCGGCACTCGTGGCTGTGGTGGCTTCGGCAGCGGCTTCAGCAGATCGGCGGCATCAGCGGTAGCA
CCAGCACTAGCAGCATGTTGAGCCGGGCAGTGTGCGGCACCAGCAGGCAGCTGGCTCCGGTTTTGGGGTA
TCTGGGCTCCAGGCAGAAGCACAGCCTCCCCGACCTGCCCTACGACTACGGCGCCCTGGAACCTCACATC
AACGCGCAGATCATGCAGCTGCACCACAGCAAGCACCACGCGGCCTACGTGAACAACCTGAACGTCACCG
AGGAGAAGTACCAGGAGGCGTTGGCCAAGGGGGAGTTGCTGGAAGCCATCAAACGTGACTTTGGTTCCTT
TGACAAGTTTAAGGAGAAGCTGACGGCTGCATCTGTTGGTGTCCAAGGCTCAGGTTGGGGTTGGCTTGGT
TTCAATAAGGAACGGGGACACTTACAAATTGCTGCTTGTCCAAATCAGGATCCACTGCAAGGAACAACAG
GCCTTATTCCACTGCTGGGGATTGATGTGTGGGAGCACGCTTACTACCTTCAGTATAAAAATGTCAGGCC
TGATTATCTAAAAGCTATTTGGAATGTAATCAACTGGGAGAATGTAACTGAAAGATACATGGCTTGCAAA
AAGTAAACCACGATCGTTATGCTGATCATACCCTAATGATCCCAGCAAGATAATGTCCTGTCTTCTAAGA
TGTGCATCAAGCCTGGTACATACTGAAAACCCTATAAGGTCCTGGATAATTTTTGTTTGATTATTCATTG
AAGAAACATTTATTTTCCAATTGTGTGAAGTTTTTGACTGTTAATAAAAGAATCTGTCAACCATCAAAAA
AAAAAAAA
NCBI Reference Sequence: NM_001024465.1
   **Homo sapiens superoxide dismutase 2, mitochondrial (SOD2), nuclear gene encoding mitochondrial protein, transcript variant 2, mRNA**
   >gi|67782306|ref|NM_001024465.1| Homo sapiens superoxide dismutase 2, mitochondrial (SOD2), nuclear gene encoding mitochondrial protein, transcript variant 2, mRNA 
GCGGTGCCCTTGCGGCGCAGCTGGGGTCGCGGCCCTGCTCCCCGCGCTTTCTTAAGGCCCGCGGGCGGCG
CAGGAGCGGCACTCGTGGCTGTGGTGGCTTCGGCAGCGGCTTCAGCAGATCGGCGGCATCAGCGGTAGCA
CCAGCACTAGCAGCATGTTGAGCCGGGCAGTGTGCGGCACCAGCAGGCAGCTGGCTCCGGTTTTGGGGTA
TCTGGGCTCCAGGCAGAAGCACAGCCTCCCCGACCTGCCCTACGACTACGGCGCCCTGGAACCTCACATC
AACGCGCAGATCATGCAGCTGCACCACAGCAAGCACCACGCGGCCTACGTGAACAACCTGAACGTCACCG
AGGAGAAGTACCAGGAGGCGTTGGCCAAGGGAGATGTTACAGCCCAGATAGCTCTTCAGCCTGCACTGAA
GTTCAATGGTGGTGGTCATATCAATCATAGCATTTTCTGGACAAACCTCAGCCCTAACGGTGGTGGAGAA
CCCAAAGGGGAGTTGCTGGAAGCCATCAAACGTGACTTTGGTTCCTTTGACAAGTTTAAGGAGAAGCTGA
CGGCTGCATCTGTTGGTGTCCAAGGCTCAGGTTGGGGTTGGCTTGGTTTCAATAAGGAACGGGGACACTT
ACAAATTGCTGCTTGTCCAAATCAGGATCCACTGCAAGGAACAACAGGCCTTATTCCACTGCTGGGGATT
GATGTGTGGGAGCACGCTTACTACCTTCAGTATAAAAATGTCAGGCCTGATTATCTAAAAGCTATTTGGA
ATGTAATCAACTGGGAGAATGTAACTGAAAGATACATGGCTTGCAAAAAGTAAACCACGATCGTTATGCT
GATCATACCCTAATGATCCCAGCAAGATAATGTCCTGTCTTCTAAGATGTGCATCAAGCCTGGTACATAC
TGAAAACCCTATAAGGTCCTGGATAATTTTTGTTTGATTATTCATTGAAGAAACATTTATTTTCCAATTG
TGTGAAGTTTTTGACTGTTAATAAAAGAATCTGTCAACCATCAAAAAAAAAAAAA
NCBI Reference Sequence: NM_000636.2
   **Homo sapiens superoxide dismutase 2, mitochondrial (SOD2), nuclear gene encoding mitochondrial protein, transcript variant 1, mRNA**
   >gi|67782304|ref|NM_000636.2| Homo sapiens superoxide dismutase 2, mitochondrial (SOD2), nuclear gene encoding mitochondrial protein, transcript variant 1, mRNA 
GCGGTGCCCTTGCGGCGCAGCTGGGGTCGCGGCCCTGCTCCCCGCGCTTTCTTAAGGCCCGCGGGCGGCG
CAGGAGCGGCACTCGTGGCTGTGGTGGCTTCGGCAGCGGCTTCAGCAGATCGGCGGCATCAGCGGTAGCA
CCAGCACTAGCAGCATGTTGAGCCGGGCAGTGTGCGGCACCAGCAGGCAGCTGGCTCCGGTTTTGGGGTA
TCTGGGCTCCAGGCAGAAGCACAGCCTCCCCGACCTGCCCTACGACTACGGCGCCCTGGAACCTCACATC
AACGCGCAGATCATGCAGCTGCACCACAGCAAGCACCACGCGGCCTACGTGAACAACCTGAACGTCACCG
AGGAGAAGTACCAGGAGGCGTTGGCCAAGGGAGATGTTACAGCCCAGATAGCTCTTCAGCCTGCACTGAA
GTTCAATGGTGGTGGTCATATCAATCATAGCATTTTCTGGACAAACCTCAGCCCTAACGGTGGTGGAGAA
CCCAAAGGGGAGTTGCTGGAAGCCATCAAACGTGACTTTGGTTCCTTTGACAAGTTTAAGGAGAAGCTGA
CGGCTGCATCTGTTGGTGTCCAAGGCTCAGGTTGGGGTTGGCTTGGTTTCAATAAGGAACGGGGACACTT
ACAAATTGCTGCTTGTCCAAATCAGGATCCACTGCAAGGAACAACAGGCCTTATTCCACTGCTGGGGATT
GATGTGTGGGAGCACGCTTACTACCTTCAGTATAAAAATGTCAGGCCTGATTATCTAAAAGCTATTTGGA
ATGTAATCAACTGGGAGAATGTAACTGAAAGATACATGGCTTGCAAAAAGTAAACCACGATCGTTATGCT
GAGTATGTTAAGCTCTTTATGACTGTTTTTGTAGTGGTATAGAGTACTGCAGAATACAGTAAGCTGCTCT
ATTGTAGCATTTCTTGATGTTGCTTAGTCACTTATTTCATAAACAACTTAATGTTCTGAATAATTTCTTA
CTAAACATTTTGTTATTGGGCAAGTGATTGAAAATAGTAAATGCTTTGTGTGATTGAATCTGATTGGACA
TTTTCTTCAGAGAGCTAAATTACAATTGTCATTTATAAAACCATCAAAAATATTCCATCCATATACTTTG
GGGACTTGTAGGGATGCCTTTCTAGTCCTATTCTATTGCAGTTATAGAAAATCTAGTCTTTTGCCCCAGT
TACTTAAAAATAAAATATTAACACTTTCCCAAGGAAACACTCGGCTTTCTATAGAAAATTGCACTTTTT
GTCGAGTAATCCTCTGCAGTGATACTTCTGGTAGATGTCACCCAGTGGTTTTTGTTAGGTCAAATGTTCC
TGTATAGTTTTTGCAAATAGAGCTGTATACTGTTTAAATGTAGCAGGTGAACTGAACTGGGGTTTGCTCA
CCTGCACAGTAAAGGCAAACTTCAACAGCAAAACTGCAAAAAGGTGGTTTTTGCAGTAGGAGAAAGGAGG
ATGTTTATTTGCAGGGCGCCAAGCAAGGAGAATTGGGCAGCTCATGCTTGAGACCCAATCTCCATGATGA
CCTACAAGCTAGAGTATTTAAAGGCAGTGGTAAATTTCAGGAAAGCAGAAGTT
NCBI Reference Sequence: NM_000576.2
   **Homo sapiens interleukin 1, beta (IL1B), mRNA**
   >gi|27894305|ref|NM_000576.2| Homo sapiens interleukin 1, beta (IL1B), mRNA 
ACCAAACCTCTTCGAGGCACAAGCCACAACACCCTGCTCTGGGATTCTCTTCAGCCAATCTTCATTGCTC
AAGTGTCTGAAGCAGCCATGGCAGAAGTACCTGAGCTCGCCAGTGAAATGATGGCTTATTACAGTGGCAA
TGAGGATGACTTGTTCTTTGAAGCTGATGGCCCTAAACAGATGAAGTGCTCCTTCCAGGACCTGGACCTC
TGCCCTCTGGATGGCGGCATCCAGCTACGAATCTCCGACCACCACTACAGCAAGGGCTTCAGGCAGGCCG
CGTCAGTTGTTGTGGCCATGGACAAGCTGAGGAAGATGCTGGTTCCCTGCCCACAGACCTTCCAGGAGAA
TGACCTGAGCACCTTCTTTCCCTTCATCTTTGAAGAAGAACCTATCTTCTTCGACACATGGGATAACGAG
GCTTATGTGCACGATGCACCTGTACGATCACTGAACTGCACGCTCCGGGACTCACAGCAAAAAAGCTTGG
TGATGTCTGGTCCATATGAACTGAAAGCTCTCCACCTCCAGGGACAGGATATGGAGCAACAAGTGGTGTT
CTCCATGTCCTTTGTACAAGGAGAAGAAAGTAATGACAAAATACCTGTGGCCTTGGGCCTCAAGGAAAAG
AATCTGTACCTGTCCTGCGTGTTGAAAGATGATAAGCCCACTCTACAGCTGGAGAGTGTAGATCCCAAAA
ATTACCCAAAGAAGAAGATGGAAAAGCGATTTGTCTTCAACAAGATAGAAATCAATAACAAGCTGGAATT
TGAGTCTGCCCAGTTCCCCAACTGGTACATCAGCACCTCTCAAGCAGAAAACATGCCCGTCTTCCTGGGA
GGGACCAAAGGCGGCCAGGATATAACTGACTTCACCATGCAATTTGTGTCTTCCTAAAGAGAGCTGTACC
CAGAGAGTCCTGTGCTGAATGTGGACTCAATCCCTAGGGCTGGCAGAAAGGGAACAGAAAGGTTTTTGAG
TACGGCTATAGCCTGGACTTTCCTGTTGTCTACACCAATGCCCAACTGCCTGCCTTAGGGTAGTGCTAAG
AGGATCTCCTGTCCATCAGCCAGGACAGTCAGCTCTCTCCTTTCAGGGCCAATCCCCAGCCCTTTTGTTG
AGCCAGGCCTCTCTCACCTCTCCTACTCACTTAAAGCCCGCCTGACAGAAACCACGGCCACATTTGGTTC
 
TAAGAAACCCTCTGTCATTCGCTCCCACATTCTGATGAGCAACCGCTTCCCTATTTATTTATTTATTTGT
TTGTTTGTTTTATTCATTGGTCTAATTTATTCAAAGGGGCAAGAAGTAGCAGTGTCTGTAAAAGAGCCT
AGTTTTTAATAGCTATGGAATCAATTCAATTTGGACTGGTGTGCTCTCTTTAAATCAAGTCCTTTAATTA
AGACTGAAAATATATAAGCTCAGATTATTTAAATGGGAATATTTATAAATGAGCAAATATCATACTGTTC
AATGGTTCTGAAATAAACTTCACTGAAG
NCBI Reference Sequence: NM_000423.2
   **Homo sapiens keratin 2 (KRT2), mRNA**
   >gi|47132619|ref|NM_000423.2| Homo sapiens keratin 2 (KRT2), mRNA 
AGCCTGTGACTTTCCTCCCTGGACAAAGGCATCATGAGTTGTCAGATCTCTTGCAAATCTCGAGGAAGAG
GAGGAGGTGGAGGAGGATTCCGGGCTTCAGCAGCGGCTCAGCTGTGGTGTCTGGTGGAAGCCGGAGATC
AACTTCCAGCTTCTCCTGCTTGAGCCGCCATGGTGGTGGTGGCGGGGGCTTCGGTGGAGGCGGCTTTGGC
AGTCGGAGTCTTGTTGGCCTTGGAGGGACCAAGAGCATCTCCATTAGTGTGGCTGGAGGAGGTGGTGGCT
TTGGCGCCGCTGGTGGATTTGGTGGCAGAGGAGGTGGTTTTGGAGGCGGCAGCAGCTTTGGAGGTGGCAG
CGGCTTCAGTGGTGGTGGTTTCGGTGGAGGCGGCTTTGGTGGAGGCCGCTTTGGAGGTTTTGGGGGCCCT
GGTGGTGTTGGAGGTTTAGGGGGTCCTGGTGGCTTTGGGCCTGGAGGATACCCTGGTGGCATCCACGAAG
TCTCTGTCAACCAGAGCCTCCTGCAGCCTCTCAACGTGAAGTTGACCCAGAGATCCAGAATGTGAAGGC
CCAAGAGCGTGAGCAGATCAAAACTCTCAACAACAAATTTGCCTCCTTCATTGACAAGGTGCGGTTCTTG
GAGCAGCAGAACCAGGTGTTACAGACCAAATGGGAGCTGCTACAACAAATGAATGTTGGCACCCGCCCCA
TCAACCTGGAGCCCATCTTCCAGGGGTATATCGACAGCCTCAAGAGATATCTGGATGGCTCACTGCAGA
AAGAACATCACAGAATTCAGAGCTGAATAACATGCAGGATCTTGTGGAGGATTATAAGAAGAAGTATGAG
GATGAAATCAATAAGCGCACAGCTGCTGAGAATGATTTTGTGACGCTTAAAAAGGACGTGGACAATGCCT
ACATGATAAAGGTGGAGTTGCAGTCCAAGGTGGACCTGCTGAACCAGGAAATTGAGTTTCTGAAAGTTCT
CTATGATGCGGAGATATCCCAGATACATCAGAGTGTCACTGACACCAACGTCATCCTCTCCATGGACAAC
AGCCGCAACCTGGACTTGGATAGCATCATCGCCGAGGTCAAGGCCCAGTATGAGGAGATCGCCCAGAGGA
GCAAGGAAGAAGCGGAGGCCCTGTACCACAGCAAGTATGAGGAGCTCCAGGTGACTGTCGGGAGACATGG
AGACAGCCTGAAAGAGATCAAGATAGAGATCAGCGAGCTGAACCGCGTGATCCAGAGGCTGCAGGGGGAG
ATCGCACATGTGAAGAAGCAGTGTAAGAATGTGCAAGATGCCATCGCAGATGCCGAGCAGCGTGGGGAGC
ATGCCCTCAAGGATGCCAGGAACAAGTTGAATGACCTGGAGGAGGCCCTGCAGCAGGCCAAGGAGGACTT
GGCGCGGCTGCTGCGTGACTACCAGGAGCTGATGAACGTGAAGCTGGCCCTAGATGTGGAGATCGCCACC
TACCGCAAACTGCTGGAGGGCGAGGAGTGCAGGATGTCTGGAGACCTCAGCAGCAATGTGACTGTGTCTG
TGACAAGCAGCACCATTTCATCAAATGTGGCATCCAAGGCTGCCTTTGGAGGTTCTGGAGGTAGAGGGTC
CAGTTCCGGAGGAGGATACAGCTCTGGAAGCAGCAGTTATGGCTCTGGAGGCCGACAGTCTGGCTCCAGA
GGCGGTAGTGGAGGAGGAGGTTCTATCTCTGGAGGAGGATATGGCTCTGGCGGTGGTTCTGGAGGAAGAT
ACGGATCTGGTGGTGGCTCTAAGGGAGGGTCCATCTCTGGAGGAGGATATGGCTCTGGAGGTGGAAAACA
CAGCTCTGGAGGTGGCTCTAGAGGAGGCTCCAGCTCTGGAGGAGGATATGGCTCTGGAGGTGGGGGTTCT
AGCTCTGTAAAGGGTAGCTCAGGTGAAGCTTTTGGTTCCAGCGTGACCTTCTCTTTTAGATAAAGATGAG
CCCCCACCACCACCGACTCTCCCAACCCAGACTCTCCCACTCCAGAATGTAGAAGCCTGTCTCTGTACCT
CTAACTGGCAGCAAGTTAAATTTTTGTCATTTATCTCTGATGGCACTTTGAGGGAAAAGAATGTCCACAT
ACAGTTTTTGAAAGATCTTCTCTCCAAACCAGTTAGTTAGAGCCAGTGACGCCTCTGTGTTCTGGGGCGG
AATCTGTGCTGTCTAGGTTTGTGCTTCTAGCCATGCCCATTCCCGCCCCCACCATGCCTCTTTGCATTGC
CCATTTTCCAGATGTGTATTCTGTTGAGGACCCAGGCCCATCCAGGGATTTCATCTCTAAGCCTGGCAGT
GCTGGGGGGAAATGTGTTTCTGTGTATATAGCTCCTCTTGTCCACTCTGCTTTCGGAAGTGCTGTGGTCT
GGGGGTCTTCATAATAAACCTCATTTGCAATTCAAAAAAAAAAAAAAAAAAAAAAAAAAAAAAAAAAAAA
AAAAAAAAA
NCBI Reference Sequence: NM_005554.3
   **Homo sapiens keratin 6A (KRT6A), mRNA**
   >gi|126273584|ref|NM_005554.3| Homo sapiens keratin 6A (KRT6A), mRNA 
ATATTTCATACCTTTCTAGAAACTGGGTGTGATCTCACTGTTGGTAAAGCCCAGCCCTTCCCAACCTGCA
AGCTCACCTTCCAGGACTGGGCCCAGCCCATGCTCTCCATATATAAGCTGCTGCCCCGAGCCTGATTCCT
AGTCCTGCTTCTCTTCCCTCTCTCCTCCAGCCTCTCACACTCTCCTCAGCTCTCTCATCTCCTGGAACCA
TGGCCAGCACATCCACCACCATCAGGAGCCACAGCAGCAGCCGCCGGGTTTCAGTGCCAACTCAGCCAG
GCTCCCTGGGGTCAGCCGCTCTGGCTTCAGCAGCGTCTCCGTGTCCCGCTCCAGGGGCAGTGGTGGCCTG
GGTGGTGCATGTGGAGGAGCTGGCTTTGGCAGCCGCAGTCTGTATGGCCTGGGGGGCTCCAAGAGGATCT
CCATTGGAGGGGGCAGCTGTGCCATCAGTGGCGGCTATGGCAGCAGAGCCGGAGGCAGCTATGGCTTTGG
TGGCGCCGGGAGTGGATTTGGTTTCGGTGGTGGAGCCGGCATTGGCTTTGGTCTGGGTGGTGGAGCCGGC
CTTGCTGGTGGCTTTGGGGGCCCTGGCTTCCCTGTGTGCCCCCCTGGAGGCATCCAAGAGGTCACCGTCA
ACCAGAGTCTCCTGACTCCCCTCAACCTGCAAATCGATCCCACCATCCAGCGGGTGCGGGCTGAGGAGCG
TGAACAGATCAAGACCCTCAACAACAAGTTTGCCTCCTTCATCGACAAGGTGCGGTTCCTGGAGCAGCAG
AACAAGGTTCTGGAAACAAAGTGGACCCTGCTGCAGGAGCAGGGCACCAAGACTGTGAGGCAGAACCTGG
AGCCGTTGTTCGAGCAGTACATCAACAACCTCAGGAGGCAGCTGGACAGCATTGTCGGGGAACGGGCCG
CCTGGACTCAGAGCTCAGAGGCATGCAGGACCTGGTGGAGGACTTCAAGAACAAATATGAGGATGAAATC
AACAAGCGCACAGCAGCAGAGAATGAATTTGTGACTCTGAAGAAGGATGTGGATGCTGCCTACATGAACA
AGGTTGAACTGCAAGCCAAGGCAGACACTCTCACAGACGAGATCAACTTCCTGAGAGCCTTGTATGATGC
AGAGCTGTCCCAGATGCAGACCCACATCTCAGACACATCTGTGGTGCTGTCCATGGACAACAACCGCAAC
CTGGACCTGGACAGCATCATCGCTGAGGTCAAGGCCCAATATGAGGAGATTGCTCAGAGAAGCCGGGCTG
AGGCTGAGTCCTGGTACCAGACCAAGTACGAGGAGCTGCAGGTCACAGCAGGCAGACATGGGGACGACCT
GCGCAACACCAAGCAGGAGATTGCTGAGATCAACCGCATGATCCAGAGGCTGAGATCTGAGATCGACCAC
GTCAAGAAGCAGTGCGCCAACCTGCAGGCCGCCATTGCTGATGCTGAGCAGCGTGGGGAGATGGCCCTCA
AGGATGCCAAGAACAAGCTGGAAGGGCTGGAGGATGCCCTGCAGAAGGCCAAGCAGGACCTGGCCCGGCT
GCTGAAGGAGTACCAGGAGCTGATGAATGTCAAGCTGGCCCTGGACGTGGAGATCGCCACCTACCGCAAG
CTGCTGGAGGGTGAGGAGTGCAGGCTGAATGGCGAAGGCGTTGGACAAGTCAACATCTCTGTGGTGCAGT
CCACCGTCTCCAGTGGCTATGGCGGTGCCAGTGGTGTCGGCAGTGGCTTAGGCCTGGGTGGAGGAAGCAG
CTACTCCTATGGCAGTGGTCTTGGCGTTGGAGGTGGCTTCAGTTCCAGCAGTGGCAGAGCCATTGGGGGT
GGCCTCAGCTCTGTTGGAGGCGGCAGTTCCACCATCAAGTACACCACCACCTCCTCCTCCAGCAGGAAGA
GCTATAAGCACTAAAGTGCGTCTGCTAGCTCTCGGTCCCACAGTCCTCAGGCCCCTCTCTGGCTGCAGAG
CCCTCTCCTCAGGTTGCCTTTCCTCTCCTGGCCTCCAGTCTCCCCTGCTGTCCCAGGTAGAGCTGGGTAT
GGATGCTTAGTGCCCTCACTTCTTCTCTCTCTCTATACCATCTGAGCACCCATTGCTCACCATCAGAT
CAACCTCTGATTTTACATCATGATGTAATCACCACTGGAGCTTCACTGTTACTAAATTATTAATTTCTTG
CCTCCAGTGTTCTATCTCTGAGGCTGAGCATTATAAGAAAATGACCTCTGCTCCTTTTCATTGCAGAAAA
TTGCCAGGGGCTTATTTCAGAACAACTTCCACTTACTTTCCACTGGCTCTCAAACTCTCTAACTTATAAG
TGTTGTGAACCCCCACCCAGGCAGTATCCATGAAAGCACAAGTGACTAGTCCTATGATGTACAAAGCCTG
TATCTCTGTGATGATTTCTGTGCTCTTCGCTGTTTGCAATTGCTAAATAAAGCAGATTTATAATACAATA
NCBI Reference Sequence: NM_021101.3
   **Homo sapiens claudin 1 (CLDN1), mRNA**
   >gi|21536297|ref|NM_021101.3| Homo sapiens claudin 1 (CLDN1), mRNA 
GAGCAACCGCAGCTTCTAGTATCCAGACTCCAGCGCCGCCCCGGGCGCGGACCCCAACCCCGACCCAGAG
CTTCTCCAGCGGCGGCGCAGCGAGCAGGGCTCCCCGCCTTAACTTCCTCCGCGGGGCCCAGCCACCTTCG
GGAGTCCGGGTTGCCCACCTGCAAACTCTCCGCCTTCTGCACCTGCCACCCCTGAGCCAGCGCGGGCGCC
CGAGCGAGTCATGGCCAACGCGGGGCTGCAGCTGTTGGGCTTCATTCTCGCCTTCCTGGGATGGATCGGC
GCCATCGTCAGCACTGCCCTGCCCCAGTGGAGGATTTACTCCTATGCCGGCGACAACATCGTGACCGCCC
AGGCCATGTACGAGGGGCTGTGGATGTCCTGCGTGTCGCAGAGCACCGGGCAGATCCAGTGCAAAGTCTT
TGACTCCTTGCTGAATCTGAGCAGCACATTGCAAGCAACCCGTGCCTTGATGGTGGTTGGCATCCTCCTG
GGAGTGATAGCAATCTTTGTGGCCACCGTTGGCATGAAGTGTATGAAGTGCTTGGAAGACGATGAGGTGC
AGAAGATGAGGATGGCTGTCATTGGGGGTGCGATATTTCTTCTTGCAGGTCTGGCTATTTTAGTTGCCAC
AGCATGGTATGGCAATAGAATCGTTCAAGAATTCTATGACCCTATGACCCCAGTCAATGCCAGGTACGAA
TTTGGTCAGGCTCTCTTCACTGGCTGGGCTGCTGCTTCTCTCTGCCTTCTGGGAGGTGCCCTACTTTGCT
 
GTTCCTGTCCCCGAAAAACAACCTCTTACCCAACACCAAGGCCCTATCCAAAACCTGCACCTTCCAGCGG
GAAAGACTACGTGTGACACAGAGGCAAAAGGAGAAAATCATGTTGAAACAAACCGAAAATGGACATTGAG
ATACTATCATTAACATTAGGACCTTAGAATTTTGGGTATTGTAATCTGAAGTATGGTATTACAAAACAAA
CAAACAAACAAAAAACCCATGTGTTAAAATACTCAGTGCTAAACATGGCTTAATCTTATTTTATCTTCTT
TCCTCAATATAGGAGGGAAGATTTTCCATTTGTATTACTGCTTCCCATTGAGTAATCATACTCAATTGG
GGGAAGGGGTGCTCCTTAAATATATATAGATATGTATATATACATGTTTTTCTATTAAAAATAGACAGTA
AAATACTATTCTCATTATGTTGATACTAGCATACTTAAAATATCTCTAAAATAGGTAAATGTATTTAATT
CCATATTGATGAAGATGTTTATTGGTATATTTTCTTTTTCGTCTATATATACATATGTAACAGTCAAATA
TCATTTACTCTTCTTCATTAGCTTTGGGTGCCTTTGCCACAAGACCTAGCCTAATTTACCAAGGATGAAT
TCTTTCAATTCTTCATGCGTGCCCTTTTCATATACTTATTTTATTTTTACCATAATCTTATAGCACTTG
CATCGTTATTAAGCCCTTATTTGTTTTGTGTTTCATTGGTCTCTATCTCCTGAATCTAACACATTTCATA
GCCTACATTTTAGTTTCTAAAGCCAAGAAGAATTTATTACAAATCAGAACTTTGGAGGCAAATCTTTCTG
CATGACCAAAGTGATAAATTCCTGTTGACCTTCCCACACAATCCCTGTACTCTGACCCATAGCACTCTTG
TTTGCTTTGAAAATATTTGTCCAATTGAGTAGCTGCATGCTGTTCCCCCAGGTGTTGTAACACAACTTTA
TTGATTGAATTTTTAAGCTACTTATTCATAGTTTTATATCCCCCTAAACTACCTTTTTGTTCCCCATTCC
TTAATTGTATTGTTTTCCCAAGTGTAATTATCATGCGTTTTATATCTTCCTAATAAGGTGTGGTCTGTTT
GTCTGAACAAAGTGCTAGACTTTCTGGAGTGATAATCTGGTGACAAATATTCTCTCTGTAGCTGTAAGCA
AGTCACTTAATCTTTCTACCTCTTTTTTCTATCTGCCAAATTGAGATAATGATACTTAACCAGTTAGAAG
AGGTAGTGTGAATATTAATTAGTTTATATTACTCTCATTCTTTGAACATGAACTATGCCTATGTAGTGTC
TTTATTTGCTCAGCTGGCTGAGACACTGAAGAAGTCACTGAACAAAACCTACACACGTACCTTCATGTGA
TTCACTGCCTTCCTCTCTCTACCAGTCTATTTCCACTGAACAAAACCTACACACATACCTTCATGTGGTT
CAGTGCCTTCCTCTCTCTACCAGTCTATTTCCACTGAACAAAACCTACGCACATACCTTCATGTGGCTCA
GTGCCTTCCTCTCTCTACCAGTCTATTTCCATTCTTTCAGCTGTGTCTGACATGTTTGTGCTCTGTTCCA
TTTTAACAACTGCTCTTACTTTTCCAGTCTGTACAGAATGCTATTTCACTTGAGCAAGATGATGTAATGG
AAAGGGTGTTGGCATTGGTGTCTGGAGACCTGGATTTGAGTCTTGGTGCTATCAATCACCGTCTGTGTTT
GAGCAAGGCATTTGGCTGCTGTAAGCTTATTGCTTCATCTGTAAGCGGTGGTTTGTAATTCCTGATCTTC
CCACCTCACAGTGATGTTGTGGGGATCCAGTGAGATAGAATACATGTAAGTGTGGTTTTGTAATTTAAAA
AGTGCTATACTAAGGGAAAGAATTGAGGAATTAACTGCATACGTTTTGGTGTTGCTTTTCAAATGTTTGA
AAACAAAAAAAATGTTAAGAAATGGGTTTCTTGCCTTAACCAGTCTCTCAAGTGATGAGACAGTGAAGTA
AAATTGAGTGCACTAAACAAATAAGATTCTGAGGAAGTCTTATCTTCTGCAGTGAGTATGGCCCGATGCT
TTCTGTGGCTAAACAGATGTAATGGGAAGAAATAAAAGCCTACGTGTTGGTAAATCCAACAGCAAGGGAG
ATTTTTGAATCATAATAACTCATAAGGTGCTATCTGTTCAGTGATGCCCTCAGAGCTCTTGCTGTTAGCT
GGCAGCTGACGCTGCTAGGATAGTTAGTTTGGAAATGGTACTTCATAATAAACTACACAAGGAAAGTCAG
CCACTGTGTCTTATGAGGAATTGGACCTAATAAATTTTAGTGTGCCTTCCAAACCTGAGAATATATGCTT
TTGGAAGTTAAAATTTAAATGGCTTTTGCCACATACATAGATCTTCATGATGTGTGAGTGTAATTCCATG
TGGATATCAGTTACCAAACATTACAAAAAAATTTATGGCCCAAAATGACCAACGAAATTGTTACAATAG
AATTTATCCAATTTTGATCTTTTTATATTCTTCTACCACACCTGGAAACAGACCAATAGACATTTTGGGG
TTTTATAATAGGAATTTGTATAAAGCATTACTCTTTTTCAATAAATTGTTTTTTAATTTAAAAAAAGGAA
AAAAAAAAAAAAAAA
NCBI Reference Sequence: NM_000427.2
   **Homo sapiens loricrin (LOR), mRNA**
   >gi|109255250|ref|NM_000427.2| Homo sapiens loricrin (LOR), mRNA 
CTCTCCTCACTCACCCTTCCTGGTGCTTTGGGCTCTCCTTCCTTCTCAGACAAGATGTCTTATCAGAAAA
AGCAGCCCACCCCTCAGCCCCCAGTGGACTGCGTGAAGACCTCTGGCGGCGGTGGCGGTGGCGGCGGCAG
CGGCGGTGGTGGCTGCGGCTTCTTCGGCGGCGGCGGCTCAGGGGGCGGTAGCAGCGGTTCTGGCTGCGGC
TACTCCGGCGGCGGTGGCTACTCTGGCGGCGGCTGCGGCGGGGGCTCCTCCGGCGGCGGGGGCGGGGGCG
GCATTGGAGGCTGCGGAGGGGGCTCCGGTGGGAGCGTCAAGTACTCCGGAGGCGGCGGCTCCTCCGGCGG
GGGCTCTGGCTGTTTCTCCAGCGGTGGGGGCGGCTCCGGCTGCTTCTCCTCCGGTGGCGGCGGCTCCTCC
GGGGGCGGCTCCGGCTGCTTCTCCAGCGGTGGGGGCGGCTCCTCCGGGGGCGGCTCCGGCTGCTTCTCCT
CCGGCGGCGGCGGCTTCTCGGCCAGGCGGTCCAGTGCCAGAGCTACGGAGGCGTCTCTAGCGGCGGCTC
CTCCGGGGGCGGCTCCGGCTGCTTCTCCAGCGGCGGGGGCGGCGGCTCTGTCTGCGGCTACTCTGGCGGC
 
GGCTCTGGCTGCGGCGGAGGCTCCTCTGGCGGCAGCGGCTCCGGCTACGTCTCCTCGCAGCAGGTCACTC
AGACCTCGTGCGCGCCCAGCCGAGTTACGGAGGGGGTCGTCCGGCGGCGGCGGCAGCGGCGGAAGCGG
CTGCTTCTCCAGCGGCGGGGGCGGCGGGAGCTCCGGCTGCGGCGGCGGCTCCTCCGGGATTGGCAGCGGC
TGCATCATCAGTGGCGGGGGCTCCGTCTGCGGAGGTGGTTCCTCTGGAGGCGGCGGCGGCTCCTCCG
TGGGTGGCTCCGGGAGTGGCAAGGGCGTCCCGATCTGCCACCAGACCCAGCAGAAGCAGGCGCCTACCTG
GCCGTCCAAATAGATCCCCCAGGGTACCACGGAGGCGAAGGAGTTGGAGGTGTTTCCAGGGGCACCGAT
GGGCTTAGAGCTCTCATGATGCTACCCGAGGTTTGCAAATCCTTCATGTCTTAACCTACCTGGAAGAAGC
CATTGAGCTCTCCGGCTGCATCTAGTTCTGCTGTTTAGCCTCTTTGGTTTCTGTACAACTACCTCCCAAC
CCCAGTGCCTCAGTCAATAAATTTGCAAATTCATGAGAA
NCBI Reference Sequence: NM_002016.1
   **Homo sapiens filaggrin (FLG), mRNA**
   >gi|60097901|ref|NM_002016.1| Homo sapiens filaggrin (FLG), mRNA 
CTTTTGGTGAACAAGGTTCACATTTATTGCCAAAAGATGTCTACTCTCCTGGAAAACATCTTTGCCATAA
TTAATCTTTTCAAGCAATATTCAAAAAAAGATAAAAACACTGACACATTGAGTAAAAAAGAGCTGAAGGA
ACTTCTGGAAAAGGAATTTCGGCAAATCCTGAAGAATCCAGATGACCCAGATATGGTTGATGTCTTCATG
GATCACTTGGATATAGACCACAACAAGAAAATTGACTTCACTGAGTTTCTTCTGATGGTATTCAAGTTGG
CTCAAGCATATTATGAGTCTACCAGAAAAGAGAATTTACCGATATCAGGACACAAGCACAGAAAGCACAG
TCATCATGATAAACATGAAGATAATAAACAGGAAGAAAACAAAGAAAACAGAAAAAGACCCTCAAGTCTG
GAAAGAAGAAACAATAGAAAAGGGAATAAGGGAAGATCCAAGAGCCCAAGAGAAACAGGGGGAAAAGGC
ATGAATCTAGTTCTGAAAAAAAAGAAAGAAAAGGATATTCACCTACTCATAGAGAAGAAGAATATGGAAA
AAACCATCATAACTCAAGTAAAAAAGAGAAAAACAAGACTGAAAATACTAGATTAGGAGACAATAGGAAG
AGGCTAAGTGAAAGACTTGAAGAGAAAGAAGACAATGAAGAAGGAGTATATGATTATGAAAATACAGGAA
GAATGACTCAAAAATGGATACAATCAGGCCATATTGCCACATATTACACAATCCAGGATGAAGCCTATGA
CACCACTGATAGTCTATTAGAAGAAAACAAAATATATGAAAGATCAAGGTCATCTGATGGCAAATCATCA
TCTCAAGTGAACAGGTCAAGACATGAAAATACAAGCCAGGTACCATTGCAGGAGTCCAGGACAAGAAAGC
GTAGGGGATCCAGAGTTAGCCAGGACAGGGACAGTGAGGGACACTCAGAAGACTCTGAGAGGCACTCTGG
GTCGGCTTCCAGAAACCATCATGGATCTGCGTGGGAGCAGTCAAGAGATGGCTCCAGACACCCCAGGTCC
CATGATGAAGACAGAGCCAGTCATGGGCACTCTGCAGACAGCTCCAGACAATCAGGCACTCGTCACGCAG
AGACTTCCTCTCGTGGACAGACTGCATCATCCCATGAACAGGCAAGATCAAGTCCAGGAGAAAGACATGG
ATCCGGCCACCAGCAGTCAGCAGACAGCTCCAGACACTCAGCCACTGGGCGCGGGCAAGCTTCATCTGCA
GTCAGCGATCGTGGACACCGGGGGTCTAGCGGTAGTCAGGCCAGTGACAGTGAGGGACATTCAGAAAACT
CAGACACACAATCAGTGTCAGGCCACGGAAAGGCTGGGCTGAGACAGCAGAGCCACCAAGAGTCCACACG
TGGCCGGTCAGGGGAACGGTCTGGACGTTCAGGGTCTTCCCTCTACCAGGTGAGCACTCATGAACAGCCT
GACTCTGCCCATGGACGGACCGGGACCAGCACTGGAGGAAGACAAGGATCGCACCACGAGCAGGCACGAG
ACAGCTCCAGGCATTCAGCGTCCCAAGAGGGTCAGGACACCATTCGTGGACACCCGGGGTCAAGCAGAGC
AGGAAGGCAGGGATCCCACCACGAGCAATCGGTAAATAGGTCTGGACACTCAGGTTCCCATCACAGCCAC
ACCACATCCCAGGGAAGGTCTGATGCCTCCCATGGGCAGTCAGGATCCAGAAGTGCAAGCAGACAAACAC
GAAATGAGGAACAATCAGGAGACGGCACCAGGCACTCAGGGTCACGTCATCATGAAGCTTCCTCTCAGGC
TGACAGCTCTAGACACTCACAGGTGGGCCAGGGACAATCATCGGGGCCCAGGACAAGTAGGAACCAGGGA
TCCAGTGTTAGCCAGGACAGTGACAGTCAGGGACACTCAGAAGACTCTGAGAGGTGGTCTGGGTCTGCTT
CCAGAAACCATCATGGATCTGCTCAGGAGCAGTCAAGAGATGGCTCCAGACACCCCAGGTCCCATCACGA
AGACAGAGCTGGTCATGGGCACTCTGCAGACAGCTCCAGAAAATCAGGCACTCGTCACACACAGAATTCC
TCTAGTGGACAGGCTGCGTCATCCCATGAACAGGCAAGATCAAGTGCAGGAGAAAGACATGGATCCCGCC
ACCAGCTCCAGTCAGCAGACAGCTCCAGACACTCAGGCACTGGGCACGGACAAGCTTCATCTGCAGTCAG
AGACAGTGGACACCGAGGGTCCAGTGGTAGTCAGGCCACTGACAGTGAGGGACATTCAGAAGACTCAGAC
ACACAGTCAGTGTCAGGCCATGGACAGGCTGGTCACCATCAGCAGAGCCACCAAGAGTCCGCACGTGACC
GGTCAGGGGAAAGGTCTCGACGTTCAGGGTCTTTCCTCTACCAGGTGAGCACTCATAAACAGTCTGAGTC
CTCCCATGGATGGACAGGGCCCAGCACTGGAGTAAGACAAGGATCCCACCATGAGCAGGCACGAGACAAC
TCCAGGCACTCAGCATCCCAAGATGGTCAGGACACCATTCGTGGACACCCGGGGTCAAGCAGAAGAGGAA
GGCAGGGGTCCCACCACGAGCAATCGGTAGATAGGTCTGGACACTCAGGGTCCCATCACAGCCACACCAC
ATCCCAGGGAAGGTCTGATGCCTCCCGTGGGCAGTCAGGATCCAGAAGTGCAAGCAGAACAACACGTAAT
 
GAGGAACAATCAAGAGACGGCTCCAGGCACTCAGGGTCACGTCACCATGAAGCTTCCTCTCATGCCGACA
TCTCTAGACACTCACAGGCAGGCCAGGGACAATCAGAGGGGTCCAGGACAAGCAGGCGCCAGGGATCCAG
TGTTAGCCAGGACAGTGACAGTGAGGGACATTCAGAAGACTCTGAGAGGTGGTCTGGGTCTGCTTCCAGA
AACCATCGTGGATCTGCTCAGGAGCAGTCAAGACATGGCTCCAGACACCCCAGGTCCCATCACGAAGACA
GAGCCGGTCACGGGCACTCTGCAGACAGCTCCAGACAATCAGGAACTCCTCACGCAGAGACTTCCTCTGG
TGGACAGGCTGCGTCATCCCATGAACAGGCAAGATCAAGTCCAGGAGAAAGACACGGATCCCGCCACCAG
CAGTCAGCAGACAGCTCCAGACACTCAGGCATTCCGCGCAGACAAGCTTCATCTGCAGTCAGAGACAGTG
GACACTGGGGGTCCAGTGGTAGTCAGGCCAGTGATAGTGAGGGACATTCAGAGGAGTCAGACACACAGTC
AGTGTCAGGCCATGGACAGGATGGGCCCCATCAGCAGAGCCACCAAGAGTCCGCACGTGACTGGTCAGGG
GGAAGGTCTGGACGTTCAGGGTCTTTCATCTACCAGGTGAGCACTCATGAACAGTCTGAGTCTGCCCATG
GGCGGACCAGGACCAGCACTGGACGAAGACAAGGATCCCACCACGAGCAGGCACGAGACAGCTCCAGGCA
CTCAGCGTCCCAAGAGGGTCAGGACACCATTCGTGCACACCCGGGGTCAAGGAGAGGAGGAAGGCAGGGA
TCCCACCATGAGCAATCGGTAGATAGATCTGGACACTCAGGGTCCCATCACAGCCACACCACATCCCAGG
GAAGGTCTGATGCCTCCCATGGGCAGTCAGGATCCAGAAGTGCAAGCAGACAAACTCGTAAGGACAAACA
ATCAGGAGACGGCTCCAGGCACTCAGGGTCACGTCACCATGAAGCTGCCTCTTGGGCTGACAGCTCTAGA
CACTCACAGGTGGGACAGGAACAATCATCGGGGTCCAGGACAAGCAGGCACCAGGGATCCAGTGTTAGCC
AGGACAGTGACAGTGAGAGACACTCAGACGACTCCGAGAGGTTGTCTGGGTCTGCTTCCAGAAACCATCA
TGGATCTTCTCGGGAGCAGTCAAGAGATGGCTCCAGACACCCTGGGTTCCATCAAGAAGACAGAGCCAGT
CACGGGCACTCTGCAGACAGCTCCAGACAATCAGGCACTCATCACACAGAGTCTTCCTCTCATGGACAGG
CTGTGTCATCCCATGAACAGGCAAGATCAAGTCCAGGAGAAAGACATGGATCCCGCCACCAGCAGTCAGC
AGACAGCTCCAGACACTCAGGCATTGGGCACAGACAAGCTTCATCTGCAGTCAGAGACAGTGGACACCGA
GGGTCCAGTGGTAGTCAGGTCACTAACAGTGAGGGACATTCAGAAGACTCAGACACACAGTCAGTGTCAG
CCCACGGACAAGCTGGGCCCCATCAGCAGAGCCACAAAGAGTCCGCACGTGGCCAGTCAGGGGAAAGCTC
TGGACGTTCAAGGTCTTTCCTCTACCAGGTGAGCTCTCATGAACAGTCTGAGTCCACACACGGACAGACT
GCACCCAGCACTGGAGGAAGACAAGGATCCCGCCATGAGCAGGCACGAAACAGCTCTAGGCACTCAGCAT
CCCAAGACGGTCAGGACACCATTCGTGGACACCCGGGGTCAAGCAGAGGAGGAAGGCAGGGATCCTACCA
CGAGCAATCAGTAGATAGGTCTGGACACTCAGGGTACCATCACAGCCACACCACACCCCAGGGAAGGTCT
GATGCCTCCCATGGGCAGTCAGGACCCAGAAGTGCAAGCAGGCAAACAAGAAATGAGGAACAATCAGGAG
ACGGCTCCAGGCACTCAGGGTCACGTCACCATGAACCTTCCACTCGGGCCGGCAGCTCTAGACACTCACA
GGTGGGCCAGGGAGAATCAGCGGGGTCCAAGACAAGCAGGCGCCAGGGATCCAGTGTTAGTCAGGACAGG
GACAGTGAGGGACACTCAGAAGACTCTGAGAGGCGGTCTGAGTCGGCTTCCAGAAACCATTATGGATCTG
CTCGGGAGCAGTCAAGACATGGCTCCAGGAACCCCAGGTCCCATCAAGAAGATAGAGCCAGTCATGGGCA
CTCTGCAGAGAGCTCCAGACAATCAGGCACTCGTCATGCAGAGACTTCCTCTGGTGGACAGGCTGCATCA
TCCCAGGAACAGGCAAGGTCAAGTCCAGGAGAAAGACATGGATCCCGCCACCAGCAGTCAGCAGACAGCT
CCACAGACTCAGGCACTGGGCGCAGACAAGATTCATCTGTAGTCGGAGACAGTGGAAACCGAGGGTCCAG
TGGTAGCCAGGCCAGTGACAGCGAGGGACACTCAGAAGAGTCAGACACACAGTCAGTGTCAGCCCACGGA
CAGGCTGGGCCCCATCAGCAGAGCCACCAAGAGTCCACACGTGGCCAGTCAGGGGAAAGGTCTGGACGTT
CAGGGTCTTTCCTCTACCAGGTGAGCACTCATGAACAGTCTGAGTCCGCCCATGGACGCACAGGGCCCAG
CACTGGAGGAAGACAAAGATCCCGCCACGAGCAGGCACGAGACAGCTCCAGGCACTCAGCGTCCCAAGAG
GGTCAGGACACCATTCGTGGACACCCAGGGTCAAGCAGAGGAGGAAGGCAGGGATCCCACTATGAGCAAT
CGGTAGATAGTTCTGGACACTCAGGGTCTCATCACAGCCACACCACGTCCCAGGAAAGGTCTGATGTCTC
CCGTGGGCAGTCAGGATCCAGAAGTGTCAGCAGACAAACACGTAATGAGAAACAATCAGGAGACGGCTCC
AGGCACTCAGGGTCGCGTCACCATGAAGCTTCCTCTCGGGCCGACAGCTCTAGACACTCGCAGGTGGGCC
AGGGACAATCATCAGGGCCCAGGACAAGCAGGAACCAGGGATCCAGTGTTAGCCAGGACAGTGACAGTCA
GGGACACTCAGAAGACTCTGAGAGGTGGTCTGGGTCTGCTTCCAGAAACCATCTTGGATCTGCTTGGGAG
CAGTCAAGAGATGGCTCCAGACACCCTGGGTCCCATCACGAAGACAGAGCCGGTCACGGGCACTCTGCAG
ACAGCTCCAGACAATCAGGCACTCGTCACACAGAGTCTTCCTCTCGTGGACAGGCTGCGTCATCCCATGA
ACAGGCAAGATCAAGTGCAGGAGAAAGACATGGATCCCACCACCAGCTCCAGTCAGCAGACAGCTCCAGA
CACTCAGGCATTGGGCATGGACAAGCTTCATCTGCAGTCAGAGACAGTGGACACCGAGGGTACAGTGGTA
GTCAGGCCAGTGACAGTGAGGGACATTCAGAAGACTCAGACACACAGTCAGTGTCAGCACAGGGAAAAGC
TGGGCCCCATCAGCAGAGCCACAAAGAGTCCGCACGTGGCCAGTCAGGGGAAAGCTCTGGACGTTCAGGG
TCTTTCCTCTACCAGGTGAGCACTCATGAACAGTCTGAGTCCACCCATGGACAGTCTGCGCCCAGCACTG
GAGGAAGACAAGGATCCCATTATGATCAGGCACAAGACAGCTCCAGGCACTCAGCATCCCAAGAGGGTCA
GGACACCATTCGTGGACACCCGGGGCCAAGCAGAGGAGGAAGACAGGGGTCCCACCAAGAGCAATCGGTA
GATAGGTCTGGACACTCAGGGTCTCATCACAGCCACACCACATCCCAGGGAAGGTCTGATGCCTCCCGTG
GGCAGTCAGGATCCAGAAGTGCAAGCAGAAAAACATATGACAAGGAACAATCAGGAGATGGCTCTAGGCA
 
CTCAGGGTCGCATCATCATGAAGCTTCCTCTTGGCCGACAGCTCTAGACACTCACTGGTGGCCAGGGA
CAATCATCAGGGCCCAGGACAAGCAGGCCCCGGGGATCCAGTGTTAGCCAGGACAGTGACAGTGAGGGAC
ACTCAGAAGATTCTGAGAGGCGGTCTGGGTCTGCGTCCAGAAACCATCATGGATCTGCTCAGGAGCAGTC
AAGAGATGGCTCCAGACACCCCAGGTCCCATCACGAAGACAGAGCCGGTCATGGGCACTCTGCAGAGAGC
TCCAGACAATCAGGCACTCATCATGCAGAGAATTCCTCTGGTGGACAGGCTGCATCATCCCATGAACAGG
CAAGATCAAGTGCAGGAGAGAGACACGGATCCCACCACCAGCAGTCAGCAGACAGCTCCAGACACTCAGG
CATTGGGCACGGACAAGCTTCATCTGCAGTCAGAGACAGTGGACACCGAGGGTCCAGTGGTAGTCAGGCC
AGTGACAGTGAGGGACATTCAGAAGACTCAGACACACAGTCAGTGTCAGCCCACGGACAGGCTGGGCCCC
ATCAGCAGAGCCACCAAGAGTCCACACGTGGCCGGTCAGCAGGAAGGTCTGGACGTTCAGGGTCTTTCCT
CTACCAGGTGAGCACTCATGAACAGTCTGAGTCCGCCCATGGACGGACCGGACCAGCACTGGAGGAAGA
CAAGGATCCCACCACAAGCAGGCACGAGACAGCTCCAGGCACTCAACGTCCCAAGAGGGTCAGGACACCA
TTCATGGACACCCGGGGTCAAGCAGTGGAGGAAGGCAGGGATCCCACTACGAGCAATTGGTAGATAGATC
TGGACACTCAGGGTCTCATCACAGCCACACCACATCCCAGGGAAGGTCTGATGCCTCCCATGGGCACTCA
GGATCCAGAAGTGCAAGCAGACAAACTCGTAACGATGAACAATCAGGAGACGGCTCCAGGCACTCAGGGT
CGCGTCACCATGAAGCTTCCTCTCGGCCGACAGCTCTGGACACTCGCAGGTGGCCAGGGACAATCAGA
GGGGCCCAGGACAAGCAGGAACTGGGGATCCAGTTTTAGCCAGGACAGTGACAGTCAGGGACACTCAGAA
GACTCTGAGAGGTGGTCTGGGTCTGCTTCCAGAAACCATCATGGATCTGCTCAGGAGCAGCTAAGAGATG
GCTCCAGACACCCCAGGTCCCATCAAGAAGACAGAGCTGGTCATGGGCACTCTGCAGACAGCTCCAGACA
ATCAGGCACTCGTCACACACAGACTTCCTCTGGTGGACAGGCTGCATCATCCCATGAACAGGCAAGATCA
AGTGCAGGAGAAAGACATGGATCCCACCACCAGCAGTCAGCAGACAGCTCCAGACACTCAGGCATTGGGC
ACGGACAAGCTTCATCTGCAGTCAGAGACAGTGGACACCGAGGGTACAGTGGTAGTCAGGCCAGTGACAA
TGAGGGACATTCAGAAGACTCAGACACACAGTCAGTGTCAGCCCACGGACAGGCTGGGTCCCATCAGCAG
AGCCACCAAGAGTCCGCACGTGGCCGGTCAGGGGAAACGTCTGGACATTCAGGATCTTTCCTCTACCAGG
TGAGCACTCATGAACAGTCTGAGTCCTCCCATGGATGGACGGGGCCCAGCACTAGAGGAAGACAAGGATC
CCGCCATGAGCAGGCACAAGACAGCTCCAGGCACTCAGCATCCCAAGACGGTCAGGACACCATTCGTGGA
CACCCGGGGTCAAGCAGAGGAGGAAGGCAGGGGTACCACCACGAGCATTCGGTAGATAGCTCTGGACACT
CAGGGTCCCATCACAGCCACACCACATCCCAGGGAAGGTCTGATGCCTCCCGTGGGCAGTCAGGATCCAG
AAGTGCAAGCAGAACAACACGTAATGAGGAACAATCAGGAGACGGCTCCAGGCACTCAGGGTCGCGTCAC
CATGAAGCTTCCACTCATGCCGACATCTCTAGACACTCACAGGCAGTCCAGGGACAATCAGAGGGGTCCA
GGAGAAGCAGGCGCCAGGGATCCAGTGTGAGCCAGGACAGTGACAGTGAGGGACATTCAGAAGACTCTGA
GAGGTGGTCTGGGTCTGCTTCCAGAAACCATCATGGATCTGCTCAGGAGCAGCTAAGAGATGGCTCCAGA
CACCCCAGGTCCCATCAAGAAGACAGAGCTGGTCATGGGCACTCTGCAGACAGCTCCAGACAATCAGGCA
CTCGTCACACACAGACTTCCTCTGGTGGACAGGCTGCATCATCCCATGAACAGGCAAGATCAAGTGCAGG
AGAAAGACATGGATCCCACCACCAGCAGTCAGCAGACAGCTCCAGACACTCAGGCATTGGGCACGGACAA
GCTTCATCTGCAGTCAGAGACAGTGGACACCGAGGGTACAGTGGTAGTCAGGCCAGTGACAATGAGGGAC
ATTCAGAAGACTCAGACACACAGTCAGTGTCAGCCCACGGACAGGCTGGGTCCCATCAGCAGAGCCACCA
AGAGTCCGCACGTGGCCGGTCAGGGGAAACGTCTGGACATTCAGGATCTTTCCTCTACCAGGTGAGCACT
CATGAACAGTCTGAGTCCTCCCATGGATGGACGGGGCCCAGCACTAGAGGAAGACAAGGATCCCGCCATG
AGCAGGCACAAGACAGCTCCAGGCACTCAGCATCCCAATACGGTCAGGACACCATTCGTGGACACCCGGG
GTCAAGCAGAGGAGGAAGGCAGGGGTACCACCACGAGCATTCGGTAGATAGCTCTGGACACTCAGGGTCC
CATCACAGCCACACCACATCCCAGGGAAGGTCTGATGCCTCCCGTGGGCAGTCAGGATCCAGAAGTGCAA
GCAGAACAACACGTAATGAGGAACAATCAGGAGACAGCTCCAGGCACTCAGTGTCACGTCACCATGAAGC
TTCCACTCATGCCGACATCTCTAGACACTCACAGGCAGTCCAGGGACAATCAGAGGGGTCCAGGAGAAGC
AGGCGCCAGGGATCCAGTGTGAGCCAGGACAGTGACAGTGAGGGACATTCAGAAGACTCTGAGAGGTGGT
CTGGGTCTGCTTCCAGAAACCATCGTGGATCTGTTCAGGAGCAGTCAAGGCACGGCTCCAGACACCCCAG
GTCCCATCACGAAGACAGAGCCGGTCACGGGCACTCTGCAGACCGCTCCAGACAATCAGGCACTCGTCAC
GCAGAGACTTCCTCTGGTGGACAGGCTGCATCATCCCATGAACAGGCAAGATCAAGTCCAGGAGAGAGAC
ACGGATCCCGCCACCAGCAGTCAGCAGACAGCTCCAGACACTCAGGCATTCCGCGTGGACAAGCTTCATC
TGCAGTCAGAGACAGTAGACACTGGGGGTCCAGTGGTAGTCAGGCCAGTGATAGTGAGGGACATTCAGAA
GAGTCAGACACACAGTCAGTGTCAGGCCATGGACAGGCTGGGCCCCATCAGCAGAGCCACCAAGAGTCCG
CACGTGACCGGTCAGGGGGAAGGTCTGGACGTTCAGGGTCTTTCCTCTACCAGGTGAGCACTCATGAACA
GTCTGAGTCTGCCCATGGGCGGACCAGGACCAGCACTGGACGAAGACAAGGATCCCACCACGAGCAGGCA
CGAGACAGCTCCAGGCACTCAGCGTCCCAAGAGGGTCAGGACACCATTCGTGGACACCCGGGGTCAAGCA
GAAGAGGAAGGCAGGGATCCCACTACGAGCAATCGGTAGATAGGTCTGGACACTCAGGGTCCCATCACAG
CCACACCACATCCCAGGGAAGGTCTGATGCCTCCCGTGGGCAGTCAGGATCCAGAAGTGCCAGCAGACAA
ACTCGTAATGACGAACAATCAGGAGATGGCTCCAGGCACTCATGGTCGCATCACCATGAAGCTTCCACTC
 
AGGCGGACAGCTCTAGACACTCACAGTCCGGCCAGGGACAATCAGCGGGGCCCAGGACAAGCAGGAACCA
GGGATCCAGTGTTAGCCAGGACAGTGACAGTCAGGGACACTCAGAAGACTCTGAGAGGTGGTCTGGGTCT
GCTTCCAGAAACCATCGTGGATCTGCTCAGGAGCAGTCAAGAGATGGCTCCAGACACCCCACGTCCCATC
ACGAAGACAGAGCCGGTCACGGCACTCTGCAGAGAGCTCCAGACAATCAGGCACTCATCATGCAGAGAA
TTCCTCTGGTGGACAGGCTGCATCATCCCATGAACAGGCAAGATCAAGTGCAGGAGAGAGACATGGATCC
CACCACCAGCAGTCAGCAGACAGCTCCAGACACTCAGGCATTGGGCACGGACAAGCTTCATCTGCAGTCA
GAGACAGTGGACACCGAGGGTCCAGTGGTAGTCAGGCCAGTGACAGTGAGGGACATTCAGAAGACTCAGA
CACACAGTCAGTGTCAGCCCACGGACAGGCTGGGCCCCATCAGCAGAGCCACCAAGAGTCCACACGTGGC
CGGTCAGCAGGAAGGTCTGGACGTTCAGGGTCTTTCCTCTACCAGGTGAGCACTCATGAACAGTCTGAGT
CTGCCCATGGACGGCTGGGCCCAGTACTGGAGGAAGACAAGGATCCCGCCACGAGCAGGCACGAGACAG
CTCCAGGCACTCAGCGTCCCAAGAGGGTCAGGACACCATTCGTGGACACCCGGGGTCAAGGAGAGGAGGA
AGACAGGGATCCTACCACGAGCAATCGGTAGATAGGTCTGGACACTCAGGGTCCCATCACAGCCACACCA
CATCCCAGGGAAGGTCTGATGCCTCCCATGGGCAGTCAGGATCCAGAAGTGCAAGCAGAGAAACACGTAA
TGAGGAACAGTCAGGAGACGGCTCCAGGCACTCAGGGTCGCGTCACCATGAAGCTTCCACTCAGGCTGAC
AGCTCTAGACACTCACAGTCCGGCCAGGGTGAATCAGCGGGGTCCAGGAGAAGCAGGCGCCAGGGATCCA
GTGTTAGCCAGGACAGTGACAGTGAGGCATACCCAGAGGACTCTGAGAGGCGATCTGAGTCTGCTTCCAG
AAACCATCATGGATCTTCTCGGGAGCAGTCAAGAGATGGCTCCAGACACCCCGGATCCTCTCACCGCGAT
ACAGCCAGTCATGTACAGTCTTCACCTGTACAGTCAGACTCTAGTACCGCTAAGGAACATGGTCACTTTA
GTAGTCTTTCACAAGATTCTGCGTATCACTCAGGAATACAGTCACGTGGCAGTCCTCACAGTTCTAGTTC
TTATCATTATCAATCTGAGGGCACTGAAAGGCAAAAAGGTCAATCAGGTTTAGTTTGGAGACATGGCAGC
TATGGTAGTGCAGATTATGATTATGGTGAATCCGGGTTTAGACACTCTCAGCACGGAAGTGTTAGTTACA
ATTCCAATCCTGTTGTTTTCAAGGAAAGATCTGATATCTGTAAAGCAAGTGCGTTTGGTAAAGATCATCC
AAGGTATTATGCAACGTATATTAATAAGGACCCAGGTTTATGTGGCCATTCTAGTGATATATCGAAACAA
CTGGGATTTAGTCAGTCACAGAGATACTATTACTATGAGTAAGAAATTAATGGCAAAGGAATTAATCCAA
GAATAGAAGAATGAAGCAAGTTCACTTTCAATCAAGAAACTTCATAATACTTTCAGGGAAGTTATCTTTT
CCTGTCAATCTGTTTAAAATATGCTATAGTATTTCATTAGTTTGGTGGTAGCTTATTTTTATTGTGTAAT
GATCTTTAAACGCTATATTTCAGAAATATTAAATGGAAGAAATCAATATCATGGAGAGCTAACTTTAGAA
AACTAGCTGGAGTATTTTAGGAGATTCTGGGTCAAGTAATGTTTTATGTTTTTGAAAGTTTAAGTTTTAG
ACACTCCCCAAATTTCTAAATTAATCTTTTTCAGAAATATCGAAGGAGCCAAAAATATAAAACAGTTCTG
TATACCAAAGTGGCTATATCAACATCAGGGCTAGCACATCTTTCTCTATTATCCTTCTATTGGAATTCTA
GTATTCTGTATTCAAAAAATCATCTTGGACATAATTAATATTATAGTAAGCTGCATCTAAATTAAAAATA
AACTATT
NCBI Reference Sequence: NM_005547.2
   **Homo sapiens involucrin (IVL), mRNA**
   >gi|44890058|ref|NM_003347.2| Homo sapiens involucrin (**I**VL), mRNA 
CTCTGCCTCAGCCTTACTGTGAGTCTGGTTGACAGTAGCTTCTAAGATGTCCCAGCAACACACACTGCCA
GTGACCCTCTCCCCTGCCCTCAGTCAGGAGCTCCTCAAGACTGTTCCTCCTCCAGTCAATACCCATCAGG
AGCAAATGAAACAGCCAACTCCACTGCCTCCCCCATGCCAGAAGGTGCCTGTCGAGCTCCCAGTGGAGGT
CCCATCAAAGCAAGAGGAAAAGCACATGACTGCTGTAAAGGACTGCCTGAGCAAGAATGTGAGCAACAG
CAGAAGGAGCCACAGGAGCAGGAGCTGCAGCAACAGCACTGGGAACAGCATGAGGAATATCAGAAAGCAG
AAAACCCAGAGCAGCAGCTTAAGCAGGAGAAAACACAAAGGGATCAGCAGCTAAACAAACAGCTGGAAGA
AGAGAAGAAGCTCTTAGACCAGCAACTGGATCAAGAGCTAGTCAAGAGAGATGAGCAACTGGGAATGAAG
AAAGAGCAACTGTTGGAGCTCCCAGAGCAGCAGGAGGGCACCTGAAGCACCTAGAGCAGCAGGAGGGAC
AGCTGAAGCACCCGGAGCAGCAGGAGGGGCAGCTGGAGCTCCCAGAGCAGCAGGAGGGGCAGCTGGAGCT
CCCAGAGCAGCAGGAGGGGCAGCTGGAGCTCCCAGAGCAGCAGGAGGGGCAGCTGGAGCTCCCAGAGCAG
CAGGAGGGGCAGCTGGAGCTCCCAGAGCAGCAGGAGGGGCAGCTGGAGCTCCCACAGCAGCAGGAGGGGC
AGCTGGAGCTCTCTGAGCAGCAGGAGGGGCAGCTGGAGCTCTCTGAGCAGCAGGAGGGACAGCTGAAGCA
CCTGGAGCACCAGGAGGGGCAGCTGGAGGTCCCAGAGGAGCAGATGGGGCAGCTGAAGTACCTGGAACAG
CAGGAGGGGCAGCTGAAGCACCTGGATCAGCAGGAGAAGCAGCCAGAGCTCCCAGAGCAGCAGATGGGGC
AGCTGAAGCACCTGGAGCAGCAGGAGGGGCAGCCTAAGCATCTGGAGCAGCAGGAGGGGCAACTGGAGCA
GCTGGAGGAGCAGGAGGGGCAGCTGAAGCACCTGGAGCAGCAGGAGGGGCAGCTGGAGCACCTGGAGCAC
 
CAGGAAGGCAGCTGGGGCTCCCAGAGCAGCAGGTGCTGCAGCTGAAGCAGCTAGAGAAGCAGCAGGGGC
AGCCAAAGCACCTGGAGGAGGAGGAGGGGCAGCTGAAGCACCTGGTGCAGCAGGAGGGGCAGCTGAAGCA
TCTGGTGCAGCAGGAGGGGCAGCTGGAGCAGCAGGAGAGGCAGGTGGAGCACCTGGAGCAGCAGGTGGGG
CAGCTGAAGCACCTAGAGGAGCAGGAGGGACAACTGAAGCATCTGGAGCAGCAGCAGGGGCAGTTGGAGG
TCCCAGAGCAGCAGGTGGGGCAGCCAAAGAACCTGGAGCAGGAGGAGAAGCAACTGGAGCTCCCAGAGCA
GCAAGAGGGCCAGGTGAAGCACCTGGAGAAGCAGGAGGCACAGCTGGAGCTCCCAGAGCAGCAGGTAGGA
CAGCCAAAGCACCTGGAACAGCAGGAAAAGCACCTAGAGCACCCAGAGCAGCAGGACGGACAACTAAAAC
ATCTGGAGCAGCAGGAGGGGCAGCTGAAGGACCTGGAGCAGCAGAAGGGGCAGCTGGAGCAGCCTGTGTT
TGCCCCAGCTCCAGGCCAGGTCCAAGACATTCAACCAGCCCTGCCCACAAAGGGAGAAGTATTGCTTCCT
GTAGAGCACCAGCAGCAGAAGCAGGAGGTGCAGTGGCCACCCAAACATAAATAACCACCCGCAGTGTCCA
GAGGCCCTCAGATCGTCTCATACAAGGGAAGAGAGAGCCACTGGCTCCACTTATTTCGGGTCCGCTAGGT
GGCCCGTCTCATCTGTGAACTTGACTCTGTCCCTCTACATGTCTCTTTAATGGGGTGAGGGTGGGGGAGA
GAGGGAATTATTGTCCAGTGCCAACCCCAATGACCCCAATCCCAACCTCAGGTGAGCAGAGCCTCTACTT
GAGGGACTATTGTTACTATAGGAATCCTTACTTCCCCAGTATTGAAGCTGAATCAGTGAGTGTGTACAAT
GATACATAATAAATCCTGGAAGTCTTGGGATCCTAAAAAAAAAAAAAAAAAAAAAAAAAAAAAA
NCBI Reference Sequence: NM_000421.3
   **Homo sapiens keratin 10 (KRT10), mRNA**
   >gi|195972865|ref|NM_000421.3| Homo sapiens keratin 10 (KRT10), mRNA 
CACTCCCTGGGCTAAACAGCATCACCATGTCTGTTCGATACAGCTCAAGCAAGCACTACTCTTCCTCCCG
CAGTGGAGGAGGAGGAGGAGGAGGATGTGGAGGAGGAGGAGTGTCATCCCTAAGAATTTCTAGC
AGCAAAGGCTCCCTTGGTGGAGGATTTAGCTCAGGGGGGTTCAGTGGTGGCTCTTTTAGCCGTGGGAGCT
CTGGTGGGGGCTGCTTTGGGGGCTCATCAGGTGGCTATGGAGGATTAGGAGGTTTTGGTGGAGGTAGCTT
TCGTGGAAGCTATGGAAGTAGCAGCTTTGGTGGGAGTTATGGAGGCAGCTTTGGAGGGGGCAGTTTCGGA
GGTGGCAGCTTTGGTGGGGGCAGCTTTGGTGGAGGCGGCTTTGGTGGAGGCGGCTTTGGAGGAGGCTTTG
GTGGTGGATTTGGAGGAGATGGTGGCCTTCTCTCTGGAAATGAAAAAGTAACCATGCAGAATCTGAATGA
CCGCCTGGCTTCCTACTTGGACAAAGTTCGGGCTCTGGAAGAATCAAACTATGAGCTGGAAGGCAAAATC
AAGGAGTGGTATGAAAAGCATGGCAACTCACATCAGGGGGAGCCTCGTGACTACAGCAAATACTACAAAA
CCATCGATGACCTTAAAAATCAGATTCTCAACCTAACAACTGATAATGCCAACATCCTGCTTCAGATCGA
CAATGCCAGGCTGGCAGCTGATGACTTCAGGCTGAAGTATGAGAATGAGGTAGCTCTGCGCCAGAGCGTG
GAGGCTGACATCAACGGCCTGCGTAGGGTGCTGGATGAGCTGACCCTGACCAAGGCTGACCTGGAGATGC
AAATTGAGAGCCTGACTGAAGAGCTGGCCTATCTGAAGAAGAACCACGAGGAGGAAATGAAAGACCTTCG
AAATGTGTCCACTGGTGATGTGAATGTGGAAATGAATGCTGCCCCGGGTGTTGATCTGACTCAACTTCTG
AATAACATGAGAAGCCAATATGAACAACTTGCTGAACAAAACCGCAAAGATGCTGAAGCCTGGTTCAATG
AAAAGAGCAAGGAACTGACTACAGAAATTGATAATAACATTGAACAGATATCCAGCTATAAATCTCAGAT
TACTGAATTGAGACGTAATGTACAAGCTCTGGAGATAGAACTACAGTCCCAACTGGCCTTGAAACAATCC
CTGGAAGCCTCCTTGGCAGAAACAGAAGGTCGCTACTGTGTGCAGCTCTCACAGATTCAGGCCCAGATAT
CCGCTCTGGAAGAACAGTTGCAACAGATTCGAGCTGAAACCGAGTGCCAGAATACTGAATACCAACAACT
CCTGGATATTAAGATCCGACTGGAGAATGAAATTCAAACCTACCGCAGCCTGCTAGAAGGAGAGGGAAGT
TCCGGAGGCGGCGGACGCGGCGGCGGAAGTTTCGGCGGCGGCTACGGCGGCGGAAGCTCCGGCGGCGGAA
GCTCCGGCGGCGGCCACGGCGGCGGCCACGGCGGCAGTTCCGGCGGCGGCTACGGAGGCGGAAGCTCCGG
CGGCGGAAGCTCCGGCGGCGGCTACGGGGGCGGAAGCTCCAGCGGCGGCCACGGCGGCAGTTCCAGCGGC
GGCTACGGTGGTGGCAGTTCCGGCGGCGGCGGCGGCGGCTACGGGGGCGGCAGCTCCGGCGGCGGCAGCA
GCTCCGGCGGCGGATACGGCGGCGGCAGCTCCAGCGGAGGCCACAAGTCCTCCTCTTCCGGGTCCGTGGG
CGAGTCTTCATCTAAGGGACCAAGATACTAACAAAACCAGAGTAATCAAGACAATTATTGAAGAGGTGGC
GCCCGACGGTAGAGTTCTTTCATCTATGGTTGAATCAGAAACCAAGAAACACTACTATTAAACTGCATCA
AGAGGAAAGAGTCTCCCTTCACACAGACCATTATTTACAGATGCATGGAAAACAAAGTCTCCAAGAAAAC
ACTTCTGTCTTGATGGTCTATGGAAATAGACCTTGAAAATAAGGTGTCTACAAGGTGTTTTGTGGTTTCT
GTATTTCTTCTTTTCACTTTACCAGAAAGTGTTCTTTAATGGAAAGAAAAACAACTTTCTGTTCTCATTT
ACTAATGAATTTCAATAAACTTTCTTACTGATGCAAACTAAAAAAAAAAAAAAAAAAAAA
NCBI Reference Sequence: NM_004925.3
   **Homo sapiens aquaporin 3 (Gill blood group) (AQP3), mRNA**
   >gi|22165421|ref|NM_004925.3| Homo sapiens aquaporin 3 (Gill blood group) (AQP3), mRNA 
CTCGCTGCGCCACCGCCTCCCGCCACCCCTGCCCGCCCGACAGCGCCGCCGCCTGCCCCGCCATGGGTCG
ACAGAAGGAGCTGGTGTCCCGCTGCGGGGAGATGCTCCACATCCGCTACCGGCTGCTCCGACAGGCGCTG
GCCGAGTGCCTGGGGACCCTCATCCTGGTGATGTTTGGCTGTGGCTCCGTGGCCCAGGTTGTGCTCAGCC
GGGGCACCCACGGTGGTTTCCTCACCATCAACCTGGCCTTTGGCTTTGCTGTCACTCTGGGCATCCTCAT
CGCTGGCCAGGTCTCTGGGGCCCACCTGAACCCTGCCGTGACCTTTGCCATGTGCTTCCTGGCTCGTGAG
CCCTGGATCAAGCTGCCCATCTACACCCTGGCACAGACGCTGGGAGCCTTCTTGGGTGCTGGAATAGTTT
TTGGGCTGTATTATGATGCAATCTGGCACTTCGCCGACAACCAGCTTTTTGTTTCGGGCCCCAATGGCAC
AGCCGGCATCTTTGCTACCTACCCCTCTGGACACTTGGATATGATCAATGGCTTCTTTGACCAGTTCATA
GGCACAGCCTCCCTTATCGTGTGTGTGCTGGCCATTGTTGACCCCTACAACAACCCCGTCCCCCGAGGCC
TGGAGGCCTTCACCGTGGGCCTGGTGGTCCTGGTCATTGGCACCTCCATGGGCTTCAACTCCGGCTATGC
CGTCAACCCTGCCCGGGACTTTGGCCCCCGCCTTTTTACAGCCCTTGCGGGCTGGGGCTCTGCAGTCTTC
ACGACCGGCCAGCATTGGTGGTGGGTGCCCATCGTGTCCCCACTCCTGGGCTCCATTGCGGGTGTCTTCG
TGTACCAGCTGATGATCGGCTGCCACCTGGAGCAGCCCCCACCCTCCAACGAGGAAGAGAATGTGAAGCT
GGCCCATGTGAAGCACAAGGAGCAGATCTGAGTGGGCAGGGGCCATCTCCCCACTCCGCTGCCCTGGCCT
TGAGCATCCACTGACTGTCCAAGGGCCACTCCCAAGAAGCCCCCTTCACGATCCACCCTTTCAGGCTAAG
GAGCTCCCTATCTACCCTCACCCCACGAGACAGCCCCTTCAGGATTTCCACTGGACCTTGCCCAAATAGC
ACCTTAGGCCACTGCCCCTAAGCTGGGGTGGAACCGGAATTTGGGTCAATACATCCTTTTGTCTCCCAAG
GGAAGAGAATGGGCAGCAGGTATGTGTGTGTGCATGTGTGTGCATGTGTGCATGTGTGTGCAGGGG
TGTGTGTGTGTGGGGGGGGTTCCCAGATATTCAGGGCAAGGGACCAGTCGGAAGGGATTCTGGCTATTGG
GGGAGCCCAGAGACAGGGGAAGGCAGCCTGTCCATCTGTGCATAAGGAGAGGAAAGTTCCAGGGTGTGTA
TGTTTCAGGGGCTTCACATGGAGGAGCTGCAGATAGATATGTGTTTCTGTGTATGTGTATGTCTGCCTTT
TTTTCTAAGTGGGGGCTTCTACAGGCTTTTGGGAAGTAGGGTGGATGTGGGTAGGGCTGGGAGGAGGGGG
CCACAGCTTAGGTTTGGAGCTCTGGATGTACATACATAAGTAGGAGCAGTGGGACGTGTTTCTGTCATAA
TGCAGGCATGAAGGGTGGAGTGAAGTCAGGTCATAAGTTTCATGTTTGCTTTTGTTTTGTTTTGTTTTTA
ATGTATGTAGCAGATGTTACAGTCTTAGGATCCGGGATGGGAGACCCCACTTTAGAAAGGGTCGTCACT
CCTTTAATCCTCTACTCAACAATGTACTCTTTTACTTTTATATTAAAAAAAATAAAATAAATATGTGCCT
AAAAAAAAAAAAAAA
NCBI Reference Sequence: NM_000526.4
   **Homo sapiens keratin 14 (KRT14), mRNA**
   >gi|197313720|ref|NM_000526.4| Homo sapiens keratin 14 (KRT14), mRNA

ACCCGAGCACCTTCTCTTCACTCAGCCAACTGCTCGCTCGCTCACCTCCCTCCTCTGCACCATGACCACC
TGCAGCCGCCAGTTCACCTCCTCCAGCTCCATGAAGGGCTCCTGCGGCATCGGGGGCGGCATCGGGGGCG
GCTCCAGCCGCATCTCCTCCGTCCTGGCCGGAGGGTCCTGCCGCGCCCCCAGCACCTACGGGGGCGGCCT
GTCTGTCTCATCCTCCCGCTTCTCCTCTGGGGGAGCCTACGGCTGGGGGGCGGCTATGGCGGTGGCTTC
AGCAGCAGCAGCAGCAGCTTTGGTAGTGGCTTTGGGGGAGGATATGGTGGTGGCCTTGGTGCTGGCTTGG
GTGGTGGCTTTGGTGGTGGCTTTGCTGGTGGTGATGGGCTTCTGGTGGGCAGTGAGAAGGTGACCATGCA
GAACCTCAATGACCGCCTGGCCTCCTACCTGGACAAGGTGCGTGCTCTGGAGGAGGCCAACGCCGACCTG
GAAGTGAAGATCCGTGACTGGTACCAGAGGCAGCGGCCTGCTGAGATCAAAGACTACAGTCCCTACTTCA
AGACCATTGAGGACCTGAGGAACAAGATTCTCACAGCCACAGTGGACAATGCCAATGTCCTTCTGCAGAT
TGACAATGCCCGTCTGGCCGCGGATGACTTCCGCACCAAGTATGAGACAGAGTTGAACCTGCGCATGAGT
GTGGAAGCCGACATCAATGGCCTGCGCAGGGTGCTGGACGAACTGACCCTGGCCAGAGCTGACCTGGAGA
TGCAGATTGAGAGCCTGAAGGAGGAGCTGGCCTACCTGAAGAAGAACCACGAGGAGGAGATGAATGCCCT

GAGAGGCCAGGTGGGTGGAGATGTCAATGTGGAGATGGACGCTGCACCTGGCGTGGACCTGAGCCGCATT
CTGAACGAGATGCGTGACCAGTATGAGAAGATGGCAGAGAAGAACCGCAAGGATGCCGAGGAATGGTTCT
TCACCAAGACAGAGGAGCTGAACCGCGAGGTGGCCACCAACAGCGAGCTGGTGCAGAGCGGCAAGAGCGA
GATCTCGGAGCTCCGGCGCACCATGCAGAACCTGGAGATTGAGCTGCAGTCCCAGCTCAGCATGAAAGCA
TCCCTGGAGAACAGCCTGGAGGAGACCAAAGGTCGCTACTGCATGCAGCTGGCCCAGATCCAGGAGATGA
TTGGCAGCGTGGAGGAGCAGCTGGCCCAGCTCCGCTGCGAGATGGAGCAGCAGAACCAGGAGTACAAGAT
CCTGCTGGACGTGAAGACGCGGCTGGAGCAGGAGATCGCCACCTACCGCCGCCTGCTGGAGGGCGAGGAC
GCCCACCTCTCCTCCTCCCAGTTCTCCTCTGGATCGCAGTCATCCAGAGATGTGACCTCCTCCAGCCGCC
AAATCCGCACCAAGGTCATGGATGTGCACGATGGCAAGGTGGTGTCCACCCACGAGCAGGTCCTTCGCAC
CAAGAACTGAGGCTGCCCAGCCCCGCTCAGGCCTAGGAGGCCCCCCGTGTGGACACAGATCCCACTGGAA
GATCCCCTCTCCTGCCCAAGCACTTCACAGCTGGACCCTGCTTCACCCTCACCCCCTCCTGGCAATCAAT
ACAGCTTCATTATCTGAGTTGCATAAAAAAAAAAAAAAAAAAA

## Claims

1. A cosmetic method for regulating the condition of mammalian skin, wherein the skin has at least three layers: a stratum corneum exterior layer; an epidermis; and a dermis, comprising the steps of:
(a) applying prior to and/or during delivery of energy a first personal care composition to an area of skin where regulation is desired, wherein the first personal care composition comprises a gel composition; and
(b) delivering energy to the dermis to heat collagen in the dermis such that the heated collagen in the dermis heats the epidermis and stratum corneum until the stratum corneum reaches an external temperature of from about 37°C to about 48°C, wherein the energy delivery to the dermis is then controlled to maintain the temperature of the stratum corneum in the range of from about 37°C to about 48°C, and wherein the energy is delivered by an RF energy delivery device and the RF energy is delivered via two or more electrodes that contact the stratum corneum via the gel composition;
the method further comprising the step of determining an expression profile of a gene panel consisting of at least one gene selected from the group consisting of SEQ ID NO: 1-9 and/or SEQ ID NO: 10-14 and at least one gene selected from the group consisting of SEQ ID NO: 15-17, wherein upregulation of the at least one gene selected from the group consisting of SEQ ID NO: 1-14 and/or SEQ ID NO: 10-14 and substantially no change in expression of the at least one gene from the group consisting of SEQ ID NO: 15-17 is indicative of induction of collagen formation and/or dermal remodeling in absence of a skin-damaging inflammatory cytokine response.

2. The cosmetic method of claim 1, wherein when the RF energy delivery device is turned on it delivers the RF energy in the range of 35% to about 65% of full power for about 20 to about 50 seconds, then the power is increased to from about 65% to about 100% for about 20 to about 50 seconds, then energy delivery is controlled such that the temperature of the stratum corneum is maintained in the range of from about 37°C to about 48°C.

3. The cosmetic method of claim 1, wherein the energy delivery device is handheld and is applied under an eye of a consumer and moved underneath the eye to just above the crows feet area, and then the direction is reversed, and the energy delivery device is moved back and forth across this path for from about 3 to about 6 minutes.

4. The cosmetic method of claim 1, wherein the energy delivery device is handheld and is applied above an eye of a consumer and moved over the eye to just below the crows feet area, and then the direction is reversed, and the energy delivery device is moved back and forth across this path for from about 3 to about 6 minutes.

5. The cosmetic method of claim 3, wherein the method is repeated underneath the other eye of the consumer.

6. The cosmetic method of claim 4, wherein the method is repeated above the other eye of the consumer.

7. The cosmetic method of claim 1 wherein the gel composition has an electrical conductivity of from about 1,000 to about 2, 000 µS/m.

8. The cosmetic method of claim 1 wherein the device is used in a multi-step regimen comprising the steps of applying the energy delivery device under one eye of a consumer and moving it to just above the crows feet area in a continuous back and forth motion for from about 3 to about 6 minutes, then applying the energy delivery device above an eye of the consumer and moving it to just below the crows feet area in a continuous back and forth motion for from about 3 to about 6 minutes, repeating these two steps on the other eye of the consumer such that the crows feet area adjacent both eyes of the consumer are each treated for from about 6 to about 12 minutes, and wherein the device preferably is handheld.

9. The cosmetic method of claim 8 wherein the multistep regimen is completed at least once per day, for a regiment period of 3 to 5 days per week, for from about 3 weeks to about 6 weeks.

10. The cosmetic method of claim 9, wherein the consumer waits for about 2 to about 8 months and then repeats the multi step regimen for the regimen period.

## Patentansprüche

1. Kosmetisches Verfahren zum Regulieren des Zustands von Säugerhaut, wobei die Haut wenigstens drei Schichten aufweist: eine äußere Stratum corneum-Schicht; eine Epidermis; und eine Dermis, welches die Schritte umfasst:
(a) Auftragen vor und/oder während einer Abgabe von Energie einer ersten persönlichen Pflegezusammensetzung auf einen Bereich der Haut, wo eine Regulierung gewünscht ist, wobei die erste persönliche Pflegezusammensetzung eine Gelzusammensetzung umfasst; und
(b) Abgeben von Energie an die Dermis, um Kollagen in der Dermis so zu erwärmen, dass das erwärmte Kollagen in der Dermis die Epidermis und das Stratum corneum erwärmt, bis das Stratum corneum eine äußere Temperatur von etwa 37°C bis etwa 48°C erreicht, wobei die Energieabgabe an die Dermis dann so kontrolliert wird, um die Temperatur des Stratum corneum in dem Bereich von etwa 37°C bis etwa 48°C zu halten, und wobei die Energie durch ein RF-Energieabgabegerät abgegeben wird und die RF-Energie über zwei oder mehr Elektroden abgegeben wird, die mit dem Stratum corneum über die Gelzusammensetzung in Kontakt sind;
wobei das Verfahren ferner den Schritt eines Bestimmens eines Exprimierungsprofils einer Gen-Auswahl umfasst, die aus wenigstens einem Gen, ausgewählt aus der Gruppe bestehend aus SEQ ID NO: 1-9 und/oder SEQ ID NO: 10-14, und wenigstens einem Gen, ausgewählt aus der Gruppe bestehend aus SEQ ID NO: 15-17, besteht, wobei eine Aufregulation des wenigstens einen Gens ausgewählt aus der Gruppe bestehend aus SEQ ID NO: 1-14 und/oder SEQ ID NO: 10-14 und im wesentlichen keine Änderung der Exprimierung des wenigstens einen Gens aus der Gruppe bestehend aus SEQ ID NO: 15-17 indikativ ist zur Induktion einer Kollagenbildung und/oder einer dermalen Remodellierung in Abwesenheit einer Haut-schädigenden Entzündungszytokin-Reaktion.

2. Kosmetisches Verfahren nach Anspruch 1, wobei, wenn das RF-Energieabgabegerät angestellt wird, es die RF-Energie in dem Bereich von 35% bis etwa 65% einer vollen Leistung für etwa 20 bis etwa 50 Sekunden liefert, dann die Leistung auf etwa 65% bis etwa 100% für etwa 20 bis etwa 50 Sekunden erhöht wird, dann die Energieabgabe so kontrolliert wird, dass die Temperatur des Stratum corneum in dem Bereich von etwa 37°C bis etwa 48°C gehalten wird.

3. Kosmetisches Verfahren nach Anspruch 1, wobei das Energieabgabegerät handgeführt wird und unter einem Auge eines Verbrauchers angewendet wird und unterhalb des Auges bis gerade oberhalb des Krähenfüßenbereichs bewegt wird und dann die Richtung umgekehrt wird, und wobei das Energieabgabegerät hin und her über diesen Weg für etwa 3 bis etwa 6 Minuten bewegt wird.

4. Kosmetisches Verfahren nach Anspruch 1, wobei das Energieabgabegerät handgeführt wird und oberhalb eines Auges eines Verbrauchers angewendet wird und über das Auge bis gerade unterhalb des Krähenfußbereichs bewegt wird, und dann die Richtung umgekehrt wird, und wobei das Energieabgabegerät hin und her über diesen Weg für etwa 3 bis etwa 6 Minuten bewegt wird.

5. Kosmetisches Verfahren nach Anspruch 3, wobei das Verfahren unterhalb des anderen Auges des Verbrauchers wiederholt wird.

6. Kosmetisches Verfahren nach Anspruch 4, wobei das Verfahren oberhalb des anderen Auges des Verbrauchers wiederholt wird.

7. Kosmetisches Verfahren nach Anspruch 1, wobei die Gelzusammensetzung eine elektrische Leitfähigkeit von etwa 1.000 bis etwa 2.000 µS/m aufweist.

8. Kosmetisches Verfahren nach Anspruch 1, wobei das Gerät in einer Mehrschrittkur verwendet wird, umfassend die Schritte eines Beaufschlagens des Energieabgabegeräts unter einem Auge eines Verbrauchers und Bewegen desselben bis gerade oberhalb des Krähenfußbereichs in einer kontinuierlichen Hin- und Herbewegung für etwa 3 bis etwa 6 Minuten, dann Beaufschlagen des Energieabgabegeräts oberhalb eines Auges des Verbrauchrs und Bewegen desselben bis gerade unterhalb des Krähenfußbereichs in einer kontinuierlichen Hin- und Herbewegung für etwa 3 bis etwa 6 Minuten, Wiederholen dieser zwei Schritte an dem anderen Auge des Verbrauchers, so dass der Krähenfußbereich angrenzend an beide Augen des Verbrauchers jeweils für etwa 6 bis etwa 12 Minuten behandelt wird, und wobei das Gerät bevorzugt handgeführt wird.

9. Kosmetisches Verfahren nach Anspruch 8, wobei die Mehrschrittkur wenigstens einmal pro Tag, für eine Kurdauer von 3 bis 5 Tagen pro Woche, für etwa 3 Wochen bis etwa 6 Wochen vervollständigt wird.

10. Kosmetisches Verfahren nach Anspruch 9, wobei der Verbraucher für etwa 2 bis etwa 8 Monate wartet und dann die Mehrschrittkur für die Kurdauer wiederholt.

## Revendications

1. Un procédé de traitement cosmétique pour réguler l'état de la peau de mammifères, la peau comportant au moins trois couches: une couche externe stratum corneum, un épiderme et un derme, lequel procédé comprend les étapes consistant en:
(a) l'application préalablement et/ou durant la fourniture d'énergie d'une première composition de soins personnels à une zone de peau dont la régulation est souhaitée, ladite première composition de soins personnels comprenant une composition de gel; et
(b) la fourniture d'énergie au derme pour chauffer le collagène qui y est contenu de sorte que le collagène chauffé du derme chauffe l'épiderme et le stratum corneum jusqu'à ce que le stratum corneum atteigne une température externe d'environ 37°C à environ 48°C, la fourniture d'énergie au derme étant alors commandée de sorte que la température du stratum corneum soit maintenue dans la plage allant d'environ 37°C à environ 48°C, l'énergie étant fournie par l'intermédiaire d'un dispositif de fourniture d'énergie RF et cette énergie RF étant amenée via deux ou plusieurs électrodes qui sont en contact avec le stratum corneum par l'intermédiaire de la composition de gel,
le procédé comprenant en outre l'étape de détermination du profil d'expression d'un panel de gènes consistant en au moins un gène choisi dans le groupe consistant en SEQ ID NO : 1-9 et/ou SEQ ID NO : 10-14 et en au moins un gène choisi dans le groupe consistant en SEQ ID NO : 15-17, dans lequel l'expression à la hausse d'au moins un gène choisi dans le groupe consistant en SEQ ID NO : 1-14 et/ou SEQ ID NO : 10-14, et sensiblement aucun changement de l'expression au moins dudit gène du groupe consistant en SEQ ID NO : 15-17, indique une induction de la formation de collagène et/ou du remodelage dermique en l'absence d'une réponse cytokinique inflammatoire endommageant la peau.

2. Le procédé de traitement cosmétique selon la revendication 1, dans lequel, lorsque le dispositif de fourniture d'énergie RF est activé, il fournit l'énergie RF dans la gamme de 35% à environ 65% de sa pleine puissance pendant environ 20 à environ 50 secondes, puis la puissance est augmentée d'environ 65% à environ 100% pendant environ 20 à environ 50 secondes, ensuite la fourniture d'énergie est commandée de telle sorte que la température du stratum corneum soit maintenue dans la plage allant d'environ 37°C à environ 48°C.

3. Le procédé de traitement cosmétique selon la revendication 1, dans lequel le dispositif de fourniture d'énergie est tenu à la main et est appliqué sous un oeil d'un consommateur puis est déplacé en-dessous de l'oeil jusqu'à juste au-dessus de la zone des pattes d'oie, puis la direction est inversée, et le dispositif de fourniture d'énergie est déplacé dans un mouvement de va et vient selon ce cheminement pendant environ 3 à environ 6 minutes.

4. Le procédé de traitement cosmétique selon la revendication 1, dans lequel le dispositif de fourniture d'énergie est tenu à la main et est appliqué au-dessus d'un oeil d'un consommateur et est déplacé au-dessus de l'oeil jusqu'à juste en-dessous de la zone des pattes d'oie, puis la direction est inversée, et le dispositif de fourniture d'énergie est déplacé dans un mouvement de va et vient selon ce cheminement pendant environ 3 à environ 6 minutes.

5. Le procédé de traitement cosmétique selon la revendication 3, dans lequel le procédé est répété au-dessous de l'autre oeil du consommateur.

6. Le procédé de traitement cosmétique selon la revendication 4, dans lequel le procédé est répété au-dessus de l'autre oeil du consommateur.

7. Le procédé de traitement cosmétique selon la revendication 1, dans lequel la composition de gel a une conductivité électrique comprise entre environ 1 000 et environ 2 000 µS/m.

8. Le procédé de traitement cosmétique selon la revendication 1, dans lequel le dispositif est utilisé dans un traitement à étapes multiples, comprenant les étapes consistant à appliquer le dispositif de fourniture d'énergie sous un oeil d'un consommateur et en le déplaçant juste au-dessus de la zone des pattes d'oie dans un mouvement continu de va et vient d'environ 3 à environ 6 minutes, puis à appliquer le dispositif de fourniture d'énergie au-dessus de l'oeil du consommateur et le déplacer juste en dessous de la zone des pattes d'oie dans un mouvement continu de va et vient pendant environ 3 à environ 6 minutes, en répétant ces deux étapes sur l'autre oeil du consommateur de telle sorte que la zone des pattes d'oie adjacente aux deux yeux du consommateur soient chacune traitée pendant environ 6 à environ 12 minutes, et dans lequel le dispositif est de préférence tenu à la main.

9. Le procédé de traitement cosmétique selon la revendication 8, dans lequel le traitement à étapes multiples est effectué au moins une fois par jour, pendant une période de traitement de 3 à 5 jours par semaine, pendant environ 3 semaines à environ 6 semaines.

10. Le procédé de traitement cosmétique selon la revendication 9, dans lequel le consommateur interrompt le traitement pendant environ 2 à environ 8 mois, puis répète le traitement à étapes multiples pendant ladite période de traitement.
